(19) Europäisches Patentamt
European Patent Office
Office européen des brevets

(11) Numéro de publication : **0 384 842 B1**

# (12) FASCICULE DE BREVET EUROPEEN

(45) Date de publication du fascicule du brevet : **29.12.93 Bulletin 93/52**

(51) Int. Cl.$^5$ : **C07J 41/00,** A61K 31/565, C07J 1/00

(21) Numéro de dépôt : **90400493.4**

(22) Date de dépôt : **22.02.90**

## (54) Nouveaux 19-nor stéroides ayant en position 11béta une chaîne carbonée comportant une fonction amide ou carbamate, leur procédé de préparation et les intermédiaires de ce procédé, leur application comme médicaments et les compositions pharmaceutiques les renfermant.

(30) Priorité : **24.02.89 FR 8902384**

(43) Date de publication de la demande : **29.08.90 Bulletin 90/35**

(45) Mention de la délivrance du brevet : **29.12.93 Bulletin 93/52**

(84) Etats contractants désignés : **AT BE CH DE DK ES FR GB GR IT LI LU NL SE**

(56) Documents cités :
**EP-A- 0 190 759**
**WO-A-83/03099**
**DE-A- 3 621 024**
**FR-A- 2 235 949**
**FR-A- 2 582 654**

(73) Titulaire : **ROUSSEL-UCLAF**
**35, Boulevard des Invalides**
**F-75007 Paris (FR)**

(72) Inventeur : **Claussner, André**
**62, rue Marc Vieville**
**F-93250 Villemomble (FR)**
Inventeur : **Nedelec, Lucien**
**45, Boulevard de l'Ouest**
**F-93340 Le Raincy (FR)**
Inventeur : **Philibert, Daniel**
**16, rue Chevalier**
**F-94210 La Varenne Saint Hilaire (FR)**
Inventeur : **Van de Velde, Patrick**
**28, rue de Meaux**
**F-75019 Paris (FR)**

(74) Mandataire : **Bourgouin, André et al**
**Département des Brevets ROUSSEL UCLAF**
**111, route de Noisy B.P. no 9**
**F-93230 Romainville (FR)**

**EP 0 384 842 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux 19-Nor stéroïdes ayant en 11béta une chaîne carbonée comportant une fonction amide ou carbamate, leur procédé de préparation et les intermédiaires de ce procédé, leur application comme médicaments et les compositions pharmaceutiques les contenant.

La demande de brevet français FR 2 235 949 décrit des dérivés de l'oestrone, ayant éventuellement une chaîne en position 11-béta terminée par une fonction carboxylique, la demande de brevet allemand DE 3621024 concerne des stéroïdes 11béta-phényl substitués et la demande de brevet européen des 11béta-phényl gonanes.

Par ailleurs les demandes française FR 2582654 et internationale WO 83/03099 décrivent des 19-nor stéroïdes subsitutés en position 11béta.

Les stéroïdes décrits dans ces demandes de brevet sont différents de ceux de la présente demande qui ont en position 11béta une chaîne comportant une fonction amide ou carbamate.

L'invention a pour objet les composés de formule (I)

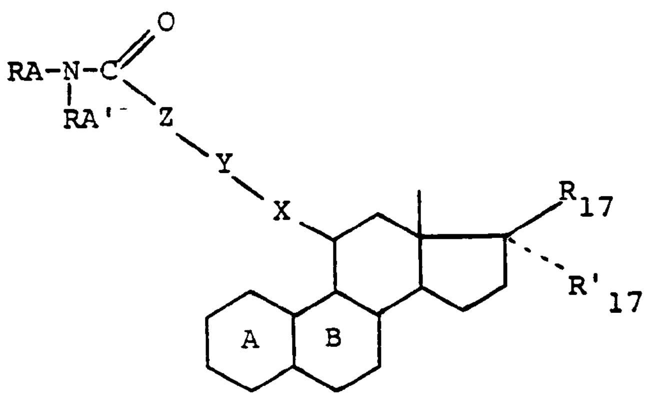

(I)

dans laquelle les cycles A et B ont l'une des structures suivantes :

a) soit A et B représentent le groupement :

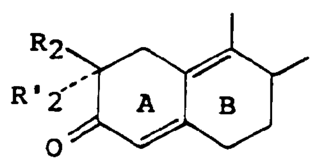

dans lequel $R_2$ et $R'_2$ identiques ou différents représentent un atome d'hydrogène ou un radical alkyle ayant de 1 à 4 atomes de carbone ;

b) soit A et B représentent le groupement :

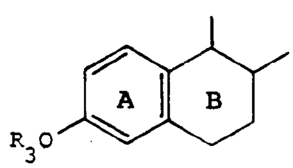

dans lequel $R_3$ représente un atome d'hydrogène ou un radical alkyle renfermant de 1 à 4 atomes de carbone ou un radical acyle,

$R_{17}$ et $R'_{17}$ sont tels que :

- soit $R_{17}$ et $R'_{17}$ forment ensemble une fonction cétone,
- soit $R_{17}$ est un radical hydroxyle ou un radical acyloxy et $R'_{17}$ représente un atome d'hydrogène, un radical alkyle, alcényle ou alcynyle ayant au plus 8 atomes de carbone, éventuellement substitué,

X, Y et Z sont tels que :

- X représente un radical méthylène, un groupement arylène, un radical $CH_2$-O ou arylènoxy lié au stéroïde par un atome de carbone,
- Y représente une simple liaison ou une chaîne aliphatique linéaire ou ramifiée, saturée ou insaturée, renfermant de 1 à 18 atomes de carbone, éventuellement interrompue par un ou plusieurs radicaux choisis parmi les radicaux arylène ou oxygène ou soufre éventuellement oxydé sous forme de sulfoxyde ou de sulfone et éventuellement terminée par un radical arylène,
- Z représente une simple liaison ou un radical $CH_2$-O lié au radical Y par l'atome de carbone, étant entendu que lorsque Y et Z sont une simple liaison, X ne peut être un radical méthylène ou $CH_2$-O, RA et RA', identiques ou différents, représentent un atome d'hydrogène, un radical alkyle linéaire ou ramifié renfermant de 1 à 8 atomes de carbone, éventuellement substitué par un ou plusieurs radicaux choisis parmi les radicaux aryle, alkyl ou dialkylamino, hydroxy, halogène ou carboxyl estérifié, ou RA et RA' forment avec l'atome d'azote auquel ils sont liés un hétérocycle à 5 ou 6 chaînons, saturé ou non, renfermant éventuellement un ou plusieurs autres hétéroatomes choisis parmi le groupe constitué par les atomes d'oxygène, d'azote et de soufre et éventuellement substitué par un radical alkyle ayant de 1 à 4 atomes de carbone, étant entendu que l'un au moins des substituants RA ou RA' n'est pas un atome d'hydrogène.

Lorsque $R_3$, $R_2$ et/ou $R'_2$ représentent un radical alkyle, il peut s'agir d'un radical méthyle, éthyle, propyle, isopropyle, butyle, isobutyle, tert-butyle et de préférence un radical méthyle.

Lorsque $R_3$ représente un radical acyle, il peut s'agir d'un radical acétyle, propionyle, butyryle ou benzoyle.

Lorsque $R_{17}$ est un radical acyloxy il peut s'agir notamment du dérivé d'un acide aliphatique ou cycloaliphatique saturé ou insaturé et notamment d'un acide alcanoïque tel que par exemple l'acide acétique, propionique, butyrique ou isobutyrique, valérique ou undécylique, d'un acide hydroxyalcanoïque tel que par exemple l'acide hydroxyacétique d'un acide cycloalcoylcarboxylique ou (cycloalcoyle) alcanoïque tel que par exemple l'acide cyclopropyl, cyclopentyl ou cyclohexylcarboxylique, cyclopentyle ou cyclohexyle acétique ou propionique, d'un acide benzoïque, d'un acide salicylique ou d'un acide phénylalcanoïque tel que l'acide phényle acétique ou phényle propionique, d'un amino acide tel que l'acide diéthylamino acétique ou aspartique ou de l'acide formique. Il s'agit de préférence du dérivé de l'acide acétique, propionique ou butyrique.

Lorsque $R'_{17}$ représente un radical alkyle, il peut s'agir du radical méthyle, éthyle, propyle, isopropyle, butyle, isobutyle, n-pentyle, n-hexyle, 2-méthyl pentyle, 2,3-diméthyl butyle, n-heptyle, 2-méthylexyle, 2,2-diméthylpentyle, 3,3-diméthyl pentyle, 3-éthylpentyle, n-octyle, 2,2-diméthylhexyle, 3,3-diméthylhexyle, 3-méthyl 3-éthylpentyle.

Il s'agit de préférence du radical méthyle.

Lorsque $R'_{17}$ représente un radical alcényle, il peut s'agir d'un radical vinyle, propényle, isopropényle, allyle, 2-méthylallyle, butényle ou isobutényle. Il s'agit de préférence du radical vinyle ou propényle.

Lorsque $R'_{17}$ représente un radical alcynyle, il peut s'agir du radical éthynyle, propynyle, propargyle, butynyle ou isobutynyle. Il s'agit de préférence du radical éthynyle ou propynyle.

L'expression éventuellement substitué appliquée aux radicaux alkyle, alcényle ou alcynyle comprend un ou plusieurs radicaux identiques ou différents choisis de préférence parmi les radicaux :

- halogène tel que fluor, chlore, brome, iode,
- alkoxy tel que méthoxy, éthoxy, propyloxy, isopropyloxy, butyloxy,
- alkylthio tel que méthylthio, éthylthio, propylthio, isopropylthio, butylthio,
- amino, alkylamino tel que méthylamino ou éthylamino, dialkylamino tel diméthylamino, diéthylamino ou méthyl éthylamino, chacun des radicaux dialkylamino étant éventuellement sous forme oxydée,
- amino alkyl tel que aminométhyle ou aminoéthyle,
- dialkylaminoalkyle tel que diméthylamino méthyle ou éthyle,
- dialkylaminoalkyloxy tel que diméthylamino éthyloxy,
- hydroxyle éventuellement acylé, par exemple acétoxy ou un radical de formule

$$-O-\underset{\displaystyle O}{\underset{\|}{C}}-(CH_2)_n\ CO_2H$$

  dans lequel n = 2 à 5 notamment,
- acyle tel que acétyle, propionyle, butyryle, benzoyle,
- carboxy libre ou estérifié tel que alkoxy carbonyle par exemple méthoxy carbonyle ou éthoxy carbonyle,
- cyano,
- trifluorométhyle,
- aryle tel que phényle, furyle, thiényle ou aralkyle tel que benzyle, ces radicaux étant eux-mêmes éventuellement substitués par des radicaux alkyle, tel que méthyle, éthyle, propyle, isopropyle, butyle, isobutyle, tert-butyle ou par des radicaux, alkoxy, alkylthio, alkylamino ou di-alkylamino indiqués ci-dessus.

Lorsque X représente un groupement arylène, il s'agit de préférence du radical phénylène.

Lorsque X représente un groupement arylènoxy, il s'agit de préférence du radical phénylènoxy.

Lorsque Y représente une chaîne aliphatique linéaire ou ramifiée, saturée ou insaturée, il peut s'agir du radical méthylène, éthylène, propylène, isopropylène, butylène, isobutylène, ou tert-butylène, n-pentylène, n-hexylène, 2-méthyl pentylène, 2,3-diméthyl butylène, n-heptylène, 2-méthylhexylène, 2,2-diméthylpentylène, 3,3-diméthyl pentylène, 3-éthylpentylène, n-octylène, 2,2-diméthylhexylène, 3,3-diméthylhexylène, 3-méthyl 3-éthylpentylène, nonylène, 2,4-diméthyl heptylène, n-décylène, n-undécylène, n-dodécylène, n-tridécylène, n-tétradécylène, n-penta décylène, n-hexadécylène, n-heptadécylène ou n-octadécylène, de préférence n-nonylène ou n-décylène. Il peut s'agir également des radicaux vinylène, isopropénylène, allylène, 2-méthylallylène ou isobuténylène, et lorsque la chaîne est interrompue ou terminée par un ou plusieurs radicaux arylènes, il s'agit de préférence du radical phénylène, étant entendu que terminée concerne l'une quelconque des deux extrémités de Y.

Lorsque RA ou RA' représente un radical alkyle, il peut s'agir du radical méthyle, éthyle, propyle, isopropyle, butyle, isobutyle ou tert-butyle, n-pentyle, n-hexyle, 2-méthyl pentyle, 2,3-diméthyl butyle, n-heptyle, 2-méthylhexyle, 2,2-diméthylpentyle, 3,3-diméthyl pentyle, 3-éthylpentyle, n-octyle, 2,2-diméthylhexyle, 3,3-diméthylhexyle, 3-méthyl 3-éthylpentyle.

Les radicaux précités peuvent être substitués par un ou plusieurs radicaux aryles tel que phényle, furyle, thiènyle, de préférence un radical phényle, par un ou plusieurs radicaux alkylamino ou dialkylamino tel que diméthylamino ou par un ou plusieurs carboxyles estérifiés par exemple par un méthoxycarbonyle ou un éthoxycarbonyle, par un ou plusieurs atomes d'halogène par exemple le fluor, le chlore ou le brome.

On peut citer notamment les radicaux 2,2,3,3,4,4,4-heptafluorobutyle ou 2-chloro 2-méthylpropyle.

Lorsque RA et RA' forment avec l'atome d'azote auquel ils sont liés un hétérocycle à 5 ou 6 chainons, il s'agit d'un hétérocycle saturé, de préférence une pyrrolidine ou une pipéridine, éventuellement substitué par un radical alkyle tel que méthyle, éthyle, propyle ou isopropyle, de préférence méthyle ou éthyle ou d'un hétérocycle insaturé, de préférence un pyrrole ou une pyridine éventuellement substitué par un radical alkyle tel que méthyle, renfermant éventuellement un autre hétéroatome, de préférence une morpholine, une pipérazine ou une pyrimidine, éventuellement substitué par un radical alkyle, de préférence méthyle ou éthyle.

Les composés de formule (I) préférés de l'invention sont ceux dans laquelle les cycles A et B représentent le groupement :

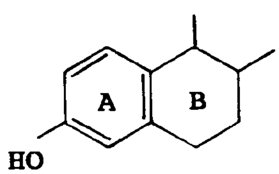

et ceux pour lesquels A et B représentent le groupement

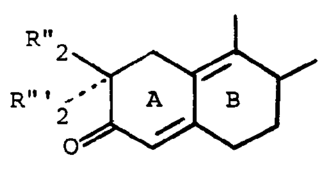

dans lequel $R''_2$ ou $R'''_2$ représente un atome d'hydrogène ou un radical méthyle, de préférence un atome d'hydrogène.

Parmi les composés de l'invention on peut citer particulièrement les composés de formule (I) pour lesquels Z est une simple liaison et plus particulièrement ceux pour lesquels $R_{17}$ est un radical hydroxyle.

Parmi les composés de l'invention on peut citer notamment les composés de formule (I) pour lesquels $R'_{17}$ est un atome d'hydrogène, un radical éthynyle ou un radical propynyle.

L'invention a tout spécialement pour objet les composés de formule (I) pour lesquels X représente un radical méthylène et Y est une chaîne linéaire saturée renfermant de 5 à 10 atomes de carbone éventuellement interrompue par un atome d'oxygène et ceux pour lesquels X représente un radical phénylène et Y est une chaîne linéaire saturée renfermant de 3 à 10 atomes de carbone, éventuellement interrompue par un atome d'oxygène et ceux pour lesquels X représente un radical phénylènoxy et Y représente une chaîne linéaire saturée renfermant de 3 à 10 atomes de carbone éventuellement interrompue par un atome d'oxygène ou de soufre.

Parmi les composés préférés de l'invention on peut citer ceux pour lesquels :

- soit RA et RA' identiques représentent un radical méthyle,
- soit RA représente un atome d'hydrogène ou un radical méthyle et RA' représente un radical butyle,
- soit RA représente un radical méthyle et RA' représente un radical isopropyle, diméthylaminoéthyle, benzyle ou heptafluorobutyle,
- soit RA et RA' forment ensemble une pipérazine éventuellement N-substituée ou une pyrrolidine.

Parmi les composés préférés de l'invention on peut donc naturellement citer les composés dont la préparation est donnée plus loin dans la partie expérimentale et plus particulièrement :

- le N-(2-diméthylaminoéthyl) 17béta-hydroxy N-méthyl 3-oxo 11béta-estra-4,9-dièn-undécanamide,
- le N-butyl 4-(3,17béta-dihydroxy estra-1,3,5(10)-trièn-11béta-yl) N-méthyl benzène octanamide,
- le 3,17béta-dihydroxy N-méthyl N-(1-méthyléthyl) 11béta-estra-1,3,5(10)-trièn-undécanamide,
- le N-butyl 3,17béta-dihydroxy N-méthyl 19-Nor-11béta-(17alphapregna-1,3,5(10)-trièn-20-yne) undécanamide,
- le 3,17béta-dihydroxy N-méthyl N-(1méthyléthyl) 19-nor 17alpha-pregna 1,3,5(10)-trièn-20-yn-11béta-undécanamide,
- le [[8-(3,17béta-dihydroxy estra 1,3,5(10)-trièn-11béta-yl) octyl] oxy] N-méthyl N-(1-méthyléthyl) acétamide,
- le N-butyl 8-[4-(3,17béta-dihydroxy estra 1,3,5(10)-trièn-11béta-yl) phénoxy] N-méthyl octanamide,
- le N-butyl [5-[4-(3,17béta-dihydroxy estra 1,3,5(10)-trièn-11béta-yl) phénoxy] pentyloxy] N-méthyl acétamide,
- le 2-[[7-[4-(3,17béta-dihydroxy estra 1,3,5(10)-trièn-11béta-yl) phényl] 6-heptylyl] oxy] N-butyl N-méthyl acétamide,
- le 3,17béta-dihydroxy N-(2,2,3,3,4,4,4-heptafluorobutyl) N-méthyl estra 1,3,5(10)-trièn-11béta-yl undécanamide,
- le 8-[4-(3,17béta-dihydroxy estra 1,3,5(10)-trièn-11béta-yl) phényl] N-butyl N-méthyl octynamide.

L'invention a également pour objet un procédé de préparation des composés de formule (I) caractérisé en ce que l'on soumet un composé de formule (II) :

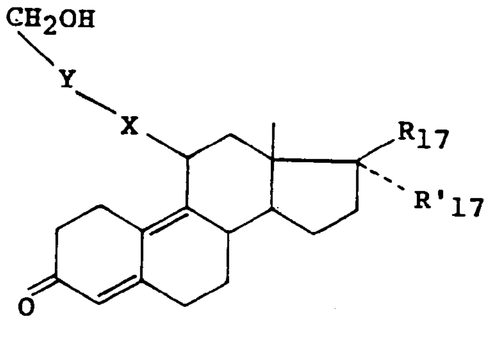

(II)

dans laquelle X, Y, $R_{17}$ et $R'_{17}$ ont la même signification que précédemment, étant entendu que $R_{17}$ ne peut représenter un radical hydroxyle,

- soit à l'action d'un agent d'oxydation pour obtenir le produit de formule (III) :

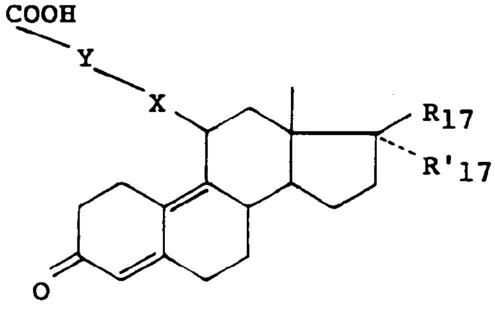

(III)

dans laquelle X, Y, $R_{17}$ et $R'_{17}$ ont la même signification que pour les composés de formule (I), que l'on

soumet à l'action d'un agent permettant d'activer la fonction carboxylique, puis à l'action d'un composé de formule (IV) :

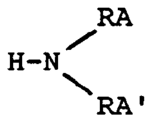

(IV)

dans laquelle RA et RA' ont la même signification que précédemment, pour obtenir le produit de formule (Ia) correspondant au composé de formule (I) dans laquelle Z est une simple liaison et les cycles A et B représentent le groupement

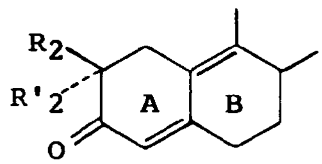

dans lequel $R_2$ et $R'_2$ sont un atome d'hydrogène,

- soit à une réaction d'introduction du radical

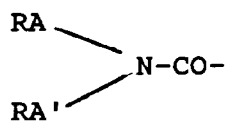

pour obtenir le produit de formule (I'a) correspondant au composé de formule (I) dans laquelle Z est un radical méthylénoxy et les cycles A et B ont la même signification que dans les produits de formule (Ia), produits (Ia) et (I'a) que, si désiré :

- soit l'on soumet à un agent de réduction lorsque $R_{17}$ et $R'_{17}$ forment ensemble une fonction cétone, puis le cas échéant, soumet à un agent d'acylation le dérivé hydroxylé en 17 ainsi obtenu,
- soit l'on soumet à un agent de saponification lorsque $R_{17}$ représente une fonction acyloxy,

pour obtenir un produit de formule (Ia) ou (I'a) dans laquelle $R_{17}$ a la définition indiquée ci-dessus, puis si désiré, l'on soumet l'un quelconque des produits de formule (Ia) ou (I'a),

- soit à une alkylation en position 2, lorsque l'un au moins des radicaux $R_2$ et $R'_2$ représente un atome d'hydrogène,
- soit l'on soumet à un agent d'aromatisation du cycle A, puis à un agent de saponification ménagée pour obtenir les produits de formule (Ib) correspondant aux produits de formule (Ia) et les produits de formule (I'b) correspondant aux produits de formule (I'a) et dans lesquelles les cycles A et B représentent le groupement :

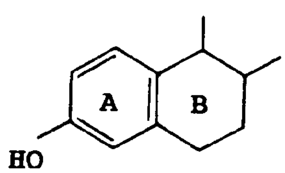

produits de formule (Ib) et (I'b) que si désiré, l'on soumet à une réaction d'alkylation ou d'acylation du radical hydroxyle en position 3, puis si désiré,

soit, lorsque $R_{17}$ et $R'_{17}$ forment ensemble une fonction cétone, l'on soumet à un agent de réduction ou l'on soumet à un complexe métallique de formule (V) :

$$M\text{-}R'_{17} \qquad (V)$$

dans laquelle M représente un atome métallique et $R'_{17}$ a la même signification que précédemment, étant entendu qu'il ne s'agit pas d'un atome d'hydrogène,

soit, lorsque $R_{17}$ est un radical hydroxyle, l'on soumet à un agent d'acylation sélective en position 17,

puis si désiré, soumet l'un quelconque des produits de formule (I) obtenus ci-dessus, soit lorsque RA ou RA' est un atome d'hydrogène, à l'action d'un agent d'alkylation approprié.

Les composés de formule (I) sont des 19-Nor stéroïdes ayant :

- soit une chaîne en 11béta comportant une fonction amide substituée ; ces composés sont alors choisis parmi les composés de formule (Ia) et (Ib), définis ci-dessus,
- soit une chaîne en 11béta comportant une fonction carbamate substituée ; ces composés sont alors choisis parmi les composés de formule (I'a) et (I'b), définis ci-dessus.

Les composés de formule (I'a) sont obtenus en faisant réagir un composé de formule (II), soit avec un activateur de la fonction hydroxyle, par exemple le phosgène, puis avec une amine primaire ou secondaire de formule (IV), dans un solvant neutre tel que le chlorure de méthylène ou le tétrahydrofuranne, en présence d'une base telle que le carbonate de potassium ou la méthylamine, soit avec un isocyanate de formule RA-N=C=O, pour obtenir alors un produit dans lequel le radical RA' est un atome d'hydrogène.

Les composés de formule (Ia) sont obtenus en faisant réagir un composé de formule (III) activé par exemple sous forme d'anhydride mixte par action d'un agent tel qu'un chloroformiate, par exemple le chloroformiate d'isobutyle, en présence d'une base telle qu'une amine tertiaire, par exemple la N-méthylmorpholine, dans un solvant anhydre tel qu'un solvant chloré, par exemple le chlorure de méthylène, avec l'amine de formule (IV).

Le produit de formule (III) est obtenu à l'aide d'un agent d'oxydation tel que, par exemple, le mélange CrO3 - acide sulfurique dans un solvant neutre tel, que par exemple, l'acétone.

Dans un mode de réalisation préféré de l'invention :

Les composés de formule (II) comprennent une chaîne en 11béta terminée par une fonction alcool choisie dans le tableau suivant :

| X | Y | $-CH_2OH$ |
|---|---|---|
| 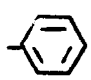 | $-(CH_2)_7$ | $-CH_2OH$ |
| " | $-C\equiv C-(CH_2)_5$ | $-CH_2OH$ |
| " | $-C\equiv C-(CH_2)_5-O-CH_2$ | $-CH_2OH$ |
| 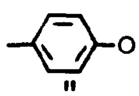 | $-(CH_2)_5-O-CH_2$ | $-CH_2OH$ |
| " | $-(CH_2)_7$ | $-CH_2OH$ |
| $-CH_2$ | $-(CH_2)_9$ | $-CH_2OH$ |
| " | $-(CH_2)_7$ $-O-CH_2)$ | $-CH_2OH$ |

comme illustré dans les exemples ci-après,

. les composés de formule (III) comprennent une chaîne en 11béta, terminée par une fonction acide carboxylique, correspondant au produit d'oxydation d'une chaîne choisie parmi les chaînes en 11béta terminée par une des fonctions alcool citées ci-dessus,

. le composé de formule (IV) est choisi parmi les amines :
butylamine, méthylbutylamine, diméthylamine, méthylisopropylamine, méthyldiméthylaminoéthylamine, méthylbenzylamine, pyrrolidine ou N-méthylpipérazine, qui sont des produits connus.

Lorsque (Ia) ou (I'a) a une cétone en 17, on obtient le stéroïde hydroxylé en 17béta correspondant, par action d'un agent de réduction tel que le borohydrure de sodium dans un solvant neutre tel que le méthanol ou l'hydrure de triterbutoxylithium aluminium dans le tétrahydrofuranne.

Lorsque (Ia) ou (I'a) a une fonction hydroxy en 17, on obtient le stéroïde acyloxylé en 17béta correspondant, par action d'un agent d'acylation, par exemple d'acétylation tel que l'anhydride acétique dans la pyridine, en présence éventuellement de 4-diméthylaminopyridine.

Lorsque (Ia) ou (I'a) a une fonction acyloxy en 17, on obtient le stéroïde hydroxylé en 17béta correspondant, par action d'un agent de saponification, tel que la potasse en milieu alcoolique.

Lorsque (Ia) ou (I'a) a un ou deux atomes d'hydrogène en position 2 ou (et) 2', on obtient le stéroïde correspondant mono ou dialkylé en 2 et 2' par action d'un agent d'alkylation, de préférence de méthylation tel que

l'iodure de méthyle.

Les composés de formule (Ib) et (I'b) qui sont des stéroïdes dérivés de l'oestradiol ayant une chaîne en 11béta comportant respectivement une fonction amide substituée ou une fonction carbamate substituée, sont obtenus à partir des composés de formule (Ia) et (I'a) respectivement, par action d'un agent d'aromatisation tel que l'hydroxyde de palladium sur magnésie au sein du méthanol ou bien le mélange bromure d'acétyle-anhydride acétique, à une température ne dépassant pas la température ambiante, suivie d'une réaction de saponification ménagée avec par exemple de la potasse dans le méthanol, du bicarbonate de sodium ou bien du méthanol en présence d'acide chlorhydrique.

Lorsque (Ib) ou (I'b) a un groupement hydroxylé en position 3, on obtient le stéroïde alkylé correspondant par action d'un réactif d'alkylation tel qu'un iodure d'alkyle ou un sulfate d'alkyle par exemple le sulfate de méthyle ou le stéroïde acylé correspondant par action d'un réactif d'acylation usuel tel qu'un halogénure d'acyle par exemple le chlorure d'acétyle.

Lorsque (Ib) ou (I'b) a une fonction cétone en 17, on obtient :

- le stéroïde 17béta hydroxylé correspondant, dans les conditions décrites ci-dessus, pour la ou l'a, par exemple par action d'un agent de réduction tel que le borohydrure de sodium dans un solvant neutre tel que le méthanol,
- le composé de formule Ib ou I'b correspondant comportant un radical $R'_{17}$ que représente un radical alkyle, alcényle ou alcynyle, éventuellement substitué, en utilisant comme complexe, par exemple, un complexe du lithium, selon le procédé décrit dans la demande de brevet EP 57115.

Lorsque (Ib) ou (I'b) a une fonction hydroxy en 17, on obtient le stéroïde 17béta acyloxylé correspondant, par action d'un agent d'acylation sélective par exemple l'anhydride acétique dans la pyridine.

Lorsque $R_A$ ou $R'_A$ est un atome d'hydrogène, on obtient le produit alkylé correspondant, par action d'un halogénure d'alkyle, par exemple un iodure de méthyle ou d'éthyle, le bromure de méthyle ou d'éthyle dans un solvant tel que le tétrahydrofuranne.

Il est bien entendu que si $R'_{17}$ comprend un radical alkyle, alcényle ou alcynyle substitué par une fonction réactive, celle-ci peut être provisoirement protégée par les méthodes usuelles.

L'invention concerne également un procédé de préparation des composés (I') correspondant aux produits de formule (I) dans lesquels X représente un radical arylène et Y représente une chaîne aliphatique éventuellement liée au groupe arylène par une double ou une triple liaison et comportant au moins 3 atomes de carbone ou liée au groupe arylène par un atome d'oxygène, caractérisé en ce que l'on soumet un composé de formule (X) :

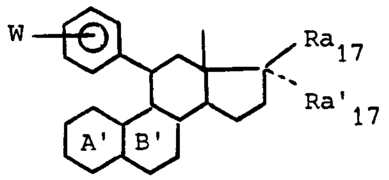

(X)

dans laquelle W représente soit un radical OH soit un radical -C≡CH, les cycles A' et B', $Ra_{17}$ et $Ra'_{17}$ ayant les mêmes significations que celles indiquées précédemment pour les cycles A et B, $R_{17}$ et $R'_{17}$ et dans lesquelles les fonctions réactives en 3 et en 17 sont éventuellement protégées ou bien, dans le cas où W représente un radical -C=CH à l'action d'un agent halogéné de formule (XI) :

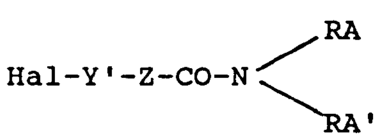

(XI)

dans laquelle Hal est un atome d'halogène, Z, RA et RA' ont la même signification que précédemment et Y' représente la chaîne aliphatique Y ci-dessus comportant 2 atomes de carbone en moins, en présence d'une base forte et soumet le cas échéant à l'action d'un agent de déprotection, pour obtenir le produit de formule ($I'_A$) :

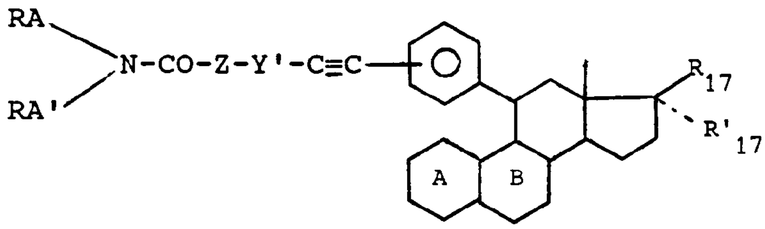

(I'A)

produit que, si désiré, l'on soumet à un agent de réduction partielle ou totale de la triple liaison pour obtenir le produit de formule ($I'_B$) :

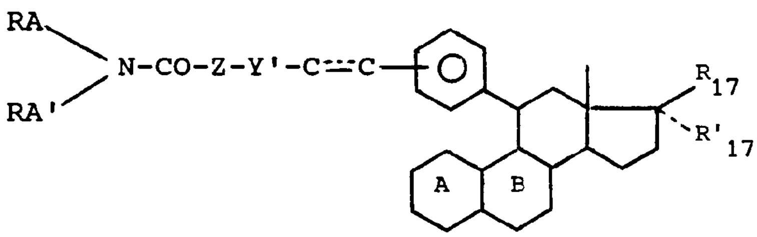

(I'B)

<u>ou bien</u>, dans le cas où W représente un radical -OH, à l'action d'un dérivé halogéné de formule (XII) :

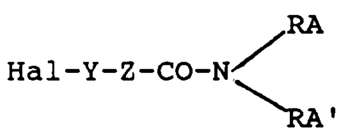

(XII)

dans laquelle Hal, Y, Z, RA et RA' ont la signification indiquée précédemment, en présence d'un agent alcalin, puis soumet le cas échéant, à l'action d'un agent de déprotection pour obtenir un produit de formule ($I''_A$) :

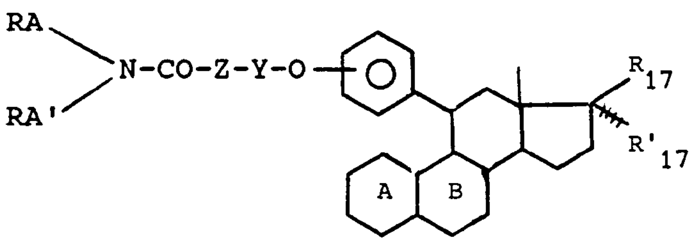

(I"A)

produit que si désiré, lorsque Y représente une chaîne aliphatique insaturée, l'on soumet à un agent de réduction partielle ou totale, et produits de formule ($I'_A$), ($I'_B$), ($I''_A$) que si désiré l'on soumet à l'une quelconque des réactions indiquées ci-dessus pour (Ia), (I'a), (Ib), (I'b).

Selon un mode préféré du procédé de l'invention décrit ci-dessus :

- les groupements de protection éventuels des fonctions hydroxyles en 3 et en 17 sont choisis parmi les groupements usuels tels que tétrahydropyrannyle et tertiobutyle,
- l'atome d'halogène que représente Hal est par exemple un atome de brome, de chlore ou d'iode,
- la base forte utilisée est par exemple le butyllithium ou l'hydrure de sodium,
- l'agent alcalin utilisé est un hydroxyde alcalin par exemple la soude,
- le déblocage des fonctions protégées est effectué à l'aide d'un agent d'hydrolyse usuel tel que l'acide chlorhydrique,
- la réduction éventuelle de la triple liaison est effectuée soit à l'aide d'hydrogène en présence de palladium sur charbon actif, sulfate de baryum et éventuellement une base telle que la pyridine ou la quinoléine, dans le cas d'une réduction partielle, soit à l'aide d'hydroxyde de palladium seul dans le cas d'une réduction totale,
- dans les composés de formule (X) utilisé au départ, le radical W est en position para.

Selon une variante des procédés décrits ci-dessus, les produits de formule (I) dans laquelle Z est une simple liaison et Y représente une chaîne aliphatique linéaire terminée par un radical vinylène précédant la fonction amide peuvent être obtenus en soumettant un produit de formule (II') :

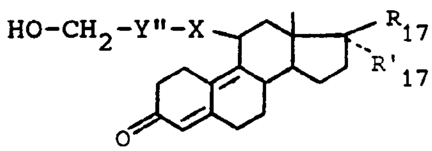

(II')

correspondant au produit de formule (II) dans laquelle Y" représente la chaîne aliphatique Y ci-dessus comportant 2 atomes de carbone en moins, à un agent d'oxydation pour obtenir l'aldéhyde correspondant, que l'on soumet à un phosphorane de formule :

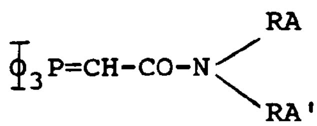

- Les produits de formule (I) dans laquelle Y représente une chaîne aliphatique ramifiée peuvent être préparés par alkylation des produits de formule (I) dans laquelle Y représente une chaîne aliphatique linéaire après avoir, le cas échéant, bloqué les fonctions réactives en 3 et en 17. L'alkylation est effectuée par exemple à l'aide d'un halogénure d'alkyle tel que l'iodure de méthyle, en présence de lithium diisopropylamide.
- Les produits de formule (III) peuvent être préparés par hydrolyse alcaline des produits correspondants comportant en position 11 une chaîne -X-Y-CN, produits obtenus à partir des produits de formule (II') tels que définis ci-dessus dont on protège les fonctions hydroxyles puis que l'on soumet à l'action d'un agent d'halogénation tel qu'un halogénure alcalin par exemple l'iodure de sodium puis un cyanure alcalin tel que le cyanure de potassium.

Des exemples de telles préparations sont données ci-dessous dans la partie expérimentale.

Les composés de formule (I) présentent d'intéressantes propriétés pharmacologiques. L'étude des produits sur les récepteurs hormonaux a permis de mettre en évidence que

- les composés de formule (Ia) et (I'a) possèdent des activités glucocorticoides ou antiglucocorticoides, progestomimétiques ou antiprogestomimétiques, androgènes ou antiandrogènes, antiminéralocorticoides, oestrogènes ou antioestrogènes,
- les composés de formule (Ib) et (I'b) possèdent en particulier une remarquable activité anti-oestrogène et des propriétés anti-prolifératives,

comme le montrent les résultats des tests donnés plus loin.

Ces propriétés rendent les composés de formule (Ia) et (I'a) utilisables pour lutter contre les effets secondaires des glucocorticoides ; ils permettent de lutter également coïdes et notamment contre le vieillissement en général et plus particulièrement contre l'hypertension, l'athérosclérose, l'ostéoporose, le diabète, l'obésité ainsi que la dépression de l'immunité et l'insomnie.

Ces produits peuvent également présenter un intérêt dans le traitement de certaines tumeurs hormono-dépendantes.

Les composés de formule (Ia) et (I'a) qui possèdent des propriétés antiprogestomimétiques peuvent être utilisés pour préparer des contraceptifs originaux ou comme agents d'interruption de grossesse.

Ces produits peuvent ainsi être utilisés comme inducteurs de règles chez la femme et plus généralement chez les animaux femelles à sang chaud.

Les produits sont alors administrés pendant les périodes où la progestérone joue un rôle physiologique essentiel, c'est-à-dire notamment pendant la phase lutéale du cycle, au moment de la nidation (ou implantation de l'embryon) et pendant la grossesse. Une méthode de contraception selon l'invention consiste à administrer à la femme un au moins des produits de formule (Ia) ou (I'a) pendant 1 à 5 jours se situant préférentiellement à la fin du cycle. Ce produit est administré alors préférentiellement par la voie orale ou in vagino mais il peut également être utilisé par la voie parentérale. Les produits peuvent également être utilisés par la voie endonasale.

Les produits de formule (Ia) et (I'a) possédant des propriétés anti-progestomimétiques peuvent également être utilisés contre les dérèglements hormonaux et, par ailleurs, ils peuvent présenter un intérêt dans le traitement des tumeurs hormono-dépendantes.

Leurs actions sur les sécrétions hypophysaires rendent les produits utilisables dans la ménopause.

Ces produits peuvent également être utilisés dans la synchronisation de l'oestrus chez les animaux d'élevage, en particulier les bovins et les ovins.

Les produits peuvent également être utilisés pour contrôler la fertilité des animaux familiers tels que chiens ou chats.

Les composés de formule (Ia) et (I'a) peuvent également présenter des propriétés progestomimétiques et peuvent ainsi être utilisés dans le traitement des aménorrhées, des dysménorrhées et des insuffisances lutéales.

Les composés de formule (Ia) et (I'a) qui possèdent des propriétés antiandrogènes peuvent être utilisés dans le traitement des hypertrophies et du cancer de la prostate, de l'hyperandrogénie, de l'anémie, de l'hirsutisme et de l'acné. Ils peuvent également être utilisés pour la contraception chez l'homme.

Les composés de formule (Ia) et (I'a) qui possèdent des propriétés oestrogènes les rendent utilisables également dans le traitement des troubles liés à une hypofolliculinie, par exemple les aménorrhées, les dysménorrhées, les avortements répétés, les troubles prémenstruels ainsi que le traitement de la ménopause et de l'ostéoporose.

Les propriétés anti-oestrogènes et anti-prolifératives des composés de formule (Ib) et (I'b) les rendent utilisables dans le traitement des carcinomes hormono-dépendants, comme par exemple les carcinomes mammaires et ses métastases et dans le traitement des tumeurs bénignes du sein.

L'invention a donc pour objet les composés de formule (I) à titre de médicaments.

Parmi les médicaments de l'invention on peut citer particulièrement les composés décrits dans la partie expérimentale et tout particulièrement les produits des exemples 8, 16, 19, 21, 35, 37, 43, 46, 55, 71 et 75.

La posologie utile varie en fonction de l'affection à traiter et de la voie d'administration ; elle peut varier par exemple de 1 mg à 100 mg par jour chez l'adulte par voie orale.

L'invention s'étend aux compositions pharmaceutiques renfermant comme principe actif au moins un médicament tel que défini ci-dessus.

Les composés de formule (I) sont utilisés par voie digestive, parentérale ou locale, par exemple par voie percutanée. Ils peuvent être prescrits sous forme de comprimés simples ou dragéifiés, de gélules, de granulés, de suppositoires, d'ovules, de préparations injectables, de pommades, de crèmes, de gels, de microsphères, d'implants, de patchs, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les composés de formule (II) ou (III) sont des produits nouveaux et l'invention a donc également pour objet les composés de formule (II) et (III) à titre de produits intermédiaires nouveaux.

Les composés de formule (II) sont préparés selon un mode opératoire dont un exemple est donné plus loin. D'une manière générale, les composés de formule (II) peuvent être préparés selon le schéma suivant :

On fait réagir en présence d'un sel de cuivre, le dérivé magnésien d'un alcool halogéné de formule (VI) :

hal-X-Y-$CH_2OR$ (VI)

dans laquelle X et Y ont la signification donnée précédemment, hal est un atome d'halogène, de préférence un atome de chlore ou de brome et R est soit un atome d'hydrogène soit un groupement protecteur de la fonction alcool par exemple un groupement :

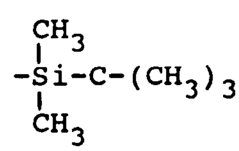

sur un composé de formule (VII) :

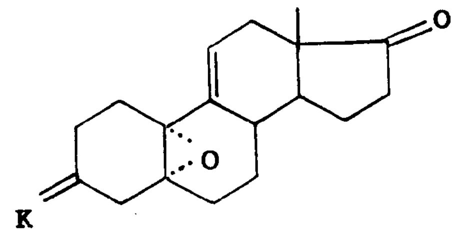

(VII)

dans laquelle K est un groupement protecteur de la fonction cétone en 3 tel qu'un cétal cyclique, pour obtenir le produit de formule (VIII) :

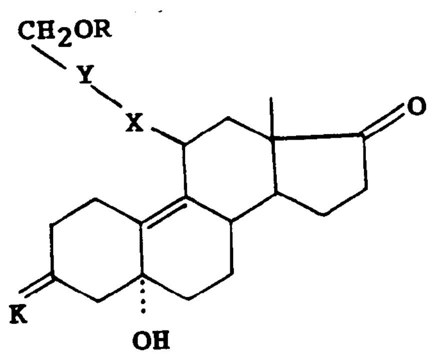

(VIII)

que l'on soumet, si désiré,

- soit au dérivé lithien du produit de formule (IX) :

  $H\text{-}R'_{17}$ (IX)

  dans laquelle $R'_{17}$ a la signification donnée précédemment, étant entendu qu'il ne s'agit pas d'un atome d'hydrogène
- soit à un agent de réduction, puis éventuellement à un agent d'acylation,

et que l'on soumet à un agent de déshydratation et d'hydrolyse susceptible de libérer la fonction 3-céto delta-4 et la fonction alcool pour obtenir le composé de formule (II).

Les produits de formule (VII) nécessaires à la mise en oeuvre du procédé sont des produits connus. Leur préparation est décrite par exemple dans le brevet EP 0057115.

Les produits de formule (X) nécessaires à la mise en oeuvre du procédé sont décrits notamment dans la demande de brevet européen EP 0 245 170. Des exemples spécifiques de préparation de produits de formule (X) sont dcrits ci-après dans les exemples.

Des exemples de préparation des produits (XI) et (XII) figurent ci-après dans la partie expérimentale.

Parmi les produits nouveaux de formule (II) préférés de l'invention, on peut citer les composés dont la préparation est donnée plus loin dans la partie expérimentale et plus particulièrement :

- la 17béta-acétyloxy 11béta-[(8-hydroxy octyl) phényl] estra-4,9-dièn-3-one,
- la 11béta-(12-hydroxy dodécyl) estra-4,9-diène-3,17-dione,
- la 11béta-(8-hydroxyoctyl) estra-4,9-dièn-3,17-dione.

Parmi les produits nouveaux de formule (III) préférés de l'invention, on peut citer ceux dont la préparation est donnée plus loin dans la partie expérimentale et plus particulièrement :

- l'acide 17béta-acétyloxy 3-oxo 11béta-estra-4,9-dièneundécanoïque,
- l'acide 3,17-dioxo 11béta-estra-4,9-dièneundécanoïque,
- l'acide 17béta-hydroxy 3-oxo 17-(1-propynyl) 11béta-estra-4,9-dièneundécanoïque.

En plus, les exemples suivants qui illustrent l'invention sans toutefois la limiter, les produits suivants constituent des produits pouvant être obtenus dans le cadre de la présente invention :

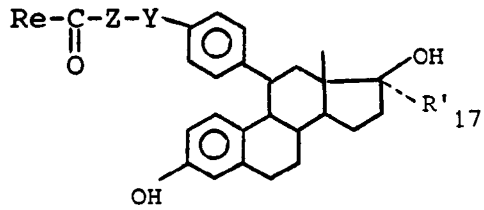

| Y | Z | Re | R'17 |
|---|---|---|---|
| $C\equiv C-(CH_2)_5$ | - | 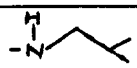 | H |
| " | - | 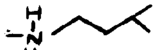 | H |
| " | - | 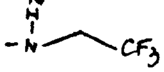 | H |
| " | - | 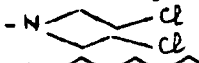 | H |
| " | - | 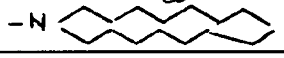 | H |

| Y | Z | Re | R'17 |
|---|---|---|---|
| C≡C-CH2-Φ-O-CH2 | - | 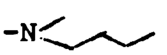 | H |
| C≡C-CH2-O-Φ-CH2 | - | 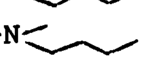 | H |
| -(CH2)7 | - | 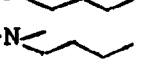 | 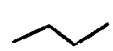 |
| | | | |

**<u>PREPARATION 1</u>: 17béta-acétyloxy 11béta-[4-(2-hydroxy méthyl) phényl] estra-4,9-dièn-3-one.**

<u>STADE A</u> : (5alpha,11béta) 3-(1,2-éthanediyl acétal cyclique) de 5-hydroxy 11-[4-[2-[[(1,1-diméthyl éthyl) diméthylsilyl] oxy] méthyl] phényl] estr-9-ène-3,17-dione.

<u>Préparation du magnésien :</u>

A une suspension de 1,4 g de magnésium en tournures dans 20 $cm^3$ de tétrahydrofuranne, on ajoute goutte à goutte 19,9 g du dérivé bromé obtenu à la préparation 9, en solution dans 60 $cm^3$ de tétrahydrofuranne. On agite 1 heure à 50°C. Titre = 0,85 mole/litre.

<u>Condensation :</u>

On agite, pendant 10 minutes à température ambiante 4,5 g de 3-(1,2-éthanediyl acétal cyclique) de 5alpha,10alpha-époxy estr-9,11-ène-3,17-dione obtenu selon EP 0057115 (ex. 7), en solution dans 45 $cm^3$ de tétrahydrofuranne et 0,4 g de chlorure de cuivre. On ajoute, en 20 minutes 50 $cm^3$ du magnésien obtenu ci-dessus, sans dépasser 27°C. Après 1 heure 30 d'agitation, on verse dans une solution glacée de chlorure d'ammonium.

La phase aqueuse est extraite 1 fois avec de l'acétate d'éthyle et 3 fois avec du chlorure de méthylène, on obtient 15,423 g de produit que l'on chromatographie sur silice (éluant : cyclohexane-acétate d'éthyle (7-3)). On obtient 6,29 g de produit recherché.

Spectre IR : ($CHCl_3$)
OH en 5 3508 $cm^{-1}$
C=O 1733 $cm^{-1}$
Aromatique 1510 $cm^{-1}$
O-Si probable.

STADE B : (5alpha,11béta,17béta) 3-(1,2-éthanediyl acétal cyclique) de 5,17-dihydroxy 11-[4-[2-[[(1,1-diméthyl éthyl) diméthylsilyl] oxy] méthyl] phényl] estr-9-èn-3-one.

A une solution de 6,15 g du produit obtenu au stade A dans 100 $cm^3$ de méthanol, on introduit en 15 minutes 6 g d'hydrure de bore et de sodium. On agite pendant 2 heures et verse dans 500 $cm^3$ d'eau, extrait avec du chlorure de méthylène. On évapore à sec et obtient 6,17 g de produit utilisé tel quel pour le stade suivant.
Spectre IR : ($CHCl_3$)
OH en 17 3611 $cm^{-1}$
OH en 5 3508 $cm^{-1}$
Aromatique 1509 $cm^{-1}$
O-Si probable

STADE C : (11béta,17béta) 17-acétyloxy 11-[4-(2-méthyl) phényl] estra-4,9-dièn-3-one.

a) Acétylation.
A une solution de 6,1 g du produit obtenu au stade B, dans 52 $cm^3$ de pyridine et 1,15 g de diméthyl aminopyridine, on ajoute 5,2 $cm^3$ d'anhydride acétique. On agite 20 minutes et coule dans 200 $cm^3$ d'une solution glacée de bicarbonate de sodium, on agite 10 minutes puis on extrait avec du chlorure de méthylène, on évapore les solvants à sec et obtient 6,828 g de produit amorphe.
b) Hydrolyse.
Le produit obtenu ci-dessus est repris avec 40 $cm^3$ d'acide chlorhydrique 2N et 50 $cm^3$ d'éthanol. On agite pendant 1 heure à température ambiante et concentre de moitié sous pression réduite. On dilue par 100 $cm^3$ d'eau, et extrait avec du chlorure de méthylène. On évapore à sec sous pression réduite et chromatographie le résidu sur silice, (éluant : cyclohexane-acétale d'éthyle (1-1)). On obtient 3,39 g de produit recherché.
Spectre IR : $CHCl_3$ (sur Nicolet)
OH 3618 $cm^{-1}$
C=O 1728 $cm^{-1}$ OAC
Diénone 1656
1602
Aromatique 1509 $cm^{-1}$

**PREPARATION 2 : Acide 3,17-dioxo 11béta-estra-4,9-diènepentanoïque**

STADE A : 3-(1,2-éthanediyl acétal cyclique) de 5alpha-hydroxy 11béta-(5-hydroxy pentyl) estr-9-ène-3,17-dione.

Préparation du magnésien de 1-chloro 5-pentanol.

A une solution de 24,6 $cm^3$ de 1-chloro 5-propanol dans 246 $cm^3$ de tétrahydrofuranne, on ajoute en 20 minutes à -20°C, 300 $cm^3$ d'une solution 0,67 M/litre de magnésien de 2-chloropropane dans le tétrahydrofuranne, on agite 20 minutes à -20°C, on ajoute alors 7,3 g de magnésien en copeaux puis 0,5 $cm^3$ de dibromoéthane, on chauffe au reflux pendant 1 heure, rajoute 0,5 $cm^3$ de dibromo-éthane et poursuit le reflux pendant 2 heures, on ramène à température ambiante. On obtient la solution de magnésien recherché titrant 0,18 M/litre.

Condensation :

A un mélange de 12 g d'époxy de 5,10alpha obtenu selon EP 0057115 (ex. 7) et 600 mg de chlorure cuivreux dans 150 $cm^3$ de tétrahydrofuranne, on ajoute, à -5°C, goutte à goutte, 570 $cm^3$ du magnésien préparé ci-dessus, agite encore 30 minutes puis verse sur un mélange de 250 $cm^3$ d'une solution saturée de chlorure d'ammonium et 250 g de glace. On extrait avec du chloroforme, lave avec une solution saturée de chlorure

de sodium, sèche et évapore à sec sous pression réduite. On chromatographie le résidu (35 g) sur silice (éluant : chlorure de méthylène-acétone (85-15)). On obtient 13,5 g de produit recherché.
Spectre IR : ($CHCl_3$)

| | |
|---|---|
| 17 céto | 1733 $cm^{-1}$ |
| OH primaire | 3623 $cm^{-1}$ |
| OH en 5 | 3567 $cm^{-1}$ |
| C = C | 1625 $cm^{-1}$ |

STADE B : 11béta-(5-hydroxy pentyl) estra-4,9-diène-3,17-dione.

On agite pendant 1 heure 30 minutes, 5 g du produit obtenu au stade A, 110 $cm^3$ d'éthanol et 28 $cm^3$ d'acide chlorhydrique 2N. On alcalinise à pH ≃ 9, avec de l'ammoniaque concentré. On évapore à sec et reprend avec de l'acétate d'éthyle, lave à l'eau, avec une solution saturée de chlorure de sodium, sèche et évapore à sec sous pression réduite. On chromatographie le résidu (4,8 g) sur silice (éluant : chlorure de méthylène-acétone (85-15)). On obtient 3,97 g de produit attendu.
Spectre IR : ($CHCl_3$)

| | |
|---|---|
| OH primaire | 3264 $cm^{-1}$ |
| cétone en 17 | 1736 $cm^{-1}$ |
| diénone | 1656 $cm^{-1}$ - 1602 $cm^{-1}$ |

STADE C : Acide 3,17-dioxo 11béta-estra-4,9-diènepentanoïque.

A une solution de 2,9 g du produit obtenu au stade B, dans 140 $cm^3$ d'acétone refroidie à -4°C, on ajoute en 25 minutes à 0°/-4°C, 7 $cm^3$ de réactif d'Heilbron-Jones, on agite encore 5 minutes à 0°C puis détruit l'excès de réactif par addition de 2,5 $cm^3$ de méthanol et ajoute une solution de 22 g de carbonate de baryum dans 220 $cm^3$ d'eau. On agite pendant 1 heure à température ambiante, filtre les sels minéraux, les lave avec 5 fois 200 $cm^3$ d'acétone. On évapore l'acétone et extrait la phase aqueuse avec 4 fois 200 $cm^3$ de chlorure de méthylène, lave à l'eau, au chlorure de sodium en solution saturée et évapore à sec sous pression réduite, on obtient 3,4 g de produit utilisé tel quel pour le stade suivant.
Spectre IR : ($CHCl_3$)

| | |
|---|---|
| 17 céto | 1736 $cm^{-1}$ |
| C = O | 1709 $cm^{-1}$ |
| cétone conjuguée | 1657 $cm^{-1}$ |
| C = C | 1602 $cm^{-1}$ |

**PREPARATION 3 : Acide 3,17-dioxo 11béta-estra-4,9-dièneheptanoïque**

STADE A : 3-(1,2-éthanediyl acétal cyclique) de 5alpha-hydroxy 11béta-(7-hydroxy heptyl) estr-9-ène-3,17-dione.

On opère comme au stade A de la préparation 2, en utilisant 14,4 g de 1-chloro 7-heptanol (obtenu selon la préparation 10) à partir de 13,9 g de l'époxyde 5(10)alpha obtenu selon EP 0057115 (ex. 7).

On obtient 26,9 g de produit brut que l'on chromatographie sur silice éluant chlorure de méthylène-acétone-triéthylamine (85-15-0,4), on obtient 9 g de produit recherché que l'on chromatographie à nouveau (éluant : acétate d'éthylecyclohexane-triéthylamine (60-40-0,4). On obtient 8,17 g de produit recherché.
Spectre IR : ($CHCl_3$)

| | |
|---|---|
| 17 céto | 1733 $cm^{-1}$ |
| Alcool primaire | 3622 $cm^{-1}$ |
| Alcool tertiaire OH | 3508 $cm^{-1}$ |

STADE B : 11béta-(7-hydroxyheptyl) estra-4,9-diène-3,17-dione.

On opère comme au stade B de la préparation 2, à partir de 2,5 g du produit obtenu au stade précédent. On obtient, après chromatographie sur silice (éluant : cyclohexane-acétate d'éthyle (1-1)) 1,935 g de produit recherché.
Spectre IR :

| | |
|---|---|
| $CH_2OH$ | 3626 $cm^{-1}$ |
| 17 céto | 1734 $cm^{-1}$ |

diénone 1654 - 1602 $cm^{-1}$

STADE C : Acide 3,17-dioxo 11béta-estra-4,9-dièneheptanoïque.

On opère comme au stade C de la préparation 2, à partir de 3,2 g de produit obtenu comme au stade B. On obtient 3,04 g de produit recherché utilisé tel quel pour le stade suivant.

**PREPARATION 4 : 17béta-acétyloxy 11béta-[4-(8-hydroxy octyl) phényl] estra-4,9-dièn-3-one.**

STADE A : (5alpha,11béta) 3-(1,2-éthanediyl acétal cyclique) de 5-hydroxy 11-[4-[8-[[(1,1-diméthyl éthyl) diméthylsilyl] oxy] octyl] phényl] estr-9-ène-3,17-dione.

On opère comme au stade A de la préparation 1 à partir de 3,171 g de l'époxyde obtenu selon EP 0057115 (ex. 7) en utilisant 9,9 g de diméthyl tert-butyl silyl octanyloxy bromobenzène préparation 11. On obtient, après chromatographie sur silice (éluant : cyclohexane-acétate d'éthyle (6-4)), 4,127 g de produit recherché.

Spectre IR : ($CHCl_3$)
OH type 5-OH 3510 $cm^{-1}$
Aromatique 1510 $cm^{-1}$
O-Si + 836 $cm^{-1}$
17 céto 1733 $cm^{-1}$

STADE B : (5alpha,11béta,17béta) 3-(1,2-éthanediyl acétal cyclique) de 5,17-dihydroxy 11-[4-[8-[[(1,1-diméthyl éthyl) diméthylsilyl] oxy] octyl] phényl] estr-9-èn-3-one.

On opère comme au stade B de la préparation 1, à partir de 2,62 g obtenu au stade A ci-dessus. On obtient 2,6 g de produit attendu utilisé tel quel pour le stade suivant.
Spectre IR : ($CHCl_3$) sur produit chromatographié
Absence de C=O
OH en 17 3612 $cm^{-1}$
OH en 5 3508 $cm^{-1}$
Aromatique 1509 $cm^{-1}$ - 1472 $cm^{-1}$
O-Si

STADE C : (5alpha,11béta,17béta) 3-(1,2-éthanediyl acétal cyclique) de 17-acétyloxy 5-hydroxy 11-[4-[8-[[(1,1-diméthyl éthyl) diméthylsilyl] oxy] octyl] phényl] estr-9-èn-3-one.

On opère comme en a) du stade C de la préparation 1. On obtient après chromatographie sur silice (éluant : cyclohexane-acétate d'éthyle (6-4)), 2,58 g de produit attendu.
Spectre IR : ($CHCl_3$)
C=O 1728 $cm^{-1}$
OH en 5 3508 $cm^{-1}$
Aromatique 1508 $cm^{-1}$
O-Si

STADE D : (11béta,17béta) 17-acétyloxy 11-[4-(8-hydroxy octyl) phényl] estra-4,9-dièn-3-one.

On opère comme en b) du stade C de la préparation 1, on obtient, après chromatographie sur silice (éluant : cyclohexane-acétate d'éthyle (5-5)) 1,2 g de produit attendu.

Spectre IR : ($CHCl_3$)

| | |
|---|---|
| Absence de O-Si | |
| OH | 3622 $cm^{-1}$ |
| C=O | 1728 $cm^{-1}$ |
| diènone | 1656 $cm^{-1}$<br>1602 $cm^{-1}$ |
| Aromatique | 1509 $cm^{-1}$ |

**PREPARATION 5 : Acide 17béta-acétyloxy 3-oxo 11béta-estra-4,9-dièneundécanoïque.**

STADE A : 3-(1,2-éthanediyl acétal cyclique) de 11béta-[11-[[diméthyl (1,1-diméthyl éthyl) silyl] oxy] undécyl] 5alpha-hydroxy estr-9-ène-3,17-dione.

On opère comme au stade A de la préparation 1 à partir de 17,5 g de l'époxyde obtenu selon EP 0057115 (ex. 7) en utilisant 500 $cm^3$ d'une suspension 0,32 M dans le tétrahydrofuranne de bromure de 11-(diméthyltert-butylsilyloxy) undécyl magnésien (préparé selon brevet ICI 85-100658). On obtient après chromatographie sur silice (éluant : cyclohexane-acétate d'éthyle (95-5 puis 5-5)) 15,3 g de produit attendu.

Spectre IR : ($CHCl_3$)

| | |
|---|---|
| 5-hydroxy | 3510 $cm^{-1}$ |
| 17 céto | 1733 $cm^{-1}$ |

STADE B : (1,2-éthanediyl acétal cyclique) de 5alpha,17béta-dihydroxy 11béta-[11-[[diméthyl (1,1-diméthyl éthyl) silyl] oxy] undécyl] estr-9-èn-3-one.

On opère comme au stade B de la préparation 1 à partir de 15,2 g du produit obtenu au stade A. On obtient 14,863 g de produit attendu, que l'on utilise tel quel pour le stade suivant.

Spectre IR : ($CHCl_3$)

| | |
|---|---|
| OH en 17 | 3613 $cm^{-1}$ |
| OH en 5 | 3508 $cm^{-1}$ |
| O-Si | |

Aliphatiques intenses.

STADE C : (1,2-éthanediyl acétal cyclique) de 17béta-acétyloxy 11béta-[11-[[diméthyl (1,1-diméthyl éthyl) silyl] oxy] undécyl] 5alpha-hydroxy estr-9-èn-3-one.

On agite 4 heures 30 à température ambiante 13,335 g de produit obtenu au stade B, 53 $cm^3$ de pyridine et 26 $cm^3$ d'anhydride acétique. On glace et neutralise en ajoutant, en 45 minutes, du bicarbonate de sodium. On extrait avec de l'acétate d'éthyle, lave à l'eau, sèche et évapore à sec sous vide. On obtient 15 g de produit que l'on utilise tel quel pour le stade suivant.

Spectre IR : ($CHCl_3$)

| | |
|---|---|
| OH en 5 | 3515 $cm^{-1}$ |
| C=O | 1728 $cm^{-1}$ |

STADE D : 17béta-acétyloxy 11béta-(11-hydroxy undécyl) estra- 4,9-dièn-3-one.

On agite pendant 2 heures 45 minutes, 15 g du produit obtenu au stade C, 300 $cm^3$ de méthanol et 75 $cm^3$ d'acide chlorhydrique 2N. On ajoute 20 $cm^3$ d'ammoniaque concentré et évapore le méthanol sous vide. On extrait avec de l'acétate d'éthyle, lave à l'eau saturée de chlorure de sodium, sèche et évapore à sec sous vide.

On obtient 12,75 g de produit que l'on chromatographie sur silice (éluant : cyclohexane-acétate d'éthyle (5-5)), on recueille 8,37 g de produit recherché.

Spectre IR : ($CHCl_3$)

| | |
|---|---|
| OH libre + associé | 3613 $cm^{-1}$ |
| C=O | 1729 $cm^{-1}$ |
| diènone | 1654 $cm^{-1}$ |
| | 1601 $cm^{-1}$ |

STADE E : Acide 17béta-acétyloxy 3-oxo 11béta-estra-4,9-diène-undécanoïque.

On opère comme au stade C de la préparation 1, à partir de 8,37 g du produit obtenu au stade D ci-dessus, on obtient après chromatographie sur silice (éluant : cyclohexane-acétate d'éthyle (5-5)) 6,67 g du produit recherché.

Spectre IR : ($CHCl_3$)

Présence d'acide d'après région OH

| | |
|---|---|
| C=O | 1728 $cm^{-1}$ |
| | 1712 $cm^{-1}$ |
| diènone | 1654 $cm^{-1}$ |
| | 1600 $cm^{-1}$ |

Aliphatiques très intenses.

**PREPARATION 6 : Acide 3,17-dioxo 11béta-estra-4,9-dièneundécanoïque.**

STADE A : 11béta-(11-hydroxy undécyl) estra-4,9-diène-3,17-dione.

On agite 1 heure 15 à température ambiante, 1 g de produit obtenu comme au stade A de la préparation 5, 20 $cm^3$ de méthanol et 5 $cm^3$ d'acide chlorhydrique 2N. On alcalinise à pH ≃ 9 avec de l'ammoniaque concentrée puis évapore à sec sous pression réduite. On reprend le résidu avec de l'acétate d'éthyle, lave avec une solution saturée de chlorure de sodium, sèche et concentre à sec sous pression réduite. L'extrait sec est chromatographié sur silice (éluant : cyclohexane-acétate d'éthyle (6-4)). On obtient 670 mg de produit recherché.

Spectre IR : ($CHCl_3$)

Présence d'acide d'après région OH

| | |
|---|---|
| OH | 3623 $cm^{-1}$ |
| 17 céto | 1736 $cm^{-1}$ |
| diènone | 1656 $cm^{-1}$ |
| | 1602 $cm^{-1}$ |

STADE B : Acide 3,17-dioxo 11béta-estra-4,9-dièneundécanoïque.

On opère comme au stade C de la préparation 2, à partir de 10,4 g de produit obtenu comme au stade A ci-dessus, et on obtient 12,4 g de produit utilisé tel quel pour le stade suivant.

<u>Spectre IR</u> : ($CHCl_3$)

Absence d'alcool

| | |
|---|---|
| C=O | 1736 $cm^{-1}$ |
| | 1709 $cm^{-1}$ |
| diènone | 1656 $cm^{-1}$ |
| | 1601 $cm^{-1}$ |

Présence d'acide d'après la région OH.

**<u>PREPARATION 7</u> : Acide 17béta-hydroxy 3-oxo 17-(1-propynyl) 11béta-estra-4,9-dièneundécanoïque.**

<u>STADE A</u> : 3,3-diméthylcétal de 5alpha,10alpha-époxy estr-9,11-ène-3,17-dione.

A une solution de 5 kg de 3,3-diméthylcétal estra-4,9-dièn-17-one (BF 1514086).dans 25 litres de chlorure de méthylène et 25 $cm^3$ de pyridine, on ajoute 430 g d'hexachloracétone et 1,3 litre d'eau oxygénée à 200 volumes. On agite pendant 24 heures à 16-18°C, puis on coule la solution dans un mélange de 1,400 k de thiosulfate de sodium et 50 litres d'eau déminéralisée. On extrait avec du chlorure de méthylène et évapore à sec sous pression réduite, on obtient 7,29 kg de produit attendu utilisé tel quel pour le stade suivant.

<u>STADE B</u> : 3,3-diméthylcétal de 11béta-(11-diméthyl tert-butyl silyloxy undécyl) 5béta-hydroxy 9-ène-3,17-dione.

A 600 $cm^3$ de magnésien de bromo undécyloxy diméthyl tert-butyl silane (brevet ICI 85-100658) dans 500 $cm^3$ de tétrahydrofuranne refroidi à 0°C, on ajoute 1,58 g de chlorure cuivreux, on agite pendant 30 minutes puis on refroidit à -30°C et ajoute en 15 minutes une solution de 18,17 g de produit obtenu comme au stade A, en solution dans 87 $cm^3$ de tétrahydrofuranne. On agite pendant 3 heures 30 à température ambiante, puis coule dans une solution de chlorure d'ammonium à 0°C, on agite 10 minutes puis extrait à l'acétate d'éthyle et ensuite au chlorure de méthylène. Les phases organiques sont lavées à l'eau saturée de chlorure de sodium, séchées puis évaporées à sec sous pression réduite. On chromatographie le résidu (123 g) sur silice, (éluant : chlorure de méthylèneacétate d'éthyle (9-1) à 1 % de triéthylamine). On obtient 5,19 g de produit attendu utilisable tel quel pour le stade suivant.

<u>STADE C</u> : 17béta-hydroxy 11béta-(11-hydroxy undécyl) 17alpha-(1-propynyl) estra-4,9-dièn-3-one.

On refroidit à -70°C, 84 $cm^3$ de butyl lithium dans l'hexane (1,6 mol/litre) et ajoute lentement 84 $cm^3$ de tétrahydrofuranne puis, on fait barboter du méthyl acétylène de façon à maintenir la température inférieure à -50°C après 10 minutes la température redescend à -70°C. On arrête le barbotage et agite 30 minutes à -70°C. On ajoute 4,18 g de produit obtenu au stade B en solution dans 80 $cm^3$ de tétrahydrofuranne. On laisse revenir à température ambiante. On agite 1 heure dans ces conditions puis coule dans une solution de chlorure d'ammonium à 0°C. On extrait acétate d'éthyle puis chlorure de méthylène, lave, sèche et amène à sec sous pression réduite. On reprend l'extrait sec obtenu (6 g) dans 50 $cm^3$ d'éthanol et 10 $cm^3$ d'acide chlorhydrique 2N et agite pendant 1 heure à température ambiante. On dilue avec 100 $cm^3$ d'eau puis extrait avec du chloroforme et évapore à sec. On chromatographie le résidu obtenu (4,9 g) sur silice (éluant : cyclohexane-acétate d'éthyle (5-5)). On obtient 2,25 g de produit attendu.

Analyse pour $C_{32}H_{48}O_3$

| | C % | H % |
|---|---|---|
| % calculés | 79,95 | 10,06 |
| % trouvés | 79,9 | 10,1 |

STADE D : (11béta,17béta) 17-acétyloxy 11-(11-hydroxy undécyl) 17-(1-propynyl) estra-4,9-dièn-3-one.

a) Diacétylation.

On agite pendant 18 heures à 20°C, une solution de 5,07 g de produit obtenu comme au stade C de l'exemple 9 dans 48 $cm^3$ de pyridine, 1 g de 4-diméthylamino pyridine et 17,6 $cm^3$ d'anhydride acétique.

On ajoute ensuite, 200 $cm^3$ de glace puis neutralisé par une solution saturée de bicarbonate de sodium. On agite pendant 30 minutes, extrait avec du chlorure de méthylène, amène à sec sous pression réduite. On chromatographie le résidu obtenu 8 g sur silice (éluant : cyclohexane-acétate d'éthyle (5-5)). On obtient 2,83 g de produit.

b) Mono-saponification.

On dissout 2,8 g du produit diacétylé dans 28 $cm^3$ de méthanol avec 0,7 g de bicarbonate de potassium. On chauffe à 70°C pendant 1 heure 30, on ajoute 100 $cm^3$ d'eau glacée puis extrait au chlorure de méthylène. On évapore les solvants et on chromatographie le résidu (2,6 g) sur silice (éluant : cyclohexane-acétate d'éthyle (5-5)). On obtient 1,18 g de produit attendu utilisable tel quel pour le stade suivant.

STADE E : Acide (11béta,17béta) 17-acétyloxy 3-oxo 17-(1-propynyl) 11-estra-4,9-dièneundécanoïque.

A une solution de 460 mg de produit obtenu au stade A dans 24 $cm^3$ d'acétone, on ajoute en 1 heure à 0°C, 0,8 $cm^3$ d'une solution préparée à partir de 57 $cm^3$ d'acide sulfurique concentré, 67 g d'oxyde chromique et de l'eau pour compléter à 250 $cm^3$.

On ajoute à la solution réactionnelle à 0°C, 8 gouttes de méthanol, 28 $cm^3$ d'eau et 8 g de carbonate de baryum. On agite pendant 1 heure à 0°C, filtre l'insoluble, extrait avec du chlorure de méthylène et évapore à sec sous pression réduite. On chromatographie le résidu obtenu (334 mg) sur silice (éluant : cyclohexane-acétate d'éthyle (5-5)). On obtient 198 mg de produit attendu.

STADE F : Acide (11béta,17béta) 17-hydroxy 3-oxo 17-(1-propynyl) 11-estra-4,9-dièneundécanoïque.

On agite pendant 1 heure à température ambiante une solution de 722 mg de produit obtenu comme au stade B, dans 8 $cm^3$ d'une solution 1M de potasse méthanolique. On ajoute ensuite 10 g de glace puis 10 $cm^3$ d'acide chlorhydrique normal, on extrait avec du chlorure de méthylène puis évapore à sec sous pression réduite. On obtient 593 mg de produit attendu utilisé tel quel pour le stade suivant.

**PREPARATION 8 : 11béta-(12-hydroxy dodécyl) estra-4,9-diène-3,17-dione.**

STADE A : (5alpha,11béta) 3-(1,2-éthane diyl acétal cyclique) de 5-hydroxy 11-[12-[[(1,1-diméthyl éthyl) diméthylsilyl] oxy] dodécyl] estr-9-ène-3,17-dione.

On opère comme au stade A de la préparation 1 à partir de 2,97 g de l'époxyde obtenu selon EP 0057115 (ex. 7) en utilisant 12 g de magnésien de bromo dodécanoxy diméthyl tert-butyl silane (préparation 13), on obtient, après chromatographie sur silice (éluant : cyclohexane-acétate d'éthyle (6-4)), 5,24 g de produit recherché.

Spectre IR : ($CHCl_3$)

| | |
|---|---|
| OH | 3510 $cm^{-1}$ |
| C=O | 1733 $cm^{-1}$ |
| O-Si + | 836 $cm^{-1}$ |

STADE B : 11béta-(12-hydroxy dodécyl) estra-4,9-diène-3,17-dione.

On opère comme au stade A de la préparation 6 à partir de 5,2 g du produit obtenu au stade A. On chromatographie sur silice (éluant : cyclohexane-acétate d'éthyle (6-4)). On obtient 2,78 g du produit attendu.

Spectre IR : ($CHCl_3$)

| | | |
|---|---|---|
| OH | 3625 $cm^{-1}$ | |
| C=O | 1736 $cm^{-1}$ | (17 acétate) |
| Diènone | 1656 $cm^{-1}$ | |
| | 1602 $cm^{-1}$ | |

**PREPARATION 9 : Bromure de phénétoxy diméthyl tert-butyl silyl.**

A une solution de 15 g d'alcool 4-bromophénéthylique dans 60 $cm^3$ de tétrahydrofuranne on ajoute, à 0°/+5°C, 10,6 g d'imidazole, on ajoute en 30 minutes une solution de 14,33 g de diméthyl tert-butyl chlorosilane dans 20 $cm^3$ de tétrahydrofuranne à 0°C ± 2°C, on dilue le milieu avec 40 $cm^3$ de tétrahydrofuranne puis agite 2 heures à température ambiante. On filtre l'insoluble, évapore à sec sous pression réduite. On chromatographie le résidu (32,89 g) sur silice (éluant : cyclohexane-acétate d'éthyle (95-5), on obtient 24 g de produit recherché.

Spectre IR : ($CHCl_3$)

Absence

Présence 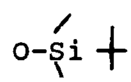

**PREPARATION 10 : 7-chloro 7-heptanol (utilisé au stade A de la préparation 3).**

On agite pendant 2 heures 30 au reflux 44 g d'heptane diol, 400 $cm^3$ d'acide chlorhydrique concentré, 150 $cm^3$ de toluène et 50 $cm^3$ d'eau. On recueille la fraction toluènique et ajoute à la fraction aqueuse 200 $cm^3$ de toluène. On chauffe à nouveau pendant 5 heures à 85/90°C, on renouvelle la même opération que ci-dessus en ajoutant, cette fois, 200 $cm^3$ de toluène et 100 $cm^3$ d'acide chlorhydrique concentré. On chauffe à nouveau 5 heures puis refroidit, sépare la fraction organique que l'on réunit aux deux précédentes, on les lave à l'eau, sèche et évapore à sec sous pression réduite puis on distille à 70°C sous 0,5 mm de mercure, on obtient 37 g de produit recherché.

Analyse pour $C_7H_{15}ClO$ = 150,65

| | C % | H % | Cl % |
|---|---|---|---|
| Calculés | 55,81 | 10,03 | 23,53 |
| Trouvés | 55,8 | 10,2 | 23,8 |

Spectre IR :

OH primaire 3615 $cm^{-1}$

**PREPARATION 11 : Diméthyl tert-butyl silyl octyloxy bromobenzène.**

STADE A : Chlorohexyloxy diméthyl tert-butyl silyl.

A un mélange de 40,93 g de 1-chlorohexanol, 42,9 g d'imidazole et 102 $cm^3$ de tétrahydrofuranne, on ajoute à 15/18°C, une solution de 56,07 g de chlorure de diméthyle tert-butyle silyle dans 114 $cm^3$ de tétrahydrofuranne. On agite pendant 15 heures à température ambiante. On essore le précipité et le chromatographie sur silice (éluant : cyclohexane-acétate d'éthyle (95-5)). On obtient 74,28 g de produit attendu.

STADE B : Diméthyl tert-butyl silyl octyloxy bromobenzène.

a) Magnésien de chlorohexyloxy diméthyl tert-butyl silyl.

A une suspension de 0,871 g de magnésien et 271 $cm^3$ de tétrahydrofuranne, on ajoute une solution de 74,82 g de produit obtenu au stade A dans 300 $cm^3$ de tétrahydrofuranne et on chauffe pendant 4 heures

au reflux. On refroidit et on obtient le magnésien attendu titrant ≃ 0,375 mol/litre.

b) Condensation :

A 640 $cm^3$ de la solution de magnésien obtenue, on ajoute à -70°C en 15 minutes : 50 g de Iodo éthyl bromobenzène (préparation 12) dans 500 $cm^3$ de tétrahydrofuranne. On agite en laissant revenir à température ambiante et maintient l'agitation pendant 15 heures. On ajoute 500 $cm^3$ d'eau saturée de chlorure d'ammonium, on agite 15 minutes, décante, lave, sèche et évapore à sec la phase organique. On chromatographie le résidu (101,3 g) sur silice (éluant : cyclohexane), on obtient 39,5 g de produit attendu.

**PREPARATION 12 : Iodure de 1-p-bromophénéthyle.**

STADE A : Alcool 1-p-bromophénéthylique.

A une solution de 95,2 g d'acide 4-bromophénylacétique dans 950 $cm^3$ de tétrahydrofuranne, on ajoute, en 35 minutes à 15/20°C, 49 $cm^3$ de complexe borane-diméthyl sulfure 10 M. On chauffe au reflux en 20 minutes et maintient 10 minutes, on refroidit et ajoute 50 $cm^3$ d'eau. On extrait avec de l'acétate d'éthyle et évapore à sec sous pression réduite. On chromatographie le résidu, 102 g sur silice (éluant : cyclohexane-acétate d'éthyle (6-4)).

STADE B : Para-toluène sulfonate de 1-p-bromophénéthyle.

A une solution de 41 g de l'alcool obtenu au stade A dans 102 $cm^3$ de pyridine, on ajoute en 35 minutes à 5°C ± 1°C, 77,72 g de chlorure de tosyle. On agite encore 30 minutes à +5°C puis laisse revenir à température ambiante. On précipite dans 500 $cm^3$ d'eau saturée de bicarbonate de sodium et extrait avec de l'acétate d'éthyle. On évapore à sec sous pression réduite, on obtient 71,4 g de produit attendu. F = 92°C.

STADE C : Iodure de 1-p-bromophénéthyle.

A une solution de 71,4 g de p-toluène sulfonate obtenu au stade B dans 1400 $cm^3$ d'acétone, on ajoute 45,12 g d'iodure de sodium et on chauffe jusqu'à un léger reflux que l'on maintient pendant 2 heures. On refroidit, essore le précipité, le lave à l'acétone et évapore le filtrat à sec sous pression réduite. On chromatographie le résidu 80,5 g sur silice (éluant : cyclohexane-acétate d'éthyle (8-2)). On obtient 60,54 g de produit attendu. F ≃ 40°C utilisé tel quel.

**PREPARATION 13 : Bromododecanoxy diméthyl tert-butyl silane.**

A une solution de 10 g de 12-bromo 1-décanol dans 40 $cm^3$ de tétrahydrofuranne, on ajoute 5,48 g d'imidazole, et en 10 minutes, 7,058 g de diméthyl tert-butyl chlorosilane en solution dans 10 $cm^3$ de tétrahydrofuranne. On agite pendant 1 heure à température ambiante, filtre l'insoluble et concentre le filtrat sous pression réduite. On purifie par chromatographie sur silice (éluant : cyclohexane-acétate d'éthyle (95-5)). On obtient 13,57 g de produit attendu utilisable tel quel pour le stade A de l'exemple 15.

Spectre IR : ($CHCl_3$)

Peu ou pas d'OH

O-Si probable $\begin{cases} 1257\ cm^{-1} \\ 837\ cm^{-1} \end{cases}$

Aliphatiques intenses.

**EXEMPLE 1 : 4-(17béta-hydroxy 3-oxo estra-4,9-dièn-11béta-yl) N-méthyl N-(1-méthyl éthyl) benzèneacétamide**

STADE A : 4-(17béta-acétyloxy 3-oxo estra-4,9-dièn-11béta-yl) N-méthyl N-(1-méthyl éthyl) benzèneacétamide.

a) Oxydation : on refroidit à 0°C une solution de 3,3 g du produit obtenu au stade C de la préparation 1,

dans 200 $cm^3$ d'acétone à laquelle on ajoute en 20 minutes, à +2/+3°C, 6,36 $cm^3$ d'une solution préparée à partir de 67 g d'oxyde chromique, 57 $cm^3$ d'acide sulfurique et eau déminéralisée q.s.p. 250 $cm^3$. On agite pendant 5 minutes puis ajoute 4 $cm^3$ de méthanol et 16 g de carbonate de baryum. On agite pendant 1 heure en laissant revenir à température ambiante. On filtre, lave à l'acétone et évapore les solvants à sec, on obtient 3,282 g de résidu.

b) Amidification.

A une solution refroidie à -10°C de 3,282 g du produit obtenu ci-dessus dans 150 $cm^3$ de chlorure de méthylène on ajoute 3 $cm^3$ de N-méthyl morpholine et 3,3 $cm^3$ de chloroformiate d'isobutyle, on agite 30 minutes à -10°C et ajoute 3,3 $cm^3$ de N-isopropyl méthylamine. On laisse la température remonter, agite 30 minutes et verse dans une solution glacée de bicarbonate de sodium, on agite pendant 10 minutes et extrait au chlorure de méthylène. On évapore les solvants à sec et chromatographie le résidu (7,6 g) sur silice (éluant : chlorure de méthylène-acétone (9-1)). On obtient 2,151 g de produit recherché.

Spectre IR : $CHCl_3$ (sur Nicolet)

| | | |
|---|---|---|
| C=O | 1728 $cm^{-1}$ | OAC |
| Diènone | 1654 $cm^{-1}$ | |
| | 1608 $cm^{-1}$ | |
| C=O | 1624 $cm^{-1}$ | Amide tertiaire |
| Aromatique | 1509 $cm^{-1}$ | |

STADE B : 4-(17béta-hydroxy 3-oxo estra-4,9-dièn-11béta-yl) N-méthyl N-(1-méthyl éthyl) benzèneacétamide.

On agite pendant 1 heure, une solution de 0,5 g du produit pobtenu au stade A, 15 $cm^3$ de méthanol et 336 mg de potasse. On refroidit à 0°C et neutralise par addition de 7,4 $cm^3$ d'acide chlorhydrique 2N.

On extrait avec du chlorure de méthylène et évapore à sec sous pression réduite, on chromatographie le résidu (459 mg) sur silice (éluant : acétate d'éthyle-cyclohexane (9-1)). On obtient 0,288 g de produit recherché.

Analyse pour $C_{30}H_{39}NO_3$ = 461,65

| | C % | H % | N % |
|---|---|---|---|
| % Calculés | 78,05 | 8,52 | 3,03 |
| % Trouvés | 77,8 | 8,6 | 3,1 |

Spectre IR : $CHCl_3$ (sur Nicolet)

| | |
|---|---|
| OH | 3612 $cm^{-1}$ |
| C=O | 1653 $cm^{-1}$ |
| | 1623 $cm^{-1}$ |
| Aromatique | 1570 $cm^{-1}$ |
| | 1509 $cm^{-1}$ |

**EXEMPLE 2 : 4-(3,17béta-dihydroxy estra-1,3,5(10)-trièn- 11béta-yl) N-méthyl N-(1-méthyl éthyl) benzènencétamide**

A une solution de 1,045 g du produit obtenu au stade A de l'exemple 1, dans 20 $cm^3$ de méthanol, on ajoute 2 g d'hydroxyde de palladium à 20 % sur oxyde de magnésium. On chauffe au reflux pendant 30 minutes. On filtre le catalyseur, lave avec du méthanol, on évapore le filtrat à sec sous pression réduite et reprend le résidu 1,03 g avec 30 $cm^3$ de méthanol à la solution obtenue, on ajoute 980 mg de potasse en pastilles et on agite 45 minutes à température ambiante. On ajoute 50 g de glace et 20 $cm^3$ d'acide chlorhydrique 2N. On extrait avec du chlorure de méthylène et évapore à sec sous pression réduite. Le résidu 1,1 g est chromatographié

sur silice (éluant : acétate d'éthyle-cyclohexane (8-2)). On obtient 467 mg de produit recherché.

Analyse pour $C_{30}H_{39}NO_3$ = 461,641

| | C % | H % | N % |
|---|---|---|---|
| % Calculés | 78,05 | 8,51 | 3,03 |
| % Trouvés | 78,2 | 8,5 | 2,9 |

Spectre IR : $CHCl_3$ (sur Nicolet)

| | |
|---|---|
| OH | 3602 $cm^{-1}$ + associé |
| C=O | 1620 $cm^{-1}$<br>1580 $cm^{-1}$ (ep.) |
| Aromatique | 1513 $cm^{-1}$<br>1501 $cm^{-1}$ (max.) |

**EXEMPLE 3 : N-butyl 3,17béta-dihydroxy 11béta-estra-1,3,5(10)-triènepentanamide**

STADE A : N-butyl 3,17-dioxo 11béta-estra-4,9-diènepentanamide.

A une solution de 3,4 g de produit obtenu au stade C de la préparation 2 dans 64 $cm^3$ de chlorure de méthylène on ajoute goutte à goutte à -10°/-15°C, 3,25 $cm^3$ de N-méthyl morpholine puis, 3,8 $cm^3$ de chloroformiate d'isobutyle. On agite pendant 30 minutes à -10°/-15°C, puis ajoute à cette température 4,1 $cm^3$ de N-butylamine. On laisse revenir à température ambiante et après 40 minutes, on ajoute 100 $cm^3$ d'une solution saturée de bicarbonate de sodium et on agite pendant 10 minutes. On décante, extrait avec du chlorure de méthylène lave avec une solution saturée de chlorure de sodium, sèche et amène à sec sous pression réduite. On chromatographie le résidu (7 g) sur silice (éluant : chlorure de méthylène-acétone (8-2)). Après évaporation à sec, on obtient 2,3 g de produit recherché.

Spectre IR : ($CHCl_3$)

| | |
|---|---|
| Cétone en 17 | 1736 $cm^{-1}$ |
| NH | 3349 $cm^{-1}$ |
| Amide II | 1519 $cm^{-1}$ |
| Diènone + C=C | 1602 $cm^{-1}$<br>868 $cm^{-1}$ |
| Diènone + amide | 1658 $cm^{-1}$ |

STADE B : 3-acétyloxy N-butyl 17-oxo 11béta-estra-1,3,5(10)-triènepentanamide.

A une solution de 2,3 g du produit obtenu au stade A dans 25 $cm^3$ de chlorure de méthylène, on ajoute à 0°/-5°C, 2,5 $cm^3$ d'anhydride acétique et 1,25 $cm^3$ de bromure d'acétyle, on agite 1 heure 20 à température ambiante puis verse sur un mélange de 150 $cm^3$ de solution saturée de bicarbonate de sodium et 50 g de glace. On agite 15 minutes puis extrait avec du chlorure de méthylène amène à sec sous pression réduite, entraîne deux fois avec du toluène et obtient 2,9 g de produit que l'on chromatographie sur silice (éluant : chlorure de méthylène-acétone (9-1)). On obtient 2,3 g de produit recherché.

Spectre IR : ($CHCl_3$)

| | | |
|---|---|---|
| Aromatiques | | 1610 $cm^{-1}$<br>1582 $cm^{-1}$<br>1494 $cm^{-1}$ |
| Amide secondaire | NH = | 3449 $cm^{-1}$ |
| | Amide II | 1518 $cm^{-1}$ |
| | C=O | 1661 $cm^{-1}$ |
| Cétone en 17 | | 1736 $cm^{-1}$<br>1408 $cm^{-1}$ |
| Acétate | | 1760 $cm^{-1}$<br>1770 $cm^{-1}$ |
| Méthyl d'acétate | | 1371 $cm^{-1}$ |

STADE C : N-butyl 3,17béta-dihydroxy 11béta-estra-1,3,5(10)-triènepentanamide.

A une solution de 1,85 g de produit obtenu au stade B dans 16 $cm^3$ de méthanol, on ajoute à 0°/+5°C, 192 mg de borohydrure de sodium, on agite 30 minutes puis ajoute 640 mg de potasse en pastilles, après 20 minutes à température ambiante, on ajoute 50 g d'eau et glace (1-1), on ajuste le pH à 4-5 avec de l'acide chlorhydrique 2N, sature avec du chlorure de sodium et extrait avec de l'acétate d'éthyle. On évapore à sec et chromatographie le résidu sur silice (éluant : chlorure de méthylène-acétone (75-25)). On obtient 1,56 g de produit brut attendu que l'on dissout dans 5 $cm^3$ de méthanol, ajoute 75 $cm^3$ de chlorure de méthylène, filtre, concentre jusqu'à début de cristallisation, glace 3 heures et essore. On obtient 1,33 g de produit recherché. F ≃ 100° (peu net).
$[alpha]_D$ +117°5 ± 2° (c = 1 % EtoH).

Analyse pour $C_{27}H_{41}O_3N$ = 427,63

| | C % | H % | N % |
|---|---|---|---|
| % Calculés | 75,83 | 9,66 | 3,27 |
| % Trouvés | 75,5 | 9,8 | 3,2 |

Spectre IR : Nujol

| | |
|---|---|
| Absorptions fortes région NH/OH | 3389 $cm^{-1}$<br>3202 $cm^{-1}$ |
| C=O Amide | 1653 $cm^{-1}$ |
| Amide II | 1532 $cm^{-1}$ |
| Aromatiques | 1610 $cm^{-1}$<br>1582 $cm^{-1}$<br>1502 $cm^{-1}$ |

**EXEMPLE 4 : N-butyl 3,17béta-dihydroxy N-méthyl 11béta-estra-1,3,5(10)-triènepentanamide.**

STADE A : N-butyl 3,7-dioxo N-méthyl 11béta-estra-4,9-diènepentanamide.

On opère comme au stade A de l'exemple 3, à partir de 3 g de l'acide obtenu au stade C de la préparation 2 et en utilisant 4,1 $cm^3$ de N-méthyl butylamine à la place de la N-butylamine, on obtient 2,82 g de produit recherché.

Spectre IR : ($CHCl_3$)

| | |
|---|---|
| Absence d'acide | |
| 17 céto | 1736 $cm^{-1}$ |
| Amide tertiaire | 1630 $cm^{-1}$ |
| Cétone conjuguée avec l'amide tertiaire | 1642 $cm^{-1}$ |
| C=C | 1603 $cm^{-1}$ |

STADE B : 3-acétyloxy N-butyl N-méthyl 17-oxo 11béta-estra-1,3,5(10)-triènepentanamide.

On opère comme au stade B de l'exemple 3 à partir de 2,8 g du produit obtenu au stade A, on obtient 2,62 g de produit recherché.

Spectre IR : ($CHCl_3$)

| | |
|---|---|
| 17 céto + acétate phénolique | 1736 $cm^{-1}$ |
| Amide tertiaire | 1627 $cm^{-1}$ |
| Aromatiques | 1586 $cm^{-1}$<br>1493 $cm^{-1}$ |

STADE C : N-butyl 3,17béta-dihydroxy N-méthyl 11béta-estra-1,3,5(10)-triènepentanamide.

On opère comme au stade C de l'exemple 3 à partir de 2,5 g du produit obtenu au stade B. On obtient 2,026 g du produit recherché.

Un échantillon analytique a été préparé en dissolvant 1,8 g du produit obtenu dans 100 $cm^3$ d'acétate d'éthyle à 60°C, après filtration on concentre à début de cristallisation, agite 3 heures à 0°C et essore. On obtient 1,58 g de produit attendu. F ≃ 165°C.

$[alpha]_D$ + 113° ± 2° (c = 1 % EtoH)

Analyse pour $C_{28}H_{43}O_3N$ = 441,66

| | C % | H % | N % |
|---|---|---|---|
| % Calculés | 76,15 | 9,81 | 3,17 |
| % Trouvés | 76,0 | 10,0 | 3,0 |

Spectre IR : ($CHCl_3$)

| | |
|---|---|
| C=O d'amide tertiaire | 1625 $cm^{-1}$ |
| OH | 3601 $cm^{-1}$ |
| Aromatique | 1585 $cm^{-1}$ - 1499 $cm^{-1}$ |

**EXEMPLE 5 : N-butyl 3,17béta-dihydroxy 11béta-estra-1,3,5(10)-trièneheptanamide.**

STADE A : N-butyl 3,7-dioxo 11béta-estra-4,9-dièneheptanamide.

On opère comme au stade A de l'exemple 3, à partir de 2,6 g de l'acide obtenu au stade C de la préparation 3. On obtient 1,845 g de produit attendu.

Spectre IR : ($CHCl_3$)

| | | |
|---|---|---|
| Absence OH | | |
| 17 céto | 1736 $cm^{-1}$ | |
| Diènone + amide | 1659 $cm^{-1}$ | |
| | 1602 $cm^{-1}$ | dont C=C |
| | 864 $cm^{-1}$ | dont C=C |
| NH | 3450 $cm^{-1}$ | |
| Amide II | 1519 $cm^{-1}$ | |

STADE B : 3-acétyloxy N-butyl 17-oxo 11béta-estra-1,3,5(10)-trièneheptanamide.

On opère comme au stade B de l'exemple 3 à partir de 2,025 g du produit obtenu au stade A ci-dessus. On obtient 1,95 g de produit recherché.

Spectre IR : ($CHCl_3$)

| | |
|---|---|
| C=O | 1735 $cm^{-1}$ |
| C=O Amide | 1660 $cm^{-1}$ |
| Amide II | 1518 $cm^{-1}$ |
| NH | 345 $cm^{-1}$ |

STADE C : N-butyl 3,17béta-dihydroxy 11béta-estra-1,3,5(10)-trièneheptanamide.

On opère comme au stade C de l'exemple 3 à partir de 1,68 g de produit obtenu au stade B, ci-dessus. On obtient, après chromatographie sur silice (éluant : chlorure de méthylène-acétone (8-2)), 1,277 g de produit attendu.

On a préparé un échantillon analytique par recristallisation de 1,2 g de produit brut dans l'acétate d'éthyle, on obtient ainsi 1,04 g de produit recherché. F ≃ 139°C.

$[alpha]_D$ + 113° ± 2° (c = 1 % éthanol).

Analyse pour $C_{29}H_{45}O_3N$ = 455,65

| | C % | H % | N % |
|---|---|---|---|
| % Calculés | 76,44 | 9,95 | 3,07 |
| % Trouvés | 76,5 | 10,1 | 3,0 |

Spectre IR : ($CHCl_3$)

| | |
|---|---|
| OH + associés | 3604 $cm^{-1}$ |
| NH | 3448 $cm^{-1}$ |
| C=O | 1657 $cm^{-1}$ |
| Amide II | 1522 $cm^{-1}$ |
| Aromatiques | 1620 $cm^{-1}$ |
| | 1609 $cm^{-1}$ |
| | 1584 $cm^{-1}$ |
| | 1499 $cm^{-1}$ |

**EXEMPLE 6 : N-butyl 3,17béta-dihydroxy N-méthyl 11béta-estra-1,3,5(10)-trièneheptanamide.**

STADE A : N-butyl 3,7-dioxo N-méthyl 11béta-estra-4,9-dièneheptanamide.

On opère comme au stade A de l'exemple 5 à partir de 3,04 g de l'acide obtenu au stade C de la préparation 3, en utilisant 3,8 $cm^3$ de N-méthyl butylamine. On obtient 3 g de produit recherché.

Spectre IR : ($CHCl_3$)
Absence d'OH

| | |
|---|---|
| 17 céto | 1736 $cm^{-1}$ |
| C=O complexe | 1656 $cm^{-1}$ (cétone conjuguée)<br>1629 $cm^{-1}$ (amide tertiaire) |
| C=C | 1603 $cm^{-1}$ |

STADE B : 3-acétyloxy N-butyl N-méthyl 17-oxo 11béta-estra-1,3,5(10)-trièneheptanamide.

On opère comme au stade B de l'exemple 5 à partir de 2,95 g du produit obtenu au stade A ci-dessus. On obtient 2,7 g de produit recherché.

Spectre IR : ($CHCl_3$)

| | |
|---|---|
| C=O | 1760 $cm^{-1}$ (ep.) région OAC du phénol<br>1736 $cm^{-1}$ (max.) 17 céto |
| Aromatique | 1627 $cm^{-1}$ Amide III<br>1585 $cm^{-1}$ (ep.)<br>1493 $cm^{-1}$ (F) |

STADE C : N-butyl 3,17béta-dihydroxy N-méthyl 11béta-estra-1,3,5(10)-trièneheptanamide.

On opère comme au stade C de l'exemple 5, à partir de 2,6 g du produit obtenu au stade B ci-dessus. On obtient 2,067 g de produit attendu.

On prépare un échantillon analytique en recristallisant 1,8 g du produit ci-dessus, dans le dichloréthane. On obtient 1,697 g de produit recherché. F = 110°C.
$[alpha]_D$ + 106°5 ± 2° (c = 1 % éthanol).

Analyse pour $C_{30}H_{47}O_3N$ = 469,68

| | C % | H % | N % |
|---|---|---|---|
| % Calculés | 76,71 | 10,09 | 2,98 |
| % Trouvés | 76,4 | 10,03 | 2,9 |

**EXEMPLE 7 : N-butyl 4-(17béta-hydroxy 3-oxo estra-4,9-dièn-11béta-yl) N-méthyl benzèneoctanamide.**

STADE A : (11béta,17béta) N-butyl N-méthyl 4-[17-acétyloxy 3-oxo estra-4,9-dièn-11-yl] benzèneoctanamide.

a) Acide 11béta-17-acétyloxy 3-oxo estra-4,9-diène-benzèneoctanoïque.

On opère comme au stade C de la préparation 2 à partir de 970 mg du produit obtenu au stade D de la préparation 4. On obtient 1,219 g de produit recherché que l'on utilise tel quel pour l'amidification.

b) Amidification.

On opère comme au stade A de l'exemple 3, à partir de 1,219 g du produit obtenu ci-dessus, en utilisant

1,1 $cm^3$ de N-méthyl butyl amine, on obtient, après chromatographie sur silice (éluant : cyclohexane-acétate d'éthyle (5-5)), 842 mg de produit attendu.

Spectre IR : ($CHCl_3$)
Absence d'acide

| | |
|---|---|
| C=O | 1728 $cm^{-1}$ (OAC) |
| Aromatique | 1509 $cm^{-1}$ |
| Diènone + Amide III | 1652 $cm^{-1}$<br>1628 $cm^{-1}$ |

STADE B : N-butyl 4-[17béta-hydroxy 3-oxo estra-4,9-dièn-11béta-yl] N-méthyl benzèneoctanamide.

On opère comme au stade B de l'exemple 1, à partir de 716 mg du produit obtenu au stade A ci-dessus, on obtient, après chromatographie sur silice (éluant : cyclohexane-acétate d'éthyle (5-5)), 508 mg du produit recherché.
$[alpha]_D$ + 169° ± 3° (c = 0,5 EtoH).

Analyse pour $C_{37}H_{53}NO_3$ = 559,79

| | C % | H % | N % |
|---|---|---|---|
| % Calculés | 79,38 | 9,54 | 2,5 |
| % Trouvés | 79,2 | 9,7 | 2,4 |

Spectre IR : ($CHCl_3$)

| | |
|---|---|
| HO + Associé | 3612 $cm^{-1}$ |
| Diènone + Amide tertiaire | 1628 $cm^{-1}$<br>1643 $cm^{-1}$ |
| Aromatique | 1508 $cm^{-1}$ |

**EXEMPLE 8 : N-butyl 4-(3,17béta-dihydroxy estra-1,3,5(10)-trièn-11béta-yl) N-méthyl benzèneoctanamide.**

STADE A : N-butyl 4-[3,17-diacétyloxy estra-1,3,5(10)-trièn-11béta-yl] N-méthyl benzèneoctanamide.

A une solution de 293 mg du produit obtenu au stade A de l'exemple 7, dans 3 $cm^3$ de chlorure de méthylène, on ajoute à 0° + 5°C, 0,15 $cm^3$ de bromure d'acétyle et 0,3 $cm^3$ d'anhydride acétique, on agite 2 heures à température ambiante. On verse dans une solution saturée de bicarbonate de sodium, agite 30 minutes et extrait avec du chlorure de méthylène. On lave à l'eau, sèche et évapore à sec sous vide. On chromatographie le résidu (300 mg) sur silice (éluant : cyclohexane-acétate d'éthyle (7-3)), on obtient 217 mg de produit attendu.

Spectre IR :

| | | |
|---|---|---|
| C=O | 1755 $cm^{-1}$<br>1728 $cm^{-1}$<br>1627 $cm^{-1}$ | <br>OAC<br>Amide tertiaire |
| Aromatique en 11 | 1509 $cm^{-1}$ | |
| Cycle A aromatique | 1494 $cm^{-1}$ | |

STADE B : N-butyl 4-[3,17béta-dihydroxy estra-1,3,5(10)-trièn-11béta-yl] N-méthyl benzèneoctanamide.

On agite 40 minutes à température ambiante : 471 mg du produit obtenu comme au stade A en solution dans 3,65 cm³ de potasse méthanolique. On ajoute de la glace puis 2,5 cm³ d'acide chlorhydrique 2N. On extrait avec de l'acétate d'éthyle, lave à l'eau, sèche et évapore à sec sous vide. On chromatographie le résidu 460 mg sur silice (éluant : cyclohexaneacétate d'éthyle (5-5)). On obtient 264 mg de produit recherché.
$[alpha]_D$ + 20° ± 2° (c = 0,5 % EtoH).

Analyse pour $C_{37}H_{53}O_3N$ = 559,79

| | C % | H % | N % |
|---|---|---|---|
| % Calculés | 79,38 | 9,54 | 2,5 |
| % Trouvés | 79,8 | 9,6 | 2,3 |

Spectre IR : ($CHCl_3$)

| | | |
|---|---|---|
| HO | 3605 $cm^{-1}$ | + Associé |
| C=O | 1621 $cm^{-1}$ | Amide tertiaire |
| Aromatique | 1583 $cm^{-1}$<br>1500 $cm^{-1}$ | |

**EXEMPLE 9 : N,N-diméthyl 17béta-hydroxy 3-oxo 11béta-estra-4,9-dièneundécanamide**

STADE A : N,N-diméthyl 17béta-acétyloxy 3-oxo 11béta-estra-4,9-dièneundécanamide.

On opère comme au stade A de l'exemple 3 à partir de 2,7 g du produit obtenu au stade E de la préparation 5, en utilisant 2,28 g de diméthyl amine en solution dans 20 cm³ de tétrahydrofuranne. On obtient 1,6 g de produit attendu.

Spectre IR : ($CHCl_3$)

Absence d'acide

| | |
|---|---|
| OAC | 1728 $cm^{-1}$ |
| Diènone + Amide III | 1336 $cm^{-1}$<br>1603 $cm^{-1}$ |

STADE B : N,N-diméthyl 17béta-hydroxy 3-oxo 11béta-estra-4,9-dièneundécanamide.

On opère comme au stade B de l'exemple 1, à partir de 780 mg du produit obtenu au stade A ci-dessus. Après chromatographie sur silice (éluant : chlorure de méthylène-acétone (8-2)), on obtient 485 mg du produit recherché.
$[alpha]_D$ -21°5 ± 2°5 (c = 0,35 % EtoH).

Analyse pour $C_{31}H_{49}NO_3$ = 483,74

| | C % | H % | N % |
|---|---|---|---|
| % Calculés | 76,97 | 10,21 | 2,89 |
| % Trouvés | 77,0 | 10,2 | 2,7 |

Spectre IR : ($CHCl_3$)

| | | |
|---|---|---|
| HO | 3616 $cm^{-1}$ | |
| Amide III | 1640 $cm^{-1}$ | (complexe) |
| Diènone | 1604 $cm^{-1}$ | C=O |
| | 964 $cm^{-1}$ | C=C |

**EXEMPLE 10 : N,N-diméthyl 3,17béta-dihydroxy 11béta-estra-1,3,5(10)-trièneundécanamide**

STADE A : 3,17béta-diacétyloxy N,N-diméthyl 11béta-estra-1,3,5(10)-trièneundécanamide.

On opère comme au stade B de l'exemple 3, à partir de 697 mg du produit obtenu au stade A de l'exemple 9. On obtient 660 mg de produit recherché.

Spectre IR : ($CHCl_3$)

Absence de diènone

| | | |
|---|---|---|
| Acétate | 1726 $cm^{-1}$ | |
| | 1745 $cm^{-1}$ | (ep.) |
| Amide III | 1632 $cm^{-1}$ | |
| Aromatiques | 1494 $cm^{-1}$ | |

STADE B : 3,17béta-dihydroxy N,N-diméthyl 11béta-estra-1,3,5(10)-trièneundécanamide.

On opère comme au stade B de l'exemple 1 et isole le produit par cristallisation dans l'isopropanol. On obtient 369 mg de produit attendu. F = 130°C.
[alpha]$_D$ +100° ± 2° (c = 0,95 % EtoH).

Analyse pour $C_{31}H_{49}O_3N$ = 483,74

| | C % | H % | N % |
|---|---|---|---|
| % Calculés | 76,97 | 10,21 | 2,89 |
| % Trouvés | 77,0 | 10,4 | 2,7 |

Spectre IR : ($CHCl_3$)

| | |
|---|---|
| HO + Associé | 3605 $cm^{-1}$ |
| Amide III | 1627 $cm^{-1}$ |
| Aromatique | 1528 $cm^{-1}$ - 1498 $cm^{-1}$ |

**EXEMPLE 11 : N-butyl 17béta-hydroxy 3-oxo 11béta-estra-4,9-dièneundécanamide.**

STADE A : 17béta-acétyloxy N-butyl 3-oxo 11béta-estra-4,9-dièneundécanamide.

On opère comme au stade A de l'exemple 3, à partir de 6,67 g du produit obtenu au stade E de la préparation 5, on obtient, après chromatographie sur silice (éluant : cyclohexane-acétate d'éthyle (1-1)), 6,911 g de produit recherché. On effectue une seconde chromatographie sur 3,443 g du produit obtenu ci-dessus, avec le même éluant, on obtient 2,898 g de produit purifié.

Analyse pour $C_{35}H_{55}O_4N$ = 553,75

| | C % | H % | N % |
|---|---|---|---|
| % Calculés | 75,9 | 10,01 | 2,52 |
| % Trouvés | 75,6 | 10,1 | 2,7 |

Spectre IR : ($CHCl_3$)

Peu ou pas d'acide

| | |
|---|---|
| -C-NH avec =C-N-H<br>‖<br>O | 3450 $cm^{-1}$ |
| Amide II | 1519 $cm^{-1}$ |
| C=O | 1657 $cm^{-1}$ |
| Acétate | 1728 $cm^{-1}$<br>1255 $cm^{-1}$ |
| Diènone | 1657 $cm^{-1}$<br>1601 $cm^{-1}$ |

STADE B : N-butyl 17béta-hydroxy 3-oxo 11béta-estra-4,9-diène-undécanamide.

On agite 40 minutes à température ambiante 0,5 g du produit obtenu au stade A, 5 $cm^3$ de méthanol et 280 mg de potasse en pastilles. On ajoute 20 $cm^3$ de glace et neutralise avec 2,5 $cm^3$ d'acide chlorhydrique 2N, on extrait avec de l'acétate d'éthyle et obtient après évaporation à sec 509 mg de produit que l'on chromatographie sur silice (éluant : cyclohexane-acétate d'éthyle (1-1)). On obtient 287 mg de produit recherché.

Spectre IR : $CHCl_3$ (sur Nicolet)

| | |
|---|---|
| OH | 3612 $cm^{-1}$ |
| =C-NH | 3450 $cm^{-1}$ |
| Amide II | 1518 $cm^{-1}$ |
| C=O | 1657 $cm^{-1}$ (amide secondaire + diénone) |

Analyse pour $C_{33}H_{53}NO_3$ = 511,8

| | C % | H % | N % |
|---|---|---|---|
| % Calculés | 77,45 | 10,44 | 2,74 |
| % Trouvés | 77,1 | 10,7 | 2,7 |

**EXEMPLE 12 : N-butyl 3,17béta-dihydroxy 11béta-estra-1,3,5(10)-trièneundécanamide**

STADE A : 3,17béta-bis(acétyloxy) N-butyl 11béta-estra-1,3,5(10)-trièneundécanamide.

On opère comme au stade B de l'exemple 3, à partir de 3 g du produit obtenu au stade A de l'exemple 11, on obtient 3,66 g de produit brut utilisé tel quel pour le stade suivant.

Spectre IR : ($CHCl_3$)

| | |
|---|---|
| -C-<br>‖<br>O | 1749 $cm^{-1}$ (ep.)<br>1726 $cm^{-1}$ (max.) |

| | | |
|---|---|---|
| Acétate | 1254 $cm^{-1}$ (F) | |
| -C(=O)- amide | 1660 $cm^{-1}$ | } -C(=O)-NH |
| =C-NH | 3450 $cm^{-1}$ | |
| Amide II | 1518 $cm^{-1}$ | |
| Aromatique | 1612 $cm^{-1}$<br>1583 $cm^{-1}$<br>1494 $cm^{-1}$ | |

STADE B : N-butyl 3,17béta-dihydroxy 11béta-estra-1,3,5(10)-trièneundécanamide.

A une solution de 2,96 g de produit obtenu au stade A, dans 74 $cm^3$ de méthanol, on ajoute 8,9 $cm^3$ d'une solution à 11 g % $cm^3$ de potasse méthanolique et on agite 2 heures à 60°C.

On glace puis neutralise avec 15 $cm^3$ d'acide chlorhydrique N, on élimine le méthanol, dilue à l'eau saturée de chlorure de sodium et extrait avec de l'acétate d'éthyle, après évaporation à sec, on obtient 2,7 g de produit que l'on chromatographie sur silice (éluant : cyclohexane-acétate d'éthyle (5-5)). On recueille 1,841 g de produit recherché.

Un échantillon analytique a été préparé en chromatographiant à nouveau le produit dans les mêmes conditions. On obtient 1,547 g du composé attendu.

$[alpha]_D$ +85° ± 3° (c = 0,5 % éthanol).

Analyse pour $C_{33}H_{53}NO_3$ = 511,75

| | C % | H % | N % |
|---|---|---|---|
| % Calculés | 77,45 | 10,43 | 2,73 |
| % Trouvés | 77,5 | 10,7 | 2,9 |

(perte sous vide 1,5 % à 100°C).

Spectre IR : ($CHCl_3$)

| | | |
|---|---|---|
| HO | | 3605 $cm^{-1}$ |
| =C-NH | | 3448 $cm^{-1}$ |
| -C(=O)- | Amide | 1657 $cm^{-1}$ |
| Amide II | | 1524 $cm^{-1}$ |
| Aromatique | | 1620 $cm^{-1}$<br>1582 $cm^{-1}$<br>1498 $cm^{-1}$ (F) |

**EXEMPLE 13 : N-butyl 3,17béta-dihydroxy N-méthyl 11béta-estra-1,3,5(10)-trièneundécanamide**

STADE A : N-butyl 11-3,17béta-bis[(tétrahydro 2H-pyrann-2-yl) oxy] 11béta-estra-1,3,5(10)-trièneundécanamide.

On agite pendant 1 heure 30 une solution de 226 mg de produit obtenu au stade B de l'exemple 12, 6,7 $cm^3$ d'éther sulfurique, 4,7 $cm^3$ de dihydropyran et 5 mg d'acide paratoluène sulfonique. On ajoute 1 $cm^3$ de

triéthylamine, lave avec une solution de bicarbonate de sodium, puis avec une solution saturée de chlorure de sodium, sèche et évapore à sec. On chromatographie le résidu (400 mg) sur silice (éluant : cyclohexane-acétate d'éthyle (7-3)). On obtient 286 mg de produit recherché.

<u>Spectre IR</u> : ($CHCl_3$)

Absence d'OH

Présence de 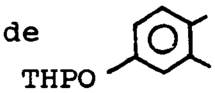 1606 $cm^{-1}$, 1574 $cm^{-1}$, 1497 $cm^{-1}$

| | |
|---|---|
| NH | 3450 $cm^{-1}$ |
| Amide II | 1518 $cm^{-1}$ |
| C=O | 1660 $cm^{-1}$ |

<u>STADE B</u> : N-butyl 3, 17béta-bis (tétrahydro 2H-pyrann-2-yl) oxy] N-méthyl 11béta-estra-1,3,5(10)-trièneundécanamide.

On agite pendant 18 heures à 50°C, sous pression, 920 mg du produit obtenu au stade A avec 14 $cm^3$ de tétrahydrofuranne, 2,27 g de bromure de tétrabutylammonium, 1,9 g de potasse pulvérisé et 14 $cm^3$ d'iodure de méthyle.

Après refroidissement, on filtre l'insoluble, le lave avec 5 fois 50 $cm^3$ de tétrahydrofuranne et évapore à sec sous pression réduite. On chromatographie le résidu (3,4 g) sur silice (éluant : cyclohexane-acétate d'éthyle-triéthylamine (70-30-0,2)). On obtient 850 mg du produit attendu (utilisé tel que pour le stade suivant).

<u>Spectre IR</u> : ($CHCl_3$)

Pas d'amide secondaire

| | |
|---|---|
| C=O | 1627 $cm^{-1}$ |
| OTHP | Probable |
| Aromatique | 1605 $cm^{-1}$, 1572 $cm^{-1}$, 1497 $cm^{-1}$ |

<u>STADE C</u> : N-butyl 3,17béta-dihydroxy N-méthyl 11béta-estra-1,3,5(10)-trièneundécanamide.

On agite pendant 1 heure à température ambiante 850 mg du produit obtenu au stade B, 35 $cm^3$ de méthanol et 3,5 $cm^3$ d'acide chlorhydrique 2N. On amène ensuite à pH 5/6 avec de l'ammoniaque concentré à 5 $cm^3$, dilue avec du chlorure de méthylène, lave à l'eau puis avec une solution saturée de chlorure de sodium, sèche et évapore à sec sous pression réduite. On chromatographie, les 638 mg obtenus, sur silice (éluant : cyclohexane-acétate d'éthyle (6-4)). On recueille 520 mg du produit recherché.

Un échantillon analytique a été préparé à partir de 618 mg de produit (98 mg d'un essai précédent étant joints aux 520 mg obtenus ci-dessus) par chromatographie sur silice (éluant : chlorure de méthylène-acétone (9-1)). On a recueilli 527 mg de produit purifié.

$[alpha]_D$ +90,5° ± 2° (c = 1 EtoH).

Analyse pour $C_{34}H_{55}O_3N$ = 525,82

| | C % | H % | N % |
|---|---|---|---|
| % Calculés | 77,67 | 10,54 | 2,66 |
| % Trouvés | 77,0 | 10,6 | 2,6 |

Spectre IR : ($CHCl_3$)

Absence d'amide II

| | |
|---|---|
| HO | 3606 $cm^{-1}$ (+ associé) |
| C=O | 1620 $cm^{-1}$ |
| Aromatique | 1582 $cm^{-1}$<br>1498 $cm^{-1}$ |

**EXEMPLE 14 : N-butyl 3,17béta-dihydroxy N-méthyl 11béta-estra-1,3,5(10)-trièneundécanamide**

STADE A : 17béta-acétyloxy N-butyl N-méthyl 3-oxo 11béta-estra-4,9-dièneundécanamide.

On opère comme au stade A de l'exemple 3, à partir de 88,5 g de l'acide obtenu au stade E de la préparation 5 et en utilisant 88,5 $cm^3$ de N-méthyl butylamine. On obtient après chromatographie sur silice (éluant : hexane-acétate d'éthyle (5-5)). On obtient 67,3 g du produit recherché.

Spectre IR : ($CHCl_3$)

| | |
|---|---|
| OAC | 1728 $cm^{-1}$<br>1255 $cm^{-1}$ |
| Diènone | 864 $cm^{-1}$ |
| Diènone + Amide III | 1628 $cm^{-1}$<br>1655 $cm^{-1}$ |

STADE B : 3,17béta-bis(acétyloxy) N-butyl N-méthyl 11béta-estra-1,3,5(10)-trièneundécanamide.

On opère comme au stade B de l'exemple 3, à partir de 67,3 g du produit obtenu au stade A ci-dessus. L'extrait sec obtenu n'est pas chromatographié, on obtient 75 g de produit recherché, utilisé tel quel pour le stade suivant.

STADE C : N-butyl 3,17béta-dihydroxy N-méthyl 11béta-estra-1,3,5(10)-trièneundécanamide.

On agite 2 heures à 60°C, 74 g du produit obtenu au stade B, 1,850 litre de méthanol et 222 $cm^3$ de potasse méthanolique à 11 g % $cm^3$. On refroidit ensuite à 0°/+ 5°C et ajuste le pH à 4/5 par addition d'acide chlorhydrique. On distille le méthanol sous pression réduite puis extrait avec du chlorure de méthylène, lave les extraits organiques à l'eau, sèche et évapore à sec sous pression réduite, on obtient 65,8 g de résine à laquelle on ajoute 1,7 g provenant d'un essai précédent.

Purification :

On recristallise par chaud et froid 67,5 g de produit brut dans 350 $cm^3$ d'acétate d'éthyle, on essore à 0°/+ 5°C et on obtient 45 g de produit attendu auxquels on joint 3 g de produit monopurifié obtenu des liqueurs-mères. On recristallise les 48 g de produits dans 4 volumes d'acétate d'éthyle et on obtient ainsi 46,1 g de produit recherché. F = 127°C.
$[alpha]_D$ +90,1° ± 2° (c = 1 % EtoH).

Analyse pour $C_{34}H_{55}O_3N$ = 525,82

| | C % | H % | N % |
|---|---|---|---|
| % Calculés | 77,67 | 10,54 | 2,66 |
| % Trouvés | 77,9 | 10,6 | 2,6 |

**EXEMPLE 15 : 17béta-hydroxy N-méthyl N-(1-méthyl éthyl) 3-oxo 11béta-estra-4,9-dièneundécanamide**

STADE A : N-(1-méthyl éthyl) 3,17-dioxo N-méthyl 11béta-estra-4,9-dièneundécanamide.

On opère comme au stade A de l'exemple 3, à partir de 610 mg de l'acide obtenu à la préparation 6 et en utilisant 0,73 $cm^3$ de N-méthyl N-isopropylamine. On obtient, après chromatographie sur silice (éluant : chlorure de méthylène-acétone (9-1)), 455 mg de produit recherché.

Spectre IR : ($CHCl_3$)

| | |
|---|---|
| 17 céto | 1736 $cm^{-1}$ |
| diénone | 1656 $cm^{-1}$ |
| Amide tertiaire | 1621 $cm^{-1}$ |

STADE B : 17béta-hydroxy N-méthyl N-(1-méthyl éthyl) 3-oxo 11béta-estra-4,9-dièneundécanamide.

A une solution de 1,18 g de produit obtenu au stade A, dans 18 $cm^3$ de tétrahydrofuranne, on ajoute à 0°/-5°C, 707 mg d'hydrure de triterbutoxy aluminium-lithium. On agite 1 heure à 0°/-5°C et verse sur 80 g d'un mélange à parties égales de glace et d'une solution saturée de chlorure d'ammonium. On agite 5 minutes et extrait 3 fois à l'acétate d'éthyle, lave la phase organique à l'eau saturée de chlorure de sodium, sèche et évapore à sec sous pression réduite, on obtient 1,14 g de résidu auquel on joint 256 mg obtenus d'une charge précédente, on chromatographie 1,4 g de produit brut sur silice (éluant : chlorure de méthylène-acétone (9-1)) et on obtient 640 mg de produit attendu.

$[alpha]_D$ -43° ± 2°5 (c = 0,4 % d'éthanol).

Analyse pour $C_{33}H_{53}O_3N$ = 511,75

| | C % | H % | N % |
|---|---|---|---|
| % Calculés | 77,44 | 10,44 | 2,74 |
| % Trouvés | 77,6 | 10,7 | 2,7 |

Spectre IR : ($CHCl_3$)

| | | |
|---|---|---|
| OH | 3613 $cm^{-1}$ | |
| C=O | 1650 $cm^{-1}$ | Diènone |
| | 1621 $cm^{-1}$ | + Amide tertiaire |

**EXEMPLE 16 : 3,17béta-dihydroxy N-méthyl N-(1-méthyl éthyl) 11béta-estra-1,3,5(10)-trièneundécanamide**

STADE A : 3-acétyloxy N-(1-méthyl éthyl) 17-oxo N-méthyl 11béta-estra-1,3,5(10)-trièneundécanamide.

On opère comme au stade B de l'exemple 3, à partir de 360 mg de produit obtenu au stade A de l'exemple 15. Après chromatographie sur silice (éluant : chlorure de méthylène-acétone (95-5)). On obtient 310 mg de produit attendu.

<u>Spectre IR</u> : ($CHCl_3$)

| | |
|---|---|
| Amide tertiaire | 1621 $cm^{-1}$ |
| Acétate phénolique | 1760 $cm^{-1}$<br>1765 $cm^{-1}$ |
| 17 céto | 1735 $cm^{-1}$ |
| Aromatique | 1493 $cm^{-1}$ |

<u>STADE B</u> : N-(1-méthyl éthyl) 3,17béta-dihydroxy N-méthyl 11béta-estra-1,3,5(10)-trièneundécanamide.

On opère comme au stade C de l'exemple 3, à partir de 272 mg du produit obtenu au stade A, ci-dessus. On obtient, après chromatographie (éluant : chlorure de méthylène-acétone (9-1)), 195 mg de produit attendu brut.

Au produit obtenu on ajoute 460 mg provenant d'un essai précédent et purifie par un triple passage sur silice (éluant : acétonitrile) on recueille 460 mg de produit que l'on chromatographie à nouveau sur silice (éluant : chlorure de méthylène-acétone (9-1)). On obtient 432 mg du produit recherché.
$[alpha]_D$ +85° ± 2° (c = 1 % EtoH).

Analyse pour $C_{33}H_{53}O_3N$ = 511,75

| | C % | H % | N % |
|---|---|---|---|
| % Calculés | 77,44 | 10,44 | 2,74 |
| % Trouvés | 77,4 | 10,7 | 2,6 |

<u>Spectre IR</u> :

| | |
|---|---|
| OH | 3606 $cm^{-1}$ + associé |
| C=O | 1618 $cm^{-1}$ |
| Aromatique | 1583 $cm^{-1}$<br>1498 $cm^{-1}$ |

**<u>EXEMPLE 17</u> : N-(phénylméthyl) 17béta-hydroxy N-méthyl 3-oxo 11béta-estra-4,9-dièneundécanamide**

<u>STADE A</u> : N-(phénylméthyl) 17béta-acétoxy N-méthyl 3-oxo 11béta-estra-4,9-dièneundécanamide.

On opère comme au stade A de l'exemple 3, à partir de 2,1 g de l'acide obtenu comme au stade E de la préparation 5 et en utilisant 2,2 $cm^3$ de benzyl méthylamine, on obtient, après chromatographie sur silice (éluant : cyclohexane-acétate d'éthyle (1-1)), 1,82 g de produit recherché.

<u>Spectre IR</u> : ($CHCl_3$)

| | |
|---|---|
| C=O | 1728 $cm^{-1}$ (OAC)<br>1645 $cm^{-1}$ (Amide III + Diénone) |
| C=C | 864 $cm^{-1}$ (def.) |
| Aromatique | 1496 $cm^{-1}$ |

<u>STADE B</u> : N-(phénylméthyl) 17béta-hydroxy N-méthyl 3-oxo 11béta-estra-4,9-dièneundécanamide.

On opère comme au stade B de l'exemple 1, à partir de 1 g du produit obtenu au stade A de l'exemple. On obtient 670 mg de produit recherché.

Analyse pour $C_{37}H_{53}O_3N$ = 559,79

| | C % | H % | N % |
|---|---|---|---|
| % Calculés | 79,39 | 9,54 | 2,5 |
| % Trouvés | 79,3 | 9,7 | 2,4 |

Spectre IR : ($CHCl_3$)

| | |
|---|---|
| C=O | 1603 $cm^{-1}$ |
| >C=O | 1642 $cm^{-1}$ (diénone + amide III) |
| C=C | 863 $cm^{-1}$ |
| Aromatique | 1496 $cm^{-1}$ |

**EXEMPLE 18 : N-(phénylméthyl) 3,17béta-dihydroxy N-méthyl 11béta-estra-1,3,5(10)-trièneundécanamide**

STADE A : 3,17béta-diacétyloxy N-(phénylméthyl) N-méthyl 11béta-estra-1,3,5(10)-trièneundécanamide.

A une solution de 829 mg du produit obtenu au stade A de l'exemple 17, dans 8 $cm^3$ de chlorure de méthylène, refroidie à 0°/+ 5°C, on ajoute 0,6 $cm^3$ d'anhydride acétique et 0,3 $cm^3$ de bromure d'acétyle, on agite pendant 2 heures, puis verse sur 60 g d'un mélange glace et solution saturée de bicarbonate de sodium (1-1), on agite 30 minutes, décante et extrait avec du chlorure de méthylène. On lave les fractions organiques avec une solution saturée de chlorure de sodium, sèche et évapore à sec sous pression réduite, on chromatographie le résidu sur silice (éluant : cyclohexane-AcOEt (75-25). On obtient 760 mg de produit recherché.

Spectre IR :

| | |
|---|---|
| Acétate phénolique | 1767-1752 $cm^{-1}$ |
| Acétate en 17 | 1727 $cm^{-1}$ |
| C=O Amide III | 1632 $cm^{-1}$ |
| Aromatiques | 1494 $cm^{-1}$<br>1585 $cm^{-1}$ |

STADE B : N-(phénylméthyl) 3,17béta-dihydroxy N-méthyl 11béta-estra-1,3,5(10)-trièneundécanamide.

On agite pendant 1 heure à température ambiante 630 mg du produit obtenu au stade A, 13 $cm^3$ de méthanol et 910 mg de potasse pastilles. On amène à pH 4/5, par addition d'acide chlorhydrique 2N, extrait à l'acétate d'éthyle, lave avec une solution saturée de chlorure de sodium et évapore à sec sous pression réduite. On chromatographie le résidu (555 mg) sur silice (éluant : chlorure de méthylène-acétone (9-1)). On obtient 390 mg de produit recherché.
$[alpha]_D$ +85°5 ± 2° (c = 0,9 % EtoH).

Analyse pour $C_{37}H_{53}O_3N$ = 559,79

| | C % | H % | N % |
|---|---|---|---|
| % Calculés | 79,38 | 9,54 | 2,5 |
| % Trouvés | 79,5 | 9,6 | 2,4 |

Spectre IR :

| | | |
|---|---|---|
| OH | 3605 $cm^{-1}$ | Libre + associé |
| C=O Amide III | 1627 $cm^{-1}$ | |
| Aromatique | 1583 $cm^{-1}$ | |
| | 1497 $cm^{-1}$ | |

**EXEMPLE 19 : N-[2-(diméthylamino) éthyl] 17béta-hydroxy N-méthyl 3-oxo 11béta-estra-4,9-dièneundécanamide**

STADE A : N-[2-(diméthylamino) éthyl] 17béta-acétyloxy N-méthyl 3-oxo 11béta-estra-4,9-dièneundécanamide.

On opère comme au stade A de l'exemple 3, à partir de 500 mg de produit obtenu comme au stade E de la préparation 5 en utilisant 0,5 $cm^3$ de N-N-N-triméthyl éthylènediamine. On chromatographie sur silice (éluant : toluène-triéthylamine (8-2)) et on obtient 380 mg de produit recherché.

Spectre IR : ($CHCl_3$)

| | |
|---|---|
| Acétate | 1729 $cm^{-1}$ |
| | 1255 $cm^{-1}$ |
| Diènone + Amide III | 1644 $cm^{-1}$ |
| C=C | 1604 $cm^{-1}$ |
| Bandes de Bohlmann. | |

STADE B : N-[2-(diméthylamino) éthyl] 17béta-hydroxy N-méthyl 3-oxo 11béta-estra-4,9-dièneundécanamide.

On agite, pendant 1 heure à température ambiante, 800 mg de produit obtenu comme au stade A de l'exemple, 8 $cm^3$ de méthanol et 500 mg de potasse en pastilles. On concentre de moitié ajoute un mélange eau et glace et extrait avec de l'acétate d'éthyle, lave les fractions organiques à l'eau puis avec une solution saturée de chlorure de sodium, sèche et évapore à sec sous pression réduite. Le résidu est chromatographié une première fois sur silice (éluant : acétate d'éthyle-isopropanol-ammoniaque (80-20-2)) puis une seconde fois le résidu (645 mg) (éluant : acétate d'éthyle-triéthylamine (6-4)), on recueille 526 mg de produit recherché. $[alpha]_D$ -26° ± 1,5° (c = 0,7 % EtoH).

$[alpha]_D$ -26° ± 1,5° (c = 0,7 % EtoH).

Analyse pour $C_{34}H_{56}O_3N_2$

| | C % | H % | N % |
|---|---|---|---|
| % Calculés | 75,51 | 10,44 | 5,18 |
| % Trouvés | 75,3 | 10,6 | 5,2 |

Spectre IR : ($CHCl_3$)

Présence OH et bande de Bohlmann

| | |
|---|---|
| Amide tertiaire et 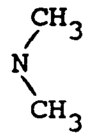 | 1642 $cm^{-1}$ |
| C=C | 1602 $cm^{-1}$ |
| =C-H dif. | 863 $cm^{-1}$ |

**EXEMPLE 20 : N-[2-(diméthylamino) éthyl] 3,17béta-dihydroxy N-méthyl 11béta-estra-1,3,5(10)-trièneundécanamide**

STADE A : N-[2-(diméthylamino) éthyl] 3,17béta-diacétyloxy N-méthyl 11béta-estra-1,3,5(10)-trièneundécanamide.

On opère comme au stade B de l'exemple 3, à partir de 800 mg de produit obtenu comme au stade A de l'exemple 19. On obtient, après chromatographie sur silice (éluant : toluène-triéthylamine (8-2)), 700 mg de produit recherché.

Spectre IR : ($CHCl_3$)

| | | | |
|---|---|---|---|
| C=O | Acétate phénolique | 1767-1750 $cm^{-1}$ | |
| | Acétate en 17 | 1727 $cm^{-1}$ | |
| | Amide tertiaire | 1630 $cm^{-1}$ | |
| Aromatiques | | 1587 $cm^{-1}$ | Bandes de |
| | | 1494 $cm^{-1}$ | Bolhmann |

STADE B : N-[2-(diméthylamino) éthyl] 3,17béta-dihydroxy N-méthyl 11béta-estra-1,3,5(10)-trièneundécanamide.

On opère comme au stade B de l'exemple 10, à partir de 880 mg de produit obtenu au stade A. On chromatographie sur silice (éluant : toluène-triéthylamine (8-2)) et recueille 550 mg de produit que l'on chromatographie à nouveau (éluant : acétate d'éthyle-isopropanol-ammoniaque (80-20-2)). On obtient 527 mg de produit recherché.

$[alpha]_D$ +77°5 ± 1,5° (c = 0,9 % EtoH).

Analyse pour $C_{34}H_{56}O_3N_2$ = 540,80

| | C % | H % | N % |
|---|---|---|---|
| % Calculés | 75,51 | 10,44 | 5,18 |
| % Trouvés | 75,3 | 10,6 | 5,2 |

Spectre IR : ($CHCl_3$)

| | |
|---|---|
| OH | 3607 $cm^{-1}$ |
| Amide III | 1627 $cm^{-1}$ |
| Aromatiques | 1582 $cm^{-1}$ |
| | 1498 $cm^{-1}$ |

**EXEMPLE 21 : N-butyl 3,17béta-dihydroxy N-méthyl 19-nor-11béta (17alpha-pregna-1,3,5(10)-trièn-20-yn) undécanamide**

STADE A : N-butyl 3,17-dioxo N-méthyl 11béta-estra-4,9-dièneundécanamide.

On opère comme au stade A de l'exemple 3, à partir de 7,5 g d'acide obtenu au stade B de la préparation 6 en utilisant 6,4 $cm^3$ de N-méthyl butylamine. On obtient 5,89 g de produit recherché.
Spectre IR : ($CHCl_3$)
Amide tertiaire 1628 $cm^{-1}$

STADE B : 3-acétyloxy N-butyl N-méthyl 17-oxo 11béta-estra-1,3,5(10)-trièneundécanamide.

On opère comme au stade B de l'exemple 3, à partir de 2,63 g du produit obtenu au stade A, ci-dessus, on recueille 2,91 g de produit brut que l'on utilise tel quel pour le stade suivant.
Spectre IR : ($CHCl_3$) (sur Nicolet)

| | |
|---|---|
| C=O | 1735 $cm^{-1}$ (17 céto + OAC phénolique) |
| Amide tertiaire | 1627 $cm^{-1}$ |
| Aromatique | 1494 $cm^{-1}$ |

STADE C : N-butyl 3,17béta-dihydroxy N-méthyl 19-nor-11béta (17alpha-pregna-1,3,5(10)-trièn-20-yn) undécanamide.

A une solution de 1 g du produit obtenu au stade B ci-dessus, dans 10 $cm^3$ d'éthylène-diamine, on ajoute 1,62 g de complexe d'acétylure de lithium-éthylène diamine et agite 4 heures 30 à 50°C. On refroidit et ajoute 20 g de glace, 10 $cm^3$ d'une solution saturée de chlorure d'ammonium et 30 $cm^3$ de chlorure de méthylène. On filtre, décante et réextrait au chlorure de méthylène, lave avec une solution saturée de chlorure de sodium, sèche, évapore à sec sous pression réduite. On chromatographie le résidu 0,805 g sur silice (éluant : chlorure de méthylène-acétone (9-1)) et obtient 0,58 g de produit que l'on chromatographie à nouveau sur silice (éluant : chlorure de méthylène-acétone (95-5)). On obtient 0,489 g de produit recherché.
$[alpha]_D$ +40°5 ± 2,5° (c = 0,5 % EtoH).

Analyse pour $C_{36}H_{55}O_3N$ = 549,80

| | C % | H % | N % |
|---|---|---|---|
| % Calculés | 78,64 | 10,08 | 2,54 |
| % Trouvés | 78,3 | 10,4 | 2,4 |

Spectre IR : $CHCl_3$ (sur Nicolet)
Absence de 17 céto

| | |
|---|---|
| OH | 3599 $cm^{-1}$ + associé |
| C=CH | 3304 $cm^{-1}$ |
| Amide | 1620 $cm^{-1}$ |
| Aromatiques | 1582 $cm^{-1}$ |
| | 1490 $cm^{-1}$ |

**EXEMPLE 22 : N-butyl 17béta-hydroxy 3-oxo-17alpha(1-propynyl) 11béta-estra-4,9-dièneundécanamide**

On opère comme au stade A de l'exemple 3, à partir de 1,37 g du produit obtenu comme à la préparation 7, en utilisant 1,4 $cm^3$ de butylamine. On chromatographie sur silice (éluant : cyclohexane-acétate d'éthyle (5-5)). On obtient 744 mg de produit recherché.

Analyse pour $C_{36}H_{55}NO_3$ = 549,844

| | C % | H % | N % |
|---|---|---|---|
| % Calculés | 78,64 | 10,08 | 2,55 |
| (solvaté à 2 % acétate éthyle) | 78,15 | 10,46 | 2,49 |
| % Trouvés | 78,2 | 10,4 | 2,4 |

Spectre IR : ($CHCl_3$)

| | |
|---|---|
| Amide secondaire =C-NH | 3450 $cm^{-1}$ |
| Amide II | 1519 $cm^{-1}$ |
| C=O | 1657 $cm^{-1}$ + cétone conjuguée |

**EXEMPLE 23 : N-butyl 17béta-hydroxy N-méthyl 3-oxo-17alpha(1-propynyl) 11béta-estra-4,9-diènedundécanamide**

On opère comme au stade A de l'exemple 3, à partir de 962 mg de l'acide obtenu à la préparation 7 et en utilisant 1 $cm^3$ de N-méthyl butylamine. Après chromatographie sur silice (éluant : cyclohexane-acétate d'éthyle (5-5)). On obtient 0,79 g de produit recherché.

Analyse pour $C_{37}H_{57}NO_3$ = 563,87

| | C % | H % | N % |
|---|---|---|---|
| % Calculés | 78,81 | 10,19 | 2,48 |
| % Trouvés | 78,5 | 10,5 | 2,3 |

Spectre IR : ($CHCl_3$)

| | |
|---|---|
| Amide tertiaire | 1630 $cm^{-1}$ |
| OH | 3602 $cm^{-1}$ |
| Cétone conjuguée | 1643 $cm^{-1}$ avec amide III |

**EXEMPLE 24 : N-butyl 3,17béta-dihydroxy N-méthyl 11béta-estra-1,3,5(10)-triènedodécanamide.**

STADE A : 11béta-N-butyl N-méthyl 3,17-dioxo estra-4,9-diènedodécanamide.

On opère comme au stade A de l'exemple 1, en utilisant 1 g de l'alcool obtenu au stade B de la préparation 8 et 1,1 $cm^3$ de N-méthyl butylamine. Après chromatographie sur silice (éluant : acétate d'éthyle-cyclohexane (8-2)). On obtient 717 mg de produit recherché.

Spectre IR : ($CHCl_3$)

Peu ou pas d'OH

| | | |
|---|---|---|
| C=O 17 céto | | 1735 $cm^{-1}$ |
| C=O | Cétone conjuguée | 1655 $cm^{-1}$ |
| | | 1643 $cm^{-1}$ |
| | Amide tertiaire | 1628 $cm^{-1}$ |
| -C=C (ep.) | | 1603 $cm^{-1}$ |

STADE B : N-butyl N-méthyl 17béta-hydroxy 11béta-estra-4,9-diènedodécanamide.

A une solution de 837 mg de produit obtenu comme au stade A, dans 17 $cm^3$ de tétrahydrofuranne à 0°C,

on ajoute 494 mg de triterbutoxyhydrure d'aluminium lithium. On agite pendant 20 minutes à 0°C. On ajoute ensuite, 20 $cm^3$ d'une solution saturée de chlorure d'ammonium puis extrait à l'acétate d'éthyle et au chlorure de méthylène. On évapore à sec sous pression réduite et chromatographie le résidu (836 mg) sur silice (éluant : acétate d'éthyle cyclohexane (6-4)). On obtient 604 mg de produit attendu.

<u>Spectre IR</u> : ($CHCl_3$)

| | | |
|---|---|---|
| OH | 3612 $cm^{-1}$ | |
| C=O | 1642 $cm^{-1}$ (ep) | Pouvant englober |
| complexe | 1628 $cm^{-1}$ (max) | Diènone + amide tertiaire |

Aliphatiques intenses.

STADE C : N-butyl 3,17béta-dihydroxy N-méthyl 11béta-estra-1,3,5(10)-triènedodécanamide.

A une solution refroidie à 0°C, de 0,2 g de produit obtenu au stade B dans 2,6 $cm^3$ de chlorure de méthylène, on ajoute à 0°C, 0,26 $cm^3$ d'anhydride acétique et 0,13 $cm^3$ de bromure d'acétyle. On laisse remonter à température ambiante. Après 1 heure, on ajoute 20 $cm^3$ de bicarbonate de sodium en solution saturée, on agite 30 minutes et extrait avec du chlorure de méthylène, évapore le solvant sous pression réduite et reprend le résidu (250 mg) dans 10 $cm^3$ de méthanol, on ajoute à la solution 150 mg de pastilles de potasse. On chauffe 2 heures à 40°C. On refroidit à 0°C puis neutralise avec de l'acide chlorhydrique concentré et extrait au chlorure de méthylène. On évapore à sec sous pression réduite et on chromatographie le résidu (188 mg) sur silice (éluant : cyclohexane-acétate d'éthyle (6-4)). On obtient 131 mg de produit attendu.
$[alpha]_D$ +89° ± 3° (c = 0,6 % EtoH).

Analyse pour $C_{35}H_{57}NO_3$

| | C % | H % | N % |
|---|---|---|---|
| % Calculés | 77,87 | 10,64 | 2,59 |
| % Trouvés | 77,8 | 10,5 | 2,5 |

<u>Spectre IR</u> : ($CHCl_3$)

| | |
|---|---|
| OH | 3606 $cm^{-1}$ + associés |
| C=O | 1620 $cm^{-1}$ |
| Aromatiques | 1582 $cm^{-1}$<br>1498 $cm^{-1}$ |

**<u>EXEMPLE 25</u> : 1-[11-(17béta-hydroxy 3-oxo estra-4,9-dièn-11béta-yl) 1-oxo-undécyl] pyrrolidine**

STADE A : 1-[11-(17béta-acétyloxy 3-oxo estra-4,9-dièn-11béta-yl) 1-oxo undécyl] pyrrolidine.

On opère comme au stade A de l'exemple 3, à partir de 2,76 g d'acide obtenu au stade E de la préparation 5, en utilisant 2,5 $cm^3$ de pyrrolidine. Après chromatographie sur silice (éluant : cyclohexane-acétate d'éthyle 4-6)), on obtient 2,18 g de produit recherché.

Spectre IR : ($CHCl_3$)
Peu ou pas d'acide

| | | |
|---|---|---|
| C=O | 1728 $cm^{-1}$ | OAC |
| | 1650 $cm^{-1}$ | Diénone + Amide tertiaire |
| | 1622 $cm^{-1}$ | |

STADE B : 1-[11-(17béta-hydroxy 3-oxo estra-4,9-dièn-11béta-yl) 1-oxo-undécyl] pyrrolidine.

On agite 40 minutes, une solution de 1,265 g du produit obtenu au stade A dans 10 $cm^3$ de potasse éthanolique N. On ajoute 12 $cm^3$ d'acide chlorhydrique N puis 2 $cm^3$ d'ammoniaque concentrée. On extrait à l'acétate d'éthyle, lave à l'eau, sèche et évapore à sec. On chromatographie le résidu (1,164 g) sur silice (éluant : acétate d'éthyle-cyclohexane (8-2)) et recueille 988 mg de produit recherché.

Analyse pour $C_{33}H_{51}O_3N$ = 509,78

| | C % | H % | N % |
|---|---|---|---|
| % Calculés | 77,75 | 10,08 | 2,74 |
| % Trouvés | 77,4 | 10,4 | 2,7 |

Spectre IR : ($CHCl_3$)

| | |
|---|---|
| OH + associé | 3614 $cm^{-1}$ |
| C=O conjuguée | 1643 $cm^{-1}$ |
| Amide tertiaire | 1623 $cm^{-1}$ |

**EXEMPLE 26 : 1-[11-(3,17béta-dihydroxy estra-1,3,5(10)-trièn-11béta-yl) 1-oxo-undécyl] pyrrolidine**

On opère à 0°/+5°C à partir de 773 mg du produit obtenu au stade A de l'exemple 25, que l'on dissout dans 8 $cm^3$ de chlorure de méthylène. On ajoute à 0°/+5°C, 0,4 $cm^3$ de bromure d'acétyle et 0,8 $cm^3$ d'anhydride acétique. On agite 2 heures en laissant revenir à température ambiante. On ajoute de la glace puis neutralise par ajout de bicarbonate de sodium, lave à l'eau, sèche et évapore à sec sous vide. On obtient 829 mg de diacétate intermédiaire.

A partir des 829 mg obtenus ci-dessus, on opère comme au stade B de l'exemple 25 et on chromatographie sur silice (éluant : acétate d'éthyle seul puis acétate d'éthyle à 20 % de méthanol) on obtient 545 mg du produit brut attendu que l'on cristallise dans le mélange chlorure de méthylène-éther isopropylique puis dans l'acétate d'éthyle. On recueille 396 mg de produit recherché.
F = 150°C.
$[alpha]_D$ +70° ± 2,5° (c = 0,5 % $CHCl_3$).

Spectre IR : ($CHCl_3$)

| | |
|---|---|
| OH libre + associé | 3607 $cm^{-1}$ |
| C=O | 1617 $cm^{-1}$ |
| Aromatiques | 1582 $cm^{-1}$ |
| | 1498 $cm^{-1}$ |

**PREPARATION DE L'EXEMPLE 27 : 11béta-[4-(8-hydroxyoctyl) phényl] estra 4,9-dièn 3,17-dione.**

On opère comme au stade A de la préparation 6 à partir de 8,5 g du produit obtenu comme au stade A de la préparation 4. On obtient après chromatographie sur silice (éluant : chlorure de méthylène-acétone 9-1), 5,65 g de produit attendu.

Spectre IR : ($CHCl_3$)

| | |
|---|---|
| -OH | 3623 $cm^{-1}$ |
| 17-céto | 1733 $cm^{-1}$ |
| cétone non conjuguée | 1712 $cm^{-1}$ |
| Aromatiques | 1605 $cm^{-1}$ |
| | 1506 $cm^{-1}$ |

**EXEMPLE 27 : 4-[3-hydroxy 17-oxo estra 1,3,5(10)trièn 11béta-yl N-méthyl N-(1méthyléthyl) benzène] octanamide.**

**STADE A** : 4-(3,17-dioxo estra 4,9-dièn 11béta-yl) N-méthyl N-(1-méthyléthyl) benzène octanamide.

On opère comme à l'exemple 1 à partir de 7,05 g du produit obtenu à la préparation 14 et en utilisant pour l'amidification 7,9 $cm_3$ de N-méthyl isopropylamine, on obtient après chromatographie sur silice (éluant : chlorure de méthylène-acétone 9-1), 5,99 g de produit attendu.

Spectre IR : ($CHCl_3$)

| | |
|---|---|
| amide III | 1621 $cm^{-1}$ |
| 17 céto | 1735 $cm^{-1}$ |
| diénone | 1657 $cm^{-1}$ |
| Aromatique | 1510 $cm^{-1}$ |

**STADE B** : 4-(3-acétyloxy 17-oxo estra 1,3,5(10)-trièn 11béta-yl) N-méthyl N-(1-méthyléthyl) benzène octanamide.

On opère comme au stade B de l'exemple 3 à partir de 4 g de produit obtenu au stade précédent en utilisant 1,9 $cm_3$ de bromure d'acétyle et 3,8 $cm_3$ d'anhydride acétique. On obtient, après chromatographie sur silice (éluant : cyclohexane-acétate d'éthyle 1-1), 4,2 g de produit attendu.

Spectre IR : ($CHCl_3$)

absence de diénone

| | |
|---|---|
| acétate phénolique | 1755 $cm^{-1}$ (ép.) |
| 17-céto | 1731 $cm^{-1}$ |
| amide III | 1621 $cm^{-1}$ |
| Aromatiques | 1513 $cm^{-1}$ |
| | 1493 $cm^{-1}$ |

**STADE C** : 4-(3-hydroxy 17-oxo estra 1,3,5(10)-trièn 11béta-yl) N-méthyl N-(1-méthyléthyl) benzène octanamide.

On opère comme à l'hydrolyse de l'exemple 2 à partir de 2,3 g du produit obtenu au stade B ci-dessus. Après chromatographie sur silice (éluant : cyclohexane-acétate d'éthyle 1-1), on obtient 2 g de produit recherché.

[$alpha_D$] = -16° ± 2° (c = 0,5% éthanol).

| Spectre IR : ($CHCl_3$) | |
|---|---|
| OH | 3600 $cm^{-1}$ |
| 17-céto | 1732 $cm^{-1}$ |
| amide III | 1617 $cm^{-1}$ |
| Aromatiques | 1583 $cm^{-1}$ |
| | 1510 $cm^{-1}$ |
| | 1501 $cm^{-1}$ |

**EXEMPLE 28 : 4-[3,17béta-dihydroxy estra 1,3,5(10)-trièn 11béta-yl] N-méthyl N-(1-méthyléthyl) benzène octanamide.**

A une solution de 480 mg de produit obtenu à l'exemple 27 et 7,5 cm3 de tétrahydrofuranne anhydre, on ajoute à 0°C+5°C 563 mg d'hydrure de triterbutoxy aluminium-lithium, on agite pendant 50 minutes et ajoute 100 mg d'hydrure. On verse dans un mélange 1-1 de glace et d'une solution saturée de chlorure d'ammonium et extrait à l'acétate d'éthyle, lave la phase organique avec une solution saturée de chlorure de sodium, sèche, filtre et évapore à sec. Après chromatographie sur silice (éluant : cyclohexane-acétate d'éthyle 1-1), on obtient 472 mg de produit recherché.
[$alpha_D$] = -34,5° ± 2,5° (c = 0,5% éthanol).

| Spectre IR : ($CHCl_3$) | |
|---|---|
| OH | 3605 $cm^{-1}$ |
| amide III | 1617 $cm^{-1}$ |
| Aromatiques | 1583 $cm^{-1}$ |
| | 1499 $cm^{-1}$ |

**EXEMPLE 29 : 4-[3,17béta-dihydroxy 19-nor 17alpha-pregna 1,3,5(10)-trièn 20-yn 11béta-yl] N-méthyl N-(1-méthyléthyl) benzène octanamide.**

A une solution de 400 mg du produit obtenu à l'exemple 27 dans 20 $cm_3$ de tétrahydrofuranne, on ajoute en 3 heures 30 minutes 825 mg d'acétylure de lithium éthylène diamine. On agite pendant 4 heures et verse dans un mélange 1-1 de glace et d'une solution saturée de chlorure d'ammonium. On extrait avec de l'acétate d'éthyle, lave à l'eau saturée de chlorure de sodium, sèche, filtre et évapore à sec. On chromatographie le résidu sur silice par deux fois en utilisant à chaque fois (éluant : chlorure de méthylène-acétone 93-7), on obtient 115 mg du produit recherché.
[$alpha_D$] = -105° ± 2° (c = 0,9% éthanol).

| Spectre IR : ($CHCl_3$) | |
|---|---|
| OH | 3599 $cm^{-1}$ |
| C=CH | 3304 $cm^{-1}$ |
| amide III | 1617 $cm^{-1}$ |
| Aromatiques | 1583 $cm^{-1}$ |
| | 1500 $cm^{-1}$ |

**EXEMPLE 30 : 4-[3,17béta-dihydroxy 17alpha-méthyl estra 1,3,5(10)-trièn 11béta-yl] N-méthyl N-(1-méthyléthyl) benzène octanamide.**

A une solution de 700 mg du produit obtenu à l'exemple 27 dans 35 $cm_3$ de tétrahydrofuranne, on ajoute à 20/25°C 12,8 $cm^3$ d'une solution 0,76M de bromure de méthyl magnésium dans le tétrahydrofuranne. Après 1 heure de réaction, on verse sur un mélange 1-1 de glace et d'une solution saturée de chlorure d'ammonium. On extrait à l'acétate d'éthyle, lave avec une solution saturée de chlorure de sodium, sèche, filtre et évapore à sec. On chromatographie le résidu sur silice (éluant : chlorure de méthylène-acétone 9-1) et obtient 411 mg du produit recherché.

Analyse : $C_{37}H_{53}NO_3$ = 559,84

| | | | |
|---|---|---|---|
| Calculé : | C% 79,38 | H% 9,54 | N% 2,50 |
| Trouvé : | 79,4 | 9,5 | 2,4 |

**PREPARATION A DE L'EXEMPLE 31 : 4-triméthylsilyléthynyl bromo benzène.**

On introduit 150 g de bromo iodo benzène à 97%, 500 $cm_3$ de diméthylformamide anhydre, 100 $cm_3$ de triéthylamine, 50 g de triméthylsilyl acétylène, 2,1 g d'iodure de cuivre et 2,22 g de bis (triphénylphosphine) palladium (II) dichlorure. On agite pendant 2 heures puis ajoute 500 $cm_3$ d'eau glacée et extrait 3 fois par 500 $cm_3$ d'acétate d'éthyle. La phase organique est lavée à l'eau salée puis séchée sur sulfate de soude. Les solvants sont évaporés sous pression réduite. On obtient 136,542 g d'huile brune. On purifie par distillation sous pression réduite et obtient 106,979 g de produit attendu.
Eb : 75-82°C sous 0,2 mbar. F = 62°C.
Spectre IR : ($CHCl_3$)
absence de C=H
C=C 2160 $cm^{-1}$

**PREPARATION B DE L'EXEMPLE 31 :**

**STADE A:** (5alpha, 11béta) 3-(1,2-éthanediyl acétal cyclique) 5-hydroxy 11-[[4-(1,1-diméthyléthyl) diméthylsilyl] éthynylphényl] estr-9-èn-3,17-dione.

On opère comme au stade A de la préparation 1 à partir de 30 g de 3-(1,2-éthanediyl acétal cyclique de 5alpha, 10alpha-époxy estr-9,11-èn-3,7-dione obtenu selon EP 0 057 115 (exemple 7) en utilisant pour la préparation du magnésien 81,254 g de dérivé bromé, obtenu à la préparation A et 7,96 g de magnésium, puis pour la condensation, 1,4 g de chlorure de cuivre. On chromatographie sur silice le produit brut obtenu auquel on joint le produit provenant d'une opération réalisée de façon identique à partir de 16,52 g d'époxyde (éluant : chlorure de méthylène-acétone 98-2). On obtient 50,8 g de produit A pur et 6 g de produit B légèrement moins pur que l'on utilise tel quel pour le stade suivant.

Spectre IR : ($CHCl_3$)

| | |
|---|---|
| OH | 3508 $cm^{-1}$ |
| C=C | 2156 $cm^{-1}$ |
| Aromatiques | 1602 $cm^{-1}$<br>1555 $cm^{-1}$<br>1502 $cm^{-1}$ |

**STADE B :** 11béta-(4-éthynylphényl) estra-4,9-dièn-3,17-dione.

On agite pendant 30 minutes une suspension de 46,8 g du produit obtenu au stade A, 200 $cm_3$ d'éthanol et 8,1 $cm_3$ de lessive de soude. On ajoute ensuite 16,7 $cm_3$ d'acide chlorhydrique concentré, on agite à température ambiante puis concentre au demi, extrait avec du chlorure de méthylène, sèche et évapore sous pression réduite. On chromatographie le résidu (38,23 g) sur silice (éluant : cyclohexane-acétate d'éthyle 1-1) et obtient 31,06 g de produit recherché. F = 184°C.

Spectre IR : ($CHCl_3$)

| | | |
|---|---|---|
| C≡CH | 3302 $cm^{-1}$ | |
| C=O | 1736 $cm^{-1}$ | (17-céto) |
| | 1659 $cm^{-1}$ | |
| | 1640 $cm^{-1}$ | diénone |

aromatique 1556, 1506 $cm^{-1}$

**STADE C** : 3béta-hydroxy estra 1,3,5(10)-trièn-11béta-yl (4-éthynylphényl) 17-one.

Acétylation en 3.

On opère comme au stade B de l'exemple 3 à partir de 31 g du composé obtenu ci-dessus en utilisant 47,1 $cm_3$ d'anhydride acétique et 23,8 $cm_3$ de bromure d'acétyle. On obtient 31,2 g d'acétate en 3 que l'on saponifie en opérant comme au stade B de l'exemple 1. Après chromatographie sur silice (éluant : cyclohexane-acétate d'éthyle 7-3), on obtient 27,03 g de produit brut que l'on empâte dans l'éther pour recueillir 22,852 g du produit recherché. F = 163°C.

Spectre IR : ($CHCl_3$)

| | |
|---|---|
| OH | 3597 $cm^{-1}$ |
| C≡CH | 3303 $cm^{-1}$ |
| C=O | 1733 $cm^{-1}$ |
| aromatique | 1606, 1582, 1556, 1503 $cm^{-1}$ |

**STADE D** : 3, 17béta-tétrahydropyrannyloxy (4-éthynylphényl) estra-1,3,5(10)-trièn-11béta-yl.

a) Réduction de la cétone en 17.

On opère comme au stade B de l'exemple 3 à partir de 14 g du composé obtenu ci-dessus en utilisant 10 g d'hydrure de bore et de sodium.

b) Dihydropyranylation.

On opère comme au stade A de l'exemple 39 à partir de 17,3 g de l'intermédiaire 17-hydroxy obtenu ci-dessus en utilisant 24,4 $cm_3$ de dihydropyranne et 0,3 g d'acide paratoluènesulfonique. Après chromatographie sur silice, on obtient 13,6 g de produit brut que l'on reprend dans l'éther isopropylique pour recueillir 10,23 g de produit recherché.

F = 213-215°C.

Spectre IR : ($CHCl_3$)

| | |
|---|---|
| C≡CH | 3302 $cm^{-1}$ |
| aromatique | 1607, 1570, 1556, 1498 $cm^{-1}$ |

**PREPARATION C DE L'EXEMPLE 31 : 1-oxo 6-bromo hexyl morpholine**

On opère comme au stade A de l'exemple 3 à partir de 5 g de l'acide 6-bromo benzylique en utilisant 3,4 $cm_3$ de N-méthyl morpholine, 3,7 $cm_3$ de chloroformiate d'isobutyle et 3,35 $cm_3$ de morpholine. Après chromatographie sur silice (éluant : chlorure de méthylène-acétone 9-1), on obtient 7 g de produit utilisé tel quel pour le stade suivant.

Spectre IR : ($CHCl_3$)

C=O 1635 $cm^{-1}$

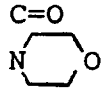 1115 $cm^{-1}$

**EXEMPLE 31 : 4-(8-(4-(3,17béta-dihydroxy estra 1,3,5(10)-trièn 11béta-yl) phényl) 1-oxo 7-octynyl) morpholine.**

A une solution refroidie à -30°C de 800 mg du composé obtenu à la préparation B de l'exemple 31 dans 6,5 $cm_3$ de tétrahydrofuranne et 6,5 $cm_3$ d'hexaméthylphosphotriamide, on ajoute goutte à goutte 1,7 $cm_3$ d'une solution 1,1M de butyllithium dans l'hexane. On agite 5 minutes à -30°C et ajoute à -25/-30°C 508 mg de la 1-oxo 6-bromo hexyl morpholine obtenue à la préparation C ci-dessus en solution dans 1 $cm_3$ de tétrahydrofuranne. On agite pendant 1 heure et verse sur 30 $cm_3$ d'une solution de chlorure de sodium. On extrait avec de l'acétate d'éthyle, lave, sèche et évapore à sec sous pression réduite, on recueille 3 g de produit brut que l'on chromatographie sur silice (éluant : cyclohexane-acétate d'éthyle 1-1), obtient 760 mg de produit que l'on dépyranyle 1 heure à température ambiante avec le mélange 8 $cm_3$ d'acide chlorhydrique 2N et 40 $cm_3$ de méthanol. On verse dans 50 g d'eau et glace (1-1), extrait au chlorure de méthylène, évapore à sec sous pression réduite. On chromatographie le résidu (665 mg) sur silice (éluant : chlorure de méthylène-acétone 8-2), on obtient 502 mg isolés de l'éther.
[alpha$_D$] = -36,5° ± 2,5° (c = 1% éthanol).
Spectre IR : ($CHCl_3$)

| | |
|---|---|
| OH | 3603 $cm^{-1}$ |
| C=O | 1631 $cm^{-1}$ |
| aromatique | 1584, 1550, 1505 $cm^{-1}$ |
| morpholine | 1115 $cm^{-1}$ |

| | | | | |
|---|---|---|---|---|
| Analyse | : | $C_{36}H_{45}NO_4$ = 555,76 | | |
| Calculé | : | C% 77,80 | H% 8,16 | N% 2,52 |
| Trouvé | : | 77,8 | 8,3 | 2,5 |

**EXEMPLE 32 : 4-(8-(4-(3,17béta-dihydroxy estra 1,3,5(10)-trièn 11béta-yl) phényl) 1-oxo octyl) morpholine.**

A une solution de 290 mg du produit obtenu à l'exemple 31 dans 15 $cm_3$ d'éthanol, on ajoute 145 mg de palladium sur charbon actif à 10% et on hydrogène sous 1300 mbar. On filtre et évapore le solvant à sec, chromatographie le résidu (280 mg) sur silice (éluant : chlorure de méthylène-acétone 85-15), reprend à l'éther et obtient 256 mg du produit recherché.
[alpha$_D$] = -30° ± 2,5° (c = 0,5% éthanol).
Spectre IR : ($CHCl_3$)

| | |
|---|---|
| OH | 3604 $cm^{-1}$ |
| C=O | 1629 $cm^{-1}$ |
| aromatique | 1583, 1500 $cm^{-1}$ |
| morpholine | 1115 $cm^{-1}$ |

| | | | | |
|---|---|---|---|---|
| Analyse | : | $C_{36}H_{49}NO_4$ = 559,8 | | |
| Calculé | : | C% 77,24 | H% 8,82 | N% 2,56 |
| Trouvé | : | 77,5 | 9,1 | 2,5 |

**PREPAPATION DE L'EXEMPLE 33 : N-dibutyl 6-bromohexanamide.**

On opère comme au stade A de l'exemple 3 à partir de 4,41 g d'acide 5-bromohexanoïque en utilisant 11,1 g de dibutylamine. On obtient le produit recherché par distillation sous pression réduite et recueille 6,141 g de produit attendu.
Eb : 139°C sous 0,5 mbar.
Spectre IR : ($CHCl_3$)
C=O 1623 $cm^{-1}$ (amide III)

**EXEMPLE 33 : 8-(4-(3,17béta-dihydroxy estra 1,3,5(10)-trièn 11béta-yl) phényl) N,N-dibutyl 7-octynamide.**

On opère comme à l'exemple 31 à partir de 0,7 g du composé obtenu à la préparation 33. En utilisant 0,463 mg de bromo dibutyl 7-octynamide (préparation de l'exemple 33). On obtient après chromatographie sur silice (éluant : cyclohexane-acétate d'éthyle 1-1) 657 mg de produit attendu.
[alpha$_D$] = -26° ± 2° (c = 0,5% éthanol).
Spectre IR : ($CHCl_3$)

| | |
|---|---|
| OH | 3603 $cm^{-1}$ |
| C=O | 1621 $cm^{-1}$ |
| aromatique | 1582 $cm^{-1}$ |

<u>Analyse</u> : $C_{40}H_{55}NO_3$ = 597,89
Calculé : C% 79,82 H% 9,88 N% 2,33
Trouvé : 79,7 10,1 2,4

**<u>EXEMPLE 34</u> : 8-(4-(3,17béta-dihydroxy estra 1,3,5(10)-trièn 11béta-yl) phényl) N,N-dibutyl 7-octanamide.**

On opère comme à l'exemple 32 à partir de 381 mg du produit obtenu à l'exemple 33 en utilisant 0,1 g de palladium sur charbon actif. Après chromatographie sur silice (éluant : cyclohexane-acétate d'éthyle 1-1), on obtient 241 mg de produit recherché.
[alpha$_D$] = -26° ± 2° (c = 0,5% éthanol).
<u>Spectre IR</u> : ($CHCl_3$)
OH 3605 $cm^{-1}$
C=O 1619 $cm^{-1}$
aromatique 1583, 1500 $cm^{-1}$

<u>Analyse</u> : $C_{40}H_{59}NO_3$ = 602,92
Calculé : C% 79,82 H% 9,88 N% 2,33
Trouvé : 79,7 10,1 2,4

**<u>PREPARATION DE L'EXEMPLE 35</u> : N-méthyl N-butyl 1-iodo hexanamide.**

A une solution de 5,288 g de N-méthyl N-butyl 1-bromo hexanamide (préparation de l'exemple 33) dans 105 $cm_3$ d'acétone, on ajoute 4,497 g d'iodure de sodium, on agite 18 heures, filtre, dilue à l'eau, extrait à l'acétate d'éthyle, lave, sèche et évapore à sec sous vide. On obtient 6,139 g de produit attendu.

**<u>EXEMPLE 35</u> : 8-(4-(3,17béta-dihydroxy estra 1,3,5(10)-trièn 11béta-yl) phényl) N-butyl N-méthyl 7-octynamide.**

On opère comme à l'exemple 33 à partir de 692 mg du produit obtenu à la préparation 33 en utilisant 516 mg de N-méthyl N-butyl 1-iodo hexanamide (préparation de l'exemple 35). Après chromatographie, on obtient 981 mg de produit brut que l'on chromatographie sur silice (éluant : cyclohexane-acétate d'éthyle 2-8) et obtient 586 mg du produit attendu.
[alpha$_D$] = -35,5° ± 2,5° (c = 0,5% éthanol).
<u>Spectre IR</u> : ($CHCl_3$)
OH 3605 $cm^{-1}$
C=O 1621 $cm^{-1}$
C=C + aromatique 1583,1500 $cm^{-1}$

<u>Analyse</u> : $C_{37}H_{49}NO_3$ = 555,81

Calculé : C% 79,95 H% 8,88 N% 2,51
Trouvé : 79,8 9,0 2,5

**<u>EXEMPLE 36</u> : (Z) 8-(4-(3,17béta-dihydroxy estra 1,3,5(10)-trièn 11béta-yl) phényl) N-butyl N-méthyl 7-octènamide.**

A une solution de 555 mg du produit obtenu à l'exemple 35 dans 11 $cm_3$ d'acétate d'éthyle, on ajoute 50 mg de palladium sur sulfate de baryum et 0,22 $cm_3$ de pyridine. On hydrogène sous une pression de 1500 mbars. On filtre et évapore à sec sous pression réduite. On obtient 620 mg de résidu que l'on chromatographie

sur silice (éluant : cyclohexane-acétate d'éthyle 1-1), on recueille 360 mg du produit recherché que l'on chromatographie une seconde fois dans ces conditions. On obtient 344 mg de composé pur.
[$alpha_D$] = -32° ± 2,5° (c = 0,5% éthanol).
Spectre IR : ($CHCl_3$)

| | |
|---|---|
| -OH | 3605 $cm^{-1}$ |
| C=O | 1621 $cm^{-1}$ |
| C=C + aromatique | 1583, 1500 $cm^{-1}$ |

| | | | | |
|---|---|---|---|---|
| Analyse : | $C_{37}H_{51}NO_3$ = 557,82 | | | |
| Calculé : | C% 79,66 | H% 9,21 | N% 2,5 |
| Trouvé : | 79,5 | 9,4 | 2,4 |

**EXEMPLE 37 : 2-((7-(4-(3,17béta-dihydroxy estra 1,3,5(10)-trièn 11béta-yl) phényl) 6-heptynyl) oxy) N-butyl N-méthyl acétamide.**

On opère comme à l'exemple 33 à partir de 0,7 g du composé obtenu à la préparation B de l'exemple 31 en utilisant 0,493 g de [(5-bromopentyl) oxy] N-butyl N-méthyl acétamide (préparation 21). Après chromatographie sur silice (éluant : acétate d'éthyle-cyclohexane 8-2), on obtient 460 mg du produit recherché.
Spectre IR : ($CHCl_3$)

| | |
|---|---|
| OH | 3603 $cm^{-1}$ + associé |
| C=O | 1634 $cm^{-1}$ |
| Aromatique | 1584, 1554, 1505 $cm^{-1}$ |

| | | | |
|---|---|---|---|
| Analyse : | $C_{38}H_{51}NO_4$ = 585,829 | | |
| Calculé : | C% 77,91 | H% 8,78 | N% 2,39 |
| Trouvé : | 78,2 | 8,9 | 2,5 |

**EXEMPLE 38 : 2-((7-(4-(3,17béta-dihydroxy estra 1,3,5(10)trièn 11béta-yl) phényl) heptyl) oxy) N-butyl N-méthyl acétamide.**

On opère comme à l'exemple 34 à partir de 300 mg du produit obtenu à l'exemple 37 en utilisant 0,1 g de palladium sur charbon actif. Après chromatographie sur silice (éluant : cyclohexane-acétate d'éthyle 1-1), on obtient 201 mg du produit recherché.
[$alpha_D$] = -19° ± 1° (c = 1% éthanol).
Spectre IR : ($CHCl_3$)

| | |
|---|---|
| -OH | 3604 $cm^{-1}$ |
| C=O | 1635 $cm^{-1}$ |
| aromatique | 1583, 1500 $cm^{-1}$ |

| | | | |
|---|---|---|---|
| Analyse : | $C_{38}H_{55}NO_4$ | | |
| Calculé : | C% 77,38 | H% 9,40 | N% 2,37 |
| Trouvé : | 77,2 | 9,7 | 2,4 |

**EXEMPLE 39 : N-butyl 4-[3,17béta-dihydroxy estra 1,3,5 (10)-trièn 11béta-yl] alpha alpha N-triméthyl benzène octanamide.**

**STADE A** : N-butyl N-méthyl 4-[3,17béta-bis [(tétrahydro 2H-pyran-2-yl) oxy] estra 1,3,5(10)-trièn 11béta-yl] benzène octanamide.

On agite pendant 2 heures 30 minutes 500 mg du produit obtenu à l'exemple 8, 20 $cm_3$ d'éther, 15 $cm_3$ de dihydropyranne et 15 mg d'acide paratoluènesulfonique. On ajoute alors 1 $cm_3$ de triéthylamine, verse dans un mélange 1-1 de glace et solution saturée de bicarbonate de sodium, extrait à l'éther, filtre et évapore à sec.

On chromatographie le résidu sur silice (éluant : cyclohexane-acétate d'éthyle 1-1) et obtient 640 mg de produit recherché.

Spectre IR : ($CHCl_3$)
Absence d'OH, présence de l'éther tétrahydropyrannylique
amide III 1627 $cm^{-1}$
Aromatiques 1574 $cm^{-1}$, 1510 $cm^{-1}$, 1497 $cm^{-1}$

**STADE B** : N-butyl alpha-N-diméthyl 4-[3,17béta-bis [(tétrahydro 2H-pyran-2-yl) oxy] estra 1,3,5(10)-trièn 11béta-yl] benzène octanamide.

A une solution de 0,7 $cm_3$ de diisopropylamine dans 5 $cm_3$ de tétrahydrofuranne anhydre, on ajoute entre 5 et 8°C 2,8 $cm_3$ d'une solution 1,6M de butyllithium dans l'hexane. On agite 10 minutes à +5°C puis refroidit à -70°C et ajoute une solution de 630 mg du produit obtenu au stade A dans 5 $cm_3$ de tétrahydrofuranne anhydre. On agite 30 minutes à -70°C et ajoute 0,5 $cm_3$ d'iodure de méthyle, agite 45 minutes, ajoute 20 $cm_3$ d'une solution saturée de chlorure d'ammonium et ramène à température ambiante. On extrait avec de l'acétate d'éthyle, lave, sèche et évapore à sec. On chromatographie le résidu obtenu sur silice (éluant : cyclohexane-acétate d'éthyle 8-2). On obtient 567 mg de produit recherché utilisé tel quel pour le stade suivant.
Spectre RMN 300 MHz :
méthyl en alpha de l'amine : 1,07 (d) et 1,08 (d).

**STADE C** : N-butyl 4-[3,17béta-dihydroxy estra 1,3,5(10)-trièn 11béta-yl] alpha alpha N-triméthyl benzène octanamide.

a) Diméthylation :

A une solution de 516 mg de produit obtenu au stade B dans 11,5 $cm_3$ de tétrahydrofuranne, on ajoute à 48°C ± 2°C° 9 $cm_3$ d'une solution de lithium diisopropylamide, préparée à +5°/+8°C par ajout de 6,2 $cm_3$ d'une solution 1,6M de butyllithium dans l'hexane à une solution de 1,4 $cm_3$ de diisopropylamine dans 10 $cm_3$ de tétrahydrofuranne. L'addition de la solution de lithium diisopropylamide terminée, on agite encore 15 minutes et ajoute en une fois 1,2 $cm_3$ d'iodure de méthyle. On agite 45 minutes, verse 50 g d'un mélange 1-1 de glace et chlorure d'ammonium en solution saturée, extrait avec de l'acétate d'éthyle, lave, sèche et évapore à sec. On obtient 560 mg de N-butyl alpha, alpha N-triméthyl 4-[3,17béta bis (tétrahydro 2H pyran-2-yl) oxy] estra 1,3,5(10)-trièn 11béta-yl] benzène octanamide intermédiaire que l'on utiise tel quel pour la dépyranylation.

b) Dépyranylation :

L'extrait sec obtenu ci-dessus est dissout dans 15 $cm_3$ de méthanol. On ajoute 2 $cm_3$ d'acide chlorhydrique 2N et agite à température ambiante pendant 1 heure. On verse sur une solution saturée de chlorure de sodium et extrait avec du chlorure de méthylène, sèche, évapore à sec et recueille 465 mg de résidu que l'on chromatographie sur silice (éluant : chlorure de méthylène-acétone 95-5) et obtient, après empâtage dans l'éther 207 mg de produit recherché.
[$alpha_D$] = -30° ± 2,5° (c = 0,5% éthanol).

| Analyse : | $C_{39}H_{57}NO_3$ = 587,85 | | |
|---|---|---|---|
| Calculé : | C% 79,68 | H% 9,77 | N% 2,38 |
| Trouvé : | 79,8 | 9,9 | 2,5 |

**PREPAPATION DE L'EXEMPLE 40 : 4-(3,7-dioxo estr-4,9-dièn 11béta-yl) benzène octanol.**

On opère comme à la préparation 6A à partir de 3,6 g de produit obtenu à la préparation 4A. On obtient après chromatographie sur silice (éluant : chlorure de méthylène-acétone 95-5) 2,234 g de produit attendu.
Spectre IR : ($CHCl_3$)

OH 3623 $cm^{-1}$
C=O 1735 $cm^{-1}$
diénone 1658, 1602 $cm^{-1}$
Aromatique 1570 (ép.), 1510 $cm^{-1}$

**EXEMPLE 40 : 4-[3,17béta-dihydroxy estra 1,3,5(10)-trièn 11béta-yl] N-méthyl N-(1-méthyléthyl) benzène nonamide.**

**STADE A** : 4(-3,17-dioxo estr-4,9-dièn-11béta-yl) benzène octane méthane sulfonate.

A une solution de 2,234 g du produit obtenu à la préparation de l'exemple 40 dans 20 $cm_3$ de pyridine anhydre, on ajoute 3,36 g de chlorure de paratoluènesulfonyle. On agite 1 heure 30 minutes, ajoute 30 $cm_3$ d'une solution saturée de bicarbonate de sodium, agite 30 minutes et extrait avec du chlorure de méthylène, les solvants sont séchés puis évaporés sous pression réduite. On chromatographie le résidu obtenu (2,9 g) sur silice (éluant : chlorure de méthylène-acétone 9-1) et obtient 2,255 g de produit recherché.
Spectre IR : ($CHCl_3$)
C=O (17-céto) 1735 $cm^{-1}$
diénone 1658, 1600 $cm^{-1}$
aromatique 1510, 1496 $cm^{-1}$
$SO_2$ 1359, 1496, 1176 $cm^{-1}$

**STADE B** : 4-(3,17-dioxo estr 4,9-dièn 11béta-yl) benzène nonane iodure.

On agite 1 heure au reflux 2,2 g du produit obtenu au stade A dans 50 $cm_3$ d'acétone avec 0,787 g d'iodure de sodium. On filtre l'insoluble et évapore à sec sous pression réduite. On reprend le résidu (3,6 g) dans 10 $cm_3$ d'éther, filtre l'insoluble et évapore le filtrat à sec sous pression réduite, on obtient 1,722 g de produit recherché.
Spectre IR : ($CHCl_3$)
C=O 1735 $cm^{-1}$
diénone 1658, 1602 $cm^{-1}$
aromatique 1510 $cm^{-1}$

**STADE C** : 4-(3,17-dioxo estr-4,9-dièn 11béta-yl) benzène décane nitrile.

On agite 2 heures au reflux 3,6 g de produit obtenu au stade B dans 55 $cm_3$ d'éthanol et 10 $cm_3$ d'eau avec 0,872 g de cyanure de potassium. On ajoute 50 $cm_3$ de glace, extrait avec du chlorure de méthylène, sèche et évapore sous pression réduite et obtient 1,68 g de produit recherché.
Spectre IR : ($CHCl_3$)
C≡N 2245 $cm^{-1}$
C=O 1735 $cm^{-1}$
diénone 1658, 1602 $cm^{-1}$
Aromatique 1500 $cm^{-1}$

**STADE D** : 4-(3-acétoxy 17-oxo estra 1,3,5(10)-trièn 11béta-yl) benzène décane nitrile.

On opère comme au stade B de l'exemple 3 à partir de 1,27 g du produit obtenu au stade C ci-dessus. On obtient après chromatographie sur silice (éluant : cyclohexane-acétate d'éthyle 1-1) 1,281 g de produit recherché.

Spectre IR : ($CHCl_3$)
C≡N 2245 $cm^{-1}$
C=O 1750 $cm^{-1}$ (OAC)
1735 $cm^{-1}$ (17-céto)
aromatique 1606, 1582, 1512, 1413 $cm^{-1}$

**STADE E** : 4-(3-isobutyl carbonyloxy 17-oxo estra 1,3,5(10)-trièn 11béta-yl) N-méthyl N-(1-méthyléthyl) benzène nonamide.

a) Hydrolyse du nitrile :

On agite au reflux pendant 60 heures 1,225 g du produit obtenu au stade D, 24 $cm_3$ d'éthanol et 1,5 $cm_3$ de lessive de soude. On refroidit et verse dans un mélange de 73 $cm_3$ d'acide chlorhydrique N et 100 $cm_3$ de glace, on agite 10 minutes, extrait avec du chlorure de méthylène, sèche, filtre et concentre à sec sous pression réduite. On obtient 1,145 g de produit.

b) Amidification :

A une solution de 1,145 g du produit obtenu cu-dessus dans 50 $cm_3$ de chlorure de méthylène, on ajoute 0,3 $cm_3$ de N-méthyl morpholine, 1 $cm_3$ de chloroformiate d'isobutyle. On agite 10 minutes puis ajoute 1 $cm_3$ d'isopropylméthylamine. On agite 30 minutes à température ambiante, verse dans 50 $cm_3$ d'une solution saturée de bicarbonate de sodium, agite 10 minutes, extrait avec du chlorure de méthylène, sèche et évapore à sec sous pression réduite. On chromatographie le résidu (2 g) sur silice (éluant : cyclohexane-acétate d'éthyle 1-1) et obtient 0,778 g de produit recherché.

Spectre IR : ($CHCl_3$)

| | |
|---|---|
| C=O | 1755 $cm^{-1}$ (ép.) |
| | 1737 $cm^{-1}$ (max) |
| | 1621 $cm^{-1}$ (amide III) |
| aromatique | 1513, 1493 $cm^{-1}$ |

**STADE F : 4-[3,17béta-dihydroxy estra 1,3,5(10)-trièn 11béta-yl] N-méthyl N-(1-méthyléthyl) benzène nonamide.**

a) Réduction du 17-céto :

Dans une solution contenant 0,778 g du produit obtenu au stade E ci-dessus dans 15 $cm_3$ de tétrahydrofuranne, on introduit par fractions 0,451 g d'hydrure de triterbutoxy aluminium-lithium. On agite 30 minutes puis verse dans une solution glacée de phosphate monosodique, agite 30 minutes et extrait avec du chlorure de méthylène. On évapore à sec sous pression réduite et obtient 0,9 g de produit 17-hydroxy.

b) Saponification :

On opère comme à l'exemple 2B à partir du produit obtenu ci-dessus et obtient après chromatographie sur silice (éluant : acétate d'éthyle-cyclohexane 6-4) 613 mg de produit attendu.

[$alpha_D$] = -17° ± 1° (c = 1% éthanol).

Spectre IR : ($CHCl_3$)

| | |
|---|---|
| OH | 3604 $cm^{-1}$ |
| C=O | 1618 $cm^{-1}$ |
| Aromatiques | 1583 $cm^{-1}$ |
| | 1500 $cm^{-1}$ |

Analyse : $C_{37}H_{53}NO_3$ = 559,84

| | | | |
|---|---|---|---|
| Calculé : | C% 79,38 | H% 9,54 | N% 2,50 |
| Trouvé : | 79,3 | 9,7 | 2,5 |

**PREPARATION DE L'EXEMPLE 41 : 3-(1,2-éthanediyl) acétal cyclique de 5alpha-hydroxy 11béta-[4-((6-hydroxy hexyl) oxy) phényl] estr-9-èn-3,17-dione.**

**STADE A** : 3-(1,2-éthanediyl) acétal cyclique de 11béta-(4-hydroxy phenyl) 5-alpha-hydroxy estr-9-èn-3,17-dione.

a) Préparation du magnésien.

On opère comme au stade A de l'exemple 1 en utilisant 7,1 g de magnésium en tournures et 50 g de 4-triméthylsilyloxy bromo benzène. On obtient une solution ≃ 0,95M de magnésien dans le tétrahydrofuranne.

b) Condensation.

On opère comme au stade A de l'exemple 1 à partir de 10 g de 3-(1,2-éthanediyl) acétal cyclique de 5alpha, 10alpha-époxy estr-9,11-èn-3,17-dione obtenu selon EP 0 057 115 (exemple 7) en utilisant 110 $cm_3$ de la solution du magnésien ci-dessus. On obtient 34,9 g de produit brut.

c) Désilylation.

Le produit brut obtenu ci-dessus est dissous dans 150 $cm_3$ de tétrahydrofuranne, on ajoute 130 $cm_3$ d'une solution 1M de fluorure de tétrabutylammonium. On agite 15 minutes à température ambiante, verse dans l'eau, extrait avec de l'acétate d'éthyle, lave à l'eau, sèche et évapore à sec sous pression réduite. On obtient 26,9 g de produit brut que l'on empâte à 40° pendant 30 minutes dans 100 $cm_3$ d'un mélange acétate d'éthyle-chlorure de méthylène (1-1). On filtre l'insoluble et obtient 5,77 g de produit recherché. Par chromatographie sur silice des liqueurs mères (éluant : acétate d'éthyle-chlorure de méthylène 1-1), on recueille 5,7 g supplémentaires de produit recherché. On réunit les 2 lots de produits obtenus (11,47 g) que l'on recristallise dans l'éthanol. On obtient 8 g du produit attendu. F = 255°C.

Spectre IR : ($CHCl_3$)

| | |
|---|---|
| Région OH | 3464, 3280 $cm^{-1}$ |
| C=O | 1720 $cm^{-1}$ |
| aromatique | 1613, 1592, 1511 $cm^{-1}$ |

**STADE B** : 3-(1,2-éthanediyl) acétal cyclique de 5alpha-hydroxy 11béta-[4-((6-hydroxy hexyl) oxy) phényl] estr-9-èn-3,17-dione.

A une solution de 3,77 g du produit obtenu au stade A dans 18 $cm_3$ d'acétone, on ajoute 13,3 $cm_3$ de soude 2N puis 3 $cm_3$ de bromohexanol. On chauffe à 50°C 3 heures, on coule dans une solution saturée de chlorure d'ammonium, extrait avec du chlorure de méthylène et évapore à sec sous pression réduite. On chromatographie le résidu (7,7 g) sur silice (éluant : chlorure de méthylène-acétate d'éthyle 6-4 puis 1-1) et obtient 4,11 g de composé recherché.

Spectre IR : ($CHCl_3$)

| | |
|---|---|
| OH- (de la chaîne) | 3620 $cm^{-1}$ |
| OH en 5alpha | 3509 $cm^{-1}$ |
| aromatique | 1609, 1578, 1509 $cm^{-1}$ |

**EXEMPLE 41 : 7-(4-(3,17béta-dihydroxy estra-1,3,5(10)-trièn 11béta-yl) phénoxy) N-méthyl N-(1-méthyléthyl) heptanamide.**

**STADE A** : 3-(1,2-éthanediyl) acétal cyclique de 5alpha-hydroxy 11béta-[4-[(6-[(4-méthylphényl) sulfonyloxy] hexyl] oxy] phényl] estr-9-èn-3,17-dione.

On opère comme au stade A de l'exemple 40 à partir de 4,08 g du composé obtenu à la préparation de l'exemple 41 en utilisant 2,85 g de chlorure de paratoluènesulfonyle. Après chromatographie sur silice (éluant : chlorure de méthylène-acétate d'éthyle 2-1), on obtient 4,19 g de produit recherché que l'on utilise tel quel pour le stade suivant.

**STADE B** : 3-(1,2-éthanediyl) acétal cyclique de 5alpha-hydroxy 11béta-[4-[(6-iodohexyl) oxy] phényl] estr-9-èn-3,17-dione.

On opère comme au stade B de l'exemple 40 à partir de 4,19 g du produit obtenu au stade A ci-dessus en utilisant 1,39 g d'iodure de sodium. ON obtient 3,9 g de produit recherché.

Spectre IR : ($CHCl_3$)

OH en 5 3508 $cm^{-1}$
17-céto 1733 $cm^{-1}$
aromatique 1609, 1575, 1508 $cm^{-1}$

**STADE C** : [4-[3,3-(1,2-éthanediyl) bis oxy 5alpha-hydroxy 17-oxo estr-9-èn-11béta-yl] phénoxy] heptan nitrile.

On opère comme au stade C de l'exemple 40 à partir de 3,75 g du composé obtenu au stade B en utilisant 780 mg de cyanure de potassium. Après chromatographie sur silice (éluant : cyclohexane-acétate d'éthyle 1-1), on obtient 2,9 g de produit recherché.
Spectre IR : ($CHCl_3$)
OH en 5alpha 3510 $cm^{-1}$
C≡N 2248 $cm^{-1}$
17-céto 1733 $cm^{-1}$
aromatique 1609, 1576, 1508 $cm^{-1}$

**STADE D** : [4-(3,20-dioxo estra-4,9-dièn-11béta-yl) phénoxy] heptan nitrile.

On agite 2 heures à température ambiante un mélange de 2,46 g du produit obtenu austade C, 13 $cm_3$ de méthanol et 4 $cm_3$ d'acide chlorhydrique 2N. Après dilution à l'eau, extraction avec du chlorure de méthylène et distillation sous pression réduite, on recueille 2,2 g de produit brut que l'on chromatographie sur silice (éluant : cyclohexane-acétate d'éthyle 1-1). On obtient 1,875 g de produit recherché.
F = 176°C.
Spectre IR : ($CHCl_3$)
C≡N 2250 $cm^{-1}$
17-céto 1735 $cm^{-1}$
3-céto 1658 $cm^{-1}$
C=C 1609 $cm^{-1}$
aromatique 1609, 1580, 1509 $cm^{-1}$

**STADE E** : 7-[4-(3-acétoxy 17béta-hydroxy estra-1,3,5(10)-trièn-11béta-yl) phénoxy] heptane nitrile.

a) Aromatisation.
On opère comme au stade B de l'exemple 3 à partir de 1,693 g du produit obtenu au stade D ci-dessus en utilisant 1,7 $cm_3$ d'anhydride acétique et 0,85 $cm_3$ de bromure d'acétyle. On obtient 2,15 g de produit brut.
b) Réduction de la cétone en 17.
On opère comme au stade B de la préparation 1 à partir de 2,15 g du produit obtenu ci-dessus en utilisant 280 mg d'hydrure de bore et de sodium. Après chromatographie sur silice (éluant : acétate d'éthyle-cyclohexane 40-60), on obtient 1,12 g du produit recherché.
Spectre IR : ($CHCl_3$)
OH 3612 $cm^{-1}$
C≡N 2250 $cm^{-1}$
C=O 1753 $cm^{-1}$
aromatique 1610, 1580, 1512, 1494 $cm^{-1}$

**STADE F** : Acide 7-[4-(3,17béta-dihydroxy estra-1,3,5(10)-trièn-11béta-yl) phénoxy] heptanoïque.

On opère comme au stade B de l'exemple 2 à partir de 900 mg de produit obtenu au stade E ci-dessus. Après chromatographie sur silice (éluant : essence G-acétone 65-35 à 1% d'acide acétique puis acétone seule), on obtient 779 mg de produit recherché.
Spectre IR : ($CHCl_3$)
OH 3602 $cm^{-1}$
C=O 1731, 1709 $cm^{-1}$
aromatique 1610, 1581, 1512 $cm^{-1}$

**STADE G** : 7-(4-(3,17béta-dihydroxy estra-1,3,5(10)-trièn 11béta-yl) phénoxy) N-méthyl N-(1-méthyléthyl) heptanamide.

On opère comme au stade A de l'exemple 3 à partir de 400 mg du produit obtenu ci-dessus en utilisant 0,34 $cm_3$ d'isopropylméthylamine. On obtient 511 mg de produit brut (carbonate, en 3, intermédiaire) que l'on saponifie en opérant comme au stade A de l'exemple 2. Après chromatographie sur silice (éluant : acétate d'éthyle puis acétone à 1% d'acide acétique) on obtient 336 mg de produit recherché.
$[alpha]_D$ = -39° ± 2° (c = 0,7% éthanol).
Spectre IR : ($CHCl_3$)

| | |
|---|---|
| OH | 3604 $cm^{-1}$ |
| C=O | 1619 $cm^{-1}$ amide III |
| aromatique | 1581, 1511 $cm^{-1}$ |

| | | | | |
|---|---|---|---|---|
| Analyse | : | $C_{35}H_{49}NO_4$ = 547,79 | | |
| Calculé | : | C% 76,74 | H% 9,01 | N% 2,55 |
| Trouvé | : | 77,0 | 9,0 | 2,6 |

**EXEMPLE 42 : N-butyl 4-[3,17béta-dihydroxy 19-nor 17alpha-pregna 1,3,5(10)-trièn-20-yn-11béta-yl] N-méthyl benzène.**

En opérant comme à l'exemple 21 à partir du produit obtenu au stade A de la préparation 4, on a obtenu 250 mg du produit recherché.
Spectre IR : ($CHCl_3$)

| | |
|---|---|
| C≡CH | 3305 $cm^{-1}$ |
| OH | 3598 $cm^{-1}$ |
| C=O | 1621 $cm^{-1}$ |
| aromatique | 1583, 1500 $cm^{-1}$ |

| | | | | |
|---|---|---|---|---|
| Analyse | : | $C_{39}H_{53}NO_3$ = 583,86 | | |
| Calculé | : | C% 80,23 | H% 9,15 | N% 2,4 |
| Trouvé | : | 80,1 | 9,3 | 2,4 |

**PREPARATION DE L'EXEMPLE 43 : 11béta-(4-hydroxyphényl) estra-4,9-dièn-3,17-dione.**

On opère comme au stade B de la préparation 3 à partir de 1 g du composé obtenu au stade A de la préparation de l'exemple 41. Après chromatographie sur silice (éluant : chlorure de méthylène-acétate d'éthyle 7-3), on obtient 703 mg de produit recherché.
Spectre IR : ($CHCl_3$)

| | |
|---|---|
| OH | 3596 $cm^{-1}$ |
| C=O | 1735 $cm^{-1}$ |
| C=O | 1657 $cm^{-1}$ |
| aromatique | 1612, 1593, 1511 $cm^{-1}$ |

**PREPARATION B DE L'EXEMPLE 43 : [(5-bromopentyl) oxy] N-butyl N-méthyl acétamide.**

**STADE A** : bromo N-butyl N-méthyl acétamide.

A une solution refroidie à -20°C de 11,9 $cm_3$ de bromure de bromoacétyle dans 180 $cm_3$ d'éther, on ajoute 26 g de butylméthylamine en solution dans 120 $cm_3$ d'éther. On ramène la température à 20°C, agite 30 minutes, dilue à l'eau et extrait à l'éther. On évapore à sec sous pression réduite. On distille le résidu (27,4 g) sous pression réduite (0,05 mbar) à 79/83°C. On obtient 19,36 g de produit recherché.

| | | | | | |
|---|---|---|---|---|---|
| <u>Analyse</u> | : | $C_7H_{14}BrNO$ = 208,105 | | | |
| Calculé | : | C% 40,40 | H% 6,78 | N% 6,73 | Br% 38,39 |
| Trouvé | : | 40,3 | 7,0 | 6,7 | 38,2 |

<u>STADE B</u> : 5-[[(diméthyl (1,1-diméthyléthyl) silyl] oxy] pentanol.

A une solution composée de 10 g de 4-pentanol, 200 $cm_3$ de chlorure de méthylène, 19,5 $cm_3$ de triéthylamine et 566 mg de 4-diméthylaminopyridine, on ajoute en refroidissant 19,14 g de chlorure de tertbutyldiméthylsilyle. On agite 1 heure à température ambiante, dilue à l'eau, décante la phase organique, la lave, la sèche et l'évapore à sec sous vide. On chromatographie le résidu (42 g) sur silice (éluant : essence G-acétate d'éthyle 95-5). On obtient 23,3 g de silyloxy pentène que l'on dissout dans 250 $cm_3$ de tétrahydrofuranne. On ajoute à 20°C 6 $cm_3$ de complexe borane-méthylsulfure. On agite 30 minutes à 20/25°C puis 30 minutes à 35°C. On ajoute à +10°C, 18 $cm_3$ de lessive de soude puis 18 $cm_3$ d'eau oxygénée, on agite 30 minutes. On dilue à l'eau, extrait avec de l'acétate d'éthyle, lave avec une solution de thiosulfate de sodium à 10%, sèche et concentre à sec sous pression réduite. On chromatographie le résidu (25,85 g) sur silice (éluant : cyclohexane-acétate d'éthyle 8-2). On recueille 22,7 g de produit que l'on distille sous pression réduite (0,06 mbar) et obtient 18,7 g du composé recherché. Eb = 73-75°C/0,06 mbar.

<u>STADE C</u> : N-butyl [(5-hydroxypentyl) oxy] N-méthyl acétamide.

A une solution de 8 g de l'alcool obtenu au stade B ci-dessus, dans 40 $cm_3$ de tétrahydrofuranne, on ajoute 2,16 g d'hydrure de sodium à 50% dans l'huile, agite 30 minutes à températur ambiante, ajoute goutte à goutte en 15 minutes une solution de 9,5 g du bromé obtenu au stade A ci-dessus dans 13 $cm_3$ de tétrahydrofuranne. On agite 16 heures à température ambiante, ajoute une solution aqueuse saturée de chlorure d'ammonium, extrait à l'acétate d'éthyle, lave, sèche et évapore à sec sous vide. On obtient 14,8 g de N-butyl [5-[(diméthyl (1,1-diméthyléthyl) silyl] oxy] pentyl) oxy] N-méthyl acétamide intermédiaire que l'on dissout dans 83 $cm_3$ de tétrahydrofuranne et 46 $cm_3$ d'une solution 1M de fluorure de tétrabutylammonium. On agite 2 heures à température ambiante, coule dans l'eau, extrait à l'acétate d'éthyle, évapore à sec sous pression réduite. On chromatographie le résidu (13,6 g) sur silice (éluant : chlorure de méthylène-isopropanol 94-6) et obtient 7,28 g du composé recherché.
<u>Spectre IR</u> : ($CHCl_3$)
-OH 3628 $cm^{-1}$
C=O 1645 $cm^{-1}$

<u>STADE D</u> : [(5-bromopentyl) oxy] N-butyl N-méthyl acétamide.

A une solution de 7,2 g du produit obtenu au stade C ci-dessus dans 73 $cm_3$ de chlorure de méthylène, on ajoute à -10°C 13 g de tétrabromométhane et 10,3 g de triphénylphosphine. On agite 1 heure à 0°C et chromatographie le milieu réactionnel sur silice (éluant : acétate d'éthyle-cyclohexane 7-3). On obtient 7,49 g du composé recherché.
<u>Spectre IR</u> : ($CHCl_3$)
C=O 1644 $cm^{-1}$

| | | | | | |
|---|---|---|---|---|---|
| <u>Analyse</u> | : | $C_{12}H_{24}BrNO_2$ = 294,24 | | | |
| Calculé | : | C% 48,98 | H% 8,22 | N% 4,76 | Br% 27,15 |
| Trouvé | : | 48,6 | 8,2 | 4,6 | 26,3 |

**<u>EXEMPLE 43</u> : N-butyl [5-[4-(3,17béta-dihydroxy estra-1,3,5(10)-trièn-11béta-yl) phénoxy] pentyloxy] N-méthyl acétamide.**

<u>STADE A</u> : N-butyl [5-[4-(3,17-dioxo estra-4,9dièn-11béta-yl) phénoxy] pentyloxy] N-méthyl acétamide.

A une solution de 2,5 g de produit obtenu au stade A de la préparation de l'exemple 41 dans 26 $cm_3$ d'acétone et 6,4 $cm_3$ de soude 2N, on ajoute 3,75 g de [(5-bromopentyl) oxy] N-butyl N-méthyl acétamide (obtenu

à la préparation ci-dessus) en solution dans 6 $cm_3$ d'acétone. On agite 5 heures à 50°C, refroidit, verse dans l'eau, acidifie avec de l'acide chlorhydrique 2N, extrait avec de l'acétate d'éthyle, lave, sèche et évapore à sec sous pression réduite. On chromatographie le résidu (6,8 g) sur silice (éluant : acétate d'éthyle) et obtient 2,63 g du produit recherché.

Spectre IR : ($CHCl_3$)

| | |
|---|---|
| C=O | 1735 $cm^{-1}$ (17-céto)<br>1657 $cm^{-1}$ |
| C=C + aromatique | 1609, 1580, 1509 $cm^{-1}$ |

**STADE B** : N-butyl [5-[4-(3-hydroxy 17-oxo estra-1,3,5(10)-trièn-11béta-yl) phénoxy] pentyloxy] N-méthyl acétamide.

On opère comme au stade A de l'exemple 2 à partir de 2,61 g du produit obtenu au stade A ci-dessus en utilisant 2,61 g d'hydroxyde de palladium sur oxyde de magnésium. Après chromatographie sur silice (éluant : acétate d'éthyle-essence G 9-1), on obtient 1,83 g du produit recherché.

Spectre IR : ($CHCl_3$)

| | |
|---|---|
| OH | 3598 $cm^{-1}$ |
| C=O | 1732 $cm^{-1}$ (17-céto)<br>1634 $cm^{-1}$ (amide III) |
| aromatique | 1611, 1581, 1511 $cm^{-1}$ |

**STADE C** : N-butyl [5-[4-(3,17béta-dihydroxy estra-1,3,5(10)-trièn-11béta-yl) phénoxy] pentyloxy] N-méthyl acétamide.

On opère comme au stade B de la préparation 1 à partir de 500 mg du produit obtenu au stade B ci-dessus en utilisant 66 mg d'hydrure de bore et de sodium. On chromatographie sur silice le résidu (514 mg) (éluant : chlorure de méthylène-isopropanol 95-5) puis une seconde fois (éluant : acétate d'éthyle). On obtient 343 mg du produit recherché.
[$alpha_D$] = -31,1° (c = 1% chloroforme).
Spectre IR : ($CHCl_3$)

| | |
|---|---|
| OH | 3603 $cm^{-1}$ |
| C=O | 1634 $cm^{-1}$ (amide III) |
| aromatique | 1611, 1581, 1511 $cm^{-1}$ |

| Analyse : | $C_{36}H_{51}NO_5$ = 577,81 | | |
|---|---|---|---|
| Calculé : | C% 74,83 | H% 8,89 | N% 2,42 |
| Trouvé : | 74,8 | 9,0 | 2,3 |

**EXEMPLE 44 : N-butyl [5-[4-(3,17béta-dihydroxy 19-nor 17alpha-pregna-1,3,5(10)-trièn-20-yn-11béta-yl) phénoxy] pentyloxy] N-méthyl acétamide.**

A une solution de 500 mg du produit obtenu à l'exemple 43 dans 4 $cm_3$ de tétrahydrofuranne, on ajoute 6 $cm_3$ d'une solution 0,44M d'acétylure de potassium dans le tétrahydrofuranne (préparée en faisant barboter de l'acétylène dans une solution de terbutylate de potassium dans le tétrhydrofuranne). On agite 30 minutes, puis verse dans une solution saturée de chlorure d'ammonium, extrait avec de l'acétate d'éthyle, lave, sèche et évapore à sec sous pression réduite. On chromatographie le résidu (504 mg) sur silice (éluant : acétate d'éthyle-essence G 85-15 puis sous pression éluant : chlorure de méthylène-acétone 90-10) et obtient 264 mg de produit recherché.
[$alpha_D$] = -107° ± 2,5° (c = 0,8% éthanol).

Spectre IR : ($CHCl_3$)

| | |
|---|---|
| OH | 3600 $cm^{-1}$ |
| C≡CH | 3304 $cm^{-1}$ |
| C=O | 1634 $cm^{-1}$ |
| aromatique | 1611, 1581, 1511 $cm^{-1}$ |

| | | | | |
|---|---|---|---|---|
| Analyse : | $C_{38}H_{51}NO_5$ = 601,83 | | | |
| Calculé : | C% | 75,83 | H% 8,54 | N% 2,32 |
| Trouvé : | | 75,5 | 8,6 | 2,2 |

**PREPARATION DE L'EXEMPLE 45 : Acide 8-[4-(3,17-dioxo estra-4,9-dièn-11béta-yl) phénoxy] octanoïque.**

**STADE A :** 3-(1,2-éthanediyl) acétal cyclique de 11béta-[4-[(8-hydroxy acétyl) oxy] phényl] 5-alpha-hydroxy-estr-9-èn-3,17-dione.

On opère comme au stade A de l'exemple 43 à partir de 1,5 g du produit obtenu au stade A de la préparation de l'exemple 41 en utilisant 2,22 g de 8-bromooctanol. Après chromatographie sur silice (éluant : acétate d'éthyle-chlorure de méthylène 6-4), on obtient 1,475 g de produit recherché.

Spectre IR : ($CHCl_3$)

| | |
|---|---|
| OH | 3620, 3509 $cm^{-1}$ |
| 17-céto | 1733 $cm^{-1}$ |
| aromatique | 1609, 1577, 1508 $cm^{-1}$ du type $-C_6H_4-OH$ |

**STADE B :** 11béta-[4-[(8-hydroxy octyl) oxy] phényl] estra-4,9-dièn-3,17-dione.

On opère comme au stade B de la préparation 2 à partir de 1,44 g du produit obtenu au stade A ci-dessus. Après chromatographie sur silice (éluant : acétate d'éthyle-chlorure de méthylène 1-1), on obtient 1,049 g du produit attendu.

Spectre IR : ($CHCl_3$)

| | |
|---|---|
| OH | 3620 $cm^{-1}$ |
| 17-céto | 1735 $cm^{-1}$ |
| diénone | 1658 $cm^{-1}$ |
| C=C + aromatique | 1609, 1580, 1509 $cm^{-1}$ |

**STADE C :** Acide 8-[4-(3,17-dioxo estra-4,9-dièn-11béta-yl) phénoxy] octanoïque.

On opère comme au stade C de la préparation 2 à partir de 1,008 g de produit obtenu au stade B ci-dessus en utilisant 1,25 $cm_3$ du réactif à Heilbron-Jones. On obtient 1,009 g du produit recherché.

Spectre IR : ($CHCl_3$)

Acide (d'après la région OH) avec C=O : 1710 $cm^{-1}$

| | |
|---|---|
| 17-céto | 1735 $cm^{-1}$ |
| diénone | 1658 $cm^{-1}$ |
| C=C + aromatique | 1609, 1580, 1509 $cm^{-1}$ |

**EXEMPLE 45 : 8-[4-(3,17béta-dihydroxy estra-1,3,5(10)-trièn-11béta-yl) phénoxy] N-méthyl N-(1-méthyléthyl) octanamide.**

**STADE A :** 8-[4-(3,17dioxo estra-4,9-dièn-11béta-yl) phénoxy] N-méthyl N-(1-méthyléthyl) octanamide.

On opère comme au stade A de l'exemple 3 à partir de 1 g du produit obtenu à la préparation ci-dessus en utilisant 1,03 $cm_3$ de N-isopropylméthylamine. Après chromatographie sur silice (éluant : acétate d'éthyle-chlorure de méthylène 60-40), on obtient 722 mg du produit recherché.

Spectre IR : ($CHCl_3$)

| | |
|---|---|
| amide III | 1621 $cm^{-1}$ |
| 17-céto | 1735 $cm^{-1}$ |

| | |
|---|---|
| diénone | 1658 $cm^{-1}$ |
| C=C + aromatique | 1580, 1509 $cm^{-1}$ |

**STADE B** : 8-[4-(3,17béta-dihydroxy estra-1,3,5(10)-trièn-11béta-yl) phénoxy] N-méthyl N-(1-méthyléthyl) octanamide.

a) Aromatisation.

On opère comme au stade A de l'exemple 2 à partir de 695 mg du produit obtenu au stade A ci-dessus et en utilisant 0,7 $cm_3$ d'anhydride acétique et 0,35 $cm_3$ de bromure d'acétyle. On obtient 815 mg de 3-acétoxy brut intermédiaire.

b) Saponification.

On opère comme au stade A de l'exemple 2. On obtient 677 mg de produit 3-hydroxy 17-céto brut.

c) Réduction de la cétone en 17.

On opère comme à l'exemple 28 en utilisant 767 mg de tri-terbutoxy alumino hydrure de lithium. Après chromatographie sur silice (éluant : acétate d'éthyle-chlorure de méthylène 6-4) puis successivement, sous pression, méthanol-eau (85-15), (75-25) et enfin deux fois acétate d'éthyle-chlorure de méthylène (6-4), on obtient 211 mg du produit recherché.

[$alpha_D$] = -45° ± 2° (c = 0,7% éthanol).

Spectre IR : ($CHCl_3$)

| | |
|---|---|
| OH | 3603 $cm^{-1}$ |
| C=O | 1619 $cm^{-1}$ (amide III) |
| aromatique | 1612, 1581, 1511 $cm^{-1}$ |

Analyse : $C_{36}H_{51}NO_4$ = 561,81

| | | | |
|---|---|---|---|
| Calculé : | C% 76,96 | H% 9,15 | N% 2,49 |
| Trouvé : | 77,3 | 9,3 | 2,5 |

**PREPARATION DE L'EXEMPLE 46 : 8-bromo N-butyl N-méthyl octanamide.**

On opère comme au stade A de l'exemple 3 à partir de 5 g d'acide 8-bromo octanoïque en utilisant 13 $cm_3$ de N-méthyl butylamine. Après chromatographie sur silice (éluant : chlorure de méthylène-acétone 95-5), on obtient 6,14 g du produit attendu.

Spectre IR : ($CHCl_3$)

C=O (type amide tertiaire) : 1627 $cm^{-1}$

**EXEMPLE 46 : N-butyl 8-[4-(3,17béta-dihydroxy estra-1,3,5(10)-trièn-11béta-yl) phénoxy] N-méthyl octanamide.**

**STADE A** : 8-[4-(3,17béta-dioxo estra-4,9-dièn-11béta-yl) phénoxy] N-butyl N-méthyl octanamide.

On opère comme au stade A de l'exemple 43 à partir de 725 mg du produit obtenu à la préparation de l'exemple 43 en utilisant 0,2 $cm_3$ de 8-bromo N-butyl N-méthyl octanamide obtenu à la préparation ci-dessus. Après chromatographie sur silice (éluant : Essence G-acétate d'éthyle 4-6), on obtient 540 mg du produit attendu.

Spectre IR : ($CHCl_3$)

| | |
|---|---|
| 17-céto | 1735 $cm^{-1}$ |
| 3-céto | 1657 $cm^{-1}$ |
| amide III | 1628 $cm^{-1}$ |
| bandes aromatiques | 1580, 1509 $cm^{-1}$ du type $-O-C_6H_5$ |

**STADE B** : 8-[4-(3-hydroxy 17-oxo estra-1,3,5(10)-trièn-11béta-yl) phénoxy] N-butyl N-méthyl octanamide.

On opère comme au stade A de l'exemple 2 à partir de 470 mg du produit obtenu au stade A ci-dessus en utilisant 260 mg d'hydroxyde de palladium sur oxyde de magnésium. Après chromatographie sur silice (éluant : acétate d'éthyle-essence G 1-1), on obtient 360 mg du composé recherché.

Spectre IR : ($CHCl_3$)

| | |
|---|---|
| OH | 3596 $cm^{-1}$ |
| C=O | 1732 $cm^{-1}$ |
| amide III | 1623 $cm^{-1}$ |
| bandes aromatiques | 1581, 1511 $cm^{-1}$ |

**STADE C** : N-butyl 8-[4-(3,17béta-dihydroxy estra-1,3,5(10)-trièn-11béta-yl) phénoxy] N-méthyl octanamide.

On opère comme au stade B de la préparation 1 à partir de 360 mg du produit obtenu au stade B ci-dessus en utilisant 72 mg d'hydrure de bore et de sodium.
Spectre IR : ($CHCl_3$)

| | |
|---|---|
| OH | 3602 $cm^{-1}$ |
| C=O | 1623 $cm^{-1}$ (amide III) |
| bandes aromatiques | 1581, 1511 $cm^{-1}$ |

**EXEMPLE 47 : N-butyl 8-[4-(3,17béta-dihydroxy 19-nor 17alpha-pregna-1,3,5(10)-trièn-20-yn-11béta-yl) phénoxy] N-méthyl octanamide.**

**STADE A** : N-butyl 8-[4-(17béta-hydroxy 3-oxo 19-nor 17alpha-pregna-4,9-dièn-20-yn-11béta-yl) phénoxy] N-méthyl octanamide.

On opère comme au stade A de l'exemple 43 à partir de 1,975 g de 17béta-hydroxy 11béta-(4-hydroxyphényl) 19-nor 17alpha-pregna 4,9-dièn-20-yn-3-one (préparé selon BF. 2 522 328) en utilisant 1,9 $cm_3$ de 8-bromo N-butyl N-méthyl octanamide (préparation A de l'exemple 46). Après chromatographie sur silice (éluant : acétate d'éthyle-cyclohexane 7-3), on obtient 2,45 g du produit attendu.
Spectre IR : ($CHCl_3$)

| | |
|---|---|
| OH | 3598 $cm^{-1}$ |
| C≡CH | 3305 $cm^{-1}$ |
| C=O | 1644 et 1628 $cm^{-1}$ amide III |
| C=C + aromatique | 1611, 1508 $cm^{-1}$ |

**STADE B** : N-butyl N-méthyl 8-[4-[3-oxo 17béta-(tétrahydro-2H-2-pyrannyloxy] 19-nor 17alpha-pregna-4,9-dièn-11béta-yl) phénoxy] octanamide.

A une solution de 2,45 g du composé obtenu au stade A ci-dessus dans 25 $cm_3$ de tétrahydrofuranne anhydre et 5 $cm_3$ de dihydropyranne, on ajoute 75 mg d'acide paratoluènesulfonique. On agite 2 heures à température ambiante, ajoute 1 $cm_3$ de triéthylamine, dilue avec une solution de bicarbonate de sodium, extrait à l'acétate d'éthyle, lave, sèche et évapore a sec sous pression réduite. On chromatographie le résidu (4,3 g) sur silice (éluant : acétate d'éthyle-cyclohexane 7-3) et obtient 2,61 g du composé attendu.
Spectre IR : ($CHCl_3$)
absence d'OH

| | |
|---|---|
| C≡CH | 3304 $cm^{-1}$ |
| C=O | 1644, 1628 $cm^{-1}$ |
| C=C et aromatique | 1610, 1508 $cm^{-1}$ |

**STADE C** : N-butyl 8-[4-(3,17béta-dihydroxy 19-nor 17alpha-pregna-1,3,5(10)-trièn-20-yn-11béta-yl) phénoxy] N-méthyl octanamide.

On opère comme à l'exemple 8 à partir de 515 mg du produit obtenu au stade B ci-dessus en utilisant 0,5 $cm_3$ d'anhydride acétique et 0,25 $cm_3$ de bromure d'acétyle et pour la saponification, 1 $cm_3$ de lessive de soude. Après chromatographie sur silice (éluant : Essence G-acétate d'éthyle 1-1), on obtient 460 mg du produit recherché.
Spectre IR : ($CHCl_3$)

| | |
|---|---|
| OH | 3599 $cm^{-1}$ |
| C≡CH | 3304 $cm^{-1}$ |
| C=O | 1623 $cm^{-1}$ amide III |
| aromatique | 1611, 1581, 1511, 1502 $cm^{-1}$ (ép.) |

| | | | |
|---|---|---|---|
| Analyse : $C_{39}H_{53}NO_4$ | | | |
| Calculé : | C% 78,09 | H% 8,90 | N% 2,33 |
| Trouvé : | 78,0 | 8,9 | 2,1 |

**PREPARATION DE L'EXEMPLE 48 : 9-bromo N-butyl N-méthyl 7-nonynamide.**

**STADE A** : 6-bromo N-butyl N-méthyl hexanamide.

On opère comme au stade A de l'exemple 3 à partir de 4,88 g d'acide 6-bromohexanoïque en utilisant 4,36 $cm_3$ de N-méthylbutylamine et obtient 7,0 g de produit attendu utilisé tel quel pour le stade suivant.

**STADE B** : N-butyl 9-hydroxy N-méthyl 7-nonynamide.

On refroidit à -60°C une solution de 1,77 $cm_3$ d'alcool propargylique, 30 $cm_3$ de tétrahydrofuranne et 7,5 $cm_3$ d'hexaméthyl phosphotriamide puis on ajoute 37,5 $cm_3$ d'une solution 1,6M de butyllithium dans l'hexane. On agite 45 minutes à -30°C. On ajoute alors 7 g du produit obtenu au stade A ci-dessus en solution dans 7 $cm_3$ de tétrahydrofuranne. On agite 16 heures à température ambiante, verse sur une solution saturée de chlorure d'ammonium et extrait avec de l'acétate d'éthyle, lave avec de l'acide chlorhydrique 2N, du bicarbonate de sodium en solution saturée, sèche et évapore à sec. On chromatographie le résidu (5,78 g) sur silice (éluant : chlorure de méthylène-acétate d'éthyle 70-30) puis (chlorure de méthylène-isopropanol 95-5) et obtient 1,65 g du produit recherché.

Spectre IR : ($CHCl_3$)
OH 3611 $cm^{-1}$
C=O 1627 $cm^{-1}$ amide

**STADE C** : 9-bromo N-butyl N-méthyl 7-nonynamide.

A une solution de 1,65 g du produit obtenu au stade B ci-dessus dans 16,5 $cm_3$ de chlorure de méthylène et refroidie à -5°C, on ajoute 2,85 g de tétrabromure de carbone et 2,25 g de triphénylphosphine. On agite 30 minutes à 0°C et chromatographie la solution réactionnelle sur silice (éluant : chlorure de méthylène-acétate d'éthyle 90-10). On obtient 1,82 g du produit recherché.

Spectre IR : ($CHCl_3$)
C=O 1628 $cm^{-1}$ amide
C≡C 2230 $cm^{-1}$

**EXEMPLE 48 : N-butyl 9-[4-(3,17béta-dihydroxy estra-1,3,5(10)-trièn-11béta-yl) phénoxy] N-méthyl 7-nonynamide.**

**STADE A** : N-butyl 9-[4-(3,17-dioxo estra-1,3,5(10)-trièn-11béta-yl) phénoxy] N-méthyl 7-nonynamide.

On opère comme au stade A de l'exemple 43 à partir de 816 mg du composé obtenu à la préparation de l'exemple 43 en utilisant 1,25 g de 9-bromo N-butyl N-méthyl 7-nonynamide (préparation ci-dessus). On obtient 2,2 g de produit brut auquel on ajoute 270 mg d'un lot précédent. On chromatographie le tout sur silice (éluant : acétate d'éthyle-Essence G 75-25) et recueille 1,126 g de produit attendu.

Spectre IR : ($CHCl_3$)

| | |
|---|---|
| C≡C | 2220 $cm^{-1}$ |
| C=O | 1735 $cm^{-1}$ (17-céto) |
| | 1657 $cm^{-1}$ (3-céto) |
| amide III | 1628 $cm^{-1}$ |
| C=C + aromatique | 1610, 1582, 1508 $cm^{-1}$ |

**STADE B** : N-butyl 9-[4-(3,17béta-dihydroxy estra-1,3,5(10)-trièn-11béta-yl) phénoxy] N-méthyl 7-nonynamide.

On opère comme aux stades B et C de l'exemple 3 à partir de 1,1 g du produit obtenu ci-dessus en utilisant 1,1 $cm_3$ d'anhydride acétique et 0,55 $cm_3$ de bromure acétyle pour l'obtention du 3-acétoxy, 2,8 $cm_3$ de soude 2N pour la saponification en 3 et 142 mg d'hydrure de bore et de sodium pour la réduction du 17-céto. Après chromatographies successives sur silice (éluant : acétate d'éthyle-Essence G 70-30, microbondapack $C_{18}$, éluant : méthanol-eau 80-20) puis à nouveau sur silice (éluant : acétate d'éthyle-Essence G 70-30), on obtient 540 mg de produit recherché.
[$alpha_D$] = -67° ± 2° (c = 0,83% chloroforme)
Spectre IR : ($CHCl_3$)

| | |
|---|---|
| OH | 3606 $cm^{-1}$ |
| C=C | 2220 $cm^{-1}$ |
| C=O | 1620 $cm^{-1}$ |
| aromatique | 1582, 1510 $cm^{-1}$ |

| | | | | |
|---|---|---|---|---|
| Analyse : | $C_{38}H_{51}NO_4$ = 585,83 | | | |
| Calculé : | C% | 77,91 | H% 8,77 | N% 2,39 |
| Trouvé : | | 78,0 | 9,0 | 2,3 |

**EXEMPLE 49 : N-butyl 9-[4-(3,17béta-dihydroxy estra-1,3,5(10)-trièn-11béta-yl) phénoxy] N-méthyl nonanamide.**

On hydrogène sous 1900 mbars pendant 1 heure une solution de 212 mg du produit obtenu à l'exemple 48 et 42 mg du catalyseur de Wilkinson en solution dans 2 $cm_3$ de toluène et 2 $cm_3$ d'éthanol. On évapore sous pression réduite et chromatographie le résidu (265 mg) sur silice (éluant : acétate d'éthyle-Essence G 70-30), on obtient 168 mg du composé recherché.
[$alpha_D$] = -32° (c = 0,49% $CHCl_3$).
Spectre IR : ($CHCl_3$)

| | |
|---|---|
| OH | 3603 $cm^{-1}$ |
| C=O | 1624 $cm^{-1}$ |
| aromatique | 1581, 1511 $cm^{-1}$ |

| | | | | |
|---|---|---|---|---|
| Analyse : | $C_{38}H_{55}NO_4$ | | | |
| Calculé : | C% | 77,37 | H% 9,4 | N% 2,38 |
| Trouvé : | | 77,4 | 9,6 | 2,4 |

**PREPARATION DE L'EXEMPLE 50 : 17béta-acétyloxy 11béta-(10-hydroxy décyl) estra-4,9-dièn-3-one.**

**STADE A** : 3-(1,2-éthanediyl acétal cyclique) de 11béta-[11-[[(11-diméthyléthyl) silyl] oxy] décyl] 5alpha-hydroxy estr-9-èn-3,17-dione.

On opère comme au stade A de la préparation 5 à partir de 19,5 g de l'époxyde obtenu selon EP 0 057 115 (ex. 7) et en utilisant 113,5 $cm^3$ d'une solution 0,59M de 10-(diméthyl tertbutylsilyloxy) décyl magnésien. On obtient 43,78 g de produit brut auquel on joint 11,3 g d'une préparation précédente. On chromatographie le tout sur silice (éluant : cyclohexane-acétate d'éthyle 7-3) et obtient 23,15 g de produit recherché.

Spectre IR : ($CHCl_3$)

| | |
|---|---|
| OH | 3616 $cm^{-1}$ (en 17) |
| | 3508 $cm^{-1}$ (en 5) |
| C=O | 1732 $cm^{-1}$ (17-céto) |

**STADE B** : (1,2-éthanediyl acétal cyclique) de 5alpha, 17béta-dihydroxy 11béta-[[10-[diméthyl (1,1-diméthyléthyl) silyl] oxy] décyl] estr-9-èn-3-one.

On opère comme au stade B de la préparation 5 à partir de 23,57 g du produit obtenu au stade A en utilisant 1,447 g d'hydrure de bore et de sodium. On obtient 22,342 g du produit recherché. On chromatographie 103 mg du produit obtenu sur silice (éluant : cyclohexane-acétate d'éthyle 1-1) et recueille 96,8 mg de produit pur.

Spectre IR : ($CHCl_3$)

| | |
|---|---|
| OH | 3612, 3510 $cm^{-1}$ |
| t-butyldiméthylsilyloxy | 1255, 836 $cm^{-1}$ |

**STADE C** : (1,2-éthanediyl acétal cyclique) de 17béta-acétyloxy 11béta-[[10-[diméthyl (1,1-diméthyléthyl) silyl] oxy] décyl] 5-alpha-hydroxy estr-9-èn-3-one.

On opère comme au stade C de la préparation 5 à partir de 22,225 g du produit obtenu au stade B ci-dessus en utilisant 44,5 $cm^3$ de pyridine et 22,25 $cm^3$ d'anhydride acétique. On obtient 23,74 g de produit attendu.

**STADE D** : 17béta-acétyloxy 11béta-(10-hydroxy décyl) estra-4,9-dièn-3-one.

On opère comme au stade D de la préparation 5 à partir de 23,74 g du produit obtenu au stade C ci-dessus en utilisant 100 $cm^3$ d'acide chlorhydrique 2N. On chromatographie sur silice (éluant : cyclohexane-acétate d'éthyle 1-1) et obtient 15,238 g de produit attendu.

Spectre IR : ($CHCl_3$)

| | |
|---|---|
| OH | 3616 $cm^{-1}$ |
| diénone | 1654, 1600 $cm^{-1}$ |

**EXEMPLE 50 : 17béta-hydroxy N-méthyl N-(1-méthyléthyl) 3-oxo-estra-4,9-dièn-11béta-décanamide.**

**STADE A** : Acide 17béta-acétoxy 3-oxo-estra-4,9-dièn-11béta-décanoïque.

On opère comme au stade E de la préparation 5 à partir de 942 mg de 17béta-acétoxy 3-oxo-estra-4,9-dièn-11béta-décanol (obtenu à la préparation ci-dessus) en utilisant 1,1 $cm_3$ de réactif de Heilbron-Jones. On obtient 964 mg de produit attendu utilisé tel quel.

**STADE B** : 17béta-acétoxy N-méthyl N-(1-méthyléthyl) 3-oxo-estra-4,9-dièn-11béta-décanamide.

On opère comme au stade A de l'exemple 3 à partir de 940 mg du produit obtenu au stade A ci-dessus en utilisant 0,41 $cm_3$ de méthylisopropylamine. Après chromatographie sur silice (éluant : Essence G-acétate d'éthyle 1-1), on obtient 705 mg de produit recherché.

Spectre IR : ($CHCl_3$)

| | |
|---|---|
| OAC | 1728 $cm^{-1}$ |
| diénone | 1653 $cm^{-1}$ |
| amide III | 1622 $cm^{-1}$ |

**STADE C** : 17béta-hydroxy N-méthyl N-(1-méthyléthyl) 3-oxo-estra-4,9-dièn-11béta-décanamide.

On opère comme au stade B de l'exemple 1 à partir de 194 mg du composé obtenu au stade ci-dessus en utilisant 0,3 $cm_3$ de soude 2N. Après chromatographie sur silice (éluant : acétate d'éthyle), on obtient 165 mg de produit attendu.

Spectre IR : ($CHCl_3$)

| | |
|---|---|
| OH | 3613 $cm^{-1}$ |
| diénone | 1644 $cm^{-1}$ |
| amide III | 1621 $cm^{-1}$ |

| | | | | |
|---|---|---|---|---|
| Analyse : | $C_{32}H_{51}NO_3$ = 497,77 | | | |
| Calculé : | C% 77,21 | H% 10,32 | N% 2,81 |
| Trouvé : | 77,1 | 10,3 | 2,8 |

**EXEMPLE 51 : 3,17béta-dihydroxy N-méthyl N-(1-méthyléthyl) estra-1,3,5(10)-trièn-11béta-décanamide.**

On opère comme à l'exemple 2 à partir de 0,5 g du composé obtenu à l'exemple 50 en utilisant 0,5 $cm_3$ d'anhydride acétique et 0,25 $cm_3$ de bromure d'acétyle lors de l'aromatisation et 1,6 $cm_3$ de soude 2N pour la saponification. Après chromatographie sur silice (éluant : acétate d'éthyle-Essence G 7-3), on obtient 357 mg de produit attendu. Après cristallisation dans l'acétate d'éthyle, on obtient 318 mg de produit recherché.
F = 150°C.
Spectre IR : ($CHCl_3$)

| | |
|---|---|
| OH | 3605 $cm^{-1}$ |
| C=O (amide) | 1617 $cm^{-1}$ |
| aromatique | 1583, 1498 $cm^{-1}$ |

| | | | | |
|---|---|---|---|---|
| Analyse | : | $C_{32}H_{51}NO_3$ = 497,77 | | |
| Calculé | : | C% 77,21 | H% 10,32 | N% 2,81 |
| Trouvé | : | 77,4 | 10,5 | 2,9 |

**EXEMPLE 52 : Méthyl (1-méthyléthyl) carbamate de 10-(17béta-hydroxy 3-oxo 4,9-dièn-11béta-yl) décyle.**

**STADE A** : Méthyl (1-méthyléthyl) carbamate de 10-(17béta acétoxy 3-oxo estra-4,9-dièn-11béta-yl) décyle.

A une solution refroidie à 0°C de 470 mg de 17béta-acétoxy 3-oxo estra-4,9-dièn-11béta-décanol (obtenu à la préparation de l'exemple 50) dans 4 $cm_3$ de toluène et 122 mg de diméthylaminopyridine, on ajoute 118 mg de triphosgène. On agite 30 minutes à température ambiante et ajoute 312 $cm_3$ de méthylisopropylamine. On agite 30 minutes à température ambiante, verse sur de l'acide chlorhydrique 0,1N, extrait avec de l'acétate d'éthyle, lave, sèche et évapore à sec. On chromatographie le résidu (642 mg) sur silice (éluant Essence G-acétate d'éthyle 6-4) et obtient 545 mg du produit recherché.
Spectre IR : ($CHCl_3$)

| | |
|---|---|
| C=O (OAC) | 1728 $cm^{-1}$ |
| C=O (amide) | 1672 $cm^{-1}$ |
| C=O (diénone) | 1657 $cm^{-1}$ |
| C=C conjugué | 1601 $cm^{-1}$ |

**STADE B** : Méthyl (1-méthyléthyl) carbamate de 10-(17béta-hydroxy 3-oxo 4,9-dièn-11béta-yl) décyle.

On opère comme au stade B de l'exemple 1 à partir de 293 mg du produit obtenu au stade ci-dessus en utilisant 1 $cm_3$ de soude 2N. Après chromatographie sur silice (éluant : Essence G-acétate d'éthyle 1-1), on obtient 244 mg du produit recherché.
Spectre IR : ($CHCl_3$)

| | |
|---|---|
| carbamate | 1672 $cm^{-1}$ |
| diénone | 1657, 1600 $cm^{-1}$ |
| OH | 3615 $cm^{-1}$ |

| | | | | |
|---|---|---|---|---|
| Analyse | : | $C_{33}H_{53}NO_4$ = 527,79 | | |
| Calculé | : | C% 75,10 | H% 10,12 | N% 2,65 |
| Trouvé | : | 74,9 | 10,1 | 2,7 |

**EXEMPLE 53 : Méthyl (1-méthyléthyl) carbamate de 10-(3,17béta-dihydroxy estra-1,3,5(10)-trièn-11béta-yl) décyle.**

STADE A : Méthyl (1-méthyléthyl) carbamate de 10-(3,17béta-diacétoxy estra-1,3,5(10)-trièn-11béta-yl) décyle.

On opère comme au stade A de l'exemmple 8 à partir de 230 mg du composé obtenu au stade A de l'exemple 52 en utilisant 230 $cm_3$ d'anhydride acétique et 115 $cm_3$ de bromure d'acétyle. On obtient 234 mg du produit attendu.

Spectre IR : ($CHCl_3$)

C=O
- 1730 $cm^{-1}$ (OAC en 17)
- 1750 $cm^{-1}$ (OAC en 3)
- 1680 $cm^{-1}$ (carbamate)

**STADE B : Méthyl (1-méthyléthyl) carbamate de 10-(3,17béta-dihydroxy estra-1,3,5(10)-trièn-11béta-yl) décyle.**

On opère comme à l'exemple 8 au stade B à partir de 260 mg du produit obtenu au stade précédent. Après chromatographie sur silice (éluant : Essence G-acétate d'éthyle 6-4), on obtient 245 mg du produit recherché.

Analyse : $C_{33}H_{53}NO_4$ = 527,79

| | | | |
|---|---|---|---|
| Calculé : | C% 75,10 | H% 10,12 | N% 2,65 |
| Trouvé : | 75,6 | 10,5 | 2,6 |

Spectre IR : ($CHCl_3$)

| | |
|---|---|
| OH | 3606 $cm^{-1}$ |
| >=O | 1672 $cm^{-1}$ |
| aromatique | 1619, 1610, 1583, 1498 $cm^{-1}$ |

**EXEMPLE 54 : 3,17béta-dihydroxy 17alpha-méthyl N-méthyl N-(1-méthyléthyl) estra-1,3,5(10)-trièn-11béta-undecanamide.**

STADE A : 3-hydroxy N-méthyl N-(1-méthyléthyl) 17-oxo-estra-1,3,5(10) -trièn-11béta-undecanamide.

On agite au reflux pendant 30 minutes 1 g du produit obtenu au stade A de l'exemple 15, avec 200 $cm_3$ de méthanol ainsi que 2,2 g d'hydroxyde de palladium sur magnésie, filtre, évapore les solvants sous pression réduite et obtient 985 mg de produit que l'on chromatographie sur silice (éluant : acétonitrile) et obtient 543 mg du produit recherché.

[$alpha_D$] = +121° ± 3° (c = 0,5% éthanol)

Spectre IR : ($CHCl_3$)

| | |
|---|---|
| OH | 3598 $cm^{-1}$ |
| C=O | 1732 $cm^{-1}$ (17-céto)<br>1619 $cm^{-1}$ |
| aromatique | 1582, 1499 $cm^3$ |

STADE B : 3,17béta-dihydroxy N-méthyl N-(1-méthyléthyl) 19-nor-17alpha-pregna-1,3,5(10)-trièn-20-yn-11béta-undecanamide.

A une solution de 0,5 g du produit obtenu au stade A ci-dessus dans 10 $cm_3$ de tétrahydrofuranne anhydre. On ajoute à 20°C 13,7 $cm_3$ de bromure d'éthyle magnésium 0,6M. On agite 2 heures, verse dans une solution

de chlorure d'ammonium, extrait avec du chlorure de méthylène, sèche et évapore à sec sous pression réduite. On chromatographie le résidu (466 mg) sur silice (éluant : chlorure de méthylène-acétone 9-1) et obtient 225 mg du produit attendu. F = 148°C.
[$alpha_D$] = +83° ± 2,5° (c = 0,5% éthanol)
Spectre IR : ($CHCl_3$)

| | |
|---|---|
| OH | 3604 $cm^{-1}$ |
| >C=O | 1617 $cm^{-1}$ (amide III) |
| aromatique | 1582, 1498 $cm^{-1}$ |

| | | | | |
|---|---|---|---|---|
| Analyse : | $C_{32}H_{55}NO_3$ = 525,82 | | | |
| Calculé : | C% 77,66 | H% 10,54 | N% 2,66 |
| Trouvé : | 77,9 | 10,8 | 2,5 |

**EXEMPLE 55 : 3,17béta-dihydroxy N-méthyl N-(1-méthyléthyl) 19-nor-17alpha-pregna-1,3,5(10)-trièn-20-yn-11béta-undecanamide.**

On opère comme au stade C de l'exemple 21 à partir de 0,954 g du produit obtenu au stade A de l'exemple 54 en utilisant 1,470 g de complexe acétylure de lithium-éthylène diamine. On obtient après chromatographie sur silice (éluant : chlorure de méthylène-acétone 9-1) 284 mg du produit recherché.
[$alpha_D$] = +43,5° ± 3° (c = 0,2% éthanol)
Spectre IR : ($CHCl_3$)

| | |
|---|---|
| OH | 3600 $cm^{-1}$ |
| C≡C | 3305 $cm^{-1}$ |
| aromatique | 1582, 1499 $cm^{-1}$ |

| | | | |
|---|---|---|---|
| Analyse : | $C_{35}H_{53}NO_3$ = 535,82 | | |
| Calculé : | C% 78,46 | H% 9,97 | N% 2,61 |
| Trouvé : | 78,5 | 9,9 | 2,4 |

**EXEMPLE 56 : Propanoate de 3-hydroxy 11béta-(11-(méthyl (1-méthyléthyl) amino) 11-oxo undecyl) estra-1,3,5(10)-trièn-17béta-yle.**

STADE A : 3,17béta dipropanoate de 11béta-N-méthyl N-(1-méthyléthyl) estra-1,3,5(10)-trièn-undécanamide.

A une solution de 0,511 g du produit obtenu au stade B de l'exemple 16 dans 5,1 $cm_3$ de pyridine, on ajoute 0,3 $cm_3$ de chlorure de propionyle, agite 1 heure 45 minutes et ajoute 0,1 $cm_3$ de chlorure de propionyle, agite 1 heure 30 minutes puis verse dans 30 g d'eau et glace, extrait avec de l'acétate d'éthyle, lave, sèche et distille à sec sous pression réduite. On obtient 0,848 g de produit que l'on chromatographie sur silice (éluant : cyclohexane-acétate d'éthyle 6-4).

Spectre IR : ($CHCl_3$)
absence d'OH

| | |
|---|---|
| >C=O | 1750 $cm^{-1}$ |
| | 1725 $cm^{-1}$ |
| | 1624 $cm^{-1}$ (amide III) |
| aromatique | 1496 $cm^{-1}$ |

**STADE B** : Propanoate de 3-hydroxy 11béta-(11-(méthyl (1-méthyléthyl) amino) 11-oxo undecyl) estra-1,3,5(10)-trièn-17béta-yle.

A une solution de 0,34 g du produit obtenu au stade A ci-dessus dans 4 $cm_3$ de méthanol, on ajoute 55 mg de bicarbonate de potassium en solution dans 0,4 $cm_3$ d'eau. On agite 22 heures à température ambiante, ajoute de l'eau, extrait avec du chlorure de méthylène, lave, sèche et évapore à sec sous pression réduite. On chromatographie le résidu (0,304 g) sur silice (éluant : cyclohexane-acétate d'éthyle 6-4) et obtient 0,285 g du produit recherché.

Spectre IR : ($CHCl_3$)

| | |
|---|---|
| OH | 3600 $cm^{-1}$ |
| carbamate | 1720 $cm^{-1}$ |
| C=O (amide) | 1617 $cm^{-1}$ |
| aromatique | 1582, 1498 $cm^{-1}$ |

| | | | | |
|---|---|---|---|---|
| Analyse : | $C_{36}H_{57}NO_4$ = 567,86 | | | |
| Calculé : | C% 76,14 | H% 10,12 | N% 2,47 |
| Trouvé : | 76,4 | 10,3 | 2,3 |

**EXEMPLE 57 : N,N-bis (1-méthyléthyl) 17béta-hydroxy 3-oxo-estra-4,9-dièn-11béta-undécanamide.**

**STADE A** : N,N-bis (1-méthyléthyl) 17béta-acétyloxy 3-oxo-estra-4,9-dièn-11béta-undécanamide.

On opère comme au stade A de l'exemple 3 à partir de 1,44 g d'acide 17béta-acétyloxy 3-oxo 11béta-estra-4,9-dièn undécanoïque obtenu au stade E de la préparation 5, en utilisant 2,1 $cm_3$ de diisopropylamine. Après chromatographie sur silice (éluant : cyclohexane-acétate d'éthyle 1-1), on obtient 0,533 g du produit recherché.

Spectre IR : ($CHCl_3$)

| | |
|---|---|
| OAC | 1728 $cm^{-1}$ |
| diénone | 1654 $cm^{-1}$ |

**STADE B** : N,N-bis (1-méthyléthyl) 17béta-hydroxy 3-oxo-estra-4,9-dièn-11béta-undécanamide.

On opère comme au stade B de l'exemple 1 à partir de 248 g du composé obtenu comme au stade A ci-dessus. Après chromatographie sur silice (éluant : cyclohexane-acétate d'éthyle 1-1), on obtient 91 mg de produit recherché.

Spectre IR : ($CHCl_3$)

| | |
|---|---|
| OH | 3618 $cm^{-1}$ |
| >C=O | 1552, 1442 $cm^{-1}$ |
| amide III | 1620 $cm^{-1}$ |

| | | | | |
|---|---|---|---|---|
| Analyse : | $C_{35}H_{57}NO_3$ = 539,85 | | | |
| Calculé : | C% 77,87 | H% 10,64 | N% 2,59 |
| Trouvé : | 77,6 | 10,7 | 2,5 |

**EXEMPLE 58 : N,N-bis (1-méthyléthyl) 3,17béta-dihydroxy estra-1,3,5(10)-trièn-11béta-undécanamide.**

On opère comme à l'exemple 8 à partir de 0,952 g de produit obtenu comme au stade A de l'exemple 57. Après chromatographie sur silice (éluant : cyclohexane-acétate d'éthyle 1-1), on obtient 302 mg de produit recherché.

Spectre IR : ($CHCl_3$)

| | |
|---|---|
| OH | 3607 $cm^{-1}$ |
| C=O | 1617 $cm^{-1}$ (amide III) |
| aromatique | 1582, 1498 $cm^{-1}$ |

**EXEMPLE 59 : 3,17béta-dihydroxy alpha,N-diméthyl N-(1-méthyléthyl) estra 1,3,5(10)-trièn-11béta-undécanamide.**

**STADE A** : 3,17béta-bis (tétrahydropyrannyloxy) N-méthyl N-(1-méthyléthyl) estra 1,3,5(10)-trièn-11béta-undécanamide.

On agite pendant 1 heure un mélange de 1,045 g de produit obtenu au stade B de l'exemple 16, 50 $cm_3$ d'éther, 3,75 $cm_3$ de dihydropyranne et 30 mg d'acide paratoluènesulfonique. On verse ensuite sur 100 $cm_3$ d'une solution saturée de bicarbonate de sodium et extrait à l'éther. On évapore à sec sous pression réduite et obtient 2,216 g de résine que l'on chromatographie sur silice (éluant : chlorure de méthylène-acétone 95-5). On recueille 1,38 g du produit recherché.
Spectre IR : ($CHCl_3$)
absence d'OH
>C=O 1621 $cm^{-1}$ (amide III)
aromatique 1573, 1497 $cm^{-1}$

**STADE B** : 3,17béta-dihydroxy alpha,N-diméthyl N-(1-méthyléthyl) estra 1,3,5(10)-trièn-11béta-undécanamide.

On opère comme au stade B de l'exemple 39 à partir de 435 mg du produit obtenu au stade A ci-dessus. On obtient 430 mg de produit dont on hydrolyse les pyrannyles par agitation dans un mélange de 4 $cm_3$ d'éthanol et 4 $cm_3$ d'acide chlorhydrique 2N. Après concentration au demi sous pression réduite, extraction au chlorure de méthylène, on chromatographie sur silice (éluant : chlorure de méthylène-méthanol 95-5) et obtient 0,178 g de produit recherché.
$[alpha]_D$ = +90° ± 2° (c = 0,15% éthanol)
Spectre IR : ($CHCl_3$)
OH 3605 $cm^{-1}$
C=O 1615 $cm^{-1}$ (amide III)
aromatique 1582, 1498 $cm^{-1}$

**EXEMPLE 60 : 3,17béta-dihydroxy alpha,alpha-diméthyl N-méthyl N-(1-méthyléthyl) estra 1,3,5(10)-trièn-11béta-undécanamide.**

On opère comme au stade C de l'exemple 39 à partir de 210 mg de produit obtenu comme au stade A de l'exemple 59. Après chromatographie sur silice (éluant : chlorure de méthylène-acétone (95-5), on obtient 103 mg du produit recherché.
$[alpha]_D$ = +72° ± 2,5° (c = 0,5% éthanol)
Spectre IR : ($CHCl_3$)
OH 3604 $cm^{-1}$
C=O 1600 $cm^{-1}$
aromatique 1580, 1498 $cm^{-1}$

| Analyse | : $C_{35}H_{57}NO_3$ | | |
|---|---|---|---|
| Calculé | : C% 77,87 | H% 10,64 | N% 2,59 |
| Trouvé | : 77,9 | 10,8 | 2,5 |

**EXEMPLE 61 : 1-(11-(17béta-hydroxy 3-oxo estra-4,9-dièn-11béta-yl) 1-oxo undécyl) 4-méthyl pipérazine.**

**STADE A** : 1-(11-(17béta-acétyloxy 3-oxo estra-4,9-dièn-11béta-yl) 1-oxo undécyl) 4-méthyl pipérazine.

On opère comme au stade A de l'exemple 3 à partir de 3,886 g de l'acide obtenu comme au stade E de la préparation 5 en utilisant 4,33 $cm_3$ de N-méthyl pipérazine. Après chromatographie sur silice (éluant : acétate d'éthyle-méthanol 95-5) puis 90-10), on obtient 3,14 g du produit recherché.
Spectre IR : ($CHCl_3$)
>C=O 1730 $cm^{-1}$

diénone + N-C=O 1646 $cm^{-1}$

**STADE B** : 1-(11-(17béta-hydroxy 3-oxo estra-4,9-dièn-11béta-yl) 1-oxo undécyl) 4-méthyl pipérazine.

On opère comme au stade B de l'exemple 1 à partir de 1,398 g du produit obtenu au stade A ci-dessus. Après chromatographie sur silice (éluant : chlorure de méthylène-méthanol 95-5), on obtient 1,012 g du produit attendu. $[alpha]_D$ = -36° ± 2,5° (c = 0,5% éthanol)
Spectre IR : ($CHCl_3$)
OH 3615 $cm^{-1}$
>C=O 1642 $cm^{-1}$

| | | | | |
|---|---|---|---|---|
| Analyse | : $C_{34}H_{54}N_2O_3$ | : 538,82 | | |
| Calculé | : | C% 75,78 | H% 10,10 | N% 5,20 |
| Trouvé | : | 75,9 | 10,1 | 5,1 |

**EXEMPLE 62 : 4-[11-(3,17béta-dihydroxy estra-1,3,5(10)-trièn-11béta-yl) 1-oxo undécyl] 4-méthyl pipérazine.**

On opère comme à l'exemple 2 à partir de 1,619 g du produit obtenu au stade A de l'exemple 61. Après deux chromatographies successives sur silice (éluant : chlorure de méthylène-méthanol 95-5), on obtient 466 mg du produit recherché.
$[alpha]_D$ = +70,5° ± 2,5° (c = 0,5% éthanol)
Spectre IR : ($CHCl_3$)
OH 3610 $cm^{-1}$
amide III 1622 $cm^{-1}$
aromatique 1582, 1498 $cm^{-1}$

| | | | | |
|---|---|---|---|---|
| Analyse | : $C_{34}H_{54}N_2O_3$ | : 538,82 | | |
| Calculé | : | C% 75,78 | H% 10,10 | N% 5,20 |
| Trouvé | : | 76,0 | 10,0 | 5,1 |

**EXEMPLE 63 : N-(2-chloro 2-méthylpropyl) 17béta-hydroxy 3-oxo-estra-4,9-dièn-11béta-undécanamide.**

**STADE A** : N-(2-chloro 2-méthylpropyl) 17béta-acétyloxy 3-oxo-estra-4,9-dièn-11béta-undécanamide.

On opère comme au stade A de l'exemple 3 à partir de 2,35 g du composé obtenu au stade E de la préparation 5 en utilisant 2,5 $cm_3$ de 2,2-diméthylaziridine. Après chromatographie sur silice (éluant : chlorure de méthylène-acétone 92,5-7,5), on obtient 2,08 g de produit.
Spectre IR : ($CHCl_3$)
=C-NH 3445 $cm^{-1}$
OAC 1728 $cm^{-1}$
diénone + amide II 1657 $cm^{-1}$
amide II 1517 $cm^{-1}$

**STADE B** : N-(2-chloro 2-méthylpropyl) 17béta-hydroxy 3-oxo-estra-4,9-dièn-11béta-undécanamide.

On opère comme au stade B de l'exemple 1 à partir de 720 mg du produit obtenu au stade A ci-dessus. Après chromatographie sur silice (éluant : cyclohexane-acétate d'éthyle 1-1), on obtient 420 mg de produit recherché.
$[alpha]_D$ = -38° ± 1° (c = 0,7% éthanol)
Spectre IR : ($CHCl_3$)
OH 3610 $cm^{-1}$
=C-NH 3444 $cm^{-1}$

| | |
|---|---|
| ›C=O | 1657 $cm^{-1}$ (amide II + diénone) |
| amide II | 1517 $cm^{-1}$ |

**EXEMPLE 64 : N-(2-chloro 2-méthylpropyl) 3,17béta-dihydroxy-estra-1,3,5(10)-trièn-11béta-undécanamide.**

STADE A : N-(2-chloro 2-méthylpropyl) 3,17béta-diacétyloxy-estra-1,3,5(10)-trièn-11béta-undécanamide.

On opère comme au stade A de l'exemple 8 à partir de 970 mg du produit obtenu à l'exemple 63 en utilisant 1 $cm_3$ de bromure d'acétyle et 2 $cm_3$ d'anhydride acétique. Après chromatographie sur silice (éluant : cyclohexane-acétate d'éthyle 7-3), on obtient 650 mg de produit recherché.

Spectre IR : ($CHCl_3$)

| | |
|---|---|
| NH | 3445 $cm^{-1}$ |
| OAC | 1748, 1727 $cm^{-1}$ |
| ›C=O | 1668 $cm^{-1}$ |
| amide II | 1516 $cm^{-1}$ |
| aromatique | 1614, 1580, 1494 $cm^{-1}$ |

STADE B : N-(2-chloro 2-méthylpropyl) 3,17béta-dihydroxy-estra-1,3,5(10)-trièn-11béta-undécanamide.

On opère comme au stade B de l'exemple 8 à partir de 640 mg du produit obtenu au stade A ci-dessus. Après chromatographie sur silice (éluant : cyclohexane-acétate d'éthyle 6-4), on obtient 512 mg de produit attendu.

$[alpha]_D$ = +83° ± 3° (c = 0,5% éthanol)

Spectre IR : ($CHCl_3$)

| | |
|---|---|
| OH | 3604 $cm^{-1}$ |
| =C-NH | 3441 $cm^{-1}$ |
| ›C=O | 1665 $cm^{-1}$ |
| amide II + aromatique | 1619, 1610, 1521, 1499 $cm^{-1}$ |

**EXEMPLE 65 : 3,17béta-dihydroxy N-méthoxy N-méthyl-estra-1,3,5(10)-trièn-11béta-undécanamide.**

STADE A : 17béta-acétyloxy N-méthoxy N-méthyl-3-oxo estra-4,9-dièn-11béta-undécanamide.

On opère comme au stade A de l'exemple 3 à partir de 1,93 g du produit obtenu comme au stade E de la préparation 5 en utilisant 1,3 $cm_3$ de N-O-diméthylhydroxylamine. Après chromatographie sur silice (éluant : cyclohexane-acétate d'éthyle 1-1), on obtient 1,867 g du produit attendu.

Spectre IR : ($CHCl_3$)

| | |
|---|---|
| OAC | 1728 $cm^{-1}$ |
| cétone conjuguée + amide III | 1653 $cm^{-1}$ |
| C=C | 1601 $cm^{-1}$ |

STADE B : 3,17bis-(acétyloxy) N-méthoxy N-méthyl-estra-1,3,5(10)-trièn-11béta-undécanamide.

On opère comme au stade A de l'exemple 8 à partir de 935 mg du produit obtenu ci-dessus en utilisant

0,75 $cm_3$ de bromure d'acétyle et 1,5 $cm_3$ d'anhydride acétique. Après chromatographie sur silice (éluant : cyclohexane-acétate d'éthyle 7-3), on obtient 898 mg du produit recherché.

Spectre IR : ($CHCl_3$)

| | |
|---|---|
| acétate | 1727, 1746 $cm^{-1}$ |
| amide III | 1647 $cm^{-1}$ |
| aromatique | 1583, 1494 $cm^{-1}$ |

**STADE C** : 3,17béta-dihydroxy N-méthoxy N-méthyl-estra-1,3,5(10)-trièn-11béta-undécanamide.

On opère comme au stade B de l'exemple 8 à partir de 850 mg de produit obtenu au stade B ci-dessus. Après chromatographie sur silice (éluant : cyclohexane-acétate d'éthyle 6-4), on obtient 630 mg du produit recherché.

$[alpha]_D$ = +92° ± 2° (c = 1% éthanol)

Spectre IR : ($CHCl_3$)

| | |
|---|---|
| OR | 3604 $cm^{-1}$ |
| amide III | 1643 $cm^{-1}$ |
| aromatique | 1583, 1498 $cm^{-1}$ |

Analyse : $C_{31}H_{49}NO_4$ : 499,74

| | | | |
|---|---|---|---|
| Calculé : | C% 74,51 | H% 9,88 | N% 2,80 |
| Trouvé : | 74,4 | 10,1 | 2,7 |

**EXEMPLE 66 : 17béta-hydroxy N-méthoxy N-méthyl-3-oxo estra-4,9-dièn-11béta-undécanamide.**

On opère comme au stade B de l'exemple 1 à partir de 770 mg du produit obtenu au stade A de l'exemple 65. Après chromatographie sur silice (éluant : cyclohexane-acétate d'éthyle 1-1), on obtient 690 mg du produit recherché.

$[alpha]_D$ = -38° ± 1° (c = 0,65% éthanol)

Spectre IR : ($CHCl_3$)

| | |
|---|---|
| OH | 3614 $cm^{-1}$ |
| diénone + amide III | 1652 $cm^{-1}$ |
| C=C | 1601 $cm^{-1}$ |

Analyse : $C_{31}H_{49}NO_4$ : 499,74

| | | | |
|---|---|---|---|
| Calculé : | C% 74,51 | H% 9,88 | N% 2,80 |
| Trouvé : | 74,5 | 9,8 | 2,6 |

**PREPARATION DE L'EXEMPLE 67 : 17béta-acétyloxy 11béta-(11-hydroxy indécyl) estra-4,9-dièn-3-one.**

On opère comme à la préparation 5 (stades B, C, D) à partir de 5,8 g de produit obtenu au stade A de la préparation 8. Après chromatographie sur silice (éluant : cyclohexane-acétate d'éthyle 6-4), on obtient 2,847 g du produit attendu.

Spectre IR : ($CHCl_3$)

| | |
|---|---|
| OH | 3624 $cm^{-1}$ |
| C=O | 1730 $cm^{-1}$ (OAC) |
| diénone | 1654, 1601 $cm^{-1}$ |

**EXEMPLE 67 : 17béta-hydroxy N-méthyl N-(1-méthyléthyl) 3-oxo-estra-4,9-dièn-11béta-dodécanamide.**

**STADE A** : 17béta-acétyloxy N-méthyl N-(1-méthyléthyl) 3-oxo-estra-4,9-dièn-11béta-dodécanamide.

On opère comme au stade A de l'exemple 1 à partir de 2,847 g de produit obtenu à la préparation ci-dessus

en utilisant 4,8 $cm_3$ de réactif d'Heilbron-Jones. On obtient 2,787 g d'acide intermédiaire. On opère l'amidification à partir de 2 g de cet acide brut en utilisant 1,6 $cm_3$ de N-méthyl isopropylamine. Après chromatographie sur silice (éluant : chlorure de méthylène-acétone 95-5), on obtient 1,8 g de produit recherché.

Spectre IR : ($CHCl_3$)

| | |
|---|---|
| C=O | 1728 $cm^{-1}$ (OAC) |
| | 1653 $cm^{-1}$ (diénone) |
| C=O | 1621 $cm^{-1}$ (amide III) |

**STADE B** : 17béta-hydroxy N-méthyl N-(1-méthyléthyl) 3-oxo-estra-4,9-dièn-11béta-dodécanamide.

On opère comme au stade B de l'exemple 1 à partir de 545 mg du produit obtenu ci-dessus. Après chromatographie sur silice (éluant : toluène-triéthylamine 95-5), on obtient 420 mg de produit attendu.
$[alpha]_D$ = -25° ± 2° (c = 0,5% éthanol)

Spectre IR : ($CHCl_3$)

| | |
|---|---|
| OH | 3613 $cm^{-1}$ |
| C=O | 1643 $cm^{-1}$ (diénone) |
| | 1621 $cm^{-1}$ (amide III) |

Analyse : $C_{34}H_{55}NO_3$ : 525,82

| | | | |
|---|---|---|---|
| Calculé : | C% 77,46 | H% 10,54 | N% 2,66 |
| Trouvé : | 77,4 | 10,6 | 2,5 |

**EXEMPLE 68 : 3,17béta-dihydroxy N-méthyl N-(1-méthyléthyl) estra-1,3,5(10)-trièn-11béta-dodécanamide.**

On opère comme à l'exemple 2 à partir de 1,276 g du produit obtenu au stade A de l'exemple 67. Après chromatographie sur silice (éluant : toluène-triéthylamine 9-1), on obtient 735 mg de produit recherché.
$[alpha]_D$ = +86,5° ± 2,5° (c = 0,6% éthanol)
Spectre IR : ($CHCl_3$)

| | |
|---|---|
| OH | 3605 $cm^{-1}$ |
| C=O | 1617 $cm^{-1}$ |
| aromatique | 1582, 1498 $cm^{-1}$ |

**EXEMPLE 69 : (E) 12-(3,17béta-dihydroxy N-méthyl N-(1-méthyléthyl) estra-1,3,5(10)-trièn-11béta-yl) 2-dodécénamide.**

**STADE A** : 10-(17béta-acétoxy 3-oxo estra-4,9-dièn-11béta-yl) décanal.

A une solution de 0,18 $cm^3$ de chlorure d'oxalyle et 5 $cm^3$ de chlorure de méthylène, refroidie à -70°C, on ajoute goutte à goutte une solution de 0,29 $cm^3$ de diméthylformamide dans 5 $cm^3$ de chlorure de méthylène. On agite 15 minutes à -60/-70°C et ajoute 650 mg du produit obtenu à la préparation de l'exemple 50, en solution dans 5 $cm^3$ de chlorure de méthylène. On agite pendant 30 minutes à -60°C et ajoute 2 $cm^3$ de triéthylamine. On amène à température ambiante, dilue à l'eau, extrait avec du chlorure de méthylène, lave à l'eau, sèche et évapore à sec sous pression réduite. On chromatographie sur silice (éluant : cyclohexane-acétate d'éthyle 7-3) et obtient 580 mg de produit recherché.
Spectre IR : ($CHCl_3$)

| | |
|---|---|
| C=O aldéhyde + acétate | 1723 $cm^{-1}$ |
| diénone | 1655, 1601 $cm^{-1}$ |

STADE B : (E) 12-(17béta-acétoxy N-méthyl N-(1-méthyléthyl) 3-oxo estra-4,9-dièn-11béta-yl) 2-dodécénamide.

Préparation du phosphorane.

A une solution de 1 $cm^3$ de N-méthyl N-isopropyl bromacétamide dans 20 $cm^3$ d'éther, on ajoute 2 g de triphénylphosphine. On agite 3 heures, sépare le précipité par décantation, l'empâte dans l'éther, le sépare à nouveau par décantation, le reprend à l'eau. On lave la solution obtenue avec de l'éther et alcalinise avec de la soude 2N, extrait à l'éther, sèche et évapore à sec sous pression réduite. On obtient 980 mg de phosphorane attendu. Des liqueurs éthérées, on récupère encore 1,190 g de phosphorane supplémentaire.

Condensation.

A une solution de l'aldéhyde obtenu au stade A ci-dessus dans 5 $cm^3$ de tétrahydrofuranne, on ajoute une solution de 653 mg de phosphorane, obtenu précédemment, dans 6 $cm^3$ de tétrahydrofuranne. On laisse 43 heures à température ambiante, évapore le solvant et chromatographie sur silice (éluant : cyclohexane-acétate d'éthyle 1-1). On obtient 580 mg du produit recherché.
Spectre IR : ($CHCl_3$)

| | |
|---|---|
| OAC | 1728 $cm^{-1}$ |
| diénone | 1655 $cm^{-1}$ |
| amide conjuguée | 1600 $cm^{-1}$ |

STADE C : (E) 12-(3,17béta-dihydroxy N-méthyl N-(1-méthyléthyl) estra-1,3,5(10)-trièn-11béta-yl) 2-dodécenamide.

On opère comme à l'exemple 8 à partir de 580 mg du composé obtenu au stade B. Après chromatographie sur silice (éluant : acétate d'éthyle-cyclohexane 1-1), on obtient 430 mg de produit recherché.
Spectre IR : ($CHCl_3$)

| | |
|---|---|
| OH | 3604 $cm^{-1}$ |
| amide III conjugé | 1655 , 1597 $cm^{-1}$ |
| bandes aromatiques | 1498 $cm^{-1}$ |

Analyse : $C_{34}H_{53}NO_3$ : 523,81

| | | | |
|---|---|---|---|
| Calculé : | C% 77,96 | H% 10,19 | N% 2,67 |
| Trouvé : | 77,8 | 10,3 | 2,4 |

**PREPARATION DE L'EXEMPLE 70 : 3-(1,2-éthanediyl acétal cyclique) de 5alpha-hydroxy 11béta-(12-hydroxy dodécyl) estr-9-èn-3,17-dione.**

On agite pendant 3 heures 4,2 g du composé obtenu au stade A de la préparation 8 avec 29 $cm^3$ d'une solution 1M de fluorure de tétrabutylammonium. On ajoute 200 $cm^3$ d'une solution de bicarbonate de sodium, agite 30 minutes puis extrait avec du chlorure de méthylène. On évapore à sec et chromatographie le résidu (6,27 g) sur silice (éluant : chlorure de méthylène-acétone 9-1). On obtient 2,43 g du produit recherché.
Spectre IR : ($CHCl_3$)

| | |
|---|---|
| OH | 3602 $cm^{-1}$ |
| C=O | 1733 $cm^{-1}$ |

**EXEMPLE 70 : 3,17béta-dihydroxy N-méthyl N-(1-méthyléthyl) estra-1,3,5(10)-trièn-11béta-tridécanamide.**

STADE A : (5alpha, 11béta)-3-(1,2-éthanediyl acétal cyclique) de 5-hydroxy 11-[12-[phénylméthyl sulfonyloxy] dodécyl] estr-9-èn-3,17-dione.

On opère comme au stade A de l'exemple 40 à partir de 3,65 g du composé obtenu à la préparation ci-dessus en utilisant 5 g de chlorure de paratoluènesulfonyle. Après chromatographie sur silice (éluant : chlorure de méthylène-acétone 9-1), on obtient 3,54 g de produit attendu utilisé tel quel pour le stade suivant.

**STADE B** : (5alpha, 11béta)-3-(1,2-éthanediyl acétal cyclique) de 5-hydroxy 11-[12-[iodo] dodécyl] estr-9-èn-3,17-dione.

On opère comme au stade B de l'exemple 40 à partir de 3,5 g du produit obtenu au stade A en utilisant 1,17 g d'iodure de sodium. On obtient 4,199 g du produit recherché.
Spectre IR : ($CHCl_3$)
OH 3506 $cm^{-1}$
C=O 1733 $cm^{-1}$

**STADE C** : 13-[(5alpha, 11béta)-3-(1,2-éthanediyl acétal cyclique) de 5-hydroxy 11béta-yl] tridécane nitrile.

On opère comme au stade C de l'exemple 40 à partir de 4,19 g du composé obtenu au stade B ci-dessus en utilisant 1,1 g de cyanure de potassium.) Après chromatographie sur silice (éluant : cyclohexane-acétate d'éthyle 6-4), on obtient 2,423 g du produit attendu.
Spectre IR : ($CHCl_3$)
OH 3504 $cm^{-1}$
C≡N 2388 $cm^{-1}$
C=O 1733 $cm^{-1}$

**STADE D** : 13-[11béta-yl estra-4,9-dièn-3,17-dione] tridécane nitrile.

On opère comme au stade B de la préparation 2 à partir de 2,4 g du produit obtenu au stade C ci-dessus. On obtient 2,11 g du composé recherché.

**STADE E** : 13-[3-acétyloxy 17-oxo 1,3,5(10)-trièn-11béta-yl] tridécanediyl.

On opère comme au stade B de l'exemple 3 à partir de 2,04 g du produit obtenu au stade D précédent. Après chromatographie sur silice (éluant : cyclohexane-acétate d'éthyle 5-5), on obtient 2 fractions A = 1,1 g et 8 = 0,817 g de produit recherché.

Spectre IR : ($CHCl_3$) sur fraction A
C≡N 2240 $cm^{-1}$
C=O 1735 $cm^{-1}$
1758 $cm^{-1}$ (ép.)
aromatique 1607, 1581, 1493 $cm^{-1}$

**STADE F** : 3-isobutylcarbonyloxy N-butyl N-méthyl 17-oxo estra 1,3,5(10)-trièn-11béta tridécanamide.

On opère comme au stade E de l'exemple 40 à partir de 0,817 g du produit B obtenu au stade précédent en utilisant 0,54 $cm^3$ de N-méthylisobutylamine. Après chromatographie sur silice (éluant : cyclohexane-acétate d'éthyle 7-3), on obtient 603 mg du produit recherché.
Spectre IR : ($CHCl_3$)
O=C 1753, 1735, 1621 $cm^{-1}$

**STADE G** : 3-isobutylcarbonyloxy 17béta-hydroxy N-butyl N-méthyl 1,3,5(10)-trièn-11béta tridécanamide.

On opère comme en a) du stade F de l'exemple 40 à partir de 573 mg du composé obtenu au stade F ci-dessus en utilisant 406 mg de triterbutoxy alumino hydrure. Après chromatographie sur silice (éluant : cyclohexane-acétate d'éthyle 7-3), on obtient 325 mg de produit recherché.
Spectre IR : ($CHCl_3$)
O=C 1756 $cm^{-1}$
amide III 1621 $cm^{-1}$
aromatique 1494 $cm^{-1}$

**STADE H** : 3,17béta-dihydroxy N-méthyl N-(1-méthyléthyl) estra-1,3,5(10)-trièn-11béta-tridécanamide.

On opère comme au stade B de l'exemple 2 à partir de 283 mg du produit obtenu au stade G ci-dessus. Après chromatographie sur silice (éluant : cyclohexane-acétate d'éthyle 4-6), on obtient 215 mg du produit attendu.
$[alpha]_D = -17° \pm 1°$ (c = 1% éthanol)

| | | | | |
|---|---|---|---|---|
| Analyse : | $C_{35}H_{57}NO_3$ : 539,85 | | | |
| Calculé : | C% 79,38 | H% 9,54 | N% 2,50 | |
| Trouvé : | 79,3 | 9,7 | 2,5 | |

**PREPARATION A DE L'EXEMPLE 71 : [(8-bromooctyl) oxy] diméthyl (1,1-diméthyléthyl) silane.**

On opère comme à la préparation 13 à partir de 3,97 g de 8-bromooctanol, 19 cm³ de diméthylformamide, 1,55 g d'imidazole et 3,32 g de diméthyl tertbutyl chlorosilane. Après chromatographie sur silice (éluant : cyclohexane-toluène 8-2), on obtient 5,4 g du produit attendu.

**PREPARATION B DE L'EXEMPLE 71 : Bromo N-méthyl N-(1-méthyléthyl) acétamide.**

A une solution de 10 cm³ de bromure d'acétyle dans 150 cm³ d'éther refroidie à -20°C, on ajoute une solution de 26 cm³ de méthylisopropylamine dans 100 cm³ d'éther. On laisse revenir à 20°C et agite 30 minutes à 20°C. On dilue à l'eau, décante, extrait à l'éther, sèche et sépare par distillation. On obtient 13 g de produit attendu. Eb : 71/72° sous 1 mmHg.

**PREPARATION C DE L'EXEMPLE 71 : 11béta-(8-hydroxyéthyl) estra-4,9-dièn-3;17-dione.**

**STADE A** : 3-(1,2-éthanediyl) acétal cyclique de 11béta-[8-(diméthyléthyl) (1,1-diméthyléthyl) silyloxy] octyl 5alpha-hydroxy estr-9-èn-3,17-dione.

On opère comme au stade A de la préparation 1 à partir de 3,96 g de 3-(1,2-éthanediyl acétal cyclique) de 5alpha, 10alpha-époxy estr-9,11-èn-3,17-dione obtenu selon EP 0057115 (ex. 7) en utilisant 5,4 g de [(8-bromo octyl) oxy] diméthyl (1,1-diméthyléthyl) silane (préparation A de l'exemple 71), 1 g de magnésium en tournures et 0,4 g de chlorure de cuivre. Après chromatographie sur Lichrosorb Rp18 (éluant : méthanol-eau 9-1), on obtient 3,85 g du composé attendu utilisé tel quel pour le stade suivant.

**STADE B** : 11béta-(8-hydroxyoctyl) estra-4,9-dièn-3,17-dione.

On opère comme au stade A de la préparation 6 à partir de 1,77 g du produit obtenu ci-dessus. Après chromatographie sur silice (éluant : chlorure de méthylène-acétate d'éthyle 1-1), on obtient 1,08 g du composé recherché.

| | |
|---|---|
| Spectre IR : ($CHCl_3$) | |
| OH | 3624 $cm^{-1}$ |
| C=O | 1735 $cm^{-1}$ (17-céto) |
| | 1656, 1602 $cm^{-1}$ (diénone) |

**EXEMPLE 71 : [[8-(3,17béta-dihydroxy estra-1,3,5(10)-trièn-11béta-yl) octyl] oxy] N-méthyl N-(1-méthyléthyl) acétamide.**

**STADE A** : [[8-(3,17-dioxo estra-4,9-dièn-11béta-yl) octyl] oxy] N-méthyl N-(1-méthyléthyl) acétamide.

A une solution de 570 mg du composé obtenu à la préparation ci-dessus dans 10 cm³ de tétrahydrofuranne, on ajoute 1,4 cm³ de bromo-N-méthyl N-(1-méthyléthyl) acétamide (obtenu à la préparation B de l'exemple 71 et 285 mg d'iodure de sodium puis 140 mg d'hydrure de sodium à 50% dans l'huile. On agite 1 heure, verse

dans une solution d'acide chlorhydrique N à 0°C et extrait avec du chlorure de méthylène. On évapore à sec et chromatographie le résidu (2 g) sur silice (éluant acétate d'éthyle-éther 8-2). On obtient 350 mg du produit recherché.

Spectre IR : ($CHCl_3$)

| | |
|---|---|
| 17-céto | 1736 $cm^{-1}$ |
| 3-céto | 1655 $cm^{-1}$ |
| amide III | 1628 $cm^{-1}$ |
| C=C | 1603 $cm^{-1}$ |

**STADE B : [[8-(3,17béta-dihydroxy estra-1,3,5(10)-trièn-11béta-yl) octyl] oxy] N-méthyl N-(1-méthyléthyl) acétamide.**

On opère comme aux stades B et C de l'exemple 3 à partir de 850 mg du produit obtenu au stade A ci-dessus. Après chromatographie sur silice (éluant : acétate d'éthyle-hexane 75-25), on obtient 442 mg de produit que l'on recristallise dans l'acétate d'éthyle. On recueille ainsi 402 mg de produit recherché. F = 126°C.

Spectre IR : ($CHCl_3$)

| | |
|---|---|
| OH | 3605 $cm^{-1}$ |
| C=O | 1626 $cm^{-1}$ |
| aromatique | 1583, 1498 $cm^{-1}$ |

| Analyse : $C_{32}H_{51}NO_4$ : 513,77 | | | |
|---|---|---|---|
| Calculé : | C% 74,81 | H% 10,0 | N% 2,72 |
| Trouvé : | 74,5 | 9,8 | 2,5 |

**PREPARATION DE L'EXEMPLE 72 :**

**STADE A :** (1,2-éthanediyl acétal cyclique) de 5alpha, 17béta-dihydroxy 11béta-[11-[diméthyl (1,1-diméthyléthyl) silyl] oxy] undécyl 17alpha-pregna-9-èn-20-yne.

On opère comme au stade C de l'exemple 21 à partir de 4 g du produit obtenu au stade A de la préparation 5 en utilisant 80 $cm^3$ d'éthylènediamine et 5,97 g de complexe acétylure de lithium-éthylènediamine. Après chromatographie sur silice (éluant : chlorure de méthylène-acétone 95-5), on obtient 2,024 g de produit recherché.

Spectre IR : ($CHCl_3$)

| | |
|---|---|
| OH en 5 et 17 | 3600, 3500 $cm^{-1}$ |
| C≡C | 3305 $cm^{-1}$ |
| bande OSi | 836 $cm^{-1}$ |

**STADE B :** 17béta-hydroxy 11béta-(11-hydroxyundécyl) 17 alpha-pregna-4,9-dièn-20-yn-3-one.

On opère comme au stade A de la préparation 6 à partir de 1,05 g de produit obtenu au stade A ci-dessus. Après chromatographie sur silice (éluant : chlorure de méthylène-acétone 9-1), on obtient 0,712 g de produit recherché.

Spectre IR : ($CHCl_3$)

| | |
|---|---|
| OH | 3608 $cm^{-1}$ |
| C≡CH | 3304 $cm^{-1}$ |
| diénone | 1652, 1598 $cm^{-1}$ |

**EXEMPLE 72 : 17béta-hydroxy N-méthyl N-(1-méthyléthyl) 3-oxo 17alpha-pregna-4,9-dièn-20-yn-11béta-undécanamide.**

On opère comme au stade A de l'exemple 1 à partir de 1,3 g du produit obtenu au stade B de la préparation ci-dessus en utilisant 2,5 $cm^3$ de réactif Heilbron-Jones, 3,11 g de carbonate de baryum puis 0,9 $cm^3$ d'isopropylméthylamine. Après chromatographie sur silice (éluant : chlorure de méthylène-acétone 9-1), on obtient 0,336 g du produit recherché.

Spectre IR : ($CHCl_3$)

OH 3600 $cm^{-1}$
C≡CH 3305 $cm^{-1}$
C=O 1651, 1621 $cm^{-1}$

| | | | | |
|---|---|---|---|---|
| Analyse : | $C_{35}H_{53}NO_3$ : 535,82 | | | |
| Calculé : | C% 78,46 | H% 9,97 | N% 2,61 |
| Trouvé : | 78,5 | 9,9 | 2,4 |

**EXEMPLE 73 : N-butyl 17béta-hydroxy 3-méthoxy N-méthyl estra-1,3,5(10)-trièn-11béta-undécanamide.**

A une solution de 194 mg du produit obtenu à l'exemple 13 dans 3 $cm^3$ d'acétone, on ajoute 0,2 $cm^2$ de soude 2N. On agite 10 minutes à température ambiante puis on ajoute 0,5 $cm^3$ d'une solution acétonique de sulfate diméthylique préparée à partir de 0,3 $cm^3$ de sulfate diméthylique et q.s.p. d'acétone pour 10 $cm^3$. On agite 10 minutes à température ambiante et ajoute 0,5 $cm^3$ de la solution de sulfate diméthylique. Après 20 minutes, on ajoute 0,2 $cm^3$ de soude 2N puis 1 $cm^3$ de solution de sulfate diméthylique. Après 20 minutes, on ajoute encore 0,2 $cm^3$ de soude 2N puis 1 $cm^3$ de solution de sulfate diméthylique. On agite encore 30 minutes, dilue avec une solution de chlorure d'ammonium et extrait avec de l'acétate d'éthyle. On évapore à sec et chromatographie le résidu (220 mg) sur silice (éluant : cyclohexane-acétate d'éthyle 1-1) et obtient 190 mg de produit recherché.
Spectre IR : ($CHCl_3$)
OH 3612 $cm^{-1}$
C=O 1626 $cm^{-1}$
aromatique 1574, 1500 $cm^{-1}$

| | | | |
|---|---|---|---|
| Analyse : | $C_{35}H_{57}NO_3$ : 539,85 | | |
| Calculé : | C% 77,87 | H% 10,64 | N% 2,59 |
| Trouvé : | 77,7 | 10,8 | 2,5 |

**EXEMPLE 74 : N-butyl 4-(17béta-hydroxy 3-méthoxy estra-1,3,5(10)-trièn-11-béta-yl) N-méthyl benzène octanamide.**

A une solution de 862 mg du produit obtenu à l'exemple 8 dans 8,6 $cm^3$ d'hexaméthylphosphotriamide, on ajoute 1,54 $cm^3$ de soude N puis goutte à goutte 218 mg d'iodure de méthyle. On agite 1 heure à température ambiante, ajoute 1,5 $cm^3$ de soude N, dilue à l'eau, extrait avec de l'acétate d'éthyle, lave, sèche, et amène à sec sous pression réduite. On recueille 1,017 g de produit que l'on chromatographie sur silice (éluant : toluène-triéthylamine 9-1) et obtient 592 mg de produit recherché.
$[alpha]_D = -38,5° \pm 2,5°$ (c = 0,5% éthanol)
Spectre IR : ($CHCl_3$)
OH 3610 $cm^{-1}$
C=O 1626 $cm^{-1}$
aromatique 1570, 1501 $cm^{-1}$

| | | | |
|---|---|---|---|
| Analyse : | $C_{38}H_{55}NO_3$ : 573,87 | | |
| Calculé : | C% 79,53 | H% 9,66 | N% 2,43 |
| Trouvé : | 79,3 | 9,9 | 2,4 |

**PREPARATION DE L'EXEMPLE 75 : Chlorhydrate de N-heptafluorobutyl N-méthylamine.**

On refroidit à 0°C 100 $cm^3$ d'éther anhydre, 100 $cm^3$ de tétrahydrofuranne anhydre puis fait barboter, pendant 10 minutes, de la méthylamine. On introduit en une demi-heure 44,98 g d'anhydride heptafluorobutyrique en maintenant un faible barbottage de méthylamine. On agite pendant 2 heures en laissant revenir à température ambiante. On distille à faible volume sous pression réduite, reprend par 200 cm³ de tétrahydrofuranne anhydre et introduit lentement 30 cm³ de complexe diborane-diméthyl sulfure. On porte à reflux pendant 16 heures. On refroidit à température ambiante puis introduit lentement 200 cm³ de méthanol. On fait ensuite barboter de l'acide chlorhydrique gazeux pendant 15 minutes. On porte à reflux pendant 1 heure puis distille les solvants sous pression réduite. On reprend le résidu par 200 cm³ de méthanol. On fait de nouveaux barbotter pendant 10 minutes de l'acide chlorhydrique gazeux puis porte à reflux pendant 2 heures. On distille le solvant, agite 10 minutes dans 100 cm³ d'acide chlorhydrique 6N glacé. On esore, lave à l'acide chlorhydrique 2N, sèche et obtient 22,699 g de produit attendu. Le chlorhydrate obtenu ci-dessus est purifié par cristallisation dans 140 cm³ d'éthanol. On ajoute alors 140 cm³ d'éther, agite une demi-heure, essore, lave à l'éther et sèche sous pression réduite. On obtient 21,7 g de produit recherché (sublime à environ 200°C).

Analyse : $C_5H_6F_7N$, HCl : 249,56

| | | | | | |
|---|---|---|---|---|---|
| Calculé : | C% 24,06 | H% 2,83 | Cl% 14,20 | F% 53,29 | N% 5,61 |
| Trouvé : | 24,0 | 2,8 | 14,4 | 52,3-52,1 | 5,6 |

**EXEMPLE 75 : 3,17béta-dihydroxy N-(2,2,3,3,4,4,4-heptafluorobutyl) N-méthyl estra-1,3,5(10)-trièn-11béta-undécanamide.**

STADE A : 3,17-dioxo N-(2,2,3,3,4,4,4-heptafluorobutyl) N-méthyl estra-4,9-dièn-11béta-undécanamide.

On opère comme au stade A de l'exemple 3 à partir de 500 mg du composé obtenu au stade B de la préparation 6 en utilisant 0,370 cm³ de N-méthylmorpholine, 0,173 cm³ de chloroformiate d'isobutyle et 412 mg de chlorhydrate d'heptafluorobutylméthylamine (préparation de l'exemple 75). Après chromatographie sur silice (éluant : essence G-acétate d'éthyle 6-4, acétate d'éthyl pur, puis acétate d'éthyle à 1% d'acide acétique), on obtient 180 mg du produit recherché.

Spectre IR : ($CHCl_3$)

| | |
|---|---|
| C=O | 1736 $cm^{-1}$ (17-céto) |
| | 1657 $cm^{-1}$ (diénone + amide III) |
| C=C | 1602 $cm^{-1}$ |

STADE B : 3-acétoxy 17-oxo N-(2,2,3,3,4,4,4-heptafluorobutyl) N-méthyl estra-1,3,5(10)-trièn-11béta-undécanamide.

On opère comme au stade B de l'exemple 3 à partir de 345 mg du produit obtenu au stade A ci-dessus en utilisant 0,4 cm³ d'anhydride acétique et 0,2 cm³ de bromure d'acétyle. On obtient 323 mg de produit attendu.

STADE C: 3,17béta-dihydroxy N-(2,2,3,3,4,4,4-heptafluorobutyl) N-méthyl estra-1,3,5(10)-trièn-11béta-undécanamide.

On opère comme au stade C de l'exemple 3 à partir de 323 mg du produit obtenu ci-dessus en utilisant 20 mg d'hydrure de bore et de sodium et 0,3 cm³ de soude 2N. Après chromatographie sur silice (éluant : essence G-acétate d'éthyle 1-1), on obtient 230 mg du produit attendu.

Spectre IR : ($CHCl_3$)

| | |
|---|---|
| OH | 3604 $cm^{-1}$ |
| C=O | 1655 $cm^{-1}$ |
| aromatique | 1610, 1584, 1498 $cm^{-1}$ |

Analyse : $C_{34}H_{48}F_7NO_3$ : 651,76

| | | | | |
|---|---|---|---|---|
| Calculé : | C% 62,66 | H% 7,42 | N% 2,15 | F% 20,4 |
| Trouvé : | 62,7 | 7,6 | 2,0 | 20,04 |

**EXEMPLE 76 : N-butyl (5-(4-(3,17béta-dihydroxy 19-nor 17alpha-pregna 1,3,5(10)-trièn-20-yn-11béta-yl) phénoxy) pentylthio) N-méthyl acétamide.**

On opère comme à l'exemple 43 à partir de 11béta-4-(hydroxyphényl) estra-4,9-dièn-3,17-dione et de (5-bromo-pentyl) thio N-butyl N-méthyl acétamide pour obtenir le N-butyl-(5-(4-(3-hydroxy 17-oxo estra-1,3,5(10)-trièn-11béta-yl) phénoxy) pentylthio) N-méthyl acétamide. L'addition d'acétylène en présence de tert-butylate de potassium conduit au produit attendu.

$[alpha]_D$ = -98,5° (c = 1% $CHCl_3$)

Spectre IR : ($CHCl_3$)

| | |
|---|---|
| OH | 3599 $cm^{-1}$ |
| éthynyle | 3305 $cm^{-1}$ |
| >C=O | 1628 $cm^{-1}$ |
| aromatique | 1581, 1512 $cm^{-1}$ |

Ultra violet (EtOH)

| | |
|---|---|
| max 281 nm | epsilon = 4000 |
| max 287 nm | epsilon = 3800 |

(EtOH + NaOH N/10)

| | |
|---|---|
| max 280 nm | epsilon = 3300 |
| max 287 nm | epsilon = 3500 |
| max 300 nm | epsilon = 3200 |

**EXEMPLE 77 : N-butyl (5-(4-(3,17béta-dihydroxy estra-1,3,5(10)-trièn-11béta-yl) phénoxy) pentylthio) N-méthyl acétamide.**

On réduit à l'aide de borhydrure de sodium dans le méthanol le N-butyl (5-(4-(3-hydroxy 17-oxo estra-1,3,5(10)-trièn-11béta-yl) phénoxy) pentylthio) N-méthyl acétamide obtenu à l'exemple 76. On obtient ainsi le produit attendu.

$[alpha]_D$ = -32,5° (c = 0,1% $CHCl_3$)

Spectre IR : ($CHCl_3$)

| | |
|---|---|
| OH | 3603 $cm^{-1}$ + OH associé |
| >C=O | 1627 $cm^{-1}$ |
| aromatique | 1581, 1511 $cm^{-1}$ |

Ultra violet (EtOH)

| | |
|---|---|
| max 281 nm | epsilon = 3800 |
| max 287 nm | epsilon = 3700 |

(EtOH + NaOH N/10)

| | |
|---|---|
| max 280 nm | epsilon = 3100 |
| max 287 nm | epsilon = 3200 |
| max 300 nm | epsilon = 2700 |

Compositions pharmaceutiques :

On a préparé des comprimés répondant à la formule suivante :

- Produit de l'exemple 21 50 mg
- Excipient (talc, amidon, stéarate de magnésien) q.s. pour un comprimé terminé à 120 mg

**Etude pharmacologique des produits de l'invention.**

1 - Etude de l'activité des produits de l'invention sur les récepteurs hormonaux.

Récepteur minéralocorticoïde du rein du rat :

Des rats mâles Sprague-Dawley EOPS, pesant 140 à 160 g, surrénalectomisés depuis 4 à 8 jours sont sacrifiés et leurs reins sont perfusés in situ avec 50 ml d'un tampon Tris 10 mM.

Saccharose 0,25 M, HCl pH 7,4. Les reins sont ensuite prélevés, décapsulés et homogénéisés à 0°C à l'aide d'un Potter teflon-verre (1 g de tissu pour 3 ml de tampon). L'homogénat est centrifugé pendant 10 minutes à 800 g à 0°C.

Afin d'éliminer la fixation de l'aldostérone tritiée sur le récepteur glucocorticoïde, le 11béta, 17béta-dihydroxy 21-méthyl pregna 1,4,6-trièn 20-yn 3-one stéroïde se fixant uniquement sur le récepteur glucocorticoïde est additionné au surnageant à la concentration finale de $10^{-6}$M. Ce surnageant est ultracentrifugé à

105 000 g pendant 60 minutes à 0°C. Des aliquotes du surnageant ainsi obtenu sont incubées à 0°C avec une concentration constante (T) d'aldostérone tritiée en présence de concentrations croissantes ($0\text{-}2500.10^{-9}$M) d'aldostérone froide ou du produit froid à étudier. Après un temps (t) d'incubation, la concentration d'aldostérone tritiée liée (B) est mesurée par la technique d'adsorption au charbon-dextran.

Récepteur androgène de la prostate de rat :

Des rats mâles Sprague Dawley EOPS de 160 à 200 g sont castrés. 24 heures après la castration, les animaux sont sacrifiés, les prostates sont prélevées, pesées et homogénéisées à 0°C à l'aide d'un Potter teflon-verre dans une solution tamponnée TS (Tris 10 mM, saccharose 0,25 M, HCl pH 7,4) (1 g de tissu pour 5 ml de TS). L'homogénat est ensuite ultracentrifugé (105 000 g pendant 60 minutes) à 0°C. Des aliquotes du surnageant ainsi obtenu, sont incubées à 0°C pendant un temps E d'incubation avec une concentration constante (T) de testostérone tritiée en présence de concentrations croissantes ($0 - 1000.10^{-9}$ M), soit de testostérone froide, soit du produit à tester. La concentration de testostérone tritiée liée (B) est ensuite mesurée dans chaque incubat par la technique d'adsorption au charbon-dextran.

Récepteur progestogène de l'utérus de lapine :

Des lapines impubères d'environ 1 kg reçoivent une application cutanée de 25 ug d'estradiol. 5 jours après ce traitement, les animaux sont sacrifiés, les utérus sont prélevés, pesés et homogénéisés à 0°C, à l'aide d'un Potter teflon-verre dans une solution tamponnée TS (Tris 10 mM, saccharose 0,25 M, HCl pH 7,4) (1 g de tissu pour 50 ml de TS). L'homogénat est ensuite ultracentrifugé (105 000 g x 90 mn) à 0°C. Des aliquotes du surnageant ainsi obtenu, sont incubées à 0°C pendant un temps t, avec une concentration constante (T) de produit R tritié (17,21-diméthyl 19-nor-4,9-pregna-diène-3,20-dione) en présence de concentrations croissantes ($0 - 2500.10^{-9}$M) soit de R froid, soit de progestérone froide, soit du produit froid à tester. La concentration de R tritié lié (B) est ensuite mesurée dans chaque incubat par la technique d'adsorption au charbon dextran.

Récepteur glucocorticoïde du thymus de rat :

Des rats mâles Sprague Dawley EOPS de 160 à 200 g sont surrénalectomisés. 4 à 8 jours après cette ablation, les animaux sont sacrifiés, et les thymus sont prélevés et homogénéisés à 0°C dans un tampon Tris 10 mM, saccharose 0,25 M, dithiothreitol 2 mM, HCl pH 7,4, à l'aide d'un Potter polytétrafluoroéthylène-verre (1 g de tissu pour 10 ml de TS). L'homogénat est ensuite ultracentrifugé (105 000 g x 90 mn) à 0°C. Des aliquotes du surnageant ainsi obtenu, sont incubées à 0°C pendant un temps (t) avec une concentration constante (T) de dexaméthasone tritiée en présence de concentrations croissantes ($0 - 2\,500.10^{-9}$ M) soit de dexaméthasone froide, soit du produit froid à tester. La concentration de la dexaméthasone tritiée liée (B) est ensuite mesurée dans chaque incubat par la technique d'adsorption au charbon-dextran.

Récepteur estrogène de l'utérus de souris :

Des souris femelles impubères âgées de 18 à 21 jours sont sacrifiées, les utérus sont prélevés puis homogénéisés à 0°C à l'aide d'un Potter teflon-verre dans une solution tamponnée TS (Tris 10 mM, saccharose 0,25 M, HCl pH 7,4 (1 g de tissu pour 25 ml de TS). L'homogénat est ensuite ultracentrifugé (105 000 g x 90 mn) à 0°C. Des aliquotes du surnageant ainsi obtenu, sont incubées à 0°C ou à 25°C pendant un temps (t) avec une concentration constante (T) d'estradiol tritié en présence de concentrations croissantes ($0 - 1000.10^{-9}$ M) soit d'estradil froid, soit du produit froid à tester. La concentration d'estradiol tritié lié (B) est ensuite mesurée dans chaque incubat par la technique d'adsorption au charbon-dextran.

Calcul de l'affinité relative de liaison :

Le calcul de l'affinité relative de liaison (ARL) est identique pour tous les récepteurs.

On trace les 2 courbes suivantes : le pourcentage de l'hormone tritiée liée $\frac{B}{T}$ en fonction du logarithme de la concentration de l'hormone de référence froide et $\frac{B}{T}$ en fonction du logarithme de la concentration du produit froid testé.

On détermine la droite d'équation $I_{50} = (\frac{B}{T}\text{max} + \frac{B}{T}\text{min})/2$. $\frac{B}{T}$ max = Pourcentage de l'hormone tritiée liée pour

une incubation de cette hormone tritiée à la concentration (T).

$\frac{B}{T}$ min = Pourcentage de l'hormone tritiée liée pour une incubation de cette hormone tritiée à la concentration (T) en présence d'un grand excès d'hormone froide ($2500.10^{-9}$M).

Les intersections de la droit $I_{50}$ et des courbes permettent d'évaluer les concentrations de l'hormone de référence froide (CH) et du produit froid testé (CX) qui inhibent de 50 % la liaison de l'hormone tritiée sur le récepteur.

L'affinité relative de liaison (ARL) du produit testé est déterminée par l'équation ARL = $100 \frac{(CH)}{(CX)}$

Les résultats obtenus sont les suivants :

| Temps d'incubation à 0°C / Produits des Exemples | Minéralocorticoïde | | androgène | | Progestogène | | Glucocoticoïde | | Oestrogène | |
|---|---|---|---|---|---|---|---|---|---|---|
| | 1H | 24H | 0,5H | 24H | 2H | 24H | 4H | 24H | 2H | 5H |
| 8 | 0,09 | 0,09 | 0 | 0,06 | 2,3 | 9 | 4,5 | 8 | 0,12 | 23 |
| 13 | 0,19 | 0,08 | 0 | 0,12 | 0,8 | 1,8 | 7 | 7 | 0,13 | 27 |
| 16 | 0 | 0 | 0 | 0,4 | 0,5 | 2 | 12 | 11 | 0,5 | 50 |
| 21 | 0,4 | 0,5 | 0 | 0 | 0,3 | 1 | 2 | 2,5 | 0,05 | 7,4 |
| 24 | 0 | 0 | 0 | 0,04 | 0 | 0 | 2 | 3 | 0,02 | 13 |
| 17 | 2 | 0,5 | 0,12 | 1,2 | 8 | 15 | 3 | 6 | 0,1 | <0,1 |
| 19 | 0,7 | 0,1 | 11 | 11 | 6 | 7 | 5 | 4,5 | 0,3 | 0,8 |
| 22 | 0 | 0 | 0,8 | 0 | 12 | 21 | 7 | 14 | <0,01 | <0,01 |
| 35 | | | | | | | | | 0,13 | 12 |
| 37 | | | | | | | | | 0,6 | 28 |
| 43 | | | | | | | | | 4,0 | 30 |
| 46 | | | | | | | | | 0,3 | 65 |
| 55 | | | | | | | | | 1,2 | 45 |
| 71 | | | | | | | | | 0,6 | 50 |

Conclusions :

Les produits étudiés, en particulier les produits des exemples 8, 13, 16, 37, 46, 55 et 71 ont une affinité marquée pour les récepteurs oestrogènes au deuxième temps.

Le produit de l'exemple 19 a une affinité modérée pour le récepteur androgène et les produits des exemples 17 et 22 pour le récepteur progestérone.

En outre la majorité des produits sont dépourvus d'activité utérotrophique.

2 - Activité anti-proliférative des produits de l'invention sur la croissance de cellules tumorales mammaires MCF-7.

Description du test :

a) Culture cellulaire :

Les lignées MCF-7 sont maintenues en culture en milieu SVF(1) à 37°C en atmosphère humide contenant 5 % $CO_2$. Les cellules à subconfluence sont récoltées par trypsination (trypsine 0,05 %, EDTA 0,02 %) puis rincées par centrifugation douce. Un échantillon des cellules en suspension est compté sur cellule de Malassez.

b) Etude de la croissance :

Les cellules resuspendues dans le milieu SVF sont ensemencées à raison de 30.000 cellules par puits dans des plaques multipuits (24 puits de 2,5 $cm^2$). Vingt quatre heures après l'ensemencement (JO), le produit à tester est ajouté au milieu en solution éthanolique (concentration finale en éthanol : 0,1 %), à la concentration de $10^{-12}$ à $10^{-6}$M, les puits contrôles recevant la même concentration en éthanol. Les milieux sont renouvelés toutes les 48 heures. En fin d'expérience (J6), le milieu est aspiré et les cellules sont immédiatement fixées par 150 microlitres de méthanol afin de doser l'ADN.

L'activité anti-proliférative des produits est évaluée par leur capacité à inhiber l'augmentation d'ADN.

c) Dosage de l'ADN :

L'ADN est dosé par une méthode fluorimétrique utilisant le DABA (Acide 3,5 diaminobenzoïque) (2) : 150 microlitres de DABA sont ajoutés dans chaque puits les plaques sont alors incubées 45 mn à 56°C, puis 2 ml d'HCl 1N sont ajoutés. La fluorescence est mesurée à l'aide d'un fluorimètre (longueur d'onde excitatrice : 408 nm, longueur d'onde d'émission 510 nm).

La quantité d'ADN par puits est évaluée par rapport à une gamme étalon obtenue en traitant dans les mêmes conditions un standard d'ADN de thymus de veau.

Résultats.

La concentration en nM qui inhibe de 50 % la croissance des cellules $MCF_7$ ($CI_{50}$) a été déterminée de la manière indiquée ci-dessus.

Résultats :

| | |
|---|---|
| Produit de l'exemple 8 | : $CI_{50}$ = 0,04 nM |
| Produit de l'exemple 13 | : $CI_{50}$ = 0,5 nM |
| Produit de l'exemple 16 | : $CI_{50}$ = 0,02 nM |
| Produit de l'exemple 21 | : $CI_{50}$ = 0,02 nM |
| Produit de l'exemple 24 | : $CI_{50}$ = 0,06 nM |
| Produit de l'exemple 35 | : $CI_{50}$ = 0,04 nM |
| Produit de l'exemple 37 | : $CI_{50}$ = 0,03 nM |
| Produit de l'exemple 43 | : $CI_{50}$ = 0,002 nM |
| Produit de l'exemple 46 | : $CI_{50}$ = 0,006 nM |
| Produit de l'exemple 55 | : $CI_{50}$ = 0,01 nM |
| Produit de l'exemple 71 | : $CI_{50}$ = 0,1 nM |

En outre, l'effet inhibiteur maximal des produits atteint au moins 90 %.

(1) Le milieu de culture sérum de veau foetal (SVF) est préparé comme suit :

Milieu MEM (minimal Essential Medium) auquel sont ajoutés :

- acides aminés non essentiels (GIBCO),
- peni-strepto (penicilline 100 U/ml, streptomycine 0,1 mg/ml),
- fungizone 0,1 %
- insuline (50 ng/ml),
- sérum de veau foetal destéroïdé (10 % concentration finale).

(2) Puzas et Goodman, Analytical Biochemistry, Vol. 86, pp. 50, 1978.

## Revendications

### Revendications pour les Etats contractants suivants : AT, BE, CH, DE, DK, FR, GB, IT, LI, LU, NL, SE

**1.** Les composés de formule (I) :

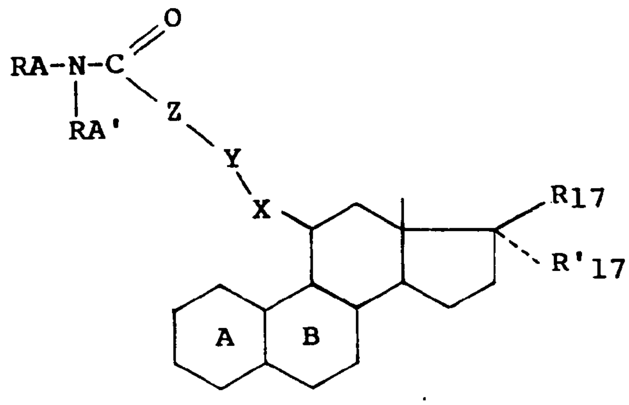

(I)

dans laquelle les cycles A et B ont l'une des structures suivantes :

a) soit A et B représentent le groupement :

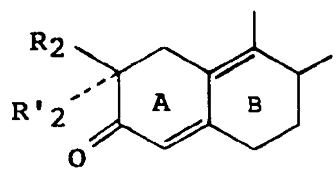

dans lequel $R_2$ et $R'_2$ identiques ou différents représentent un atome d'hydrogène ou un radical alkyle ayant de 1 à 4 atomes de carbone ;

b) soit A et B représentent le groupement :

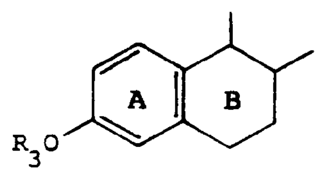

dans lequel $R_3$ représente un atome d'hydrogène ou un radical alkyle renfermant de 1 à 4 atomes de carbone ou un radical acyle,

$R_{17}$ et $R'_{17}$ sont tels que :

- soit $R_{17}$ et $R'_{17}$ forment ensemble une fonction cétone,
- soit $R_{17}$ est un radical hydroxyle ou un radical acyloxy et $R'_{17}$ représente un atome d'hydrogène, un radical alkyle, alcényle ou alcynyle ayant au plus 8 atomes de carbone, éventuellement substitué,

X, Y et Z sont tels que :

- X représente un radical méthylène, un groupement arylène, un radical $CH_2$-O ou arylènoxy lié au stéroïde par un atome de carbone,
- Y représente une simple liaison ou une chaîne aliphatique linéaire ou ramifiée, saturée ou insaturée, renfermant de 1 à 18 atomes de carbone, éventuellement interrompue par un ou plusieurs radicaux choisis parmi les radicaux arylène, oxygène ou soufre éventuellement oxydé sous forme de sulfoxyde ou de sulfone et éventuellement terminée par un radical arylène,
- Z représente une simple liaison ou un radical $CH_2$-O lié au radical Y par l'atome de carbone, étant entendu que lorsque Y et Z sont une simple liaison, X ne peut être un radical méthylène ou $CH_2$-O,

RA et RA', identiques ou différents, représentent un atome d'hydrogène, un radical alkyle linéaire ou ramifié renfermant de 1 à 8 atomes de carbone, éventuellement substitué par un ou plusieurs radicaux choisis parmi les radicaux aryle, alkyl ou dialkylamino, hydroxy, halogène ou carboxyl estérifié, ou RA et RA' forment avec l'atome d'azote auquel ils sont liés un hétérocycle à 5 ou 6 chaînons, saturé ou non, renfermant éventuellement un ou plusieurs autres hétéroatomes choisis parmi le groupe constitué par les atomes d'oxygène, d'azote et de soufre et éventuellement substitué par un radical alkyle ayant de 1 à 4 atomes de carbone, étant entendu que l'un au moins des substituants RA ou RA' n'est pas un atome d'hydrogène.

2. Les composés de formule (I) telle que définie à la revendication 1, dans lesquels les cycles A et B représentent le groupement :

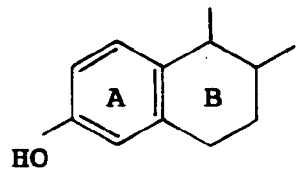

3. Les composés de formule (I) telle que définie à la revendication 1, dans lesquels les cycles A et B représentent le groupement :

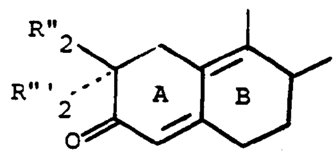

dans lequel $R"_2$ ou $R'''_2$ représente un atome d'hydrogène ou un radical méthyle, de préférence un atome d'hydrogène.

4. Les composés de formule (I) telle que définie à la revendication 1, 2 ou 3 pour lesquels Z est une simple liaison.

5. Les composés de formule (I) telle que définie à l'une quelconque des revendications 1 à 4 pour lesquels R 17 est un radical hydroxyle.

6. Les composés de formule (I) telle que définie à l'une quelconque des revendications 1 à 5 pour lesquels $R'_{17}$ est un atome d'hydrogène, un radical éthynyle ou un radical propynyle.

7. Les composés de formule (I) telle que définie à l'une quelconque des revendications 1 à 6 pour lesquels X représente un radical méthylène et Y est une chaîne linéaire saturée renfermant de 5 à 10 atomes de carbone éventuellement interrompue par un atome d'oxygène.

8. Les composés de formule (I) telle que définie à l'une quelconque des revendications 1 à 6 pour lesquels X représente un radical phénylène et Y est une chaîne linéaire saturée ou insaturée renfermant de 3 à 10 atomes de carbone, éventuellement interrompue par un atome d'oxygène.

9. Les composés de formule (I) telle que définie à l'une quelconque des revendications 1 à 6 pour lesquels X représente un radical phénylénoxy et Y est une chaîne linéaire saturée renfermant de 3 à 10 atomes de carbone, éventuellement interrompue par un atome d'oxygène ou de soufre.

10. Les composés de formule (I) telle que définie à l'une quelconque des revendications 1 à 9 pour lesquels :
- soit RA et RA' identiques représentent un radical méthyle,
- soit RA représente un atome d'hydrogène ou un radical méthyle et RA' représente un radical butyle,
- soit RA représente un radical méthyle et RA' représente un radical isopropyle, diméthylaminoéthyle, benzyle ou heptafluorobutyle,
- soit RA et RA' forment ensemble une pipérazine éventuellement N-substituée ou une pyrrolidine.

11. Les composés de formule (I) telle que définie à l'une quelconque des revendications 1 à 10 dont les noms suivent :
- le N-(2-diméthylaminoéthyl) 17béta-hydroxy N-méthyl 3-oxo 11béta-estra-4,9-dièn-undécanamide,
- le N-butyl 4-(3,17béta-dihydroxy estra-1,3,5(10)-trièn-11béta-yl) N-méthyl benzène octanamide,
- le 3,17béta-dihydroxy N-méthyl N-(1-méthyléthyl) 11béta-estra-1,3,5(10)-trièn-undécanamide,

- le N-butyl 3,17béta-dihydroxy N-méthyl 19-Nor 11béta-(17alpha-pregna-1,3,5(10)-trièn-20-yne) undécanamide.
- le 3,17béta-dihydroxy N-méthyl N-(1méthyléthyl) 19-nor 17alpha-pregna 1,3,5(10)-trièn-20-yn-11béta-undécanamide,
- le [[8-(3,17béta-dihydroxy estra 1,3,5(10)-trièn-11béta-yl) octyl] oxy] N-méthyl N-(1-méthyléthyl) acétamide,
- le N-butyl 8-[4-(3,17béta-dihydroxy estra 1,3,5(10)-trièn-11béta-yl) phénoxy] N-méthyl octanamide,
- le N-butyl [5-[4-(3,17béta-dihydroxy estra 1,3,5(10)-trièn-11béta-yl) phénoxy] pentyloxy] N-méthyl acétamide,
- le 2-[[7-[4-(3,17béta-dihydroxy estra 1,3,5(10)-trièn-11béta-yl) phényl] 6-heptylyl] oxy] N-butyl N-méthyl acétamide,
- le 3,17béta-dihydroxy N-(2,2,3,3,4,4,4-heptafluorobutyl) N-méthyl estra 1,3,5(10)-trièn-11béta-yl undécanamide,
- le 8-[4-(3,17béta-dihydroxy estra 1,3,5(10)-trièn-11béta-yl) phényl] N-butyl N-méthyl octynamide.

**12.** Procédé de préparation des composés de formule (I) telle que définie à la revendication 1, caractérisé en ce que l'on soumet un composé de formule (II) :

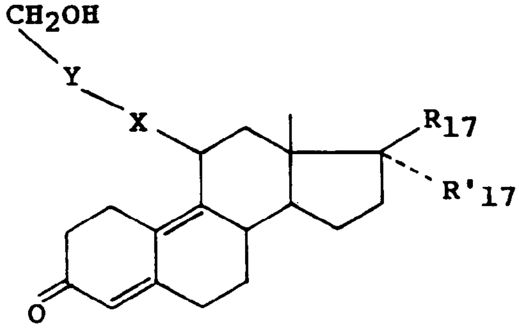

(II)

dans laquelle X, Y, $R_{17}$ et $R'_{17}$ ont la même signification qu'à la revendication 1, étant entendu que $R_{17}$ ne peut représenter un radical hydroxyle,

- soit à l'action d'un agent d'oxydation pour obtenir le produit de formule (III) :

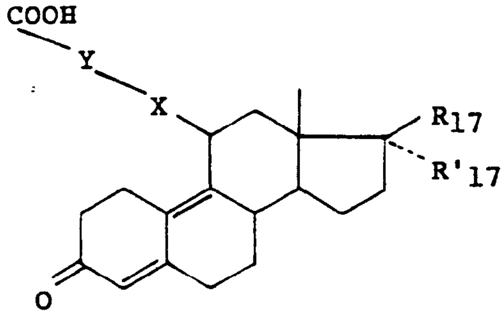

(III)

dans laquelle $R_{17}$, $R'_{17}$, X et Y ont la même signification qu'à la revendication 1 que l'on soumet à l'action d'un agent permettant d'activer la fonction carboxylique, puis à l'action d'un composé de formule (IV) :

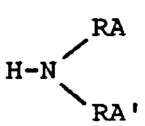

(IV)

dans laquelle RA et RA' ont la même signification qu'à la revendication 1, pour obtenir le produit de formule (Ia) correspondant au composé de formule (I) dans laquelle Z est une simple liaison et les cycles A et B représentent le groupement

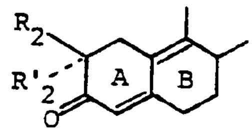

dans lequel $R_2$ et $R'_2$ sont un atome d'hydrogène,

- soit à une réaction d'introduction du radical

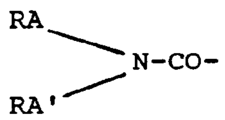

pour obtenir le produit de formule (I'a) correspondant au composé de formule (I) dans laquelle Z est un radical méthylènoxy et les cycles A et B ont la même signification que dans les produits de formule (Ia),

produits (Ia) et (I'a) que, si désiré,

- soit l'on soumet à un agent de réduction lorsque $R_{17}$ et $R'_{17}$ forment ensemble une fonction cétone, puis le cas échéant, soumet à un agent d'acylation le dérivé hydroxylé en 17 ainsi obtenu,
- soit l'on soumet à un agent de saponification lorsque $R_{17}$ représente une fonction acyloxy, pour obtenir un produit de formule (Ia) ou (I'a) dans laquelle $R_{17}$ a la définition indiquée ci-dessus, puis si désiré, l'on soumet l'un quelconque des produits de formule (Ia) ou (I'a),
- soit à une alkylation en position 2, lorsque l'un au moins des radicaux $R_2$ et $R'_2$ représente un atome d'hydrogène,
- soit l'on soumet à un agent d'aromatisation du cycle A, puis à un agent de saponification ménagée pour obtenir les produits de formule (Ib) correspondant aux produits de formule (Ia) et les produits de formule (I'b) correspondant aux produits de formule (I'a) et dans lesquelles les cycles A et B représentent le groupement :

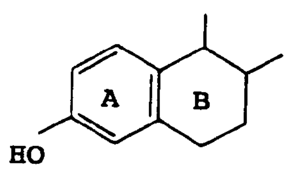

produits de formule (Ib) et I'b) que si désiré, l'on soumet à une réaction d'alkylation ou d'acylation du radical hydroxyle en position 3, puis si désiré,

soit, lorsque $R_{17}$ et $R'_{17}$ forment ensemble une fonction cétone, l'on soumet à un agent de réduction ou l'on soumet à un complexe métallique de formule (V) :

$$M\text{-}R'_{17} \qquad (V)$$

dans laquelle M représente un atome métallique et $R'_{17}$ a la même signification que précédemment, étant entendu qu'il ne s'agit pas d'un atome d'hydrogène,

soit, lorsque $R_{17}$ est un radical hydroxyle, l'on soumet à un agent d'acylation sélective en position 17, puis si désiré, soumet l'un quelconque des produits de formule (I) obtenus ci-dessus, soit lorsque RA ou RA' est un atome d'hydrogène, à l'action d'un agent d'alkylation approprié.

**13.** Procédé de préparation des composés de formule (I) dans lesquels X représente un radical arylène et Y représente une chaîne aliphatique éventuellement liée au groupe arylène par une double ou une triple liaison et comportant au moins 3 atomes de carbone ou liée au groupe arylène par un atome d'oxygène, caractérisé en ce que l'on soumet un composé de formule (X) :

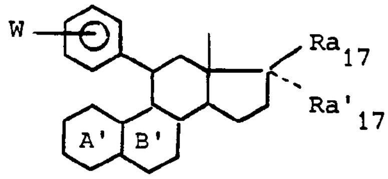

(X)

dans laquelle W représente soit un radical OH soit un radical -C=CH, les cycles A' et B', $Ra_{17}$ et $Ra'_{17}$ ayant les mêmes significations que celles indiquées précédemment que pour les cycles A et B, $R_{17}$ et $R'_{17}$ et dans lesquelles les fonctions réactives en 3 et en 17 sont éventuellement protégées ou bien, dans le cas où W représente un radical -C=CH à l'action d'un agent halogéné de formule (XI) :

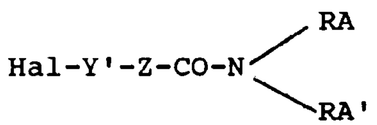

(XI)

dans laquelle Hal est un atome d'alogène, Z, RA et RA' ont la même signification que précédemment et Y' représente la chaîne aliphatique Y ci-dessus comportant 2 atomes de carbone en moins, en présence d'une base forte et soumet le cas échéant à l'action d'un agent de déprotection, pour obtenir le produit de formule ($I'_A$) :

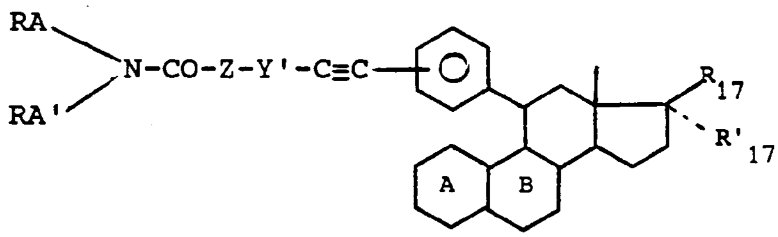

($I'_A$)

produit que, si désiré, l'on soumet à un agent de réduction partielle ou totale de la triple liaison pour obtenir le produit de formule ($I'_B$) :

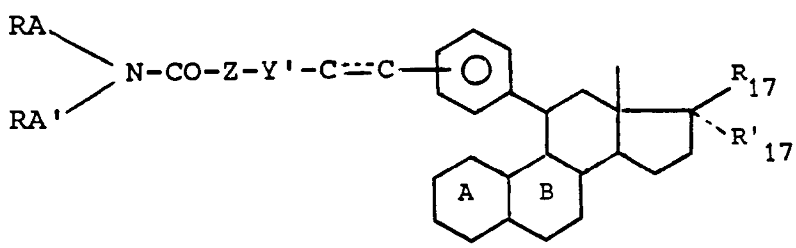

($I'_B$)

<u>ou bien</u>, dans le cas où W représente un radical -OH, à l'action d'un dérivé halogéné de formule (XII) :

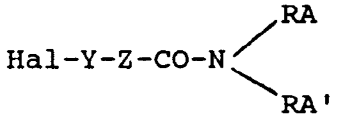

(XII)

dans laquelle Hal, Y, Z, RA et RA' on la signification indiquée précédemment, en présence d'un agent alcalin, puis soumet le cas échéant, à l'action d'un agent de déprotection pour obtenir un produit de formule ($I''_A$) :

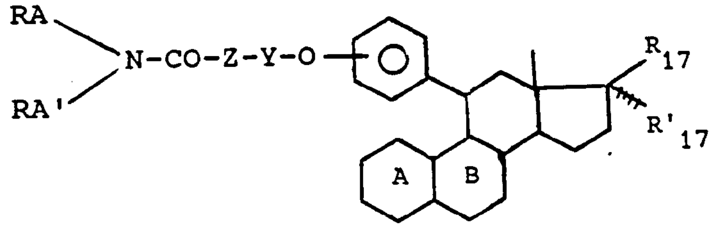

(I"A)

produit que si désiré, lorsque Y représente une chaîne aliphatique insaturée, l'on soumet à un agent de réduction partielle ou totale, et produits de formule (I'$_A$), (I'$_B$), (I"$_A$) que si désiré l'on soumet à l'une quelconque des réactions indiquées ci-dessus pour (Ia), (I'a), (Ib), (I'b).

**14.** A titre de médicament, les composés de formule (I) telle que définie à l'une quelconque des revendications 1 à 10.

**15.** A titre de médicament, les composés de formule (I) définis à la revendication 11.

**16.** Les compositions pharmaceutiques renfermant comme principe actif au moins un médicament tel que défini à la revendication 14 ou 15.

**17.** A titre de produits intermédiaires nouveaux, les composés de formules (II) et (III), telles que définies à la revendication 12.

**18.** Les produits intermédiaires nouveaux selon la revendication 17 répondant à la formule :
- 17béta-acétyloxy 11béta-(8-hydroxy octyl) phényl] estra-4,9-dièn-3-one,
- 11béta-(12-hydroxy dodécyl) estra-4,9-diène-3,17-dione,
- 11béta (8-hydroxy octyl) estra-4,9-dièn-3,17-dione,
- acide 17béta-acétyloxy 3-oxo 11béta-estra-4,9-dièneundécanoïque,
- acide 3,17-dioxo 11béta-estra-4,9-dièneundécanoïque,
- acide 17béta-hydroxy 3-oxo 17-(1-propynyl) 11béta-estra-4,9-dièneundécanoïque.

**Revendications pour l'Etat contractant suivant: GR**

**1.** Procédé de préparation des composés de formule (I) :

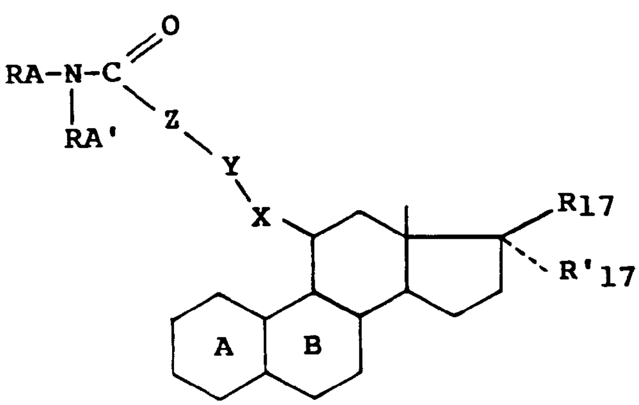

(I)

dans laquelle les cycles A et B ont l'une des structures suivantes :
a) soit A et B représentent le groupement :

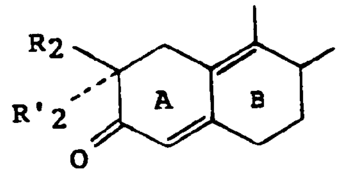

dans lequel $R_2$ et $R'_2$ identiques ou différents représentent un atome d'hydrogène ou un radical alkyle ayant de 1 à 4 atomes de carbone ;

b) soit A et B représentent le groupement :

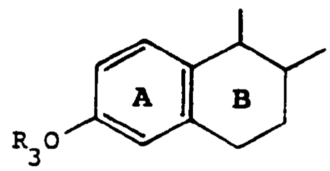

dans lequel $R_3$ représente un atome d'hydrogène ou un radical alkyle renfermant de 1 à 4 atomes de carbone ou un radical acyle,

$R_{17}$ et $R'_{17}$ sont tels que :

- soit $R_{17}$ et $R'_{17}$ forment ensemble une fonction cétone,
- soit $R_{17}$ est un radical hydroxyle ou un radical acyloxy et $R'_{17}$ représente un atome d'hydrogène, un radical alkyle, alcényle ou alcynyle ayant au plus 8 atomes de carbone, éventuellement substitué,

X, Y et Z sont tels que :

- X représente un radical méthylène, un groupement arylène, un radical $CH_2$-O ou arylènoxy lié au stéroïde par un atome de carbone,
- Y représente une simple liaison ou une chaîne aliphatique linéaire ou ramifiée, saturée ou insaturée, renfermant de 1 à 18 atomes de carbone, éventuellement interrompue par un ou plusieurs radicaux choisis parmi les radicaux arylène, oxygène ou soufre éventuellement oxydé sous forme de sulfoxyde ou de sulfone et éventuellement terminée par un radical arylène,
- Z représente une simple liaison ou un radical $CH_2$-O lié au radical Y par l'atome de carbone, étant entendu que lorsque Y et Z sont une simple liaison, X ne peut être un radical méthylène ou $CH_2$-O,

  RA et RA', identiques ou différents, représentent un atome d'hydrogène, un radical alkyle linéaire ou ramifié renfermant de 1 à 8 atomes de carbone, éventuellement substitué par un ou plusieurs radicaux choisis parmi les radicaux aryle, alkyl ou dialkylamino, hydroxy, halogène ou carboxyl estérifié, ou RA et RA' forment avec l'atome d'azote auquel ils sont liés un hétérocycle à 5 ou 6 chaînons, saturé ou non, renfermant éventuellement un ou plusieurs autres hétéroatomes choisis parmi le groupe constitué par les atomes d'oxygène, d'azote et de soufre et éventuellement substitué par un radical alkyle ayant de 1 à 4 atomes de carbone, étant entendu que l'un au moins des substituants RA ou RA' n'est pas un atome d'hydrogène, caractérisé en ce que l'on soumet un composé de formule (II) :

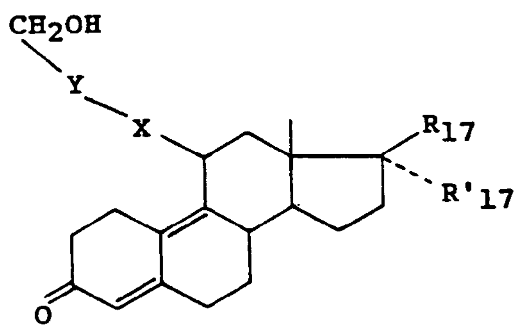

(II)

dans laquelle X, Y, $R_{17}$ et $R'_{17}$ ont la même signification que précédemment, étant entendu que $R_{17}$ ne peut représenter un radical hydroxyle,

- soit à l'action d'un agent d'oxydation pour obtenir le produit de formule (III) :

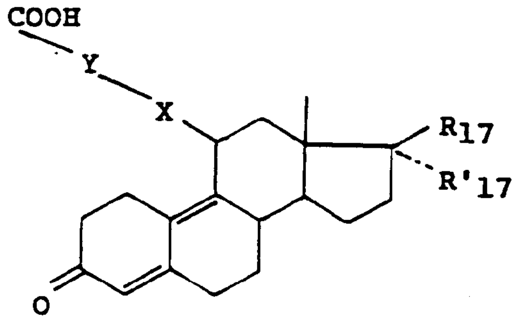

(III)

dans laquelle $R_{17}$, $R'_{17}$, X et Y ont la même signification qu'à la revendication 1 que l'on soumet à l'action d'un agent permettant d'activer la fonction carboxylique, puis à l'action d'un composé de formule (IV) :

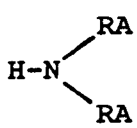

(IV)

dans laquelle RA et RA' ont la même signification que précédement, pour obtenir le produit de formule (Ia) correspondant au composé de formule (I) dans laquelle Z est une simple liaison et les cycles A et B représentent le groupement

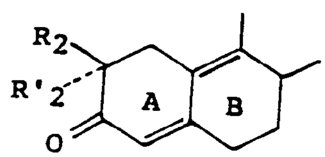

dans lequel $R_2$ et $R'_2$ sont un atome d'hydrogène,

- soit à une réaction d'introduction du radical

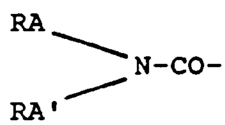

pour obtenir le produit de formule (I'a) correspondant au composé de formule (I) dans laquelle Z est un radical méthylènoxy et les cycles A et B ont la même signification que dans les produits de formule (Ia),

produits (Ia) et (I'a) que, si désiré,

- soit l'on soumet à un agent de réduction lorsque $R_{17}$ et $R'_{17}$ forment ensemble une fonction cétone, puis le cas échéant, soumet à un agent d'acylation le dérivé hydroxylé en 17 ainsi obtenu,
- soit l'on soumet à un agent de saponification lorsque $R_{17}$ représente une fonction acyloxy, pour obtenir un produit de formule (Ia) ou (I'a) dans laquelle $R_{17}$ a la définition indiquée ci-dessus, puis si désiré, l'on soumet l'un quelconque des produits de formule (Ia) ou (I'a),
- soit à une alkylation en position 2, lorsque l'un au moins des radicaux $R_2$ et $R'_2$ représente un atome d'hydrogène,
- soit l'on soumet à un agent d'aromatisation du cycle A, puis à un agent de saponification ménagée pour obtenir les produits de formule (Ib) correspondant aux produits de formule (Ia) et les produits

de formule (I'b) correspondant aux produits de formule (I'a) et dans lesquelles les cycles A et B représentent le groupement :

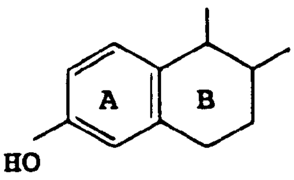

produits de formule (Ib) et (I'b) que si désiré, l'on soumet à une réaction d'alkylation ou d'acylation du radical hydroxyle en position 3, puis si désiré,
soit, lorsque $R_{17}$ et $R'_{17}$ forment ensemble une fonction cétone, l'on soumet à un agent de réduction ou l'on soumet à un complexe métallique de formule (V) :

$$M\text{-}R'_{17} \qquad (V)$$

dans laquelle M représente un atome métallique et $R'_{17}$ a la même signification que précédemment, étant entendu qu'il ne s'agit pas d'un atome d'hydrogène,
soit, lorsque $R_{17}$ est un radical hydroxyle, l'on soumet à un agent d'acylation sélective en position 17, puis si désiré, soumet l'un quelconque des produits de formule (I) obtenus ci-dessus, soit lorsque RA ou RA' est un atome d'hydrogène, à l'action d'un agent d'alkylation approprié.

**2.** Procédé selon la revendication 1 pour la préparation des composés de formule ($I_A$) :

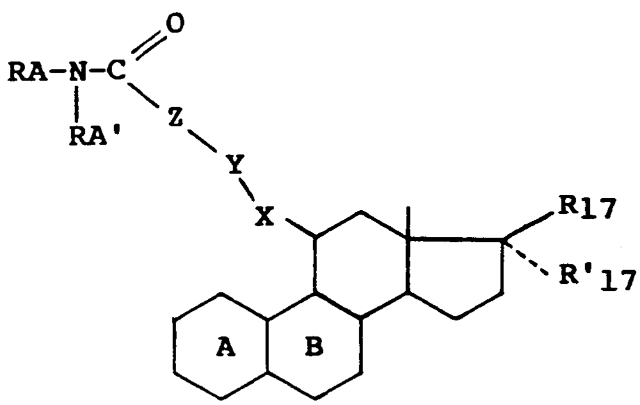

($I_A$)

dans laquelle les cycles A et B ont l'une des structures suivantes :

a) soit A et B représentent le groupement :

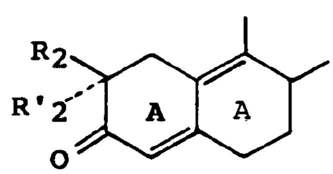

dans lequel $R_2$ et $R'_2$ identiques ou différents représentent un atome d'hydrogène ou un radical alkyle ayant de 1 à 4 atomes de carbone ;

b) soit A et B représentent le groupement :

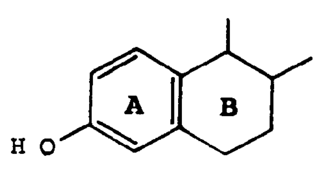

$R_{17}$ et $R'_{17}$ sont tels que :

- soit $R_{17}$ et $R'_{17}$ forment ensemble une fonction cétone,
- soit $R_{17}$ est un radical hydroxyle ou un radical acyloxy et $R'_{17}$ représente un atome d'hydrogène, un radical alkyle, alcényle ou alcynyle ayant au plus 8 atomes de carbone, éventuellement substitué,

X, Y et Z sont tels que :

- X représente un radical méthylène, un groupement arylène, un radical $CH_2$-O ou arylènoxy lié au stéroïde par un atome de carbone,
- Y représente une simple liaison ou une chaîne aliphatique linéaire ou ramifiée, saturée ou insaturée, renfermant de 1 à 18 atomes de carbone, éventuellement interrompue par un ou plusieurs radicaux choisis parmi les radicaux arylène, oxygène et éventuellement terminée par un radical arylène,
- Z représente une simple liaison ou un radical $CH_2$-O lié au radical Y par l'atome de carbone, étant entendu que lorsque Y et Z sont une simple liaison, X ne peut être un radical méthylène ou $CH_2$-O,

RA et RA', identiques ou différents, représentent un atome d'hydrogène, un radical alkyle linéaire ou ramifié renfermant de 1 à 8 atomes de carbone, éventuellement substitué par un radical aryle, alkyl ou dialkylamino, hydroxy, halogène ou carboxyl estérifié, ou RA et RA' forment avec l'atome d'azote auquel ils sont liés un hétérocycle à 5 ou 6 chaînons, saturé ou non, renfermant éventuellement un ou plusieurs autres hétéroatomes choisis parmi le groupe constitué par les atomes d'oxygène, d'azote et de soufre et éventuellement substitué par un radical alkyle ayant de 1 à 4 atomes de carbone, étant entendu que l'un au moins des substituants RA ou RA' n'est pas un atome d'hydrogène, caractérisé en ce que l'on soumet un composé de formule (II) :

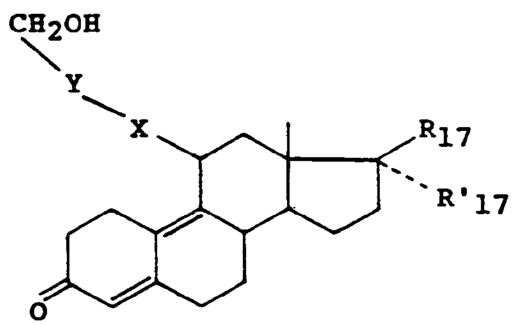

(II)

dans laquelle X, Y, $R_{17}$ et $R'_{17}$ ont la même signification qu'à la revendication 1, étant entendu que $R_{17}$ ne peut représenter un radical hydroxyle,

- soit à l'action d'un agent d'oxydation pour obtenir le produit de formule (III) :

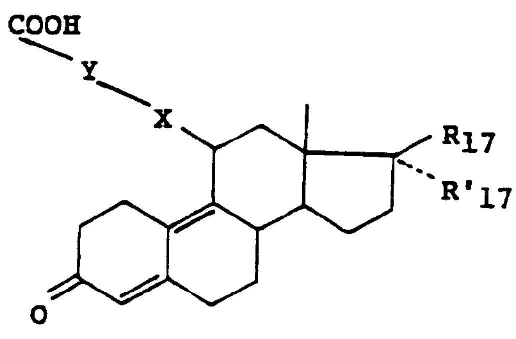

(III)

dans laquelle $R_{17}$, $R'_{17}$, X et Y ont la même signification qu'à la revendication 1 que l'on soumet à l'action d'un agent permettant d'activer la fonction carboxylique, puis à l'action d'un composé de formule (IV) :

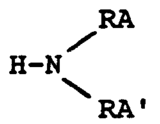

(IV)

dans laquelle RA et RA' ont la même signification qu'à la revendication 1 pour obtenir le produit de formule (Ia) correspondant au composé de formule ($I_A$) dans laquelle Z est une simple liaison et les cycles A et B représentent le groupement

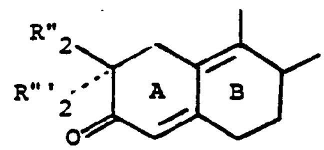

dans lequel $R_2$ et $R'_2$ sont un atome d'hydrogène,

- soit à une réaction d'introduction du radical

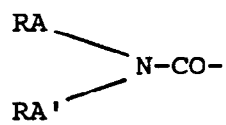

pour obtenir le produit de formule (I'a) correspondant au composé de formule ($I_A$) dans laquelle Z est un radical méthylènoxy et les cycles A et B ont la même signification que dans les produits de formule (Ia),

produits (Ia) et (I'a) que, si désiré,

- soit l'on soumet à un agent de réduction lorsque $R_{17}$ et $R'_{17}$ forment ensemble une fonction cétone, puis le cas échéant, soumet à un agent d'acylation le dérivé hydroxylé en 17 ainsi obtenu,
- soit l'on soumet à un agent de saponification lorsque $R_{17}$ représente une fonction acyloxy, pour obtenir un produit de formule (Ia) ou (I'a) dans laquelle $R_{17}$ a la définition indiquée ci-dessus, puis si désiré, l'on soumet l'un quelconque des produits de formule (Ia) ou (I'a),
- soit à une alkylation en position 2, lorsque l'un au moins des radicaux $R_2$ et $R'_2$ représente un atome d'hydrogène,
- soit l'on soumet à un agent d'aromatisation du cycle A, puis à un agent de saponification ménagée pour obtenir les produits de formule (Ib) correspondant aux produits de formule (Ia) et les produits de formule (I'b) correspondant aux produits de formule (I'a) et dans lesquelles les cycles A et B représentent le groupement :

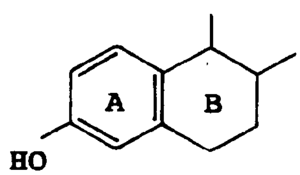

produits de formule (Ib) et (I'b) que si désiré,

soit, lorsque $R_{17}$ et $R'_{17}$ forment ensemble une fonction cétone, l'on soumet à un agent de réduction ou l'on soumet à un complexe métallique de formule (V) :

$$M\text{-}R'_{17} \qquad (V)$$

dans laquelle M représente un atome métallique et $R'_{17}$ a la même signification que précédemment, étant entendu qu'il ne s'agit pas d'un atome d'hydrogène,

soit, lorsque $R_{17}$ est un radical hydroxyle, l'on soumet à un agent d'acylation sélective en position 17, puis si désiré, soumet l'un quelconque des produits de formule (I) obtenus ci-dessus, soit lorsque RA ou RA' est un atome d'hydrogène, à l'action d'un agent d'alkylation approprié.

3. Procédé selon la revendication 2, caractérisé en ce que l'on utilise au départ un produit de formule (II) dans laquelle $R'_{17}$ est un atome d'hydrogène, un radical éthynyle ou un radical propynyle.

4. Procédé selon la revendication 2, caractérisé en ce que l'on utilise au départ un produit de formule (II) dans laquelle X représente un radical méthylène et Y est une chaîne linéaire saturée renfermant de 5 à 10 atomes de carbone, éventuellement interrompue par un atome d'oxygène.

5. Procédé selon la revendication 2, caractérisé en ce que l'on utilise au départ un produit de formule (II) dans laquelle X représente un radical phénylène et Y est une chaîne linéaire saturée ou insaturée renfermant de 3 à 10 atomes de carbone, éventuellement interrompue par un atome d'oxygène.

6. Procédé selon la revendication 1, caractérisé en ce que l'on utilise au départ un produit de formule (II) dans laquelle X représente un radical phénylènoxy et Y est une chaîne linéaire saturée renfermant de 3 à 10 atomes de carbone, éventuellement interrompue par un atome d'oxygène ou de soufre.

7. Procédé selon la revendication 2, caractérisé en ce que l'on utilise au départ un composé de formule (IV) ou bien un réactif d'introduction du radical

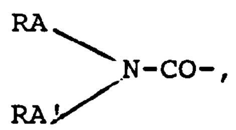

ou bien le cas échéant un réactif tel que
- soit RA et RA'identiques représentent un radical méthyle,
- soit RA représente un atome d'hydrogène ou un radical méthyle et RA' représente un radical butyle,
- soit RA représente un radical méthyle et RA' représente un radical isopropyle, diméthylaminoéthyle, benzyle ou heptafluorobutyle,
- soit RA et RA' forment ensemble une pipérazine éventuellement N-substituée ou une pyrrolidine.

8. Procédé selon la revendication 1, caractérisé en ce que l'on choisit les produits de départ de manière telle que l'on prépare :
- le N-(2-diméthylaminoéthyl) 17béta-hydroxy N-méthyl 3-oxo 11béta-estra-4,9-dièn-undécanamide,
- le N-butyl 4-(3,17béta-dihydroxy estra-1,3,5(10)-trièn-11béta-yl) N-méthyl benzène octanamide,
- le 3,17béta-dihydroxy N-méthyl N-(1-méthyléthyl) 11béta-estra-1,3,5(10)-trièn-undécanamide,
- le N-butyl 3,17béta-dihydroxy N-méthyl 19-Nor 11béta-(17alpha-pregna-1,3,5(10)-trièn-20-yne) undécanamide.
- le 3,17béta-dihydroxy N-méthyl N-(1méthyléthyl) 19-nor 17alpha-pregna 1,3,5(10)-trièn-20-yn-11béta-undécanamide,
- le [[8-(3,17béta-dihydroxy estra 1,3,5(10)-trièn-11béta-yl) octyl] oxy] N-méthyl N-(1-méthyléthyl) acétamide,
- le N-butyl 8-[4-(3,17béta-dihydroxy estra 1,3,5(10)-trièn-11béta-yl) phénoxy] N-méthyl octanamide,
- le N-butyl [5-[4-(3,17béta-dihydroxy estra 1,3,5(10)-trièn-11béta-yl) phénoxy] pentyloxy] N-méthyl acétamide,
- le 2-[[7-[4-(3,17béta-dihydroxy estra 1,3,5(10)-trièn-11béta-yl) phényl] 6-heptylyl] oxy] N-butyl N-méthyl acétamide,
- le 3,17béta-dihydroxy N-(2,2,3,3,4,4,4-heptafluorobutyl) N-méthyl estra 1,3,5(10)-trièn-11béta-yl undécanamide,
- le 8-[4-(3,17béta-dihydroxy estra 1,3,5(10)-trièn-11béta-yl) phényl] N-butyl N-méthyl octynamide.

9. Procédé de préparation des produits de formule (I) telle que définie à la revendication 1, dans laquelle X représente un radical arylène et Y représente une chaîne aliphatique éventuellement liée au groupe arylène par une double ou une triple liaison et comportant au moins 3 atomes de carbone ou liée au groupe arylène par un atome d'oxygène, caractérisé en ce que l'on soumet un composé de formule (X) :

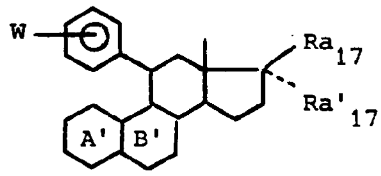

(X)

dans laquelle W représente soit un radical OH soit un radical -C=CH, les cycles A' et B', $Ra_{17}$ et $Ra'_{17}$ ayant les mêmes significations que celles indiquées précédemment que pour les cycles A et B, $R_{17}$ et $R'_{17}$ et dans lesquelles les fonctions réactives en 3 et en 17 sont éventuellement protégées ou bien, dans le cas où W représente un radical -C=CH à l'action d'un agent halogéné de formule (XI)

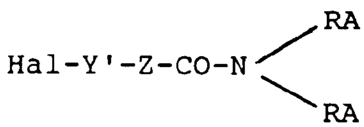

(XI)

dans laquelle Hal est un atome d'halogène, Z, RA et RA' ont la même signification que précédemment et Y' représente la chaîne aliphatique Y ci-dessus comportant 2 atomes de carbone en moins, en présence d'une base forte et soumet le cas échéant à l'action d'un agent de déprotection, pour obtenir le produit de formule ($I'_A$) :

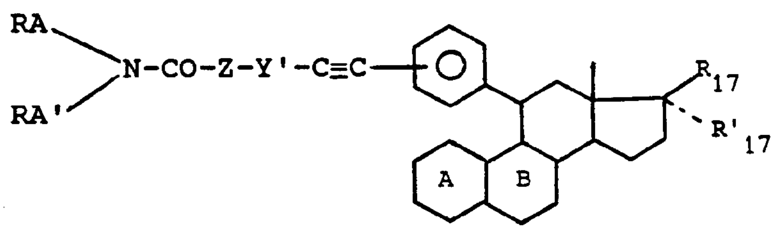

($I'_A$)

produit que, si désiré, l'on soumet à un agent de réduction partielle ou totale de la triple liaison pour obtenir le produit de formule ($I'_B$) :

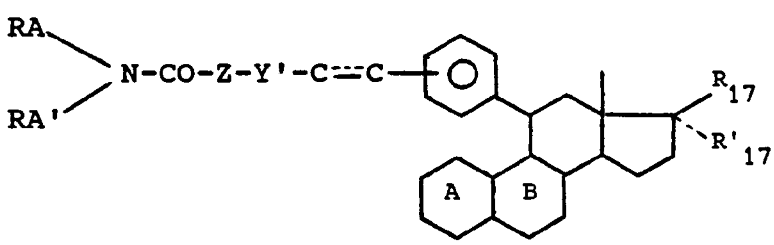

($I'_B$)

<u>ou bien</u>, dans le cas où W représente un radical -OH, à l'action d'un dérivé halogéné de formule (XII) :

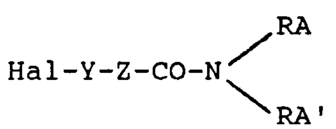

(XII)

dans laquelle Hal, Y, Z, RA et RA' ont la signification indiquée précédemment, en présence d'un agent alcalin, puis soumet le cas échéant, à l'action d'un agent de déprotection pour obtenir un produit de formule ($I''_A$) :

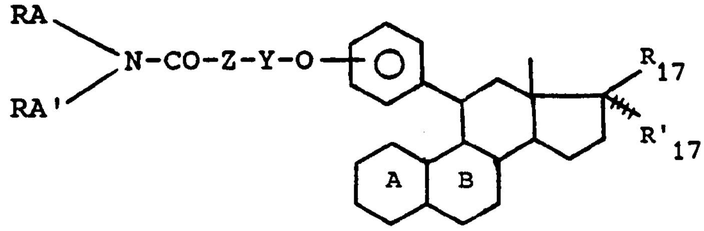

(I"A)

produit que si désiré, lorsque Y représente une chaîne aliphatique insaturée, l'on soumet à un agent de réduction partielle ou totale, et produits de formule (I'$_A$ ), (I'$_B$), (I"$_A$) que si désiré l'on soumet à l'une quelconque des réactions indiquées ci-dessus pour (Ia), (I'a), (Ib), (I'b).

**10.** Procédé selon la revendication 8, caractérisé en ce que l'on utilise au départ un composé de formule (X) dans laquelle R'$_{17}$ est un atome d'hydrogène, un radical éthynyle ou un radical propynyle.

**11.** Procédé selon la revendication 9, caractérisé en ce que l'on utilise au départ un composé de formule (X) dans laquelle W représente un radical -C=CH et un composé de formule (XI) dans laquelle Y est une chaîne linéaire saturée renfermant de 5 à 10 atomes de carbone, éventuellement interrompue par un atome d'oxygène.

**12.** Procédé selon la revendication 9, caractérisé en ce que l'on utilise au départ un composé de formule (X) dans laquelle W représente un radical OH et un composé de formule (XII) dans laquelle Y est une chaîne linéaire saturée renfermant de 3 à 10 atomes de carbone éventuellement interrompue par un atome d'oxygène ou de soufre.

**13.** Procédé selon la revendication 9, caractérisé en ce que l'on utilise au départ un composé de formule (XI) ou un bien un composé de formule (XII) ou le bien le cas échéant un réactif d'alkylation tel que
- soit RA et RA'identiques représentent un radical méthyle,
- soit RA représente un atome d'hydrogène ou un radical méthyle et RA' représente un radical butyle,
- soit RA représente un radical méthyle et RA' représente un radical isopropyle, diméthylaminoéthyle, benzyle ou hepta-fluorobutyle,
- soit RA et RA' forment ensemble une pipérazine éventuellement N-substituée ou une pyrrolidine.

**14.** Procédé selon la revendication 9, caractérisé en ce que l'on choisit les produits de départ de manière telle que l'on prépare :
- le N-butyl 4-(3,17béta-dihydroxy estra-1,3,5(10)-trièn-11béta-yl) N-méthyl benzène octanamide,
- le N-butyl 8-[4-(3,17béta-dihydroxy estra 1,3,5(10)-trièn-11béta-yl) phénoxy] N-méthyl octanamide,
- le N-butyl [5-[4-(3,17béta-dihydroxy estra 1,3,5(10)-trièn-11béta-yl) phénoxy] pentyloxy] N-méthyl acétamide,
- le 2-[[7-[4-(3,17béta-dihydroxy estra 1,3,5(10)-trièn-11béta-yl) phényl] 6-heptylyl] oxy] N-butyl N-méthyl acétamide,
- le 8-[4-(3,17béta-dihydroxy estra 1,3,5(10)-trièn-11béta-yl) phényl] N-butyl N-méthyl octynamide.

**15.** A titre de produits industriels nouveaux, les composés de formules (II) et (III), telles que définies à la revendication 1.

**16.** Les produits nouveaux selon la revendication 16 répondant à la formule :
- 17béta-acétyloxy 11béta-[(8-hydroxy octyl) phényl] estra-4,9-dièn-3-one,
- 11béta-(12-hydroxy dodécyl) estra-4,9-diène-3,17-dione,
- 11béta (8-hydroxy octyl) estra-4,9-dièn-3,17-dione,
- acide 17béta-acétyloxy 3-oxo 11béta-estra-4,9-dièneundécanoïque,
- acide 3,17-dioxo 11béta-estra-4,9-dièneundécanoïque,
- acide 17béta-hydroxy 3-oxo 17-(1-propynyl) 11béta-estra-4,9-dièneundécanoïque.

**17.** Procédé de préparation de compositions pharmaceutiques caractérisés en ce que l'on met à titre de principe actif l'un au moins des produits de formule générale (I) telle que définie à la revendication 1 ou 9 sous une forme destinée à cet usage.

18. Procédé de préparation de compositions pharmaceutiques caractérisés en ce que l'on met à titre de principe actif l'un au moins des produits de formule générale ($I_A$) telle que définie à la revendication 2 sous une forme destinée à cet usage.

19. Procédé de préparation de compositions pharmaceutiques caractérisés en ce que l'on met à titre de principe actif l'un au moins des produits de formule générale (I) telle que définie à la revendication 8 ou 15 sous une forme destinée à cet usage.

**Revendications pour l'Etat contractant suivant : ES**

1. Procédé de préparation des composés de formule (I) :

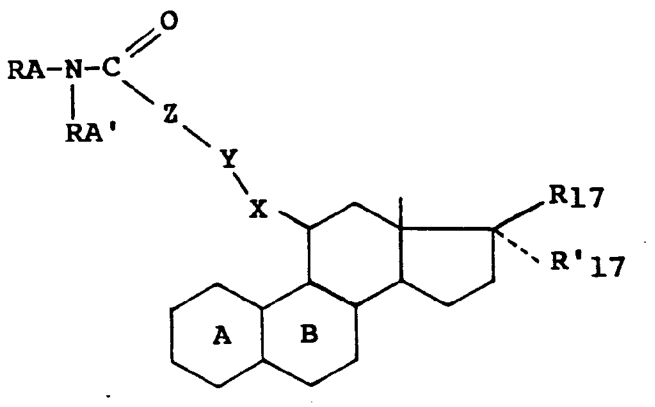

(I)

dans laquelle les cycles A et B ont l'une des structures suivantes :

a) soit A et B représentent le groupement :

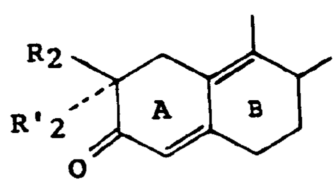

dans lequel $R_2$ et $R'_2$ identiques ou différents représentent un atome d'hydrogène ou un radical alkyle ayant de 1 à 4 atomes de carbone ;

b) soit A et B représentent le groupement :

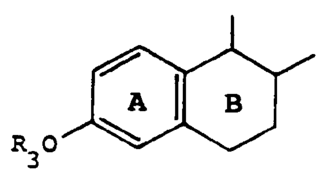

dans lequel $R_3$ représente un atome d'hydrogène ou un radical alkyle renfermant de 1 à 4 atomes de carbone ou un radical acyle,

$R_{17}$ et $R'_{17}$ sont tels que :

- soit $R_{17}$ et $R'_{17}$ forment ensemble une fonction cétone,
- soit $R_{17}$ est un radical hydroxyle ou un radical acyloxy et $R'_{17}$ représente un atome d'hydrogène, un radical alkyle, alcényle ou alcynyle ayant au plus 8 atomes de carbone, éventuellement substitué,

X, Y et Z sont tels que :

- X représente un radical méthylène, un groupement arylène, un radical $CH_2$-O ou arylènoxy lié au stéroïde par un atome de carbone,
- Y représente une simple liaison ou une chaîne aliphatique linéaire ou ramifiée, saturée ou insaturée, renfermant de 1 à 18 atomes de carbone, éventuellement interrompue par un ou plusieurs radicaux choisis parmi les radicaux arylène, oxygène ou soufre éventuellement oxydé sous forme de sulfoxyde ou de sulfone et éventuellement terminée par un radical arylène,

- Z représente une simple liaison ou un radical $CH_2$-O lié au radical Y par l'atome de carbone, étant entendu que lorsque Y et Z sont une simple liaison, X ne peut être un radical méthylène ou $CH_2$-O,

RA et RA', identiques ou différents, représentent un atome d'hydrogène, un radical alkyle linéaire ou ramifié renfermant de 1 à 8 atomes de carbone, éventuellement substitué par un ou plusieurs radicaux choisis parmi les radicaux aryle, alkyl ou dialkylamino, hydroxy, halogène ou carboxyl estérifié, ou RA et RA' forment avec l'atome d'azote auquel ils sont liés un hétérocycle à 5 ou 6 chaînons, saturé ou non, renfermant éventuellement un ou plusieurs autres hétéroatomes choisis parmi le groupe constitué par les atomes d'oxygène, d'azote et de soufre et éventuellement substitué par un radical alkyle ayant de 1 à 4 atomes de carbone, étant entendu que l'un au moins des substituants RA ou RA' n'est pas un atome d'hydrogène, caractérisé en ce que l'on soumet un composé de formule (II) :

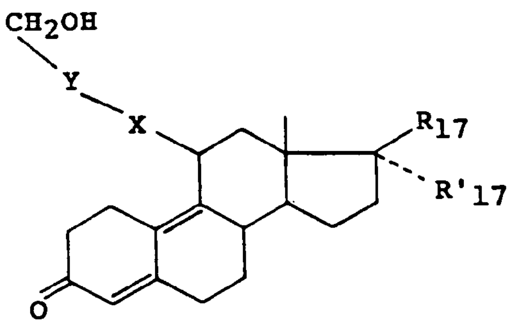

(II)

dans laquelle X, Y, $R_{17}$ et $R'_{17}$ ont la même signification que précédemment, étant entendu que $R_{17}$ ne peut représenter un radical hydroxyle,

- soit à l'action d'un agent d'oxydation pour obtenir le produit de formule (III) :

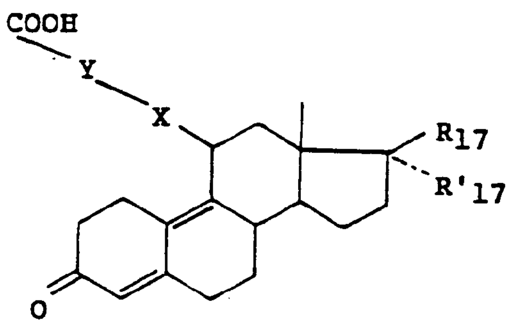

(III)

dans laquelle $R_{17}$, $R'_{17}$, X et Y ont la même signification qu'à la revendication 1 que l'on soumet à l'action d'un agent permettant d'activer la fonction carboxylique, puis à l'action d'un composé de formule (IV) :

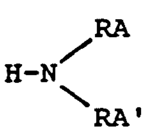

(IV)

dans laquelle RA et RA' ont la même signification que précédemment, pour obtenir le produit de formule (Ia) correspondant au composé de formule (I) dans laquelle Z est une simple liaison et les cycles A et B représentent le groupement

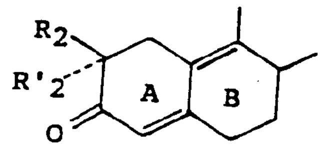

dans lequel $R_2$ et $R'_2$ sont un atome d'hydrogène,

- soit à une réaction d'introduction du radical

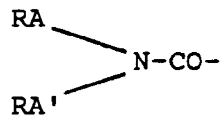

pour obtenir le produit de formule (I'a) correspondant au composé de formule (I) dans laquelle Z est un radical méthylènoxy et les cycles A et B ont la même signification que dans les produits de formule (Ia),

produits (Ia) et (I'a) que, si désiré,

- soit l'on soumet à un agent de réduction lorsque $R_{17}$ et $R'_{17}$ forment ensemble une fonction cétone, puis le cas échéant, soumet à un agent d'acylation le dérivé hydroxylé en 17 ainsi obtenu,
- soit l'on soumet à un agent de saponification lorsque $R_{17}$ représente une fonction acyloxy, pour obtenir un produit de formule (Ia) ou (I'a) dans laquelle $R_{17}$ a la définition indiquée ci-dessus, puis si désiré, l'on soumet l'un quelconque des produits de formule (Ia) ou (I'a),
- soit à une alkylation en position 2, lorsque l'un au moins des radicaux $R_2$ et $R'_2$ représente un atome d'hydrogène,
- soit l'on soumet à un agent d'aromatisation du cycle A, puis à un agent de saponification ménagée pour obtenir les produits de formule (Ib) correspondant aux produits de formule (Ia) et les produits de formule (I'b) correspondant aux produits de formule (I'a) et dans lesquelles les cycles A et B représentent le groupement :

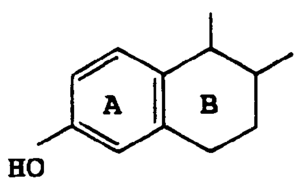

produits de formule (Ib) et I'b) que si désiré, l'on soumet à une réaction d'alkylation ou d'acylation du radical hydroxyle en position 3, puis si désiré,

soit, lorsque $R_{17}$ et $R'_{17}$ forment ensemble une fonction cétone, l'on soumet à un agent de réduction ou l'on soumet à un complexe métallique de formule (V) :

$$M\text{-}R'_{17} \qquad (V)$$

dans laquelle M représente un atome métallique et $R'_{17}$ a la même signification que précédemment, étant entendu qu'il ne s'agit pas d'un atome d'hydrogène,

soit, lorsque $R_{17}$ est un radical hydroxyle, l'on soumet à un agent d'acylation sélective en position 17, puis si désiré, soumet l'un quelconque des produits de formule (I) obtenus ci-dessus, soit lorsque RA ou RA' est un atome d'hydrogène, à l'action d'un agent d'alkylation approprié.

**2.** Procédé selon la revendication 1 pour la préparation des composés de formule ($I_A$) :

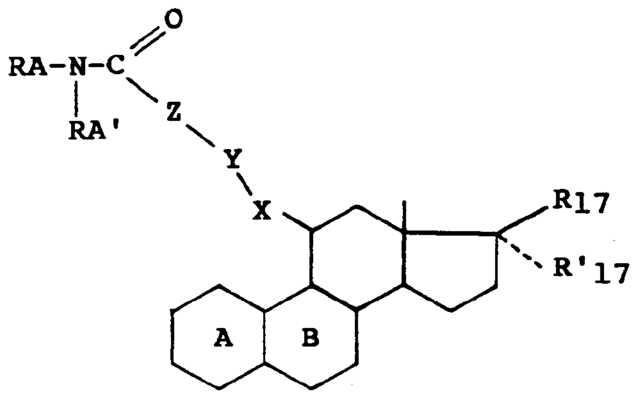

($I_A$)

dans laquelle les cycles A et B ont l'une des structures suivantes :

a) soit A et B représentent le groupement :

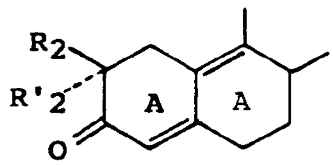

dans lequel $R_2$ et $R'_2$ identiques ou différents représentent un atome d'hydrogène ou un radical alkyle ayant de 1 à 4 atomes de carbone ;

b) soit A et B représentent le groupement :

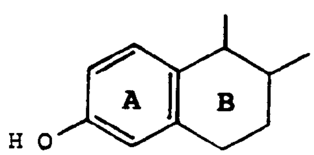

$R_{17}$ et $R'_{17}$ sont tels que :

- soit $R_{17}$ et $R'_{17}$ forment ensemble une fonction cétone,
- soit $R_{17}$ est un radical hydroxyle ou un radical acyloxy et $R'_{17}$ représente un atome d'hydrogène, un radical alkyle, alcényle ou alcynyle ayant au plus 8 atomes de carbone, éventuellement substitué,

X, Y et Z sont tels que :

- X représente un radical méthylène, un groupement arylène, un radical $CH_2$-O ou arylènoxy lié au stéroïde par un atome de carbone,
- Y représente une simple liaison ou une chaîne aliphatique linéaire ou ramifiée, saturée ou insaturée, renfermant de 1 à 18 atomes de carbone, éventuellement interrompue par un ou plusieurs radicaux choisis parmi les radicaux arylène, oxygène et éventuellement terminée par un radical arylène,
- Z représente une simple liaison ou un radical $CH_2$-O lié au radical Y par l'atome de carbone, étant entendu que lorsque Y et Z sont une simple liaison, X ne peut être un radical méthylène ou $CH_2$-O,

RA et RA', identiques ou différents, représentent un atome d'hydrogène, un radical alkyle linéaire ou ramifié renfermant de 1 à 8 atomes de carbone, éventuellement substitué par un radical aryle, alkyl ou dialkylamino, hydroxy, halogène ou carboxyl estérifié, ou RA et RA' forment avec l'atome d'azote auquel ils sont liés un hétérocycle à 5 ou 6 chaînons, saturé ou non, renfermant éventuellement un ou plusieurs autres hétéroatomes choisis parmi le groupe constitué par les atomes d'oxygène, d'azote et de soufre et éventuellement substitué par un radical alkyle ayant de 1 à 4 atomes de carbone, étant entendu que l'un au moins des substituants RA ou RA' n'est pas un atome d'hydrogène, caractérisé en ce que l'on soumet un composé de formule (II) :

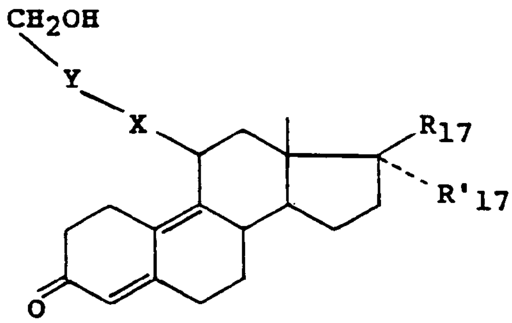

(II)

dans laquelle X, Y, $R_{17}$ et $R'_{17}$ ont la même signification qu'à la revendication 1, étant entendu que $R_{17}$ ne peut représenter un radical hydroxyle,

- soit à l'action d'un agent d'oxydation pour obtenir le produit de formule (III) :

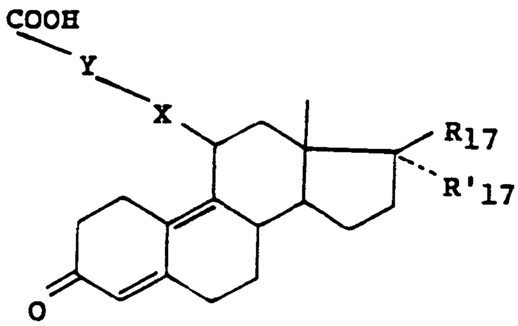

(III)

dans laquelle $R_{17}$, $R'_{17}$, X et Y ont la même signification qu'à la revendication 1 que l'on soumet à l'action d'un agent permettant d'activer la fonction carboxylique, puis à l'action d'un composé de formule (IV) :

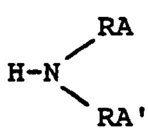

(IV)

dans laquelle RA et RA' ont la même signification qu'à la revendication 1 pour obtenir le produit de formule (Ia) correspondant au composé de formule ($I_A$) dans laquelle Z est une simple liaison et les cycles A et B représentent le groupement

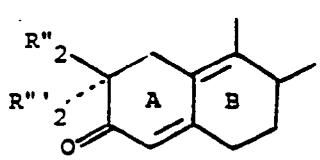

dans lequel $R_2$ et $R'_2$ sont un atome d'hydrogène,

- soit à une réaction d'introduction du radical

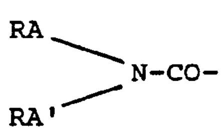

pour obtenir le produit de formule (I'a) correspondant au composé de formule ($I_A$) dans laquelle Z est un radical méthylènoxy et les cycles A et B ont la même signification que dans les produits

de formule (Ia),

produits (Ia) et (I'a) que, si désiré,

- soit l'on soumet à un agent de réduction lorsque $R_{17}$ et $R'_{17}$ forment ensemble une fonction cétone, puis le cas échéant, soumet à un agent d'acylation le dérivé hydroxylé en 17 ainsi obtenu,
- soit l'on soumet à un agent de saponification lorsque $R_{17}$ représente une fonction acyloxy, pour obtenir un produit de formule (Ia) ou (I'a) dans laquelle $R_{17}$ a la définition indiquée ci-dessus, puis si désiré, l'on soumet l'un quelconque des produits de formule (Ia) ou (I'a),
- soit à une alkylation en position 2, lorsque l'un au moins des radicaux $R_2$ et $R'_2$ représente un atome d'hydrogène,
- soit l'on soumet à un agent d'aromatisation du cycle A, puis à un agent de saponification ménagée pour obtenir les produits de formule (Ib) correspondant aux produits de formule (Ia) et les produits de formule (I'b) correspondant aux produits de formule (I'a) et dans lesquelles les cycles A et B représentent le groupement :

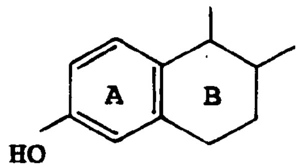

produits de formule (Ib) et (I'b) que si désiré,

soit, lorsque $R_{17}$ et $R'_{17}$ forment ensemble une fonction cétone, l'on soumet à un agent de réduction ou l'on soumet à un complexe métallique de formule (V) :

$$M\text{-}R'_{17} \qquad (V)$$

dans laquelle M représente un atome métallique et $R'_{17}$ a la même signification que précédemment, étant entendu qu'il ne s'agit pas d'un atome d'hydrogène,

soit, lorsque $R_{17}$ est un radical hydroxyle, l'on soumet à un agent d'acylation sélective en position 17, puis si désiré, soumet l'un quelconque des produits de formule (I) obtenus ci-dessus, soit lorsque RA ou RA' est un atome d'hydrogène, à l'action d'un agent d'alkylation approprié.

3. Procédé selon la revendication 2, caractérisé en ce que l'on utilise au départ un produit de formule (I) dans laquelle $R_{17}$ est un atome d'hydrogène, un radical éthynyle ou un radical propynyle.

4. Procédé selon la revendication 2, caractérisé en ce que l'on utilise au départ un produit de formule (II) dans laquelle X représente un radical méthylène et Y est une chaîne linéaire saturée renfermant de 5 à 10 atomes de carbone, éventuellement interrompue par un atome d'oxygène.

5. Procédé selon la revendication 2, caractérisé en ce que l'on utilise au départ un produit de formule (II) dans laquelle X représente un radical phénylène et Y est une chaîne linéaire saturée ou insaturée renfermant de 3 à 10 atomes de carbone, éventuellement interrompue par un atome d'oxygène.

6. Procédé selon la revendication 1, caractérisé en ce que l'on utilise au départ un produit de formule (II) dans laquelle X représente un radical phénylènoxy et Y est une chaîne linéaire saturée renfermant de 3 à 10 atomes de carbone, éventuellement interrompue par un atome d'oxygène ou de soufre.

7. Procédé selon la revendication 2, caractérisé en ce que l'on utilise au départ un composé de formule (IV) ou bien un réactif d'introduction du radical

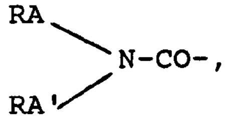

ou bien le cas échéant un réactif tel que

- soit RA et RA'identiques représentent un radical méthyle,
- soit RA représente un atome d'hydrogène ou un radical méthyle et RA' représente un radical butyle,

- soit RA représente un radical méthyle et RA' représente un radical isopropyle, diméthylaminoéthyle, benzyle ou heptafluorobutyle,
- soit RA et RA' forment ensemble une pipérazine éventuellement N-substituée ou une pyrrolidine.

**8.** Procédé de préparation des produits de formule (I) telle que définie à la revendication 1, dans laquelle X représente un radical arylène et Y représente une chaîne aliphatique éventuellement liée au groupe arylène par une double ou une triple liaison et comportant au moins 3 atomes de carbone ou liée au groupe arylène par un atome d'oxygène, caractérisé en ce que l'on soumet un composé de formule (X) :

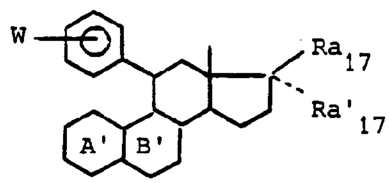

(X)

dans laquelle W représente soit un radical OH soit un radical -C=CH, les cycles A' et B', $Ra_{17}$ et $Ra'_{17}$ ayant les mêmes significations que celles indiquées précédemment que pour les cycles A et B, $R_{17}$ et $R'_{17}$ et dans lesquelles les fonctions réactives en 3 et en 17 sont éventuellement protégées ou bien, dans le cas où W représente un radical -C=CH à l'action d'un agent halogéné de formule (XI) :

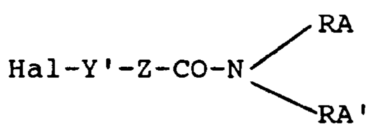

(XI)

dans laquelle Hal est un atome d'halogène, Z, RA et RA' ont la même signification que précédemment et Y' représente la chaîne aliphatique Y ci-dessus comportant 2 atomes de carbone en moins, en présence d'une base forte et soumet le cas échéant à l'action d'un agent de déprotection, pour obtenir le produit de formule ($I'_A$) :

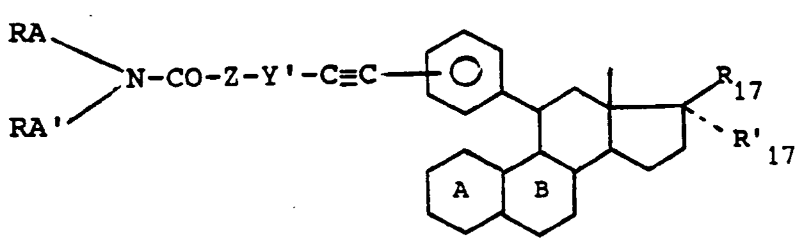

($I'_A$)

produit que, si désiré, l'on soumet à un agent de réduction partielle ou totale de la triple liaison pour obtenir le produit de formule ($I'_B$) :

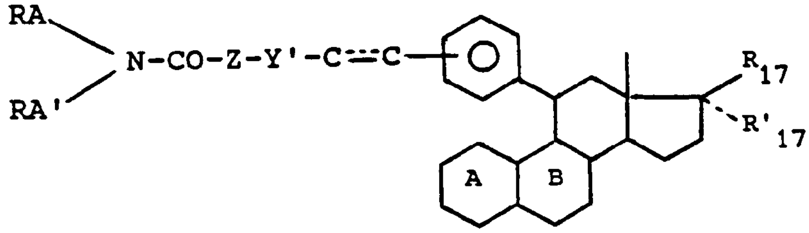

($I'_B$)

ou bien, dans le cas où W représente un radical -OH, à l'action d'un dérivé halogéné de formule (XII) :

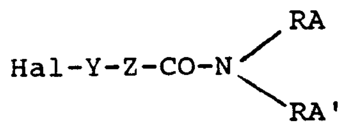

(XII)

dans laquelle Hal, Y, Z, RA et RA' ont la signification indiquée précédemment, en présence d'un agent alcalin, puis soumet le cas échéant, à l'action d'un agent de déprotection pour obtenir un produit de formule $(I''_A)$ :

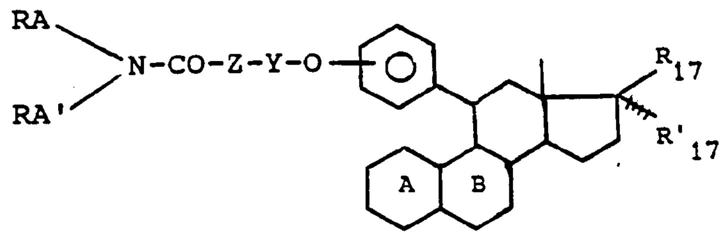

$(I''_A)$

produit que si désiré, lorsque Y représente une chaîne aliphatique insaturée, l'on soumet à un agent de réduction partielle ou totale, et produits de formule $(I'_A)$,$(I'_B)$, $(I''_A)$ que si désiré l'on soumet à l'une quelconque des réactions indiquées ci-dessus pour (Ia), (I'a), (Ib), (I'b).

**9.** Procédé selon la revendication 8, caractérisé en ce que l'on utilise au départ un composé de formule (X) dans laquelle $R'_{17}$ est un atome d'hydrogène, un radical éthynyle ou un radical propynyle.

**10.** Procédé selon la revendication 9, caractérisé en ce que l'on utilise au départ un composé de formule (X) dans laquelle W représente un radical -C=CH et un composé de formule (XI) dans laquelle Y est une chaîne linéaire saturée renfermant de 5 à 10 atomes de carbone, éventuellement interrompue par un atome d'oxygène.

**11.** Procédé selon la revendication 9, caractérisé en ce que l'on utilise au départ un composé de formule (X) dans laquelle W représente un radical OH et un composé de formule (XII) dans laquelle Y est une chaîne linéaire saturée renfermant de 3 à 10 atomes de carbone éventuellement interrompue par un atome d'oxygène ou de soufre.

**12.** Procédé selon la revendication 9, caractérisé en ce que l'on utilise au départ un composé de formule (XI) ou un bien un composé de formule (XII) ou le bien le cas échéant un réactif d'alkylation tel que
- soit RA et RA' identiques représentent un radical méthyle,
- soit RA représente un atome d'hydrogène ou un radical méthyle et RA' représente un radical butyle,
- soit RA représente un radical méthyle et RA' représente un radical isopropyle, diméthylaminoéthyle, benzyle ou hepta-fluorobutyle,
- soit RA et RA' forment ensemble une pipérazine éventuellement N-substituée ou une pyrrolidine.

## Claims

### Claims for the following Contracting States : AT, BE, CH, DE, FR, GB, IT, LI, LU, NL, SE, DK

**1.** The compounds of formula (I):

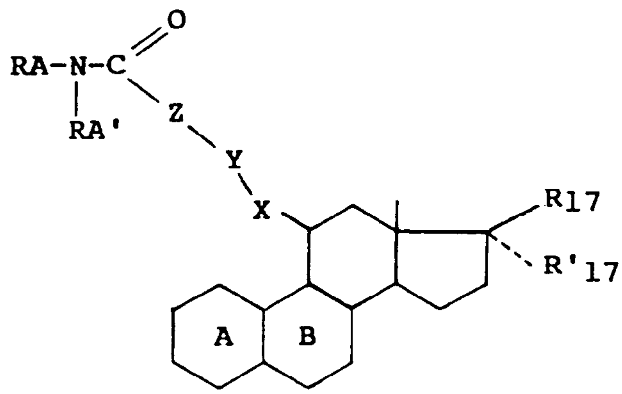

(I)

in which rings A and B have one of the following structures:

a) either A and B represent the group:

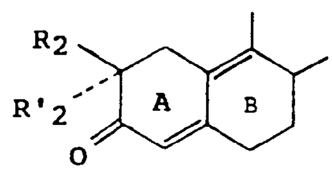

in which $R_2$ and $R'_2$ being identical or different represent a hydrogen atom or an alkyl radical having 1 to 4 carbon atoms;

b) or A and B represent the group:

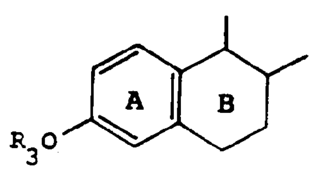

in which $R_3$ represents a hydrogen atom or an alkyl radical containing 1 to 4 carbon atoms or an acyl radical,

$R_{17}$ and $R'_{17}$ are such that:

- either $R_{17}$ and $R'_{17}$ together form a ketone function,
- or $R_{17}$ is a hydroxyl radical or an acyloxy radical and $R'_{17}$ represents a hydrogen atom, an optionally substituted alkyl, alkenyl or alkynyl radical having at most 8 carbon atoms,

  X, Y and Z are such that:
- X represents a methylene radical, an arylene group, a $CH_2$-O radical or an arylenoxy radical linked to the steroid by a carbon atom,
- Y represents a single bond or a saturated or unsaturated, linear or branched aliphatic chain containing 1 to 18 carbon atoms, optionally interrupted by one or more radicals chosen from the arylene radicals, oxygen or sulphur optionally oxidized in the form of the sulphoxide or sulphone and optionally terminated by an arylene radical,
- Z represents a single bond or a $CH_2$-O radical linked to the Y radical by the carbon atom,

  it being understood that when Y and Z are a single bond, X cannot be a methylene or $CH_2$-O radical,

  RA and RA', identical or different, represent a hydrogen atom, a linear or branched alkyl radical containing 1 to 8 carbon atoms, optionally substituted by one or more radicals chosen from aryl, alkyl or dialkylamino, hydroxy, halogen or esterified carboxyl radicals, or RA and RA' form with the nitrogen atom to which they are linked a saturated or unsaturated heterocycle with 5 or 6 links, optionally containing one or more other heteroatoms chosen from the group constituted by oxygen, nitrogen and sulphur atoms and optionally substituted by an alkyl radical having 1 to 4 carbon atoms, it being understood that at least one of the RA or RA' substituents is not a hydrogen atom.

2. The compounds of formula (I) as defined in claim 1, in which the rings A and B represent the group:

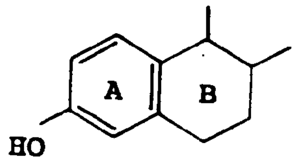

3. The compounds of formula (I) as defined in claim 1, in which rings A and B represent the group:

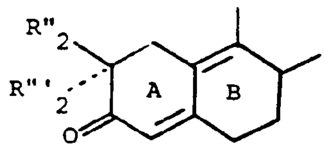

in which $R''_2$ or $R'''_2$ represents a hydrogen atom or a methyl radical, preferably a hydrogen atom.

4. The compounds of formula (I) as defined in claim 1, 2 or 3 in which Z is a single bond.

5. The compounds of formula (I) as defined in any one of claims 1 to 4 in which $R_{17}$ is a hydroxyl radical.

6. The compounds of formula (I) as defined in any one of claims 1 to 5 in which $R'_{17}$ is a hydrogen atom, an ethynyl radical or a propynyl radical.

7. The compounds of formula (I) as defined in any one of claims 1 to 6 in which X represents a methylene radical and Y is a saturated linear chain containing 5 to 10 carbon atoms optionally interrupted by an oxygen atom.

8. The compounds of formula (I) as defined in any one of claims 1 to 6 in which X represents a phenylene radical and Y is a saturated or unsaturated linear chain containing 3 to 10 carbon atoms, optionally interrupted by an oxygen atom.

9. The compounds of formula (I) as defined in any one of claims 1 to 6 in which X represents a phenylenoxy radical and Y is a saturated linear chain containing 3 to 10 carbon atoms, optionally interrupted by an oxygen or sulphur atom.

10. The compounds of formula (I) as defined in any one of claims 1 to 9 in which:
- either RA and RA' are identical and represent a methyl radical,
- or RA represents a hydrogen atom or a methyl radical and RA' represents a butyl radical,
- or RA represents a methyl radical and RA' represents an isopropyl, dimethylaminoethyl, benzyl or heptafluorobutyl radical,
- or RA and RA' together form an optionally N-substituted piperazine or a pyrrolidine radical.

11. The compounds of formula (I) as defined in any one of claims 1 to 10 whose names follow:
- N-(2-dimethylaminoethyl) 17beta-hydroxy N-methyl 3-oxo 11beta-estra-4, 9-dien-undecanamide,
- N-butyl 4-(3,17beta-dihydroxy estra-1,3,5(10)-trien-11beta-yl) N-methyl benzene octanamide,
- 3,17beta-dihydroxy N-methyl N-(1-methylethyl) 11beta-estra-1,3,5(10)-trien-undecanamide,
- N-butyl 3,17beta-dihydroxy N-methyl 19-Nor 11beta-(17alpha-pregna-1,3,5(10)-trien-20-yne) undecanamide,
- 3,17beta-dihydroxy N-methyl N-(1-methylethyl) 19-nor 17alpha-pregna 1,3,5(10)-trien-20-yn-11beta-undecanamide,
- [[8-(3,17beta-dihydroxy estra 1,3,5(10)-trien-11beta-yl) octyl] oxy] N-methyl N-(1-methylethyl) acetamide,
- N-butyl 8-[4-(3,17beta-dihydroxy estra 1,3,5(10)-trien-11beta-yl) phenoxy] N-methyl octanamide,
- N-butyl [5-[4-(3,17beta-dihydroxy estra 1,3,5(10)-trien-11beta-yl) phenoxy] pentyloxy] N-methyl acetamide,
- 2-[[7-[4-(3,17beta-dihydroxy estra 1,3,5(10)-trien-11beta-yl) phenyl] 6-heptylyl] oxy] N-butyl N-methyl acetamide,

- 3,17beta-dihydroxy N-(2,2,3,3,4,4,4-heptafluorobutyl) N-methyl estra 1,3,5(10)-trien-11beta-yl undecanamide,
- 8-[4-(3,17beta-dihydroxy estra 1,3,5(10)-trien-11beta-yl) phenyl] N-butyl N-methyl octynamide.

**12.** Preparation process for the compounds of formula (I) as defined in claim 1, characterized in that a compound of formula (II):

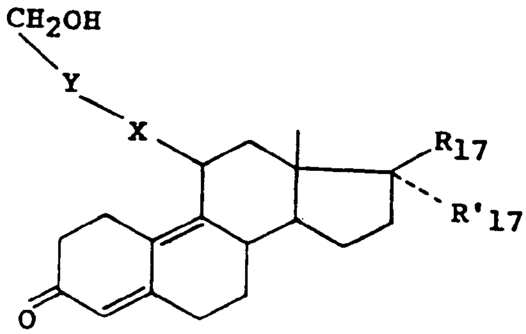

(II)

in which X, Y, $R_{17}$ and $R'_{17}$ have the same meaning as in claim 1, it being understood that $R_{17}$ cannot represent a hydroxyl radical, is subjected,

- either to the action of an oxidizing agent in order to obtain the product of formula (III):

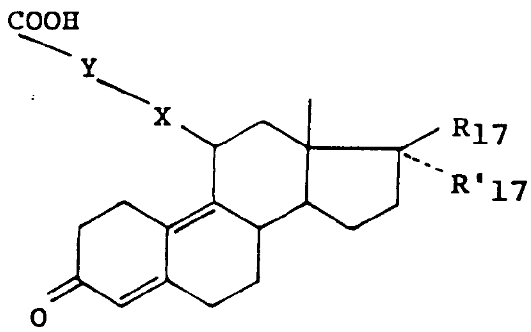

(III)

in which $R_{17}$, $R'_{17}$, X and Y have the same meaning as in claim 1 which is subjected to the action of an agent allowing the activation of the carboxylic function, then to the action of a compound of formula (IV):

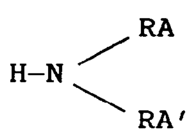

(IV)

in which RA and RA' have the same meaning as in claim 1, in order to obtain the product of formula (Ia) corresponding to the compound of formula (I) in which Z is a single bond and rings A and B represent the group

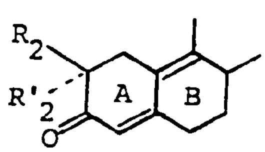

in which $R_2$ and $R'_2$ are a hydrogen atom,

- or to a reaction for introducing the radical

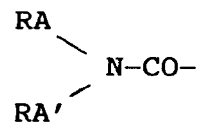

in order to obtain the product of formula (I'a) corresponding to the compound of formula (I) in which Z is a methylenoxy radical and rings A and B have the same meaning as in the products of formula (Ia),

which products (Ia) and (I'a), if desired,

- either are subjected to a reducing agent when $R_{17}$ and $R'_{17}$ together form a ketone function, then if appropriate, the derivative hydroxylated in position 17 thus obtained is subjected to an acylation agent,
- or are subjected to a saponification agent when $R_{17}$ represents an acyloxy function,

in order to obtain a product of formula (Ia) or (I'a) in which $R_{17}$ has the meaning indicated above, then if desired, any one of the products of formula (Ia) or (I'a) is subjected,

- either to an alkylation in position 2, when at least one of the $R_2$ and $R'_2$ radicals represents a hydrogen atom,
- or to an aromatisation agent of ring A, then to a saponification agent used sparingly in order to obtain the products of formula (Ib) corresponding to the products of formula (Ia) and the products of formula (I'b) corresponding to the products of formula (I'a) and in which rings A and B represent the group:

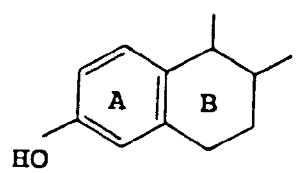

which products of formulae (Ib) and (I'b), if desired, are subjected to an alkylation or acylation reaction of the hydroxyl radical in position 3, then if desired, either, when $R_{17}$ and $R'_{17}$ together form a ketone function, are subjected to a reducing agent or subjected to a metal complex of formula (V):

$$M\text{-}R'_{17} \qquad (V)$$

in which M represents a metal atom and $R'_{17}$ has the same meaning as previously, it being understood that it is not a hydrogen atom,

or, when $R_{17}$ is a hydroxyl radical, are subjected to a selective acylation agent in position 17, then if desired, any one of the products of formula (I) obtained above, or when RA or RA' is a hydrogen atom, is subjected to the action of an appropriate alkylation agent.

**13.** Preparation process for the compounds of formula (I) in which X represents an arylene radical and Y represents an aliphatic chain optionally linked to the arylene group by a double or a triple bond and containing at least 3 carbon atoms or linked to the arylene group by an oxygen atom, characterized in that a compound of formula (X):

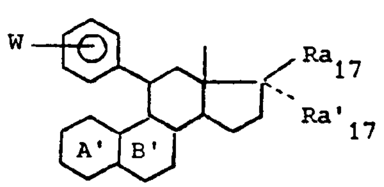

(X)

in which W represents either an OH radical or a -C=CH radical, rings A' and B', $Ra_{17}$ and $Ra'_{17}$ having the same meanings as those indicated previously for rings A and B, $R_{17}$ and $R'_{17}$ and in which the reactive functions in positions 3 and 17 are optionally protected <u>either</u>, in the case where W represents a -C=CH radical, is subjected to the action of a halogenation agent of formula (XI):

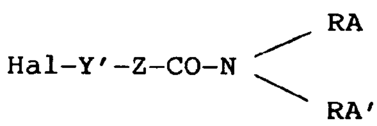

(XI)

in which Hal is a halogen atom, Z, RA and RA' have the same meaning as previously and Y' represents the above aliphatic chain Y containing at least 2 carbon atoms, in the presence of a strong base, and if appropriate is subjected to the action of a deprotection agent, in order to obtain the product of formula ($I'_A$):

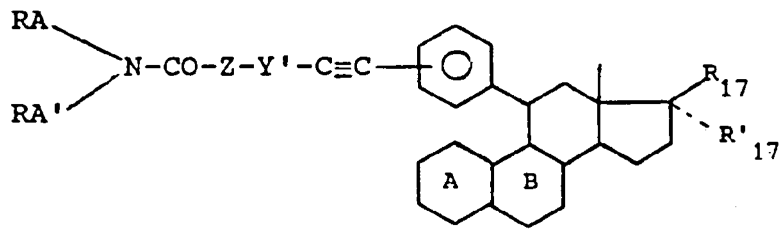

($I'_A$)

which product, if desired, is subjected to a partial or total reducing agent of triple bond in order to obtain the product of formula ($I'_B$):

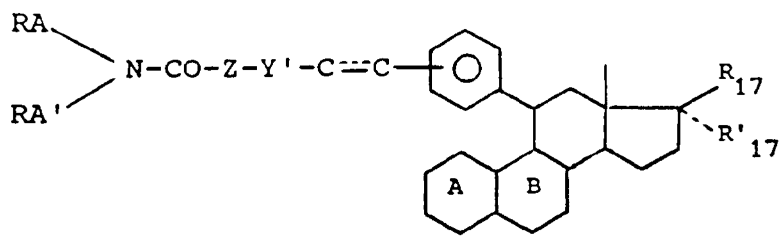

($I'_B$)

<u>or</u>, in the case where W represents an -OH radical, is subjected to the action of a halogenated derivative of formula (XII):

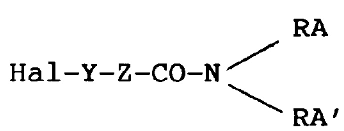

(XII)

in which Hal, Y, Z, RA and RA' have the meaning indicated previously, in the presence of an alkaline agent, then if appropriate, is subjected to the action of a deprotection agent in order to obtain a product of formula ($I''_A$):

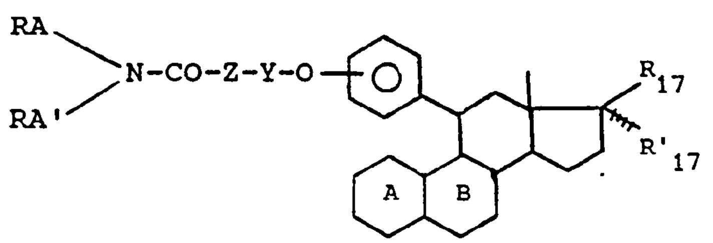

($I''_A$)

which product, if desired, when Y represents an unsaturated aliphatic chain, is subjected to a partial or total reducing agent, and which products of formulae ($I'_A$), ($I'_B$), ($I''_A$), if desired, are subjected to any one

of the reactions indicated above for (Ia), (I'a), (Ib), (I'b).

**14.** As a medicament, the compounds of formula (I) defined in any one of claims 1 to 10.

**15.** As a medicament, the compounds of formula (I) defined in claim 11.

**16.** Pharmaceutical compositions containing as active ingredient at least one medicament as defined in claim 14 or 15.

**17.** As new intermediate products, the compounds of formulae (II) and (III), as defined in claim 12.

**18.** The new intermediate products according to claim 17 corresponding to the formula:
- 17beta-acetyloxy 11beta-[(8-hydroxy octyl) phenyl] estra-4,9-dien-3-one,
- 11beta-(12-hydroxydodecyl) estra-4,9-diene-3,17-dione,
- 11beta (8-hydroxy octyl) estra-4,9-dien-3,17-dione,
- 17beta-acetyloxy 3-oxo 11beta-estra-4,9-dieneundecanoic acid,
- 3,17-dioxo 11beta-estra-4, 9-dieneundecanoic acid,
- 17beta-hydroxy 3-oxo 17-(1-propynyl) 11beta-estra-4,9-dieneundecanoic acid.

**Claims for the following Contracting State : GR**

**1.** Preparation process for the compounds of formula (I):

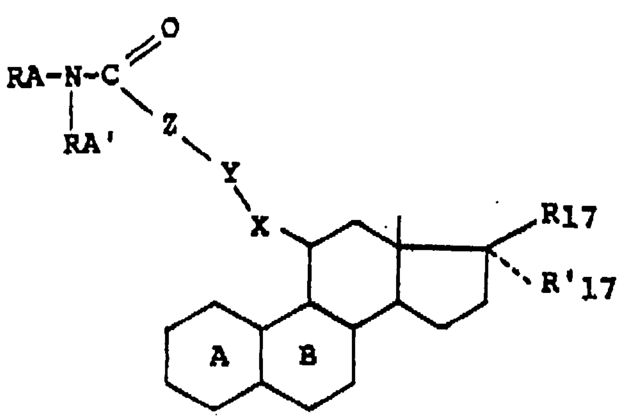

(I)

in which rings A and B have the one of the following structures:
a) either A and B represent the group:

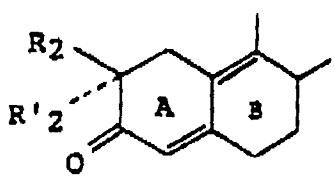

in which $R_2$ and $R'_2$ being identical or different represent a hydrogen atom or an alkyl radical having 1 to 4 carbon atoms;
b) or A and B represent the group:

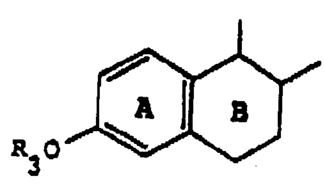

in which $R_3$ represents a hydrogen atom or an alkyl radical containing 1 to 4 carbon atoms or an acyl radical,

$R_{17}$ and $R'_{17}$ are such that:

- either $R_{17}$ and $R'_{17}$ together form a ketone function,
- or $R_{17}$ is a hydroxyl radical or an acyloxy radical and $R'_{17}$ represents a hydrogen atom, an optionally substituted alkyl, alkenyl or alkynyl radical having at most 8 carbon atoms,

X, Y and Z are such that:

- X represents a methylene radical, an arylene group, a $CH_2$-O radical or an arylenoxy radical linked to the steroid by a carbon atom,
- Y represents a single bond or a saturated or unsaturated, linear or branched aliphatic chain containing 1 to 18 carbon atoms, optionally interrupted by one or more radicals chosen from the arylene radicals, oxygen or sulphur optionally oxidized in the form of the sulphoxide or sulphone and optionally terminated by an arylene radical,
- Z represents a single bond or a $CH_2$-O radical linked to the Y radical by the carbon atom, it being understood that when Y and Z are a single bond, X cannot be a methylene or $CH_2$-O radical,

RA and RA', identical or different, represent a hydrogen atom, a linear or branched alkyl radical containing 1 to 8 carbon atoms, optionally substituted by one or more radicals chosen from aryl, alkyl or dialkylamino, hydroxy, halogen or esterified carboxy radicals, or RA and RA' form with the nitrogen atom to which they are linked a saturated or unsaturated heterocycle with 5 or 6 links, optionally containing one or more other heteroatoms chosen from the group constituted by oxygen, nitrogen and sulphur atoms and optionally substituted by an alkyl radical having 1 to 4 carbon atoms, it being understood that at least one of the RA or RA' substituents is not a hydrogen atom, characterized in that a compound of formula (II):

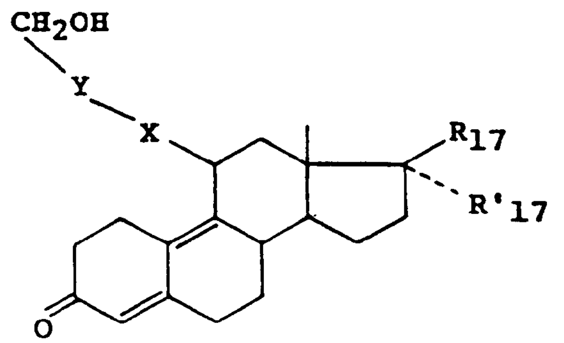

(II)

in which X, Y, $R_{17}$ and $R'_{17}$ have the same meaning as previously, it being understood that $R_{17}$ cannot represent a hydroxyl radical, is subjected,

- either to the action of an oxidizing agent in order to obtain the product of formula (III):

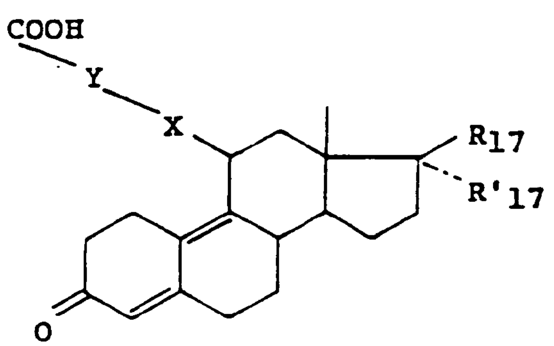

(III)

in which $R_{17}$, $R'_{17}$, X and Y have the same meaning as previously which is subjected to the action of an agent allowing the activation of the carboxylic function, then to the action of a compound of formula (IV):

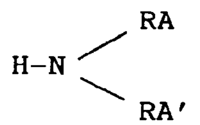

(IV)

in which RA and RA' have the same meaning as in claim 1, in order to obtain the product of formula (Ia) corresponding to the compound of formula (I) in which Z is a single bond and rings A and B represent the group

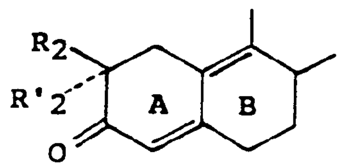

in which $R_2$ and $R'_2$ are a hydrogen atom,

- or to an introduction reaction of the radical

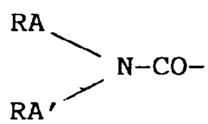

in order to obtain the product of formula (I'a) corresponding to the compound of formula (I) in which Z is a methylenoxy radical and rings A and B have the same meaning as in the products of formula (Ia),

which products (Ia) and (I'a), if desired,

- either are subjected to a reducing agent when $R_{17}$ and $R'_{17}$ together form a ketone function, then if appropriate, the derivative hydroxylated in position 17 thus obtained is subjected to an acylation agent,
- or are subjected to a saponification agent when $R_{17}$ represents an acyloxy function,

in order to obtain a product of formula (Ia) or (I'a) in which $R_{17}$ has the meaning indicated above, then if desired, any one of the products of formula (Ia) or (I'a) is subjected,

- either to an alkylation in position 2, when at least one of the $R_2$ and $R'_2$ radicals represents a hydrogen atom,
- or to an aromatisation agent of ring A, then to a saponification agent used sparingly in order to obtain the products of formula (Ib) corresponding to the products of formula (Ia) and the products of formula (I'b) corresponding to the products of formula (I'a) and in which rings A and B represent the group:

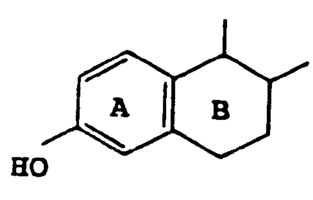

which products of formulae (Ib) and (I'b), if desired, are subjected to an alkylation or acylation reaction of the hydroxyl radical in position 3, then if desired, either, when $R_{17}$ and $R'_{17}$ together form a ketone function, are subjected to a reducing agent or subjected to a metal complex of formula (V):

$$M\text{-}R'_{17} \qquad (V)$$

in which M represents a metal atom and $R'_{17}$ has the same meaning as previously, it being understood that it is not a hydrogen atom,

or, when $R_{17}$ is a hydroxyl radical, are subjected to a selective acylation agent in position 17, then

if desired, any one of the products of formula (I) obtained above, or when RA or RA' is a hydrogen atom, is subjected to the action of an appropriate alkylation agent.

2. Process according to claim 1 for the preparation of the compounds of formula ($I_A$):

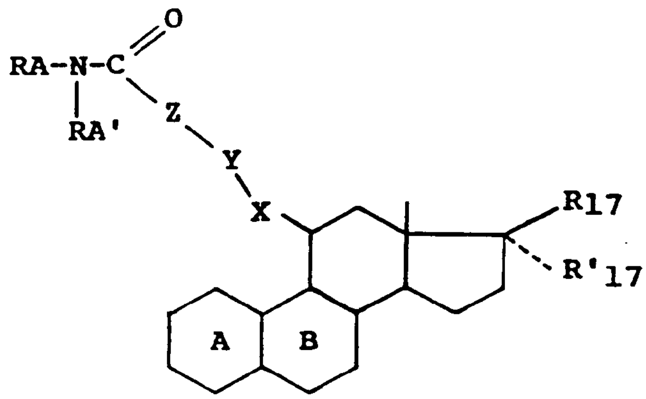

($I_A$)

in which rings A and B have one of the following structures:

a) either A and B represent the group:

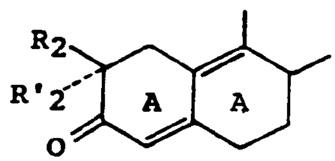

in which $R_2$ and $R'_2$ being identical or different represent a hydrogen atom or an alkyl radical having 1 to 4 carbon atoms;

b) or A and B represent the group:

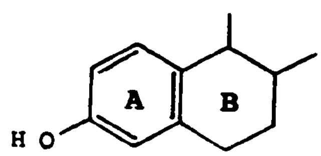

$R_{17}$ and $R'_{17}$ are such that:

- either $R_{17}$ and $R'_{17}$ together form a ketone function,
- or $R_{17}$ is a hydroxyl radical or an acyloxy radical and $R'_{17}$ represents a hydrogen atom, an optionally substituted alkyl, alkenyl or alkynyl radical having at most 8 carbon atoms, X, Y and Z are such that:
- X represents a methylene radical, an arylene group, a $CH_2$-O radical or an arylenoxy radical linked to the steroid by a carbon atom,
- Y represents a single bond or a saturated or unsaturated, linear or branched aliphatic chain containing 1 to 18 carbon atoms, optionally interrupted by one or more radicals chosen from the arylene radicals, oxygen radicals and optionally terminated by an arylene radical,
- Z represents a single bond or a $CH_2$-O radical linked to the Y radical by the carbon atom, it being understood that when Y and Z are a single bond, X cannot be a methylene or $CH_2$-O radical,

RA and RA', identical or different, represent a hydrogen atom, a linear or branched alkyl radical containing 1 to 8 carbon atoms, optionally substituted by an aryl, alkyl or dialkylamino, hydroxy, halogen or esterified carboxyl radical, or RA and RA' form with the nitrogen atom to which they are linked a saturated or unsaturated heterocycle with 5 or 6 links, optionally containing one or more other heteroatoms chosen from the group constituted by oxygen, nitrogen and sulphur atoms and optionally substituted by an alkyl radical having 1 to 4 carbon atoms, it being understood that at least one of the RA or RA' substituents is not a hydrogen atom, characterized in that a compound of formula (II):

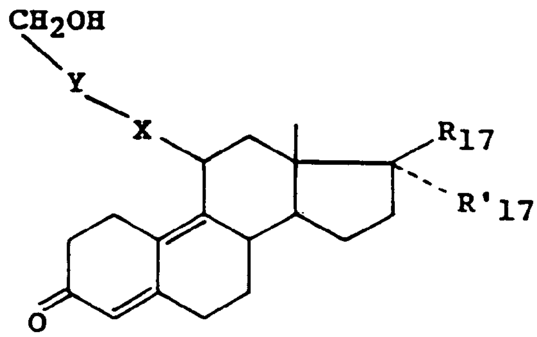

(II)

in which X, Y, $R_{17}$ and $R'_{17}$ have the same meaning as in claim 1, it being understood that $R_{17}$ cannot represent a hydroxyl radical, is subjected,

- either to the action of an oxidizing agent in order to obtain the product of formula (III):

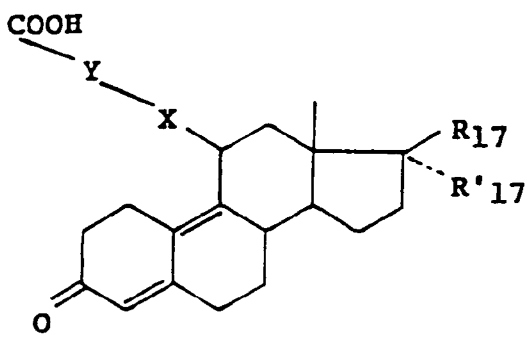

(III)

in which $R_{17}$, $R'_{17}$, X and Y have the same meaning as in claim 1 which is subjected to the action of an agent allowing the activation of the carboxylic function, then to the action of a compound of formula (IV):

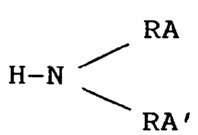

(IV)

in which RA and RA' have the same meaning as in claim 1, in order to obtain the product of formula (Ia) corresponding to the compound of formula (I) in which Z is a single bond and rings A and B represent the group

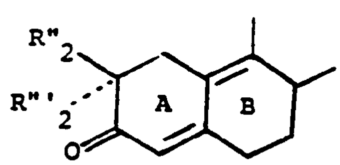

in which $R_2$ and $R'_2$ are a hydrogen atom,

- or to an introduction reaction of the radical

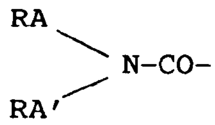

in order to obtain the product of formula (I'a) corresponding to the compound of formula ($I_A$) in which Z is a methylenoxy radical and rings A and B have the same meaning as in the products of formula (Ia),

which products (Ia) and (I'a), if desired,

- either are subjected to a reducing agent when $R_{17}$ and $R'_{17}$ together form a ketone function, then if appropriate, the derivative hydroxylated in position 17 thus obtained is subjected to an acylation agent,
- or are subjected to a saponification agent when $R_{17}$ represents an acyloxy function,

in order to obtain a product of formula (Ia) or (I'a) in which $R_{17}$ has the meaning indicated above, then if desired, any one of the products of formula (Ia) or (I'a) is subjected,

- either to an alkylation in position 2, when at least one of the $R_2$ and $R'_2$ radicals represents a hydrogen atom,
- or to an aromatisation agent of ring A, then to a saponification agent used sparingly in order to obtain the products of formula (Ib) corresponding to the products of formula (Ia) and the products of formula (I'b) corresponding to the products of formula (I'a) and in which rings A and B represent the group:

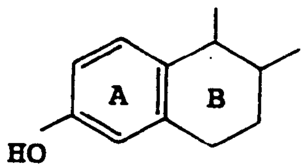

which products of formulae (Ib) and (I'b), if desired, either are subjected, when $R_{17}$ and $R'_{17}$ together form a ketone function, to a reducing agent or subjected to a metal complex of formula (V):

$$M\text{-}R'_{17} \qquad (V)$$

in which M represents a metal atom and $R'_{17}$ has the same meaning as previously, it being understood that it is not a hydrogen atom,

or, when $R_{17}$ is a hydroxyl radical, are subjected to a selective acylation agent in position 17, then if desired, any one of the products of formula (I) obtained above, or when RA or RA' is a hydrogen atom, is subjected to the action of an appropriate alkylation agent.

3. Process according to claim 2, characterized in that a product of formula (II) is used at the start in which $R'_{17}$ is a hydrogen atom, an ethynyl radical or a propynyl radical.

4. Process according to claim 2, characterized in that a product of formula (II) is used at the start in which X is a methylene radical and Y is a saturated linear chain containing 5 to 10 carbon atoms, optionally interrupted by an oxygen atom.

5. Process according to claim 2, characterized in that a product of formula (II) is used at the start in which X represents a phenylene radical and Y is a saturated or unsaturated linear chain containing 3 to 10 carbon atoms, optionally interrupted by an oxygen atom.

6. Process according to claim 1, characterized in that a product of formula (II) is used at the start in which X represents a phenylenoxy radical and Y is a saturated linear chain containing 3 to 10 carbon atoms, optionally interrupted by an oxygen or sulphur atom.

7. Process according to claim 2, characterized in that the following are used at the start: a compound of formula (IV) or a reagent for intorducing the

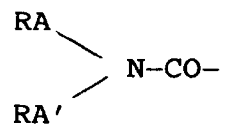

radical, or if appropriate a reagent such that:

- either RA and RA' being identical represent a methyl radical,
- or RA represents a hydrogen atom or a methyl radical and RA' represents a butyl radical,
- or RA represents a methyl radical and RA' represents an isopropyl, dimethylaminoethyl, benzyl or heptafluorobutyl radical,
- or RA and RA' together form an optionally N-substituted piperazine radical or a pyrrolidine radical.

8. Process according to claim 1, characterized in that the starting products are chosen so as to prepare the following:
   - N-(2-dimethylaminoethyl) 17beta-hydroxy N-methyl 3-oxo 11beta-estra-4,9-dien-undecanamide,
   - N-butyl 4-(3,17beta-dihydroxy estra-1,3,5(10)-trien-11beta-yl) N-methyl benzene octanamide,
   - 3,17beta-dihydroxy N-methyl N-(1-methylethyl) 11beta-estra-1,3,5(10)-trien-undecanamide,
   - N-butyl 3,17beta-dihydroxy N-methyl 19-Nor 11beta-(17alpha-pregna-1,3,5(10)-trien-20-yne) undecanamide,
   - 3,17beta-dihydroxy N-methyl N-(1-methylethyl) 19-nor 17alpha-pregna 1,3,5(10)-trien-20-yn-11beta-undecanamide,
   - [[8-(3,17beta-dihydroxy estra 1,3,5(10)-trien-11beta-yl) octyl] oxy] N-methyl N-(1-methylethyl) acetamide,
   - N-butyl 8-[4-(3,17beta-dihydroxy estra 1,3,5(10)-trien-11beta-yl) phenoxy] N-methyl octanamide,
   - N-butyl [5-[4-(3,17beta-dihydroxy estra 1,3,5(10)-trien-11beta-yl) phenoxy] pentyloxy] N-methyl acetamide,
   - 2-[[7-[4-(3,17beta-dihydroxy estra 1,3,5(10)-trien-11beta-yl) phenyl] 6-heptylyl] oxy] N-butyl N-methyl acetamide,
   - 3,17beta-dihydroxy N-(2,2,3,3,4,4,4-heptafluobutyl) N-methyl estra 1,3,5(10)-trien-11beta-yl undecanamide,
   - 8-[4-(3,17beta-dihydroxy estra 1,3,5(10)-trien-11beta-yl) phenyl] N-butyl N-methyl octynamide.

9. Preparation process for the products of formula (I) as defined in claim 1, in which X represents an arylene radical and Y represents an aliphatic chain optionally linked to the arylene group by a double or a triple bond and containing at least 3 carbon atoms or linked to the arylene group by an oxygen atom, characterized in that a compound of formula (X):

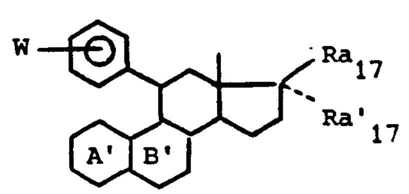

(X)

in which W represents either an OH radical or a -C=CH radical, rings A' and B', $Ra_{17}$ and $Ra'_{17}$ having the same meanings as those indicated previously for rings A and B, $R_{17}$ and $R'_{17}$ and in which the reactive functions in positions 3 and 17 are optionally protected either, in the case where W represents a -C=CH radical is subjected to the action of a halogenation agent of formula (XI):

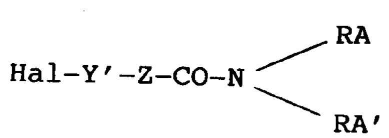

(XI)

in which Hal is a halogen atom, Z, RA and RA' have the same meaning as previously and Y' represents the above aliphatic chain Y containing at least 2 carbon atoms in the presence of a strong base, and if

appropriate is subjected, to the action of a deprotection agent, in order to obtain the product of formula ($I'_A$):

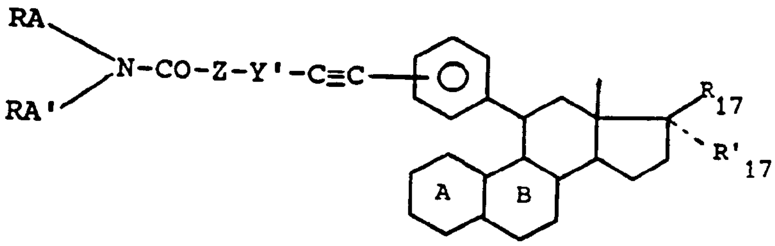

($I'_A$)

which product, if desired, is subjected to a partial or total reducing agent of the triple bond in order to obtain the product of formula ($I'_B$):

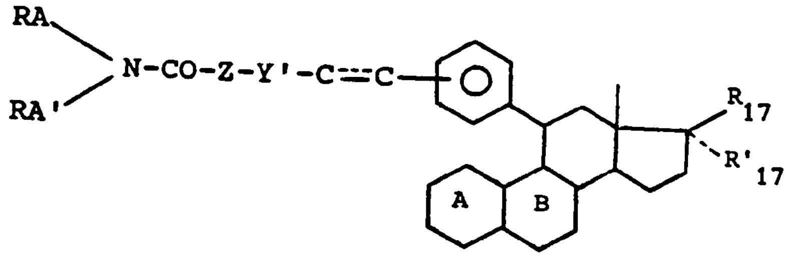

($I'_B$)

or, in the case where W represents an -OH radical, is subjected to the action of a halogenated derivative of formula (XII):

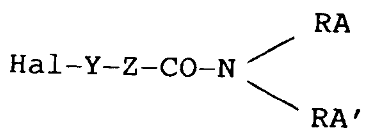

(XII)

in which Hal, Y, Z, RA and RA' have the meaning indicated previously, in the presence of an alkaline agent, then if appropriate, is subjected to the action of a deprotection agent in order to obtain a product of formula ($I''_A$):

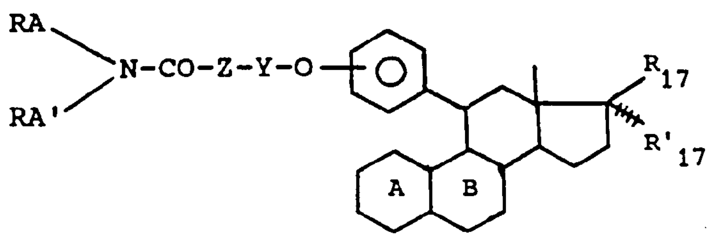

($I''_A$)

which product, if desired, when Y represents an unsaturated aliphatic chain, is subjected to a partial or total reducing agent, and which products of formulae ($I'_A$), ($I'_B$), ($I''_A$), if desired, are subjected to any one of the reactions indicated above for (Ia), (I'a), (Ib), (I'b).

**10.** Process according to claim 8, characterized in that a compound of formula (X) is used at the start in which $R'_{17}$ is a hydrogen atom, an ethynyl radical or a propynyl radical.

11. Process according to claim 9, characterized in that a compound of formula (X) is used at the start in which W represents a -C=CH radical and a compound of formula (XI) is used at the start in which Y is a saturated linear chain containing 5 to 10 carbon atoms optionally interrupted by an oxygen atom.

12. Process according to claim 9, characterized in that a compound of formula (X) is used at the start in which W represents an OH radical and a compound of formula (XII) is used at the start in which Y is a saturated linear chain containing 3 to 10 carbon atoms, optionally interrupted by an oxygen or sulphur atom.

13. Process according to claim 9, characterized in that a compound of formula (XI) or a compound of formula (XII) or, if appropriate, an alkylation reagent such that
- either RA and RA' are identical and represent a methyl radical,
- or RA represents a hydrogen atom or a methyl radical and RA' represents a butyl radical,
- or RA represents a methyl radical and RA' represents an isopropyl, dimethylaminoethyl, benzyl or heptafluorobutyl radical,
- or RA and RA' together form an optionally N-substituted piperazine radical or a pyrrolidine radical, are used at the start.

14. Process according to claim 9, characterized in that the starting products are chosen so as to prepare the following:
- N-butyl 4-(3,17beta-dihydroxy estra-1,3,5(10)-trien-11beta-yl) N-methyl benzene octanamide,
- N-butyl 8-[4-(3,17beta-dihydroxy estra 1,3,5(10)-trien-11beta-yl) phenoxy] N-methyl acetamide,
- N-butyl [5-[4-(3,17beta-dihydroxy estra 1,3,5(10)-trien-11beta-yl) phenoxy] pentyloxy] N-methyl acetamide,
- 2-[[7-[4-(3,17beta-dihydroxy estra 1,3,5(10)-trien-11beta-yl) phenyl] 6-heptylyl] oxy] N-butyl N-methyl acetamide,
- 8-[4-(3,17beta-dihydroxy estra 1,3,5(10)-trien-11beta-yl) phenyl] N-butyl N-methyl octynamide.

15. As new industrial products, the compounds of formulae (II) and (III), as defined in claim 1.

16. The new products according to claim 16 corresponding to the following formula:
- 17beta-acetyloxy 11beta-[(8-hydroxy octyl) phenyl] estra-4,9-dien-3-one,
- 11beta-(12-hydroxy dodecyl) estra-4,9-diene-3,17-dione,
- 11beta (8-hydroxy octyl) estra-4,9-dien-3,17-dione,
- 17beta-acetyloxy 3-oxo 11beta-estra-4,9-dieneundecanoic acid,
- 3,17-dioxo 11beta-estra-4,9-dieneundecanoic acid,
- 17beta-hydroxy 3-oxo 17-(1-propynyl) 11beta-estra-4,9-dieneundecanoic acid.

17. Preparation process for pharmaceutical compositions characterized in that at least one of the products of general formula (I) as defined in claim 1 or 9 is used as active ingredient in a form intended for this use.

18. Preparation process for pharmaceutical compositions characterized in that at least one of the products of general formula ($I_A$) as defined in claim 2 is used as active ingredient in a form intended for this use.

19. Preparation process for pharmaceutical compositions characterized in that at least one of the products of general formula (I) as defined in claim 8 or 15 is used as active ingredient in a form intended for this use.

**Claims for the following Contracting State : ES**

1. Preparation process for the compounds of formula (I):

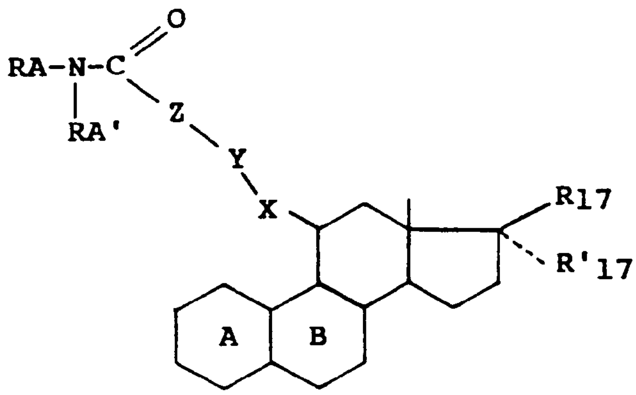

(I)

in which rings A and B have the one of the following structures:

a) either A and B represent the group:

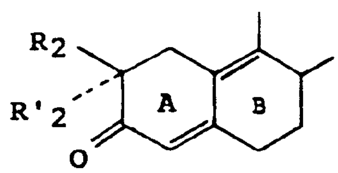

in which $R_2$ and $R'_2$ being identical or different represent a hydrogen atom or an alkyl radical having 1 to 4 carbon atoms;

b) or A and B represent the group:

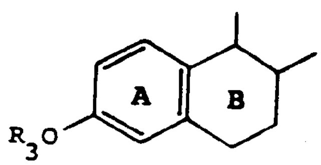

in which $R_3$ represents a hydrogen atom or an alkyl radical containing 1 to 4 carbon atoms or an acyl radical,

$R_{17}$ and $R'_{17}$ are such that:

- either $R_{17}$ and $R'_{17}$ together form a ketone function,
- or $R_{17}$ is a hydroxyl radical or an acyloxy radical and $R'_{17}$ represents a hydrogen atom, an optionally substituted alkyl, alkenyl or alkynyl radical having at most 8 carbon atoms, X, Y and Z are such that:
- X represents a methylene radical, an arylene group, a $CH_2$-O radical or an arylenoxy radical linked to the steroid by a carbon atom,
- Y represents a single bond or a saturated or unsaturated, linear or branched aliphatic chain containing 1 to 18 carbon atoms, optionally interrupted by one or more radicals chosen from the arylene radicals, oxygen or sulphur optionally oxidized in the form of the sulphoxide or sulphone and optionally terminated by an arylene radical,
- Z represents a single bond or a $CH_2$-O radical linked to the Y radical by the carbon atom,
  it being understood that when Y and Z are a single bond, X cannot be a methylene or $CH_2$-O radical,
  RA and RA', identical or different, represent a hydrogen atom, a linear or branched alkyl radical containing 1 to 8 carbon atoms, optionally substituted by one or more radicals chosen from aryl, alkyl or dialkylamino, hydroxy, halogen or esterified carboxyl radicals, or RA and RA' form with the nitrogen atom to which they are linked a saturated or unsaturated heterocycle with 5 or 6 links, optionally containing one or more other heteroatoms chosen from the group constituted by oxygen, nitrogen and sulphur atoms and optionally substituted by an alkyl radical having 1 to 4 carbon atoms, it being understood that at least one of the RA or RA' substituents is not a hydrogen atom,

characterized in that a compound of formula (II):

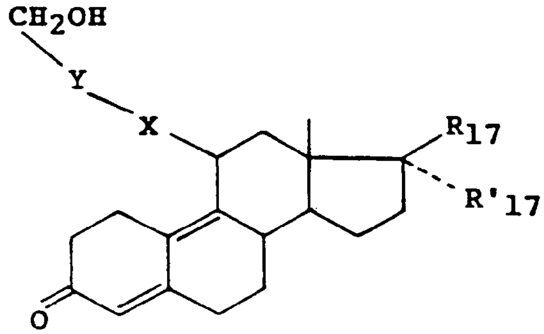

(II)

in which X, Y, $R_{17}$ and $R'_{17}$ have the same meaning as previously, it being understood that $R_{17}$ cannot represent a hydroxyl radical, is subjected,

- either to the action of an oxidizing agent in order to obtain the product of formula (III):

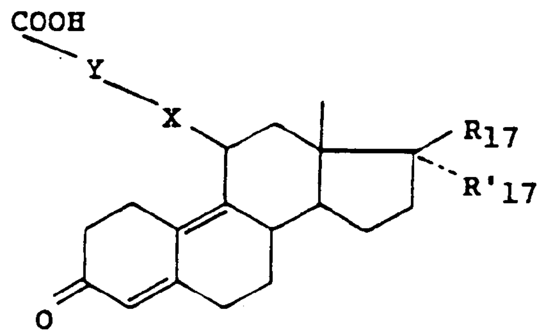

(III)

in which $R_{17}$, $R'_{17}$, X and Y have the same meaning as in claim 1 which is subjected to the action of an agent allowing the activation of the carboxylic function, then to the action of a compound of formula (IV):

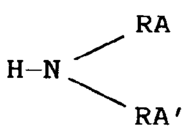

(IV)

in which RA and RA' have the same meaning as previously, in order to obtain the product of formula (Ia) corresponding to the compound of formula (I) in which Z is a single bond and rings A and B represent the group

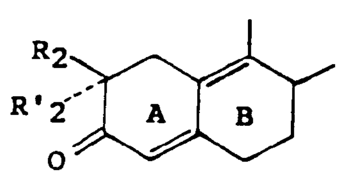

in which $R_2$ and $R'_2$ are a hydrogen atom,

- or to an introduction reaction of the radical

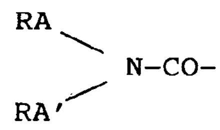

in order to obtain the product of formula (I'a) corresponding to the compound of formula (I) in which Z is a methylenoxy radical and rings A and B have the same meaning as in the products of formula (Ia),
which products (Ia) and (I'a), if desired,

- either are subjected to a reducing agent when $R_{17}$ and $R'_{17}$ together form a ketone function, then if appropriate, the derivative hydroxylated in position 17 thus obtained is subjected to an acylation agent,
- or are subjected to a saponification agent when $R_{17}$ represents an acyloxy function,

in order to obtain a product of formula (Ia) or (I'a) in which $R_{17}$ has the meaning indicated above, then if desired, any one of the products of formula (Ia) or (I'a) is subjected,

- either to an alkylation in position 2, when at least one of the $R_2$ and $R'_2$ radicals represents a hydrogen atom,
- or to an aromatisation agent of ring A, then to a saponification agent used sparingly in order to obtain the products of formula (Ib) corresponding to the products of formula (Ia) and the products of formula (I'b) corresponding to the products of formula (I'a) and in which rings A and B represent the group:

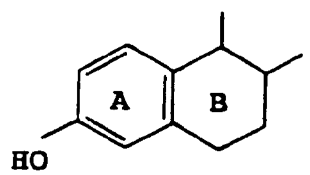

which products of formula (Ib) and (I'b), if desired, are subjected to an alkylation or acylation reaction of the hydroxyl radical in position 3, then if desired,
either, when $R_{17}$ and $R'_{17}$ together form a ketone function, are subjected to a reducing agent or subjected to a metal complex of formula (V):

$$M\text{-}R'_{17} \qquad (V)$$

in which M represents a metal atom and $R'_{17}$ has the same meaning as previously, it being understood that it is not a hydrogen atom,
or, when $R_{17}$ is a hydroxyl radical, are subjected to a selective acylation agent in position 17, then if desired, any one of the products of formula (I) obtained above, or when RA or RA' is a hydrogen atom, is subjected to the action of an appropriate alkylation agent.

2. Process according to claim 1 for the preparation of the compounds of formula ($I_A$):

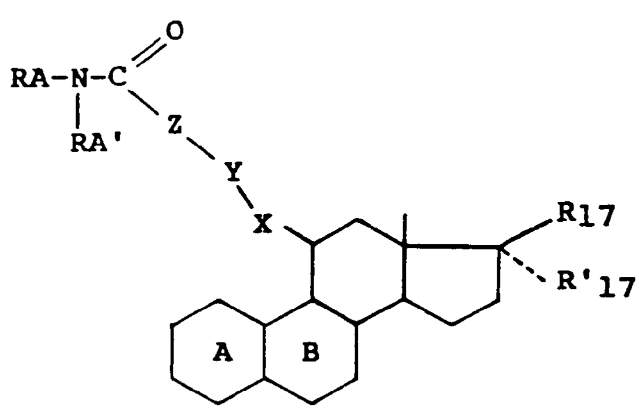

($I_A$)

in which rings A and B have one of the following structures:

a) either A and B represent the group:

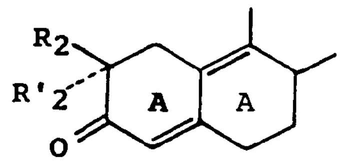

in which $R_2$ and $R'_2$ being identical or different represent a hydrogen atom or an alkyl radical having 1 to 4 carbon atoms;
b) or A and B represent the group:

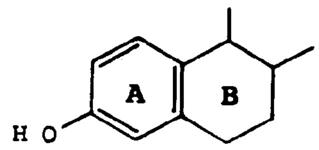

$R_{17}$ and $R'_{17}$ are such that:

- either $R_{17}$ and $R'_{17}$ together form a ketone function,
- or $R_{17}$ is a hydroxyl radical or an acyloxy radical and $R'_{17}$ represents a hydrogen atom, an optionally substituted alkyl, alkenyl or alkynyl radical having at most 8 carbon atoms,
  X, Y and Z are such that:
- X represents a methylene radical, an arylene group, a $CH_2$-O radical or an arylenoxy radical linked to the steroid by a carbon atom,
- Y represents a single bond or a saturated or unsaturated, linear or branched aliphatic chain containing 1 to 18 carbon atoms, optionally interrupted by one or more radicals chosen from the arylene radicals, oxygen radicals and optionally terminated by an arylene radical,
- Z represents a single bond or a $CH_2$-O radical linked to the Y radical by the carbon atom,
  it being understood that when Y and Z are a single bond, X cannot be a methylene or $CH_2$-O radical,
  RA and RA', identical or different, represent a hydrogen atom, a linear or branched alkyl radical containing 1 to 8 carbon atoms, optionally substituted by an aryl, alkyl or dialkylamino, hydroxy, halogen or esterified carboxyl radical, or RA and RA' form with the nitrogen atom to which they are linked a saturated or unsaturated heterocycle with 5 or 6 links, optionally containing one or more other heteroatoms chosen from the group constituted by oxygen, nitrogen and sulphur atoms and optionally substituted by an alkyl radical having 1 to 4 carbon atoms, it being understood that at least one of the RA or RA' substituents is not a hydrogen atom, characterized in that a compound of formula (II):

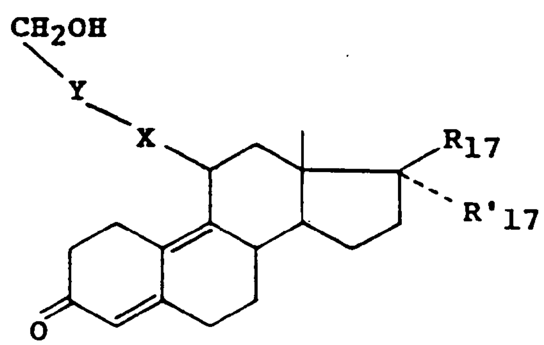

(II)

in which X, Y, $R_{17}$ and $R'_{17}$ have the same meaning as in claim 1, it being understood that $R_{17}$ cannot represent a hydroxyl radical, is subjected,

- either to the action of an oxidizing agent in order to obtain the product of formula (III):

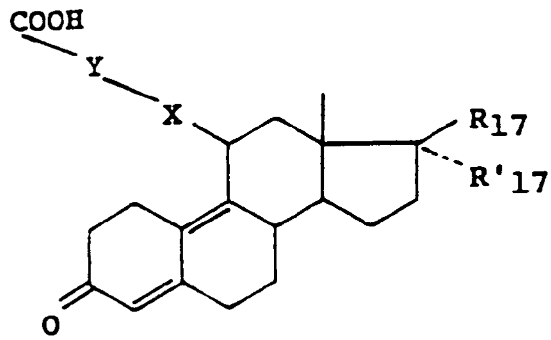

(III)

in which $R_{17}$, $R'_{17}$, X and Y have the same meaning as in claim 1 which is subjected to the action of an agent allowing the activation of the carboxylic function, then to the action of a compound of formula (IV):

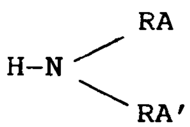

(IV)

in which RA and RA' have the same meaning as in claim 1, in order to obtain the product of formula (Ia) corresponding to the compound of formula (I) in which Z is a single bond and rings A and B represent the group

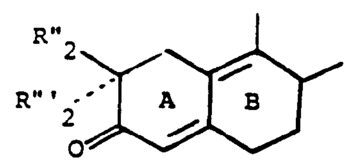

in which $R_2$ and $R'_2$ are a hydrogen atom,

- or to an introduction reaction of the radical

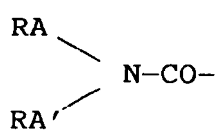

in order to obtain the product of formula (I'a) corresponding to the compound of formula (I) in which Z is a methylenoxy radical and rings A and B have the same meaning as in the products of formula (Ia),
which products (Ia) and (I'a), if desired,

- either are subjected to a reducing agent when $R_{17}$ and $R'_{17}$ together form a ketone function, then if appropriate, the derivative hydroxylated in position 17 thus obtained is subjected to an acylation agent,
- or are subjected to a saponification agent when $R_{17}$ represents an acyloxy function,

in order to obtain a product of formula (Ia) or (I'a) in which $R_{17}$ has the meaning indicated above, then if desired, any one of the products of formula (Ia) or (I'a) is subjected,

- either to an alkylation in position 2, when at least one of the $R_2$ and $R'_2$ radicals represents a hydrogen atom,
- or to an aromatisation agent of ring A, then to a saponification agent used sparingly in order to obtain the products of formula (Ib) corresponding to the products of formula (Ia) and the products of formula (I'b) corresponding to the products of formula (I'a) and in which rings A and B represent the group:

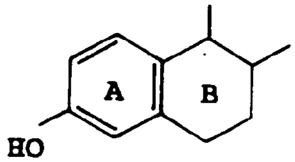

which products of formula (Ib) and (I'b), if desired, either, when $R_{17}$ and $R'_{17}$ together form a ketone function, are subjected to a reducing agent or are subjected to a metal complex of formula (V):

$$M\text{-}R'_{17} \qquad (V)$$

in which M represents a metal atom and $R'_{17}$ has the same meaning as previously, it being understood that it is not a hydrogen atom,

or, when $R_{17}$ is a hydroxyl radical, are subjected to a selective acylation agent in position 17, then if desired, subject any one of the products of formula (I) obtained above, or when RA or RA' is a hydrogen atom, is subjected to the action of an appropriate alkylation agent.

**3.** Process according to claim 2, characterized in that a product of formula (II) is used at the start in which $R'_{17}$ is a hydrogen atom, an ethynyl radical or a propynyl radical.

**4.** Process according to claim 2, characterized in that a product of formula (II) is used at the start in which X is a methylene radical and Y is a saturated linear chain containing 5 to 10 carbon atoms, optionally interrupted by an oxygen atom.

**5.** Process according to claim 2, characterized in that a product of formula (II) is used at the start in which X represents a phenylene radical and Y is a saturated or unsaturated linear chain containing 3 to 10 carbon atoms, optionally interrupted by an oxygen atom.

**6.** Process according to claim 1, characterized in that a product of formula (II) is used at the start in which X represents a phenylenoxy radical and Y is a saturated linear chain containing 3 to 10 carbon atoms, optionally interrupted by an oxygen or sulphur atom.

**7.** Process according to claim 2, characterized in that there are used at the start: a product of formula (II) is used at the start or a reagent for introducing the

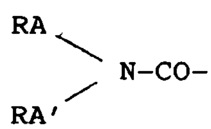

radical, or if appropriate a reagent such that:

- either RA and RA' being identical represent a methyl radical,
- or RA represents a hydrogen atom or a methyl radical and RA" radicals a butyl radical,
- or RA represents a methyl radical and RA' represents an isopropyl, dimethylaminoethyl, benzyl or heptafluorobutyl radical,
- or RA and RA' together form an optionally N-substituted piperazine radical or a pyrrolidine radical.

**8.** Preparation process for the products of formula (I) as defined in claim 1, in which X represents an arylene radical and Y represents an aliphatic chain optionally linked to the arylene group by a double or a triple bond and containing at least 3 carbon atoms or linked to the arylene group by an oxygen atom, characterized in that a compound of formula (X):

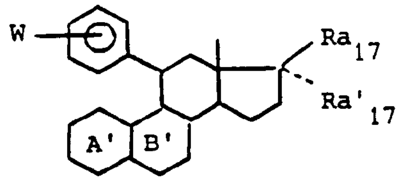

(X)

in which W represents either an OH radical or a -C=CH radical, rings A' and B', $Ra_{17}$ and $Ra'_{17}$ having the same meanings as those indicated previously for rings A and B, $R_{17}$ and $R'_{17}$ and in which the reactive functions in positions 3 and 17 are optionally protected either, in the case where W represents a -C=CH radical is subjected to the action of a halogenation agent of formula (XI):

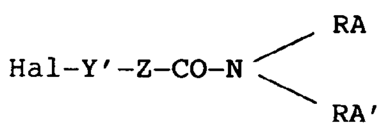

(XI)

in which Hal is a halogen atom, Z, RA and RA' have the same meaning as previously and Y' represents the above aliphatic chain Y containing at least 2 carbon atoms, in the presence of a strong base, and if appropriate is subjected to the action of a deprotection agent, in order to obtain the product of formula ($I'_A$):

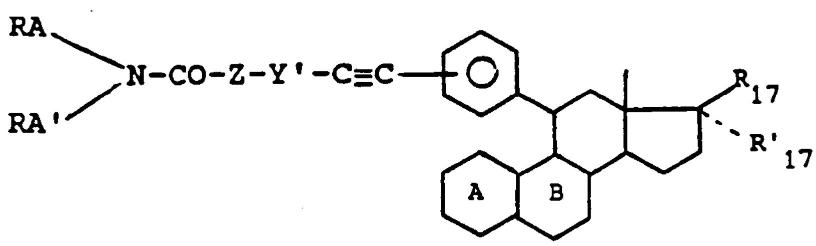

($I'_A$)

which product, if desired, is subjected to a partial or total reducing agent of the triple bond in order to obtain the product of formula ($I'_B$):

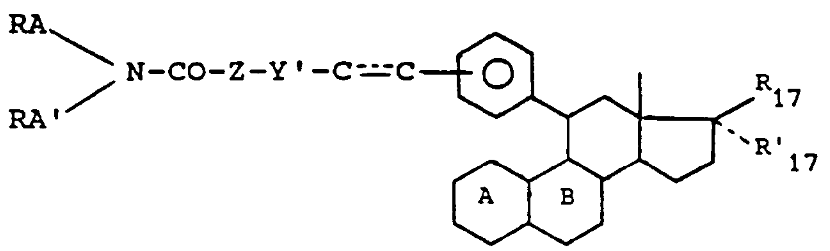

($I'_B$)

or, in the case where W represents an -OH radical, is subjected to the action of a halogenated derivative of formula (XII):

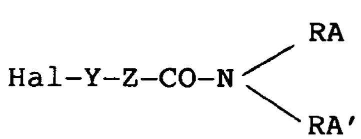

(XII)

in which Hal, Y, Z, RA and RA' have the meaning indicated previously, in the presence of an alkaline agent, then if appropriate, subjected to the action of a deprotection agent in order to obtain a product of formula ($I''_A$):

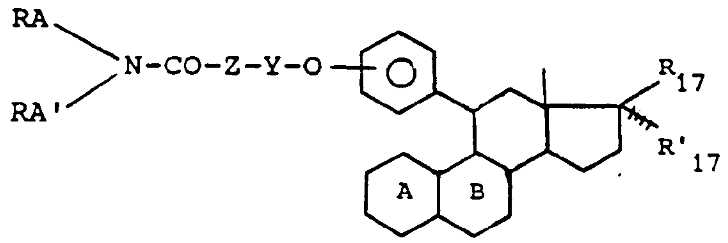

($I''_A$)

which product, if desired, when Y represents an unsaturated aliphatic chain, is subjected to a partial or total reducing agent, and which products of formulae ($I'_A$), ($I'_B$), ($I''_A$), if desired, are subjected to any one of the reactions indicated above for (Ia), (I'a), (Ib), (I'b).

9. Process according to claim 8, characterized in that in that a compound of formula (X) is used at the start in which $R'_{17}$ is a hydrogen atom, an ethynyl radical or a propynyl radical.

10. Process according to claim 9, characterized in that a compound of formula (X) is used at the start in which W represents a -C=CH radical and a compound of formula (XI) is used at the start in which Y is a saturated linear chain containing 5 to 10 carbon atoms optionally interrupted by an oxygen atom.

11. Process according to claim 9, characterized in that a compound of formula (X) is used at the start in which W represents an OH radical and a compound of formula (XII) is used at the start in which Y is a saturated linear chain containing 3 to 10 carbon atoms, optionally interrupted by an oxygen or sulphur atom.

12. Process according to claim 9, characterized in that a compound of formula (X) or a compound of formula (XII) or, if appropriate, an alkylation reagent such that
    - either RA and RA' are identical and represent a methyl radical,
    - or RA represents a hydrogen atom or a methyl radical and RA' represents a butyl radical,
    - or RA represents a methyl radical and RA' represents an isopropyl, dimethylaminoethyl, benzyl or heptafluorobutyl radical,
    - or RA and RA' together form an optionally N-substituted piperazine or a pyrrolidine radical, are used at the start.

**Patentansprüche**

**Patentansprüche für folgende Vertragsstaaten : AT, BE, CH, DE, FR, GB, IT, LI, LU, NL, SE, DK**

1. Verbindungen der Formel (I)

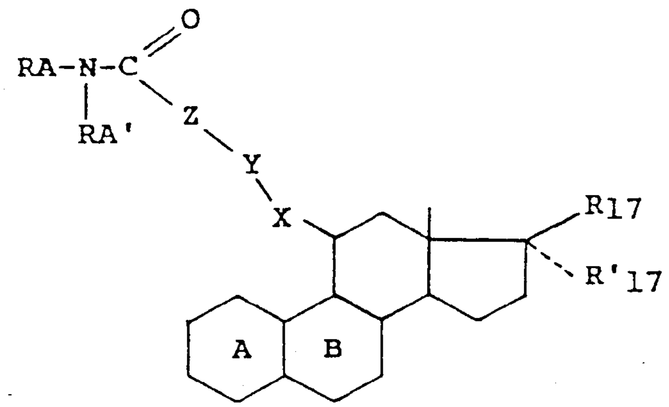

(I)

worin die Ringe A und B eine der folgenden Strukturen besitzen:

(a) entweder bedeuten A und B die Gruppe

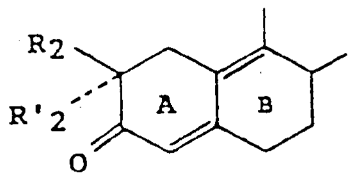

worin $R_2$ und $R'_2$, identisch oder verschieden, ein Wasserstoffatom oder eine Alkylgruppe mit 1 bis 4 Kohlenstoffatomen wiedergeben;

(b) oder A und B bedeuten die Gruppe

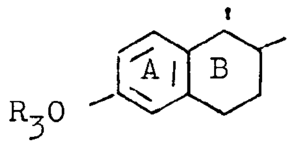

worin $R_3$ für ein Wasserstoffatom oder eine Alkylgruppe mit 1 bis 4 Kohlenstoffatomen oder einen Acylrest steht;

$R_{17}$ und $R'_{17}$ derart sind, daß

- entweder $R_{17}$ und $R'_{17}$ gemeinsam eine Ketofunktion bilden,
- oder $R_{17}$ ein Hydroxylrest oder ein Acyloxyrest ist und $R'_{17}$ ein Wasserstoffatom, einen Alkyl-, Alkenyl- oder Alkinylrest mit höchstens 8 Kohlenstoffatomen, der gegebenenfalls substituiert ist, bedeutet;

X, Y und Z derart sind, daß

- X eine Methylengruppe, eine Arylengruppe, einen Rest $CH_2$-O oder einen Arylenoxyrest, gebunden an das Steroid über ein Kohlenstoffatom, bedeutet,
- Y eine einfache Bindung oder eine gesättigte oder ungesättigte, aliphatische, lineare oder verzweigte Kette mit 1 bis 18 Kohlenstoffatomen, gegebenenfalls unterbrochen durch einen oder mehrere Reste, ausgewählt unter den Arylenresten, Sauerstoff oder in Form des Sulfoxids oder Sulfons oxiertem Schwefel und gegebenenfalls durch eine Arylengruppe beendet, darstellt,
- Z eine einfache Bindung oder einen Rest $CH_2$-O, der an den Rest Y durch das Kohlenstoffatom gebunden ist, wiedergibt,

wobei, wenn Y und Z eine einfache Bindung darstellen, X keinen Methylenrest oder $CH_2$-O bedeuten kann; RA und RA', identisch oder verschieden, ein Wasserstoffatom, einen linearen oder verzweigten Alkylrest mit 1 bis 8 Kohlenstoffatomen, gegebenenfalls substituiert durch einen oder mehrere Reste, ausgewählt unter den Aryl-,Alkyl- oder Dial-kylamino-, Hydroxy-, Halogen- oder veresterten Carboxylresten, darstellen oder RA und RA' mit dem Stickstoffatom, an das sie gebunden sind, einen gesättigten oder ungesättigten Heterocyclus mit 5 oder 6 Kettengliedern bilden, der gegebenenfalls ein oder mehrere weitere Heteroatome, ausgewählt unter den Sauerstoff-, Stickstoff- und Schwefelatomen, enthält und gegebenenfalls durch einen Alkylrest mit 1 bis 4 Kohlenstoffatomen substituiert ist, wobei zumindest einer der Substituenten RA oder RA' kein Wasserstoffatom ist,

2. Verbindungen der Formel (I), wie in Anspruch 1 definiert, worin die Ringe A und B die Gruppe

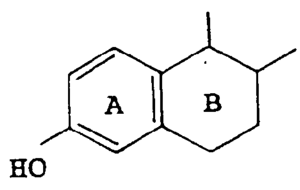

wiedergeben.

3. Verbindungen der Formel (I), wie in Anspruch 1 definiert, worin die Ringe A und B die Gruppe

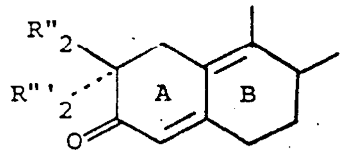

wiedergeben, worin R"$_2$ oder R'"$_2$ ein Wasserstoffatom oder eine Methylgruppe, vorzugsweise ein Wasserstoffatom, bedeutet.

**4.** Verbindungen der Formel (I), wie in Anspruch 1, 2 oder 3 definiert, worin Z eine Einfachbindung ist.

**5.** Verbindungen der Formel (I), wie in einem der Ansprüche 1 bis 4 definiert, worin $R_{17}$ eine Hydroxylgruppe ist.

**6.** Verbindungen der Formel (I), wie in einem der Ansprüche 1 bis 5 definiert, worin $R'_{17}$ ein Wasserstoffatom, einen Ethinylrest oder einen Propinylrest bedeutet.

**7.** Verbindungen der Formel (I), wie in einem der Ansprüche 1 bis 6 definiert, worin X einen Methylenrest bedeutet und Y eine gesättigte, lineare Kette mit 5 bis 10 Kohlenstoffatomen ist, die gegebenenfalls durch ein Sauerstoffatom unterbrochen ist.

**8.** Verbindungen der Formel (I), wie in einem der Ansprüche 1 bis 6 definiert, worin X eine Phenylengruppe ist und Y eine gesättigte oder ungesättigte, lineare Kette mit 3 bis 10 Kohlenstoffatomen bedeutet, die gegebenenfalls durch ein Sauerstoffatom unterbrochen ist.

**9.** Verbindungen der Formel (I), wie in einem der Ansprüche 1 bis 6 definiert, worin X ein Phenylenoxyrest ist und Y eine gesättigte, lineare Kette mit 3 bis 10 Kohlenstoffatomen wiedergibt, die gegebenenfalls durch ein Sauerstoff- oder Schwefelatom unterbrochen ist.

**10.** Verbindungen der Formel (I), wie in einem der Ansprüche 1 bis 9 definiert, worin
- entweder RA und RA' identisch eine Methylgruppe sind,
- oder RA ein Wasserstoffatom oder eine Methylgruppe bedeuten und RA' für eine Butylgruppe steht,
- oder RA eine Methylgruppe ist und RA' für einen Isopropyl-, Dimethylaminoethyl-, Benzyl- oder Heptafluorobutylrest bedeutet oder
- RA und RA' gemeinsam ein gegebenenfalls N-substituiertes Piperazin oder ein Pyrrolidin bilden.

**11.** Verbindungen der Formel (I), wie in einem der Ansprüche 1 bis 10 definiert, mit den folgenden Bezeichnungen:
N-(2-Dimethylaminoethyl)-17β-hydroxy-N-methyl-3-oxo-11β-östra-4,9-dien-undecanamid,
N-Butyl-4-(3,17β-dihydroxy-östra-1,3,5(10)-trien-11β-yl)-N-methylbenzoloctanamid,
3,17β-Dihydroxy-N-methyl-N-(1-methylethyl)-11β-östra-1,3,5(10)-trien-undecanamid,
N-Butyl-3,17β-dihydroxy-N-methyl-19-nor-11β-(17α-pregna-1,3,5(10)-trien-20-in)-undecanamid,
3,17β-Dihydroxy-N-methyl-N-(1-methylethyl)-19-nor-17α-pregna-1,3,5(10)-trien-20-in-11β-undecanamid,
[[8-(3,17β-Dihydroxy-ostra-1,3,5(10)-trien-11β-yl)-octyl]-oxy]-N-methyl-N-(1-methylethyl)-acetamid,
N-Butyl-8-[4-(3,17β-dihydroxy-östra-1,3,5(10)-trien-11β-yl)-phenoxy]-N-methyl-octanamid,
N-Butyl-[5-[4-(3,17β-dihydroxy-östra-1,3,5(10)-trien-11β-yl)-phenoxy]-pentyloxy]-N-methyl-acetamid,
2-[[7-[4-(3,17β-Dihydroxy-östra-1,3,5(10)-trien-11β-yl)-phenyl]-6-heptylyl]-oxy]-N-butyl-N-methylacetamid,
3,17β-Dihydroxy-N-(2,2,3,3,4,4,4-heptafluorbutyl)-N-methyl-östra-1,3,5(10)-trien-11β-yl-undecanamid,
8-[4-(3,17β-Dihydroxy-östra-1,3,5(10)-trien-11β-yl)-phenyl]-N-butyl-N-methyl-octinamid.

**12.** Verfahren zur Herstellung der Verbindungen der Formel (I), wie in Anspruch 1 definiert, dadurch gekennzeichnet, daß man eine Verbindung der Formel (II)

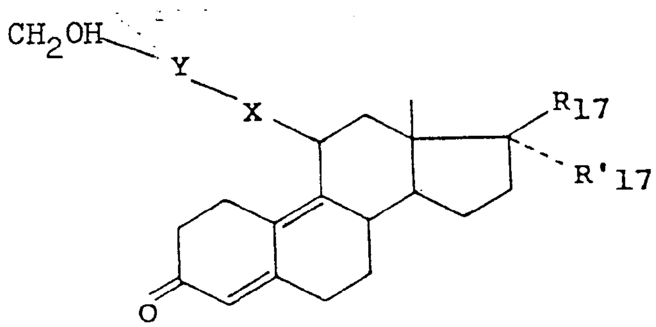

(II)

worin X, Y, $R_{17}$ und $R'_{17}$ die in Anspruch 1 gegebene Bedeutung besitzen, wobei jedoch $R_{17}$ keine Hydroxylgruppe sein kann,

- entweder der Einwirkung eines Oxidationsmittels unterzieht, um zu dem Produkt der Formel (III)

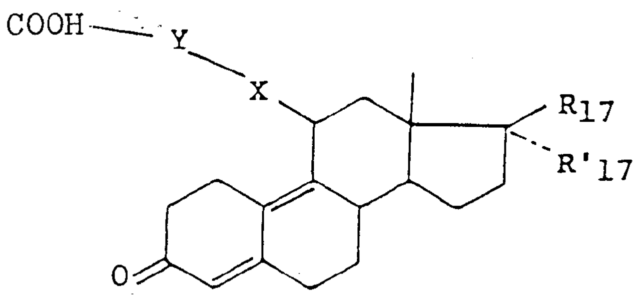

(III)

zu gelangen, worin $R_{17}$, $R'_{17}$, X und Y die in Anspruch 1 angegebene Bedeutung besitzen, welches man der Einwirkung eines Mittels, das die Aktivierung der Carboxylfunktion erlaubt, danach der Einwirkung einer Verbindung der Formel (IV)

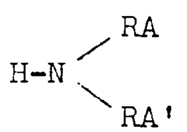

(IV)

unterzieht, worin RA und RA' die in Anspruch 1 gegebene Bedeutung besitzen, um zu dem Produkt der Formel (Ia) zu gelangen, welches der Verbindung der Formel (I) entspricht, worin Z eine einfache Bindung darstellt und die Ringe A und B die Gruppe

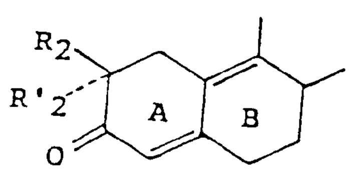

wiedergeben, worin $R_2$ und $R'_2$ ein Wasserstoffatom bedeuten,

- oder einer Reaktion zur Einführung des Restes

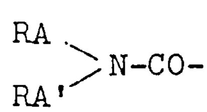

unterzieht, um das Produkt der Formel (I'a) zu erhalten, welches der Verbindung der Formel (I) entspricht, worin Z einen Methylenoxyrest darstellt und die Ringe A und B die gleiche Bedeutung wie in den Produkten der Formel (Ia) besitzen,

die Produkte (Ia) und (I'a) gewünschtenfalls

- entweder der Einwirkung eines Reduktionsmittels unterzieht, wenn $R_{17}$ und $R'_{17}$ gemeinsam eine Ketofunktion bilden, danach gegebenenfalls derjenigen eines Mittels zur Acylierung des so erhaltenen, in 17-Stellung hydroxylierten Derivats,
- oder der Einwirkung eines Verseifungsmittels unterzieht, wenn $R_{17}$ eine Acyloxyfunktion wiedergibt, um ein Produkt der Formel (Ia) oder (I'a) zu erhalten, worin $R_{17}$ die vorstehend angegebene Bedeutung besitzt,wonach man gewünschtenfalls irgendeines der Produkte der Formel (Ia) oder (I'a)
- entweder einer Alkylierung in 2-Stellung unterzieht, wenn zumindest einer der Reste $R_2$ und $R'_2$ ein Wasserstoffatom bedeutet,
- oder der Einwirkung eines Mittels zur Aromatisierung des Ringes A, danach derjenigen eines Mittels zur milden Verseifung unterzieht, um zu den Produkten der Formel (Ib) entsprechend den Produkten der Formel (Ia) und den Produkten der Formel (I'b) entsprechend den Produkten der Formel (I'a), worin die Ringe A und B für die Gruppe

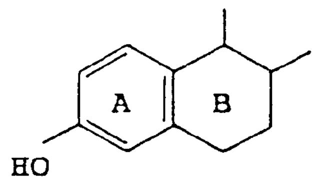

stehen, zu gelangen, die Produkte der Formel (Ib) und (I'b) gewünschtenfalls einer Reaktion zur Alkylierung oder Acylierung der Hydroxylgruppe in 3-Stellung unterzieht, hiernach gewünschtenfalls

- entweder, wenn $R_{17}$ und $R'_{17}$ gemeinsam eine Ketofunktion bedeuten, der Einwirkung eines Reduktionsmittels unterzieht oder derjenigen eines Metallkomplexes der Formel (V)

$$M\text{-}R'_{17} \qquad (V)$$

worin M für ein Metallatom steht und $R'_{17}$ die vorstehend angegebene Bedeutung besitzt, wobei es sich jedoch nicht um ein Wasserstoffatom handelt,
- oder, wenn $R_{17}$ für einen Hydroxylrest steht, der Einwirkung eines Mittels für die selektive Acylierung in 17-Stellung unterzieht, anschließend gewünschtenfalls irgendeines der vorstehend erhaltenen Produkte der Formel (I), oder wenn RA oder RA' ein Wasserstoffatom ist, der Einwirkung eines geeigneten Alkylierungsmittels unterzieht.

**13.** Verfahren zur Herstellung der Verbindungen der Formel (I), worin X einen Arylenrest bedeutet und Y für eine aliphatische Kette steht, die gegebenenfalls an die Arylengruppe über eine Doppel- oder eine Dreifachbindung gebunden ist und zumindest 3 Kohlenstoffatome enthält oder an die Arylengruppe über ein Sauerstoffatom gebunden ist, dadurch gekennzeichnet, daß man eine Verbindung der Formel (X)

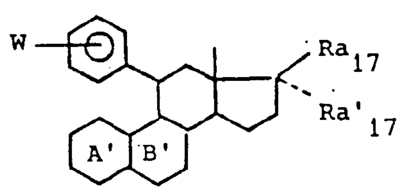

(X)

worin W entweder einen Rest OH oder einen Rest -C=CH bedeutet, die Ringe A' und B', $Ra_{17}$ und $Ra'_{17}$ die vorstehend für die Ringe A und B, $R_{17}$ und $R'_{17}$ angegebenen Bedeutungen besitzen und worin die reaktiven Funktionen in 3- und in 17-Stellung gegebenenfalls geschützt sind, oder aber, wenn W einen Rest -C=CH wiedergibt, der Einwirkung eines Halogenierungsmittels der Formel (XI)

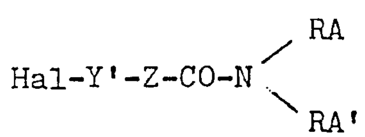

(XI)

worin Hal ein Halogenatom bedeutet, Z, RA und RA' die vorstehend angegebene Bedeutung besitzen und

Y' die vorstehende aliphatische Kette Y mit zumindest 2 Kohlenstoffatomen wiedergibt, in Gegenwart einer starken Base unterzieht und gegebenenfalls der Einwirkung eines Mittels zur Schutzgruppenabspaltung unterzieht, um zu dem Produkt der Formel ($I'_A$)

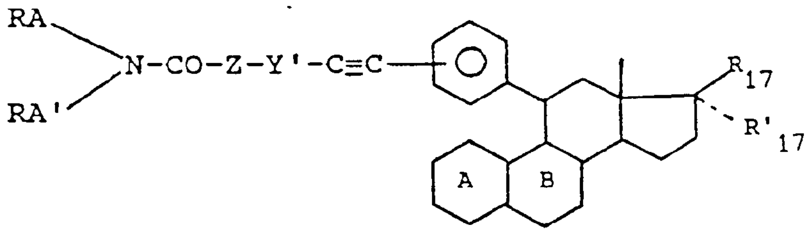

($I'_A$)

zu gelangen, welches man gewünschtenfalls der Einwirkung eines Mittels für die partielle oder vollständige Reduktion der Dreifachbindung unterzieht, um zu dem Produkt der Formel ($I'_B$)

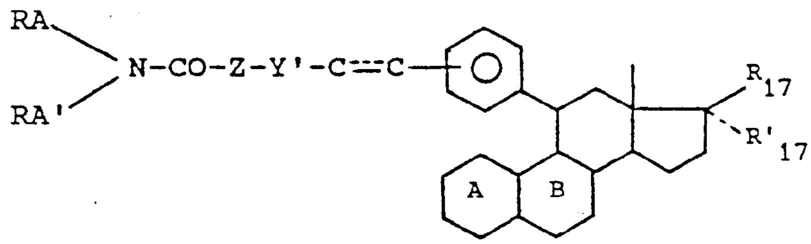

($I'_B$)

zu gelangen, oder wenn W einen Rest -OH bedeutet, der Einwirkung eines halogenierten Derivats der Formel (XII)

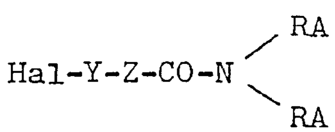

(XII)

worin Hal, Y, Z, RA und RA' die vorstehend angegebene Bedeutung besitzen, in Gegenwart eines alkalischen Mittels unterzieht, anschließend gegebenenfalls der Einwirkung eines Mittels zur Schutzgruppenabspaltung unterzieht, um zu einem Produkt der Formel ($I''_A$)

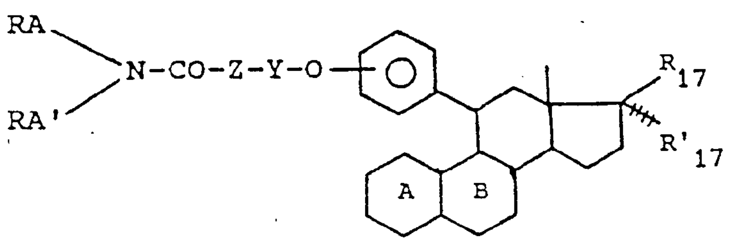

($I''_A$)

zu gelangen, welches Produkt man gewünschtenfalls, wenn Y eine ungesättigte, aliphatische Kette bedeutet, der Einwirkung eines Mittels für die partielle oder vollständige Reduktion unterzieht und die Produkte der Formel ($I'_A$), ($I'_B$), ($I''_A$) gewünschtenfalls irgendeiner der vorstehend für (Ia), (I'a),(Ib), (I'b) angegebenen Reaktionen unterzieht.

**14.** Als Arzneimittel die Verbindungen der Formel (I), wie in einem der Ansprüche 1 bis 10 definiert.

**15.** Als Arzneimittel die Verbindungen der Formel (I), wie in Anspruch 11 definiert.

**16.** Pharmazeutische Zusammensetzungen, enthaltend als Wirkstoff zumindest ein Arzneimittel, wie in Anspruch 14 oder 15 definiert.

17. Als neue Zwischenprodukte die Verbindungen der Formeln (II) und (III), wie in Anspruch 12 definiert.

18. Als neue Zwischenprodukte gemäß Anspruch 17 diejenigen der folgenden Bezeichnung:
17β-Acetyloxy-11β-[(8-hydroxyoctyl)-phenyl]-östra-4,9-dien-3-on,
11β-(12-Hydroxydodecyl)-östra-4,9-dien-3,17-dion,
11β-(8-Hydroxyoctyl)-östra-4,9-dien-3,17-dion,
17β-Acetyloxy-3-oxo-11β-östra-4,9-dienundecansäure,
3,17-Dioxo-11β-östra-4,9-dienundecansäure,
17β-Hydroxy-3-oxo-17-(1-propinyl)-11β-östra-4,9-dienundecansäure.

**Patentansprüche für folgenden Vertragsstaat : GR**

1. Verfahren zur Herstellung der Verbindungen der Formel (I)

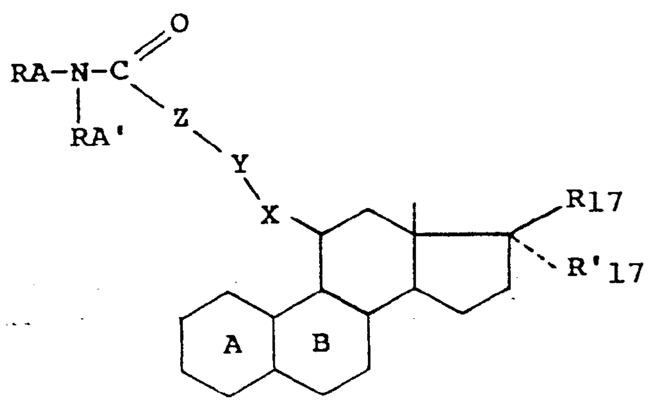

(I)

worin die Ringe A und B eine der folgenden Strukturen besitzen:
(a) entweder bedeuten A und B die Gruppe

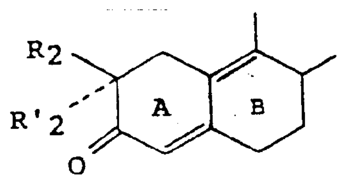

worin $R_2$ und $R'_2$, identisch oder verschieden, ein Wasserstoffatom oder eine Alkylgruppe mit 1 bis 4 Kohlenstoffatomen wiedergeben;
(b) oder A und B bedeuten die Gruppe

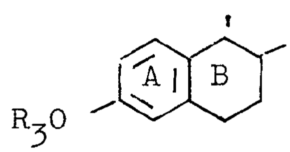

worin $R_3$ für ein Wasserstoffatom oder eine Alkylgruppe mit 1 bis 4 Kohlenstoffatomen oder einen Acylrest steht;
$R_{17}$ und $R'_{17}$ derart sind, daß
- entweder $R_{17}$ und $R'_{17}$ gemeinsam eine Ketofunktion bilden,
- oder $R_{17}$ ein Hydroxylrest oder ein Acyloxyrest ist und $R'_{17}$ ein Wasserstoffatom, einen Alkyl-, Alkenyl- oder Alkinylrest mit höchstens 8 Kohlenstoffatomen, der gegebenenfalls substituiert ist, bedeutet;

X, Y und Z derart sind, daß

- X eine Methylengruppe, eine Arylengruppe, einen Rest $CH_2$-O oder einen Arylenoxyrest, gebunden an das Steroid über ein Kohlenstoffatom, bedeutet,
- Y eine einfache Bindung oder eine gesättigte oder ungesättigte, aliphatische, lineare oder verzweigte Kette mit 1 bis 18 Kohlenstoffatomen, gegebenenfalls unterbrochen durch einen oder mehrere Reste, ausgewählt unter den Arylenresten, Sauerstoff oder in Form des Sulfoxids oder Sulfons oxiertem Schwefel und gegebenenfalls durch eine Arylengruppe beendet, darstellt,
- Z eine einfache Bindung oder einen Rest $CH_2$-O, der an den Rest Y durch das Kohlenstoffatom gebunden ist, wiedergibt,

wobei, wenn Y und Z eine einfache Bindung darstellen, X keinen Methylenrest oder $CH_2$-O bedeuten kann;

RA und RA', identisch oder verschieden, ein Wasserstoffatom, einen linearen oder verzweigten Alkylrest mit 1 bis 8 Kohlenstoffatomen, gegebenenfalls substituiert durch einen oder mehrere Reste, ausgewählt unter den Aryl-,Alkyl- oder Dial-kylamino-, Hydroxy-, Halogen- oder veresterten Carboxylresten, darstellen oder RA und RA' mit dem Stickstoffatom, an das sie gebunden sind, einen gesättigten oder ungesättigten Heterocyclus mit 5 oder 6 Kettengliedern bilden, der gegebenenfalls ein oder mehrere weitere Heteroatome, ausgewählt unter den Sauerstoff-, Stickstoff- und Schwefelatomen, enthält und gegebenenfalls durch einen Alkylrest mit 1 bis 4 Kohlenstoffatomen substituiert ist, wobei zumindest einer der Substituenten RA oder RA' kein Wasserstoffatom ist, dadurch gekennzeichnet, daß man eine Verbindung der Formel (II)

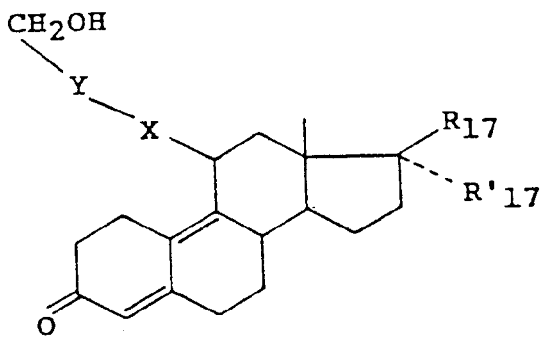

(II)

worin X, Y, $R_{17}$ und $R'_{17}$ die vorstehend angegebene Bedeutung besitzen, wobei jedoch $R_{17}$ keine Hydroxylgruppe bedeuten kann,

- entweder der Einwirkung eines Oxidationsmittels unterwirft, um zu dem Produkt der Formel (III)

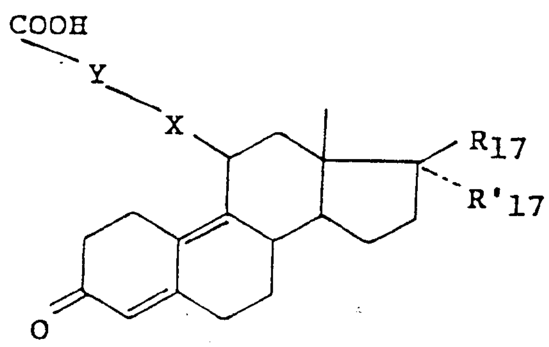

(III)

zu gelangen, worin $R_{17}$, $R'_{17}$, X und Y die in Anspruch 1 angegebene Bedeutung besitzen, welches man der Einwirkung eines Mittels, das die Aktivierung der Carboxylfunktion erlaubt, danach der Einwirkung einer Verbindung der Formel (IV)

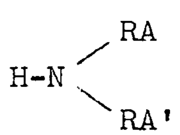

(IV)

unterzieht, worin RA und RA' die vorstehend angegebene Bedeutung besitzen, um zu dem Produkt der Formel (Ia) zu gelangen, welches der Verbindung der Formel (I) entspricht, worin Z eine einfache Bindung darstellt und die Ringe A und B die Gruppe

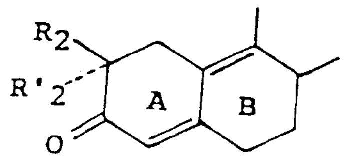

wiedergeben, worin $R_2$ und $R'_2$ ein Wasserstoffatom bedeuten,

- oder einer Reaktion zur Einführung des Restes

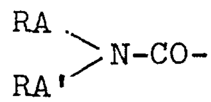

unterzieht, um das Produkt der Formel (I'a) zu erhalten, welches der Verbindung der Formel (I) entspricht, worin Z einen Methylenoxyrest darstellt und die Ringe A und B die gleiche Bedeutung wie in den Produkten der Formel (Ia) besitzen,
die Produkte (Ia) und (I'a) gewünschtenfalls

- entweder der Einwirkung eines Reduktionsmittels unterzieht, wenn $R_{17}$ und $R'_{17}$ gemeinsam eine Ketofunktion bilden, danach gegebenenfalls derjenigen eines Mittels zur Acylierung des so erhaltenen, in 17-Stellung hydroxylierten Derivats,
- oder der Einwirkung eines Verseifungsmittels unterzieht, wenn $R_{17}$ eine Acyloxyfunktion wiedergibt,

um ein Produkt der Formel (Ia) oder (I'a) zu erhalten, worin $R_{17}$ die vorstehend angegebene Bedeutung besitzt,wonach man gewünschtenfalls irgendeines der Produkte der Formel (Ia) oder (I'a)

- entweder einer Alkylierung in 2-Stellung unterzieht, wenn zumindest einer der Reste $R_2$ und $R'_2$ ein Wasserstoffatom bedeutet,
- oder der Einwirkung eines Mittels zur Aromatisierung des Ringes A, danach derjenigen eines Mittels zur milden Verseifung unterzieht, um zu den Produkten der Formel (Ib) entsprechend den Produkten der Formel (Ia) und den Produkten der Formel (I'b) entsprechend den Produkten der Formel (I'a), worin die Ringe A und B für die Gruppe

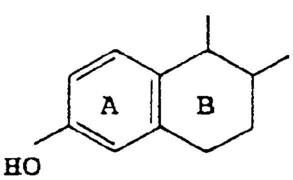

stehen, zu gelangen, die Produkte der Formel (Ib) und (I'b) gewünschtenfalls einer Reaktion zur Alkylierung oder Acylierung der Hydroxylgruppe in 3-Stellung unterzieht, hiernach gewünschtenfalls

- entweder, wenn $R_{17}$ und $R'_{17}$ gemeinsam eine Ketofunktion bedeuten, der Einwirkung eines Reduktionsmittels unterzieht oder derjenigen eines Metallkomplexes der Formel (V)

$$M\text{-}R'_{17} \qquad (V)$$

worin M für ein Metallatom steht und $R'_{17}$ die vorstehend angegebene Bedeutung besitzt, wobei es sich jedoch nicht um ein Wasserstoffatom handelt,

- oder, wenn $R_{17}$ für einen Hydroxylrest steht, der Einwirkung eines Mittels für die selektive Acylierung in 17-Stellung unterzieht, anschließend gewünschtenfalls irgendeines der vorstehend erhaltenen Produkte der Formel (I),oder wenn RA oder RA' ein Wasserstoffatom ist, der Einwirkung eines geeigneten Alkylierungsmittels unterzieht.

2. Verfahren gemäß Anspruch 1 zur Herstellung der Verbindungen der Formel ($I_A$)

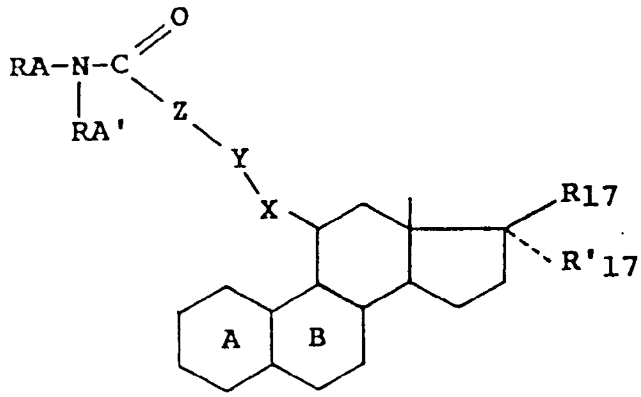

($I_A$)

worin die Ringe A und B eine der folgenden Strukturen aufweisen:

(a) entweder bedeuten A und B die Gruppe

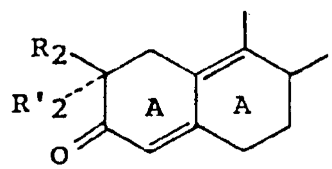

worin $R_2$ und $R'_2$, identisch oder voneinander verschieden, ein Wasserstoffatom oder einen Alkylrest mit 1 bis 4 Kohlenstoffatomen darstellen;

(b) oder A und B bedeuten die Gruppe

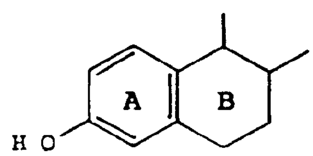

$R_{17}$ und $R'_{17}$ sind derart, daß

- entweder $R_{17}$ und $R'_{17}$ gemeinsam eine Ketofunktion bilden
- oder $R_{17}$ einen Hydroxylrest oder eine Acyloxygruppe ist und $R'_{17}$ ein Wasserstoffatom, einen Alkyl-, Alkenyl- oder Alkinylrest mit höchstens 8 Kohlenstoffatomen, der gegebenenfalls substituiert ist, bedeutet,

X, Y und Z derart sind, daß

- X eine Methylengruppe, eine Arylengruppe, einen Rest $CH_2$-O oder einen Arylenoxyrest, gebunden an das Steroid über ein Kohlenstoffatom, wiedergibt,
- Y eine einfache Bindung oder eine gesättigte oder ungesättigte, lineare oder verzweigte, aliphatische Kette mit 1 bis 18 Kohlenstoffatomen, gegebenenfalls unterbrochen durch einen oder mehrere Reste, ausgewählt unter den Arylenresten, Sauerstoff und gegebenenfalls beendet durch einen Arylenrest, bedeutet,
- Z eine einfache Bindung oder einen Rest $CH_2$-O, der an den Rest Y über das Kohlenstoffatom gebunden ist, wiedergibt, wobei jedoch, wenn Y und Z eine einfache Bindung darstellen, X kein Methylenrest oder $CH_2$-O sein kann,

RA und RA', identisch oder verschieden, ein Wasserstoffatom, einen linearen oder verzweigten Alkylrest mit 1 bis 8 Kohlenstoffatomen, gegebenenfalls substituiert durch einen Aryl-, Alkyl- oder Dialkylamino-, Hydroxyrest, Halogen oder veresterten Carboxylrest, darstellen oder RA und RA' mit dem Stickstoffatom, an das sie gebunden sind, einen gesättigten oder ungesättigten Heterocyclus mit 5 oder 6 Kettengliedern bilden, der gegebenenfalls ein oder mehrere weitere Heteroatome, ausgewählt unter den Sauerstoff-, Stickstoff- und Schwefelatomen, enthält und gegebenenfalls durch einen Alkylrest mit 1 bis 4 Kohlenstoffatomen substituiert ist, wobei jedoch

zumindest einer der Substituenten RA oder RA' kein Wasserstoffatom ist, dadurch gekennzeichnet, daß man eine Verbindung der Formel (II)

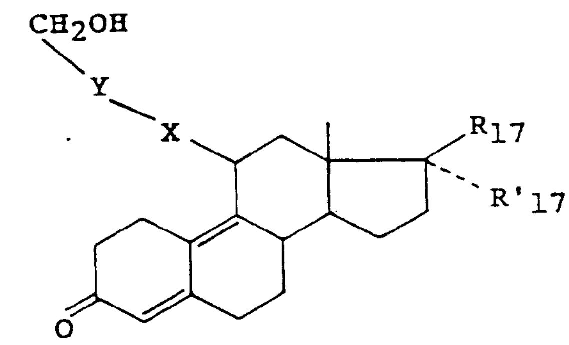

(II)

worin X, Y, $R_{17}$ und $R'_{17}$ die in Anspruch 1 angegebene Bedeutung besitzen, mit der Maßgabe, daß $R_{17}$ keine Hydroxylgruppe bedeuten kann,

- entweder der Einwirkung eines Oxidationsmittels unterzieht, um das Produkt der Formel (III)

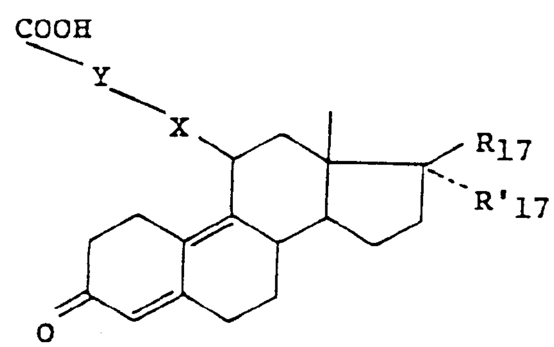

(III)

zu erhalten, worin $R_{17}$, $R'_{17}$, X und Y die in Anspruch 1 angegebene Bedeutung besitzen, welches man der Einwirkung eines Mittels unterzieht, das die Aktivierung der Carboxylfunktion ermöglicht, anschließend der Einwirkung einer Verbindung der Formel (IV)

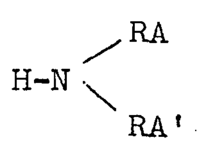

(IV)

worin RA und RA' die in Anspruch 1 angegebene Bedeutung besitzen, um zu dem Produkt der Formel (Ia) zu gelangen, welches der Verbindung der Formel ($I_A$) entspricht, worin Z eine einfache Bindung darstellt und die Ringe A und B die Gruppe

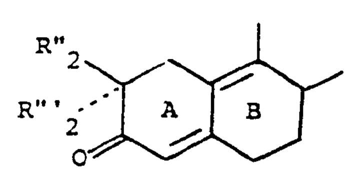

bedeuten, worin $R_2$ und $R'_2$ ein Wasserstoffatom darstellen,

- oder einer Reaktion zur Einführung des Restes

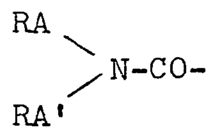

unterzieht, um zu dem Produkt der Formel (I'a) zu gelangen, welches der Verbindung der Formel ($I_A$) entspricht, worin Z ein Methylenoxyrest ist und die Ringe A und B die bei den Produkten der Formel (Ia) angegebene Bedeutung besitzen,

die Produkte (Ia) und (I'a) gewünschtenfalls

- entweder der Einwirkung eines Reduktionsmittels unterzieht, wenn $R_{17}$ und $R'_{17}$ gemeinsam eine Ketofunktion bilden, danach gegebenenfalls der Einwirkung eines Mittels zur Acylierung des so erhaltenen, in 17-Stellung hydroxylierten Derivats,
- oder der Einwirkung eines Verseifungsmittels unterzieht, wenn $R_{17}$ eine Acyloxyfunktion bedeutet, um zu einem Produkt der Formel (Ia) oder (I'a) zu gelangen, worin $R_{17}$ die vorstehend angegebene Bedeutung besitzt, dann gewünschtenfalls irgendeines der Produkte der Formel (Ia) oder (I'a)
- entweder einer Alkylierung in 2-Stellung unterzieht, wenn zumindest einer der Reste $R_2$ und $R'_2$ ein Wasserstoffatom bedeutet,
- oder der Einwirkung eines Mittels zur Aromatisierung des Ringes A unterzieht, danach derjenigen eines Mittels für die milde Verseifung, um zu den Produkten der Formel (Ib), die den Produkten der Formel (Ia) entsprechen, und den Produkten der Formel (I'b), die den Produkten der Formel (I'a) entsprechen, und worin die Ringe A und B für die Gruppe

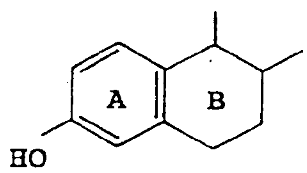

stehen, zu gelangen,

die Produkte der Formel (Ib) und (I'b) gewünschtenfalls

- entweder, wenn $R_{17}$ und $R'_{17}$ gemeinsam eine Ketofunktion bilden, der Einwirkung eines Reduktionsmittels unterzieht oder derjenigen eines Metallkomplexes der Formel (V)

$$M - R'_{17} \qquad (V)$$

  worin M ein Metallatom bedeutet und $R'_{17}$ die vorstehend angegebene Bedeutung besitzt, wobei es sich jedoch nicht um ein Wasserstoffatom handelt,
- oder, wenn $R_{17}$ ein Hydroxylrest ist, der Einwirkung eines Mittels für die selektive Acylierung in 17-Stellung unterzieht, danach gewünschtenfalls irgendeines der vorstehend erhaltenen Produkte der Formel (I), oder wenn RA oder RA' ein Wasserstoffatom ist, der Einwirkung eines geeigneten Alkylierungsmittels unterzieht.

3. Verfahren gemäß Anspruch 2, dadurch gekennzeichnet, daß man von einem Produkt der Formel (II) ausgeht, worin $R'_{17}$ ein Wasserstoffatom, einen Ethinylrest oder einen Propinylrest bedeutet.

4. Verfahren gemäß Anspruch 2, dadurch gekennzeichnet, daß man von einem Produkt der Formel (II) ausgeht, worin X eine Methylengruppe bedeutet und Y eine gesättigte, lineare Kette mit 5 bis 10 Kohlenstoffatomen ist, die gegebenenfalls durch ein Sauerstoffatom unterbrochen ist.

5. Verfahren gemäß Anspruch 2, dadurch gekennzeichnet, daß man von einem Produkt der Formel (II) ausgeht, worin X eine Phenylengruppe bedeutet und Y für eine gesättigte oder ungesättigte, lineare Kette mit 3 bis 10 Kohlenstoffatomen steht, die gegebenenfalls durch ein Sauerstoffatom unterbrochen ist.

6. Verfahren gemäß Anspruch 1, dadurch gekennzeichnet, daß man von einem Produkt der Formel (II) ausgeht, worin X für einen Phenylenoxyrest steht und Y eine gesättigte, lineare Kette mit 3 bis 10 Kohlenstoffatomen ist, die gegebenenfalls durch ein Sauerstoff- oder Schwefelatom unterbrochen ist.

7. Verfahren gemäß Anspruch 2, dadurch gekennzeichnet, daß man von einer Verbindung der Formel (IV) oder aber einem Reagens zur Einführung des Restes

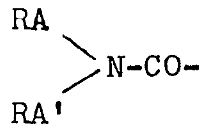

oder gegebenenfalls einem derartigen Reagens ausgeht, daß
- entweder RA und RA' identisch eine Methylgruppe bedeuten
- oder RA ein Wasserstoffatom oder einen Methylrest wiedergibt und RA' für einen Butylrest steht,
- oder RA eine Methylgruppe bedeutet und RA' für einen Isopropyl-, Dimethylaminoethyl-, Benzyl- oder Heptafluorobutylrest steht,
- oder RA und RA' gemeinsam ein gegebenenfalls N-substituiertes Piperazin oder ein Pyrrolidin bilden.

8. Verfahren gemäß Anspruch 1, dadurch gekennzeichnet, daß man die Ausgangsprodukte derart wählt, daß man herstellt:
N-(2-Dimethylaminoethyl)-17β-hydroxy-N-methyl-3-oxo-11β-östra-4,9-dien-undecanamid,
N-Butyl-4-(3,17β-dihydroxy-östra-1,3,5(10)-trien-11β-yl)-N-methylbenzoloctanamid,
3,17β-Dihydroxy-N-methyl-N-(1-methylethyl)-11β-östra-1,3,5(10)-trien-undecanamid,
N-Butyl-3,17β-dihydroxy-N-methyl-19-nor-11β-(17α-pregna-1,3,5(10)-trien-20-in)-undecanamid,
3,17β-Dihydroxy-N-methyl-N-(1-methylethyl)-19-nor-17α-pregna-1,3,5(10)-trien-20-in-11β-undecanamid,
[[8-(3,17β-Dihydroxy-östra-1,3,5(10)-trien-11β-yl)-octyl]-oxy]-N-methyl-N-(1-methylethyl)-acetamid,
N-Butyl-8-[4-(3,17β-dihydroxy-östra-1,3,5(10)-trien-11β-yl)-phenoxy]-N-methyl-octanamid,
N-Butyl-[5-[4-(3,17β-dihydroxy-östra-1,3,5(10)-trien-11β-yl)-phenoxy]-pentyloxy]-N-methyl-acetamid,
2-[[7-[4-(3,17β-Dihydroxy-östra-1,3,5(10)-trien-11β-yl)-phenyl]-6-heptylyl]-oxy]-N-butyl-N-methyl-acetamid,
3,17β-Dihydroxy-N-(2,2,3,3,4,4,4-heptafluorbutyl)-N-methyl-östra-1,3,5(10)-trien-11β-yl-undecanamid,
8-[4-(3,17β-Dihydroxy-östra-1,3,5(10)-trien-11β-yl)- phenyl]-N-butyl-N-methyl-octinamid.

9. Verfahren zur Herstellung der Produkte der Formel (I), wie in Anspruch 1 definiert, worin X einen Arylenrest bedeutet und Y für eine aliphatische Kette steht, die gegebenenfalls an die Arylengruppe über eine Doppel- oder eine Dreifachbindung gebunden ist und zumindest 3 Kohlenstoffatome enthält oder an die Arylengruppe über ein Sauerstoffatom gebunden ist, dadurch gekennzeichnet, daß man eine Verbindung der Formel (X)

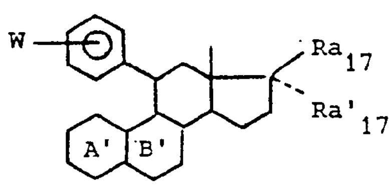

(X)

worin W entweder einen Rest OH oder einen Rest -C=CH bedeutet, die Ringe A' und B', $Ra_{17}$ und $Ra'_{17}$ die vorstehend für die Ringe A und B, $R_{17}$ und $R'_{17}$ angegebenen Bedeutungen besitzen und worin die reaktiven Funktionen in 3- und in 17-Stellung gegebenenfalls geschützt sind, oder aber, wenn W einen Rest -C=CH wiedergibt, der Einwirkung eines Halogenierungsmittels der Formel (XI)

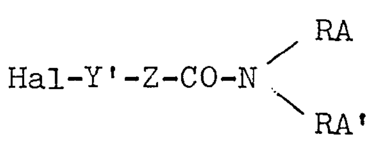

(XI)

worin Hal ein Halogenatom bedeutet, Z, RA und RA' die vorstehend angegebene Bedeutung besitzen und

Y' die vorstehende aliphatische Kette Y mit zumindest 2 Kohlenstoffatomen wiedergibt, in Gegenwart einer starken Base unterzieht und gegebenenfalls der Einwirkung eines Mittels zur Schutzgruppenabspaltung unterzieht, um zu dem Produkt der Formel ($I'_A$)

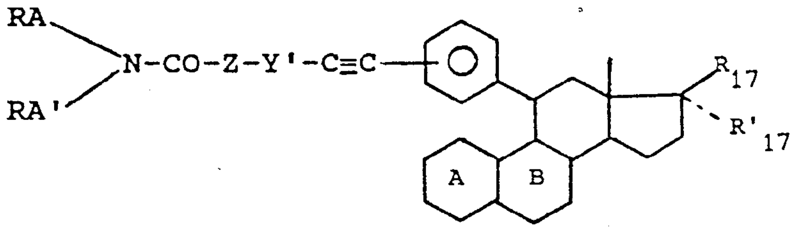

($I'_A$)

zu gelangen, welches man gewünschtenfalls der Einwirkung eines Mittels für die partielle oder vollständige Reduktion der Dreifachbindung unterzieht, um zu dem Produkt der Formel ($I'_B$)

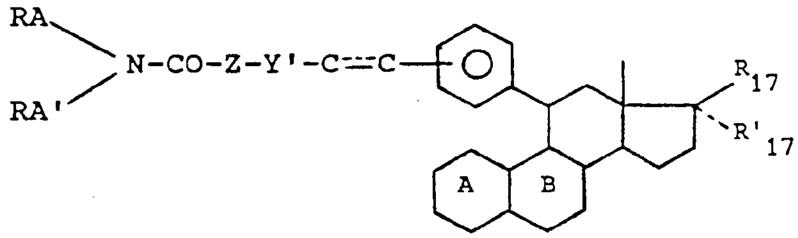

($I'_B$)

zu gelangen, <u>oder</u> wenn W einen Rest -OH bedeutet, der Einwirkung eines halogenierten Derivats der Formel (XII)

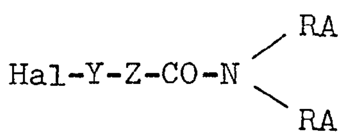

(XII)

worin Hal, Y, Z, RA und RA' die vorstehend angegebene Bedeutung besitzen, in Gegenwart eines alkalischen Mittels unterzieht, anschließend gegebenenfalls der Einwirkung eines Mittels zur Schutzgruppenabspaltung unterzieht, um zu einem Produkt der Formel ($I''_A$)

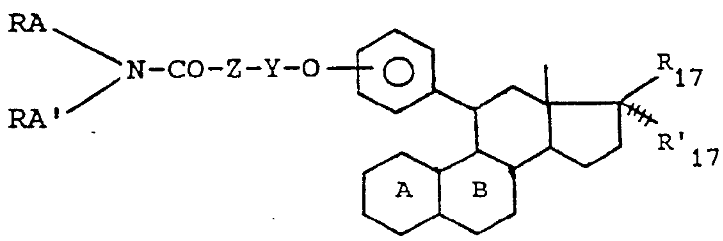

($I''_A$)

zu gelangen, welches Produkt man gewünschtenfalls, wenn Y eine ungesättigte, aliphatische Kette bedeutet, der Einwirkung eines Mittels für die partielle oder vollständige Reduktion unterzieht und die Produkte der Formel ($I'_A$), ($I'_B$), ($I''_A$) gewünschtenfalls irgendeiner der vorstehend für (Ia), (I'a), (Ib), (I'b)angegebenen Reaktionen unterzieht.

**10.** Verfahren gemäß Anspruch 8, dadurch gekennzeichnet, daß man von einer Verbindung der Formel (X) ausgeht, worin $R'_{17}$ ein Wasserstoffatom, einen Ethinylrest oder einen Propinylrest bedeutet.

**11.** Verfahren gemäß Anspruch 9, dadurch gekennzeichnet, daß man von einer Verbindung der Formel (X), worin W einen Rest -C=CH bedeutet, und einer Verbindung der Formel (XI), worin Y eine gesättigte, lineare Kette mit 5 bis 10 Kohlenstoffatomen bedeutet, die gegebenenfalls durch ein Sauerstoffatom unterbrochen ist, ausgeht.

**12.** Verfahren gemäß Anspruch 9, dadurch gekennzeichnet, daß man von einer Verbindung der Formel (X), worin W einen Rest OH bedeutet, und einer Verbindung der Formel (XII), worin Y eine gesättigte, lineare Kette mit 3 bis 10 Kohlenstoffatomen bedeutet, die gegebenenfalls durch ein Sauerstoff- oder Schwefelatom unterbrochen ist, ausgeht.

**13.** Verfahren gemäß Anspruch 9, dadurch gekennzeichnet, daß man von einer Verbindung der Formel (XI) oder einer Verbindung der Formel (XII) oder gegebenenfalls einem Alkylierungsmittel ausgeht, derart, daß

- entweder RA und RA' identisch eine Methylgruppe bedeuten
- oder RA ein Wasserstoffatom oder einen Methylrest bedeutet und RA' für einen Butylrest steht
- oder RA eine Methylgruppe bedeutet und RA' einen Isopropyl-, Dimethylaminoethyl-, Benzyl- oder Heptafluorobutylrest wiedergibt
- oder RA und RA' gemeinsam ein gegebenenfalls N-substituiertes Piperazin oder ein Pyrrolidin bilden.

**14.** Verfahren gemäß Anspruch 9, dadurch gekennzeichnet, daß man die Ausgangsprodukte derart wählt, daß man herstellt:

N-Butyl-4-(3,17β-dihydroxy-östra-1,3,5(10)-trien-11β-yl)-N-methyl-benzol-octanamid,
N-Butyl-8-[4-(3,17β-dihydroxy-östra-1,3,5(10)-trien-11β-yl)-phenoxy]-N-methyl-octanamid,
N-Butyl-[5-[4-(3,17β-dihydroxy-östra-1,3,5(10)-trien-11β-yl)-phenoxyl-pentyloxy]-N-methyl-acetamid,
2-[[7-[4-(3,17β-Dihydroxy-östra-1,3,5(10)-trien-11β-yl)-phenyl]-6-heptylyl]-oxy]-N-butyl-N-methyl-acetamid,
8-[4-(3,17β-Dihydroxy-östra-1,3,5(10)-trien-11β-yl)-phenyl]-N-butyl-N-methyl-octinamid.

**15.** Als neue industrielle Produkte die Verbindungen der Formeln (II) und (III), wie in Anspruch 1 definiert.

**16.** Als neue Produkte gemäß Anspruch 16 diejenigen mit der folgenden Bezeichnung:

17β-Acetyloxy-11β-[(8-hydroxyoctyl)-phenyl]-östra-4,9-dien-3-on,
11β-(12-Hydroxydodecyl)-östra-4,9-dien-3,17-dion,
11β-(8-Hydroxyoctyl)-östra-4,9-dien-3,17-dion,
17β-Acetyloxy-3-oxo-11β-östra-4,9-dienundecansäure,
3,17-Dioxo-11β-östra-4,9-dienundecansäure,
17β-Hydroxy-3-oxo-17-(1-propinyl)-11β-östra-4,9-dienundecansäure.

**17.** Verfahren zur Herstellung von pharmazeutischen Zusammensetzungen, dadurch gekennzeichnet, daß man als Wirkstoff zumindest eines der Produkte der allgemeinen Formel (I), wie in Anspruch 1 oder 9 definiert, in eine für diese Verwendung geeignete Form überführt.

**18.** Verfahren zur Herstellung von pharmazeutischen Zusammensetzungen, dadurch gekennzeichnet, daß man als Wirkstoff zumindest eines der Produkte der allgemeinen Formel ($I_A$), wie in Anspruch 2 definiert, in eine für diese Verwendung geeignete Form überführt.

**19.** Verfahren zur Herstellung von pharmazeutischen Zusammensetzungen, dadurch gekennzeichnet, daß man als Wirkstoff zumindest eines der Produkte der allgemeinen Formel (I), wie in Anspruch 8 oder 15 definiert, in eine für diese Verwendung geeignete Form überführt.

**Patentansprüche für folgenden Vertragsstaat : ES**

**1.** Verfahren zur Herstellung der Verbindungen der Formel (I)

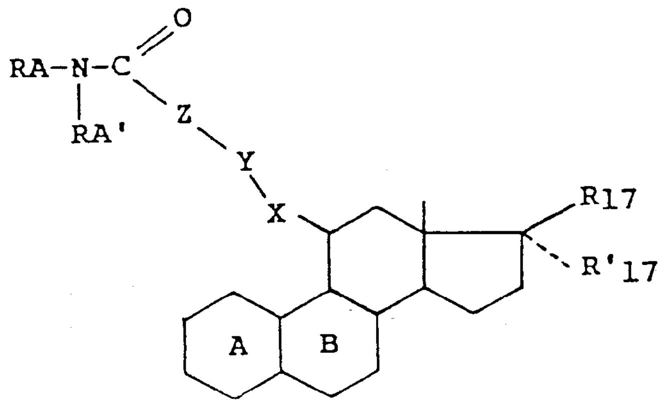

(I)

worin die Ringe A und B eine der folgenden Strukturen besitzen:
(a) entweder bedeuten A und B die Gruppe

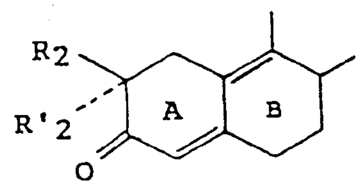

worin $R_2$ und $R'_2$, identisch oder verschieden, ein Wasserstoffatom oder eine Alkylgruppe mit 1 bis 4 Kohlenstoffatomen wiedergeben;
(b) oder A und B bedeuten die Gruppe

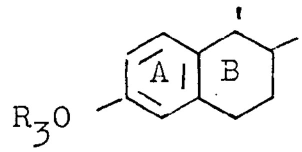

worin $R_3$ für ein Wasserstoffatom oder eine Alkylgruppe mit 1 bis 4 Kohlenstoffatomen oder einen Acylrest steht;

$R_{17}$ und $R'_{17}$ derart sind, daß

- entweder $R_{17}$ und $R'_{17}$ gemeinsam eine Ketofunktion bilden,
- oder $R_{17}$ ein Hydroxylrest oder ein Acyloxyrest ist und $R'_{17}$ ein Wasserstoffatom, einen Alkyl-, Alkenyl- oder Alki-nylrest mit höchstens 8 Kohlenstoffatomen, der gegebenenfalls substituiert ist, bedeutet;

X, Y und Z derart sind, daß

- X eine Methylengruppe, eine Arylengruppe, einen Rest $CH_2$-O oder einen Arylenoxyrest, gebunden an das Steroid über ein Kohlenstoffatom, bedeutet,
- Y eine einfache Bindung oder eine gesättigte oder ungesättigte, aliphatische, lineare oder verzweigte Kette mit 1 bis 18 Kohlenstoffatomen, gegebenenfalls unterbrochen durch einen oder mehrere Reste, ausgewählt unter den Arylenresten, Sauerstoff oder in Form des Sulfoxids oder Sulfons oxiertem Schwefel und gegebenenfalls durch eine Arylengruppe beendet, darstellt,
- Z eine einfache Bindung oder einen Rest $CH_2$-O, der an den Rest Y durch das Kohlenstoffatom gebunden ist, wiedergibt,

wobei, wenn Y und Z eine einfache Bindung darstellen, X keinen Methylenrest oder $CH_2$-O bedeuten kann;
RA und RA', identisch oder verschieden, ein Wasserstoffatom, einen linearen oder verzweigten Alkylrest mit 1 bis 8 Kohlenstoffatomen, gegebenenfalls substituiert durch einen oder mehrere Reste, ausgewählt unter den Aryl-,Alkyl- oder Dialkylamino-, Hydroxy-, Halogen- oder veresterten Carboxylresten, darstellen oder RA und RA' mit dem Stickstoffatom, an das sie gebunden sind, einen gesättigten oder ungesättigten Heterocyclus mit 5 oder 6 Kettengliedern bilden, der gegebenenfalls ein oder mehrere weitere Heteroatome, ausgewählt unter den Sauerstoff-, Stickstoff- und Schwefelatomen, enthält und gegebenenfalls durch einen Alkylrest mit 1 bis 4 Kohlenstoffatomen substituiert ist, wobei zumindest einer der Substituenten

RA oder RA' kein Wasserstoffatom ist, dadurch gekennzeichnet, daß man eine Verbindung der Formel (II)

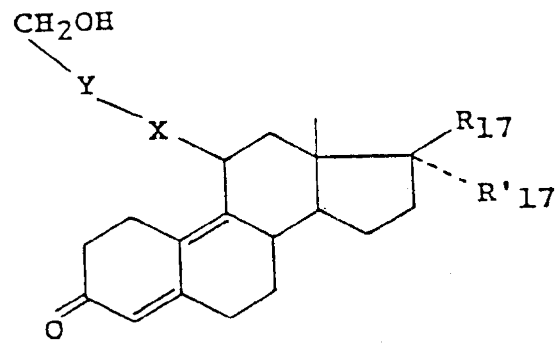

(II)

worin X, Y, $R_{17}$ und $R'_{17}$ die vorstehend angegebene Bedeutung besitzen, wobei jedoch $R_{17}$ keine Hydroxylgruppe bedeuten kann,

- entweder der Einwirkung eines Oxidationsmittels unterwirft, um zu dem Produkt der Formel (III)

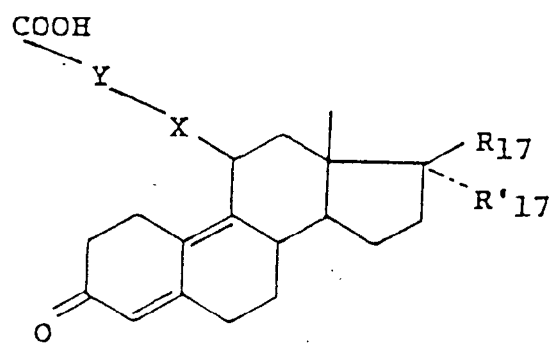

(III)

zu gelangen, worin $R_{17}$, $R'_{17}$, X und Y die in Anspruch 1 angegebene Bedeutung besitzen, welches man der Einwirkung eines Mittels, das die Aktivierung der Carboxylfunktion erlaubt, danach der Einwirkung einer Verbindung der Formel (IV)

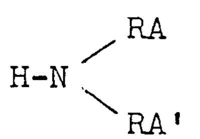

(IV)

unterzieht, worin RA und RA' die vorstehend angegebene Bedeutung besitzen, um zu dem Produkt der Formel (Ia) zu gelangen, welches der Verbindung der Formel (I) entspricht, worin Z eine einfache Bindung darstellt und die Ringe A und B die Gruppe

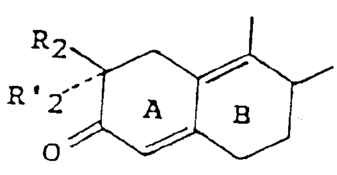

wiedergeben, worin $R_2$ und $R'_2$ ein Wasserstoffatom bedeuten,

- oder einer Reaktion zur Einführung des Restes

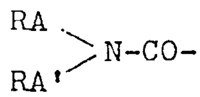

unterzieht, um das Produkt der Formel (I'a) zu erhalten, welches der Verbindung der Formel (I) entspricht, worin Z einen Methylenoxyrest darstellt und die Ringe A und B die gleiche Bedeutung wie in den Produkten der Formel (Ia) besitzen,
die Produkte (Ia) und (I'a) gewünschtenfalls

- entweder der Einwirkung eines Reduktionsmittels unterzieht, wenn $R_{17}$ und $R'_{17}$ gemeinsam eine Ketofunktion bilden, danach gegebenenfalls derjenigen eines Mittels zur Acylierung des so erhaltenen, in 17-Stellung hydroxylierten Derivats,
- oder der Einwirkung eines Verseifungsmittels unterzieht, wenn $R_{17}$ eine Acyloxyfunktion wiedergibt, um ein Produkt der Formel (Ia) oder (I'a) zu erhalten, worin $R_{17}$ die vorstehend angegebene Bedeutung besitzt,wonach man gewünschtenfalls irgendeines der Produkte der Formel (Ia) oder (I'a)
- entweder einer Alkylierung in 2-Stellung unterzieht, wenn zumindest einer der Reste $R_2$ und $R'_2$ ein Wasserstoffatom bedeutet,
- oder der Einwirkung eines Mittels zur Aromatisierung des Ringes A, danach derjenigen eines Mittels zur milden Verseifung unterzieht, um zu den Produkten der Formel (Ib) entsprechend den Produkten der Formel (Ia) und den Produkten der Formel (I'b) entsprechend den Produkten der Formel (I'a), worin die Ringe A und B für die Gruppe

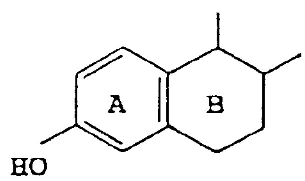

stehen, zu gelangen, die Produkte der Formel (Ib) und (I'b) gewünschtenfalls einer Reaktion zur Alkylierung oder Acylierung der Hydroxylgruppe in 3-Stellung unterzieht, hiernach gewünschtenfalls

- entweder, wenn $R_{17}$ und $R'_{17}$ gemeinsam eine Ketofunktion bedeuten, der Einwirkung eines Reduktionsmittels unterzieht oder derjenigen eines Metallkomplexes der Formel (V)

$$M\text{-}R'_{17} \qquad (V)$$

worin M für ein Metallatom steht und $R'_{17}$ die vorstehend angegebene Bedeutung besitzt, wobei es sich jedoch nicht um ein Wasserstoffatom handelt,
- oder, wenn $R_{17}$ für einen Hydroxylrest steht, der Einwirkung eines Mittels für die selektive Acylierung in 17-Stellung unterzieht, anschließend gewünschtenfalls irgendeines der vorstehend erhaltenen Produkte der Formel (I),oder wenn RA oder RA' ein Wasserstoffatom ist, der Einwirkung eines geeigneten Alkylierungsmittels unterzieht.

2. Verfahren gemäß Anspruch 1 zur Herstellung der Verbindungen der Formel ($I_A$)

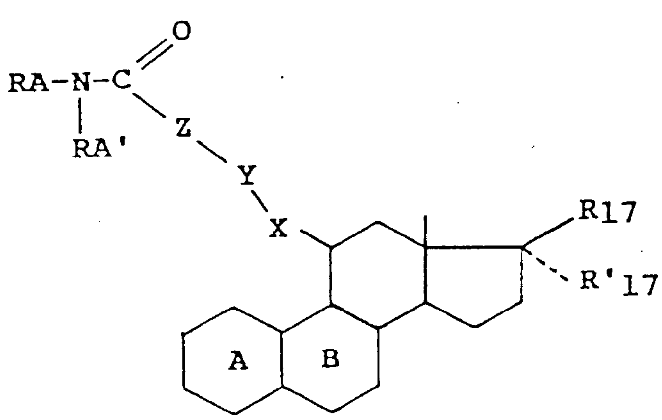

($I_A$)

worin die Ringe A und B eine der folgenden Strukturen aufweisen:

(a) entweder bedeuten A und B die Gruppe

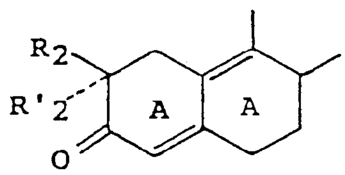

worin $R_2$ und $R'_2$, identisch oder voneinander verschieden, ein Wasserstoffatom oder einen Alkylrest mit 1 bis 4 Kohlenstoffatomen darstellen;

(b) oder A und B bedeuten die Gruppe

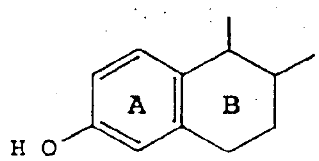

$R_{17}$ und $R'_{17}$ sind derart, daß

- entweder $R_{17}$ und $R'_{17}$ gemeinsam eine Ketofunktion bilden
- oder $R_{17}$ einen Hydroxylrest oder eine Acyloxygruppe ist und $R'_{17}$ ein Wasserstoffatom, einen Alkyl-, Alkenyl- oder Alkinylrest mit höchstens 8 Kohlenstoffatomen, der gegebenenfalls substituiert ist, bedeutet,

X, Y und Z derart sind, daß

- X eine Methylengruppe, eine Arylengruppe, einen Rest $CH_2$-O oder einen Arylenoxyrest, gebundenan das Steroid über ein Kohlenstoffatom, wiedergibt,
- Y eine einfache Bindung oder eine gesättigte oder ungesättigte, lineare oder verzweigte, aliphatische Kette mit 1bis 18 Kohlenstoffatomen, gegebenenfalls unterbrochen durch einen oder mehrere Reste, ausgewählt unter den Arylenresten, Sauerstoff und gegebenenfalls beendet durch einen Arylenrest, bedeutet,
- Z eine einfache Bindung oder einen Rest $CH_2$-O, der an den Rest Y über das Kohlenstoffatom gebunden ist, wiedergibt,

wobei jedoch, wenn Y und Z eine einfache Bindung darstellen, X kein Methylenrest oder $CH_2$-O sein kann, RA und RA', identisch oder verschieden, ein Wasserstoffatom, einen linearen oder verzweigten Alkylrest mit 1 bis 8 Kohlenstoffatomen, gegebenenfalls substituiert durch einen Aryl-, Alkyl- oder Dialkylamino-, Hydroxyrest, Halogen oder veresterten Carboxylrest, darstellen oder RA und RA' mit dem Stickstoffatom, an das sie gebunden sind, einen gesättigten oder ungesättigten Heterocyclus mit 5 oder 6 Kettengliedern bilden, der gegebenenfalls ein oder mehrere weitere Heteroatome, ausgewähltunter den Sauerstoff-, Stickstoff- und Schwefelatomen, enthält und gegebenenfalls durch einen Alkylrest mit 1 bis 4 Kohlenstoffatomen substituiert ist, wobei jedoch zumindest einer der Substituenten RA oder RA' kein Wasserstoffatom ist, dadurch gekennzeichnet, daß man eine Verbindung der Formel (II)

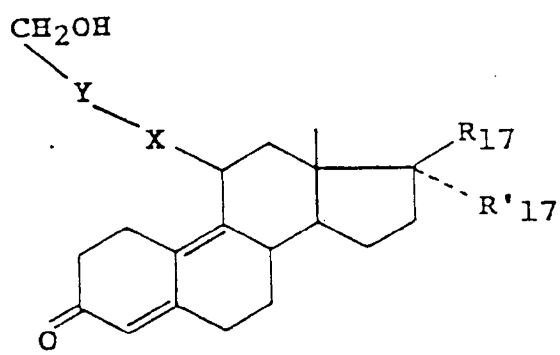

(II)

worin X, Y, $R_{17}$ und $R'_{17}$ die in Anspruch 1 angegebene Bedeutung besitzen, mit der Maßgabe, daß $R_{17}$

keine Hydroxylgruppe bedeuten kann,

- entweder der Einwirkung eines Oxidationsmittels unterzieht, um das Produkt der Formel (III)

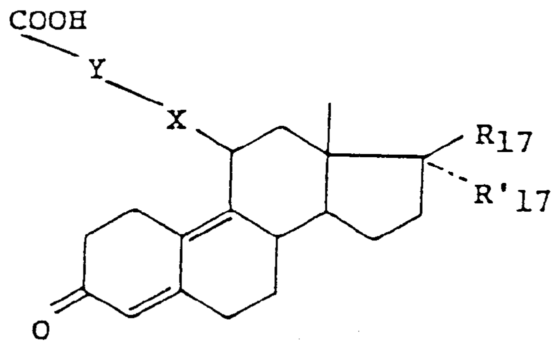

(III)

zu erhalten, worin $R_{17}$, $R'_{17}$, X und Y die in Anspruch 1 angegebene Bedeutung besitzen, welches man der Einwirkung eines Mittels unterzieht, das die Aktivierung der Carboxylfunktion ermöglicht, anschließend der Einwirkung einer Verbindung der Formel (IV)

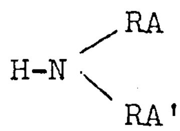

(IV)

worin RA und RA' die in Anspruch 1 angegebene Bedeutung besitzen, um zu dem Produkt der Formel (Ia) zu gelangen, welches der Verbindung der Formel ($I_A$) entspricht, worin Z eine einfache Bindung darstellt und die Ringe A und B die Gruppe

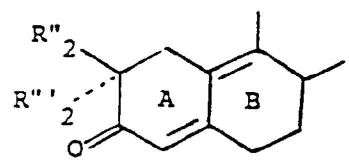

bedeuten, worin $R_2$ und $R'_2$ ein Wasserstoffatom darstellen,

- oder einer Reaktion zur Einführung des Restes

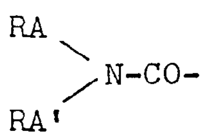

unterzieht, um zu dem Produkt der Formel (I'a) zu gelangen, welches der Verbindung der Formel ($I_A$) entspricht, worin Z ein Methylenoxyrest ist und die Ringe A und B die bei den Produkten der Formel (Ia) angegebene Bedeutung besitzen,

die Produkte (Ia) und (I'a) gewünschtenfalls

- entweder der Einwirkung eines Reduktionsmittels unterzieht, wenn $R_{17}$ und $R'_{17}$ gemeinsam eine Ketofunktion bilden, danach gegebenenfalls der Einwirkung eines Mittels zur Acylierung des so erhaltenen, in 17-Stellung hydroxylierten Derivats,
- oder der Einwirkung eines Verseifungsmittels unterzieht, wenn $R_{17}$ eine Acyloxyfunktion bedeutet, um zu einem Produkt der Formel (Ia) oder (I'a) zu gelangen, worin $R_{17}$ die vorstehend angegebene Bedeutung besitzt, dann gewünschtenfalls irgendeines der Produkte der Formel (Ia) oder (I'a)
- entweder einer Alkylierung in 2-Stellung unterzieht, wenn zumindest einer der Reste $R_2$ und $R'_2$ ein Wasserstoffatom bedeutet,

- oder der Einwirkung eines Mittels zur Aromatisierung des Ringes A unterzieht, danach derjenigen eines Mittels für die milde Verseifung, um zu den Produkten der Formel (Ib), die den Produkten der Formel (Ia) entsprechen, und den Produkten der Formel (I'b), die den Produkten der Formel (I'a) entsprechen, und worin die Ringe A und B für die Gruppe

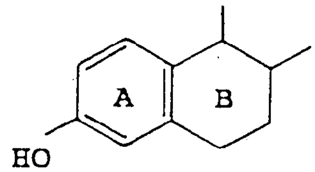

stehen, zu gelangen,
die Produkte der Formel (Ib) und (I'b) gewünschtenfalls

- entweder, wenn $R_{17}$ und $R'_{17}$ gemeinsam eine Ketofunktion bilden, der Einwirkung eines Reduktionsmittels unterzieht oder derjenigen eines Metallkomplexes der Formel (V)

$$M - R'_{17} \qquad (V)$$

worin M ein Metallatom bedeutet und $R'_{17}$ die vorstehend angegebene Bedeutung besitzt, wobei es sich jedoch nicht um ein Wasserstoffatom handelt,
- oder, wenn $R_{17}$ ein Hydroxylrest ist, der Einwirkung eines Mittels für die selektive Acylierung in 17-Stellung unterzieht, danach gewünschtenfalls irgendeines der vorstehend erhaltenen Produkte der Formel (I), oder wenn RA oder RA' ein Wasserstoffatom ist, der Einwirkung eines geeigneten Alkylierungsmittels unterzieht.

**3.** Verfahren gemäß Anspruch 2, dadurch gekennzeichnet, daß man von einem Produkt der Formel (II) ausgeht, worin $R'_{17}$ ein Wasserstoffatom, einen Ethinylrest oder einen Propinylrest bedeutet.

**4.** Verfahren gemäß Anspruch 2, dadurch gekennzeichnet, daß man von einem Produkt der Formel (II) ausgeht, worin X eine Methylengruppe bedeutet und Y eine gesättigte, lineare Kette mit 5 bis 10 Kohlenstoffatomen ist, die gegebenenfalls durch ein Sauerstoffatom unterbrochen ist.

**5.** Verfahren gemäß Anspruch 2, dadurch gekennzeichnet, daß man von einem Produkt der Formel (II) ausgeht, worin X eine Phenylengruppe bedeutet und Y für eine gesättigte oder ungesättigte, lineare Kette mit 3 bis 10 Kohlenstoffatomen steht, die gegebenenfalls durch ein Sauerstoffatom unterbrochen ist.

**6.** Verfahren gemäß Anspruch 1, dadurch gekennzeichnet, daß man von einem Produkt der Formel (II) ausgeht, worin X für einen Phenylenoxyrest steht und Y eine gesättigte, lineare Kette mit 3 bis 10 Kohlenstoffatomen ist, die gegebenenfalls durch ein Sauerstoff- oder Schwefelatom unterbrochen ist.

**7.** Verfahren gemäß Anspruch 2, dadurch gekennzeichnet, daß man von einer Verbindung der Formel (IV) oder aber einem Reagens zur Einführung des Restes

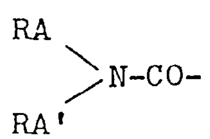

oder gegebenenfalls einem derartigen Reagens ausgeht, daß

- entweder RA und RA' identisch eine Methylgruppe bedeuten
- oder RA ein Wasserstoffatom oder einen Methylrest wiedergibt und RA' für einen Butylrest steht,
- oder RA eine Methylgruppe bedeutet und RA' für einen Isopropyl-, Dimethylaminoethyl-, Benzyl- oder Heptafluorobutylrest steht,
- oder RA und RA' gemeinsam ein gegebenenfalls N-substituiertes Piperazin oder ein Pyrrolidin bilden.

**8.** Verfahren zur Herstellung der Produkte der Formel (I), wie in Anspruch 1 definiert, worin X einen Arylenrest bedeutet und Y für eine aliphatische Kette steht, die gegebenenfalls an die Arylengruppe über eine Doppel- oder eine Dreifachbindung gebunden ist und zumindest 3 Kohlenstoffatome enthält oder an

die Arylengruppe über ein Sauerstoffatom gebunden ist, dadurch gekennzeichnet, daß man eine Verbindung der Formel (X)

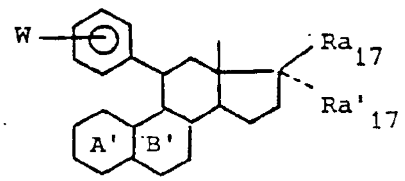

(X)

worin W entweder einen Rest OH oder einen Rest -C=CH bedeutet, die Ringe A' und B', $Ra_{17}$ und $Ra'_{17}$ die vorstehend für die Ringe A und B, $R_{17}$ und $R'_{17}$ angegebenen Bedeutungen besitzen und worin die reaktiven Funktionen in 3- und in 17-Stellung gegebenenfalls geschützt sind, oder aber, wenn W einen Rest -C=CH wiedergibt, der Einwirkung eines Halogenierungsmittels der Formel (XI)

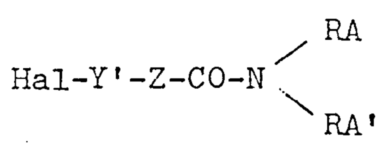

(XI)

worin Hal ein Halogenatom bedeutet, Z, RA und RA' die vorstehend angegebene Bedeutung besitzen und Y' die vorstehende aliphatische Kette Y mit zumindest 2 Kohlenstoffatomen wiedergibt, in Gegenwart einer starken Base unterzieht und gegebenenfalls der Einwirkung eines Mittels zur Schutzgruppenabspaltung unterzieht, um zu dem Produkt der Formel ($I'_A$)

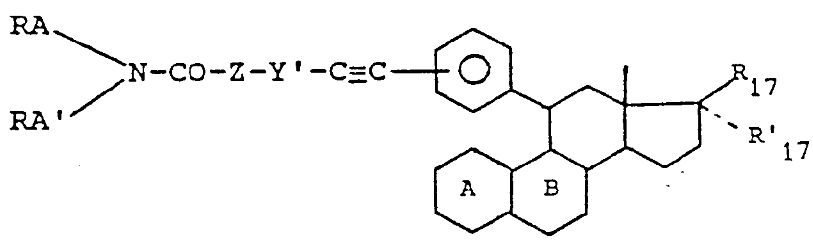

($I'_A$)

zu gelangen, welches man gewünschtenfalls der Einwirkung eines Mittels für die partielle oder vollständige Reduktion der Dreifachbindung unterzieht, um zu dem Produkt der Formel ($I'_B$)

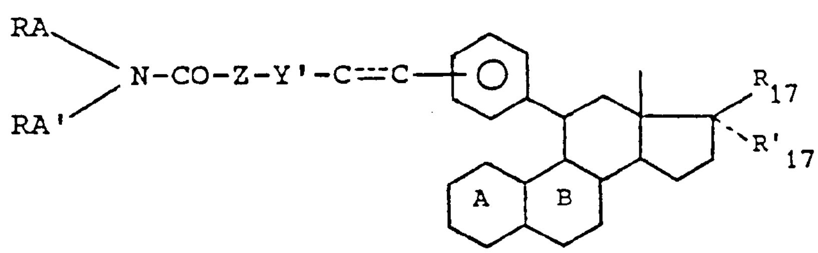

($I'_B$)

zu gelangen, oder wenn W einen Rest -OH bedeutet, der Einwirkung eines halogenierten Derivats der Formel (XII)

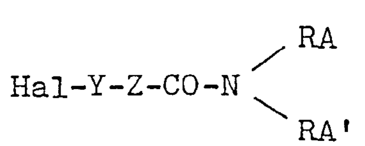

(XII)

worin Hal, Y, Z, RA und RA' die vorstehend angegebene Bedeutung besitzen, in Gegenwart eines alkalischen Mittels unterzieht, anschließend gegebenenfalls der Einwirkung eines Mittels zur Schutzgruppenabspaltung unterzieht, um zu einem Produkt der Formel (I"$_A$)

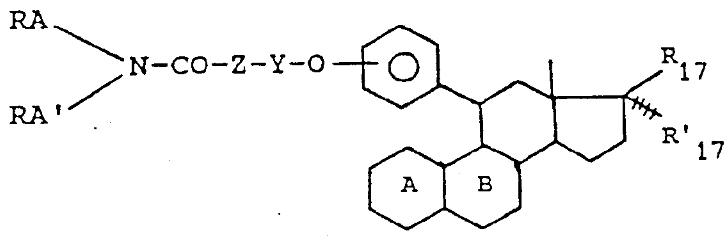

(I"$_A$)

zu gelangen, welches Produkt man gewünschtenfalls, wenn Y eine ungesättigte, aliphatische Kette bedeutet, der Einwirkung eines Mittels für die partielle oder vollständige Reduktion unterzieht und die Produkte der Formel (I'$_A$), (I'$_B$), (I"$_A$) gewünschtenfalls irgendeiner der vorstehend für (Ia), (I'a), (Ib), (I'b) angegebenen Reaktionen unterzieht.

**9.** Verfahren gemäß Anspruch 8, dadurch gekennzeichnet, daß man von einer Verbindung der Formel (X) ausgeht, worin R'$_{17}$ ein Wasserstoffatom, einen Ethinylrest oder einen Propinylrest bedeutet.

**10.** Verfahren gemäß Anspruch 9, dadurch gekennzeichnet, daß man von einer Verbindung der Formel (X), worin W einen Rest -C=CH bedeutet, und einer Verbindung der Formel (XI), worin Y eine gesättigte, lineare Kette mit 5 bis 10 Kohlenstoffatomen bedeutet, die gegebenenfalls durch ein Sauerstoffatom unterbrochen ist, ausgeht.

**11.** Verfahren gemäß Anspruch 9, dadurch gekennzeichnet, daß man von einer Verbindung der Formel (X), worin W einen Rest OH bedeutet, und einer Verbindung der Formel (XII), worin Y eine gesättigte, lineare Kette mit 3 bis 10 Kohlenstoffatomen bedeutet, die gegebenenfalls durch ein Sauerstoff- oder Schwefelatom unterbrochen ist, ausgeht.

**12.** Verfahren gemäß Anspruch 9, dadurch gekennzeichnet, daß man von einer Verbindung der Formel (XI) oder einer Verbindung der Formel (XII) oder gegebenenfalls einem Alkylierungsmittel ausgeht, derart, daß
- entweder RA und RA' identisch eine Methylgruppe bedeuten
- oder RA ein Wasserstoffatom oder einen Methylrest bedeutet und RA' für einen Butylrest steht
- oder RA eine Methylgruppe bedeutet und RA' einen Isopropyl-, Dimethylaminoethyl-, Benzyl- oder Heptafluorobutylrest wiedergibt
- oder RA und RA' gemeinsam ein gegebenenfalls N-substituiertes Piperazin oder ein Pyrrolidin bilden.